# EUROPEAN PATENT APPLICATION

(11) **EP 4 428 227 A1**
(43) Date of publication of application: **11.09.2024**
(21) Application number: 22895708.0
(22) Date of filing: 18.11.2022
(51) Int. Cl.: C12N 5/071, A61K 35/30, A61L 27/36, A61L 27/38, A61L 27/52, A61L 27/58, A61P 27/02, C07K 14/78, C12M 3/00, C12N 5/074

(54) **PRODUCTION METHOD FOR SHEET-LIKE RETINAL TISSUE**

(30) Priority: 19.11.2021 JP 2021188941
(71) Applicant: RIKEN, Wako-shi, Saitama 351-0198 (JP); Sumitomo Pharma Co., Ltd., Osaka 541-0045 (JP)
(72) Inventor: MANDAI, Michiko, Wako-shi, Saitama 351-0198 (JP); TAKAHASHI, Masayo, Wako-shi, Saitama 351-0198 (JP); YAMASAKI, Suguru, Kobe-shi, Hyogo 650-0047 (JP); HORIUCHI, Matsuri, Kobe-shi, Hyogo 650-0047 (JP)
(74) Representative: Hoffmann Eitle
(86) International application number: PCT/JP2022/042876
(87) International publication number: WO 2023/090427

(57) **Abstract**

A method for manufacturing a cell aggregate comprising retinal tissue having a layer structure, comprising suspension-culturing or adhesion-culturing a dispersed retinal cell population in a culture medium containing a Wnt signaling pathway agonist, wherein the retinal cell population comprises one or more cells selected from the group consisting of a retinal progenitor cell and a photoreceptor progenitor cell.

## Description

### Technical Field

The present invention relates to a method for manufacturing a sheet-shaped retinal tissue and a sheet-shaped retinal tissue.

### Background Art

In recent years, it has been reported that photoreceptor progenitor cells, when transplanted at an appropriate stage of differentiation into a normal retina in a mouse living body, are functionally engrafted (Non Patent Literature 1), and the possibility of transplantation therapies for degenerative diseases of photoreceptor cells such as retinitis pigmentosa has been shown.

Many methods for differentiating pluripotent stem cells into three-dimensional retinal tissues by self-organization culture have been reported, and it is becoming possible to manufacture and transplant three-dimensional retinal tissues having a layer structure. For example, there have been reported: a method for obtaining a multilayered retinal tissue from pluripotent stem cells (Non-Patent Literature 2 and Patent Literature 1); a method for obtaining a multilayered retinal tissue by forming an aggregate of homogeneous pluripotent stem cells in a serum-free medium containing a Wnt signaling pathway inhibitor, and suspension-culturing the obtained aggregate in the presence of a basal membrane preparation and then in a serum medium (Non Patent Literature 3 and Patent Literature 2); and a method for obtaining retinal tissue by suspension-culturing an aggregate of pluripotent stem cells in a culture medium containing a BMP signaling pathway agonist (Non-Patent Literature 4 and Patent Literature 3). However, these retinal tissues have been manufactured as sphere-like cell aggregates, and any method for manufacturing a sheet-shaped (flattened) retinal tissue has not yet been known.

Meanwhile, it has been reported that Wnt2b exhibits an effect on the epithelial structure formation of a retina in a chicken (Non Patent Literature 5), though any similar effect has not been reported for organisms other than chickens.

### Citation List

### Patent Literature

Patent Literature 1: WO2011/055855
Patent Literature 2: WO2013/077425
Patent Literature 3: WO2015/025967

### Non Patent Literature

Non Patent Literature 1: Maclaren RE et al., "Retinal Repair by Transplantation of Photoreceptor Precursors", Nature, 444, 203-207, (2006)
Non Patent Literature 2: Eiraku M. et al., "Self-organizing optic-cup morphogenesis in three-dimensional culture", Nature, 472, 51-56, (2011)
Non Patent Literature 3: Nakano T. et al., "Self-formation of Optic Cups and Storable Stratified Neural Retina From Human ESCs" Cell Stem Cell, 10(6), 771-785, (2012)
Non Patent Literature 4: Kuwahara A. et al., "Generation of a ciliarymargin-like stem cell niche from self-organizing human retinal tissue", Nature Communications, 6, 6286, (2015)
Non Patent Literature 5: Nakagawa, S. et. al., "Identification of the laminar-inducing factor Wnt-signal from the anterior rim induces correct laminar formation of the neural retina in vitro. " Developmental Biology, 260, 414-425, (2003)

### Summary of Invention

### Technical Problem

Accordingly, in light of the situation described above, an object of the present invention is to provide a method for reforming the neuroepithelial structure of retinal tissue from a retinal cell and a method for manufacturing a sheet-shaped retinal tissue by applying the method, and a sheet-shaped retinal tissue. Another object is to secondarily provide a method for purifying a retinal progenitor cell more preferable as a starting cell, and a method for detaching a wide sheet-shaped retinal tissue manufactured as described above.

### Solution to Problem

The present inventors have conceived the idea that a sheet-shaped retinal tissue is manufactured by reforming a neuroepithelial structure with dispersed retinal cells as starting cells. However, the problem has been found that retinal cells are dispersed and thereby disrupt an apical-basal polarity of the cells, so that the neuroepithelial structure cannot be reformed.

The present inventors have conducted diligent studies on this problem and consequently found that the problem is solved by adding a Wnt signaling pathway agonist to dispersed retinal cells. The present inventors have also conducted diligent studies in order to reform a more favorable neuroepithelial structure. As a result, the present inventors have further found that impure cells such as retinal pigment epithelial cells (RPE) are removed by (1) further adding a ROCK inhibitor, a SHH signaling pathway agonist and/or a fibroblast growth factor, (2) performing culture on a culture plate coated with an extracellular matrix serving as a scaffold of cell adhesion, particularly, in order to reform a favorable sheet-shaped neuroepithelial structure, and (3) improving the purity of retinal progenitor cells as starting cells, reaching the completion of the present invention.

Specifically, the present invention relates to each of the following aspects.
[1]A method for manufacturing retinal tissue having an epithelial structure, comprising
   suspension-culturing or adhesion-culturing a dispersed retinal cell population in a culture medium comprising a Wnt signaling pathway agonist, wherein
   the retinal cell population comprises one or more cells selected from the group consisting of a retinal progenitor cell and a photoreceptor progenitor cell.
[2] The manufacturing method according to [1], wherein the Wnt signaling pathway agonist is one or more substances selected from the group consisting of CHIR99021, BIO, Wnt2b and Wnt3a.
[3] The manufacturing method according to [1] or [2], wherein the culture medium further comprises one or more substances selected from the group consisting of a ROCK inhibitor, a SHH signaling pathway agonist and an FGF signaling pathway agonist.
[4] The manufacturing method according to [3], wherein the ROCK inhibitor is one or more substances selected from the group consisting of Y-27632, fasudil (HA1077) and H-1152.
[5] The manufacturing method according to [3] or [4], wherein the SHH signaling pathway agonist is one or more substances selected from the group consisting of SAG, PMA and SHH.
[6] The manufacturing method according to any of [3] to [5], wherein the FGF signaling pathway agonist is one or more fibroblast growth factors selected from the group consisting of FGF2, FGF4 and FGF8.
[7] The manufacturing method according to any of [1] to [6], comprising performing the adhesion culture in the culture medium comprising the Wnt signaling pathway agonist, wherein the retinal tissue having an epithelial structure is a sheet-shaped retinal tissue.
[8] The manufacturing method according to [7], wherein the adhesion culture is performed using a culture container coated with an extracellular matrix and/or a temperature-responsive polymer.
[9] The manufacturing method according to [8], wherein a culture surface of the culture container is coated with the temperature-responsive polymer, and an upper surface of the temperature-responsive polymer is coated with the extracellular matrix.
[10] The manufacturing method according to [8] or [9], wherein the extracellular matrix is one or more substances selected from the group consisting of collagen, laminin, fibronectin, Matrigel and vitronectin.
[11] The manufacturing method according to any of [8] to [10], further comprising the step of exposing the culture container coated with the temperature-responsive polymer to a temperature that changes the properties of the temperature-responsive polymer to thereby detach the sheet-shaped retinal tissue from the culture container.
[12] The manufacturing method according to any of [1] to [11], comprising the step of dispersing a cell aggregate comprising one or more cells selected from the group consisting of a retinal progenitor cell and a photoreceptor progenitor cell to obtain the dispersed retinal cell population.
[13] The manufacturing method according to [12], comprising the step of differentiating pluripotent stem cells to obtain the cell aggregate comprising one or more cells selected from the group consisting of the retinal progenitor cell and the photoreceptor progenitor cell.
[14] The manufacturing method according to any of [1] to [13], further comprising the step of increasing the percentage of a retinal progenitor cell comprised in the dispersed retinal cell population before suspension-culturing or adhesion-culturing the dispersed retinal cell population.
[15] The method according to [14], wherein contamination with or differentiation into retinal pigment epithelial cells is suppressed.
[16] The manufacturing method according to [14] or [15], wherein the step of increasing the percentage of a retinal progenitor cell comprises the step of contacting the dispersed retinal cell population with a substance that binds to one or more antigens selected from the group consisting of CD9, CD39, CD90 and CXCR4 to obtain a cell population expressing the antigens.
[17] The manufacturing method according to [16], wherein the step of increasing the percentage of a retinal progenitor cell comprises the step of further contacting the dispersed retinal cell population with a substance that binds to one or more antigens selected from the group consisting of SSEA1, CD66b, CD69 and CD84 to obtain a cell population having expression levels of the antigens that are equal to or less than a reference.
[18] The manufacturing method according to any of [14] to [17], wherein the step of increasing the percentage of a retinal progenitor cell comprises the following steps:
   (1) culturing pluripotent stem cells in the presence of one or more selected from the group consisting of a Shh signaling pathway agonist, ATP and an A2A receptor agonist to manufacture a cell aggregate;
   (2) differentiating the cell aggregate into a retinal progenitor cell; and
   (3) dispersing the cell aggregate and contacting it with a substance that binds to CD39.
[19] The manufacturing method according to any of [1] to [18], wherein the retinal progenitor cell and/or the photoreceptor progenitor cell occupies 50% or more of the total number of cells comprised in the retinal cell population.
[20] The manufacturing method according to any of [1] to [18], wherein the retinal progenitor cell and/or the photoreceptor progenitor cell occupies 80% or more of the total number of cells comprised in the retinal cell population.
[21] The manufacturing method according to any of [1] to [20], wherein from the start of the suspension culture or the adhesion culture, the dispersed retinal cell population is cultured in the culture medium comprising the Wnt signaling pathway agonist.
[22] The manufacturing method according to any of [1] to [21], wherein in the epithelial structure, the orientation of cells is a direction roughly perpendicular to the layer direction.
[23] The manufacturing method according to any of [1] to [22], further comprising the step of dissecting a size necessary for transplantation from the retinal tissue having an epithelial structure obtained by the suspension culture or the adhesion culture.
[24] The manufacturing method according to any of [1] to [23], wherein the epithelial structure is a multilayered structure.
[25] A sheet-shaped retinal tissue consisting of a retinal cell layer having a multilayered structure, wherein
   (1) the retinal cell layer having a multilayered structure has polarities of a basal surface and an apical surface,
   (2) the retinal cell layer having a multilayered structure comprises one or more cells selected from the group consisting of a retinal progenitor cell, a photoreceptor progenitor cell and a photoreceptor cell,
   (3) in each layer of the retinal cell layer, the orientation of cells is a direction roughly perpendicular to the layer direction, and
   (4) a diameter is 8 mm or more.
[26] The sheet-shaped retinal tissue according to [25], wherein the retinal cell layer having a multilayered structure further comprises a sheet-shaped retinal pigment epithelial cell joined to the retinal cell layer, wherein the tangent directions of the respective surfaces of the retinal cell layer and the sheet-shaped retinal pigment epithelial cell are roughly in parallel, the apical surface of the retinal cell layer and the apical surface of the sheet-shaped retinal pigment epithelial cell face each other, and the retinal cell layer and the sheet-shaped retinal pigment epithelial cell are joined through an adhesion factor present therebetween.
[27] The sheet-shaped retinal tissue according to [26], wherein the adhesion factor is an extracellular matrix or a hydrogel.
[28] The sheet-shaped retinal tissue according to [27], wherein the adhesion factor is one or more substances selected from gelatin, fibrin, fibronectin, hyaluronic acid, laminin, IV-type collagen, heparan sulfate proteoglycan and entactin.
[29] The sheet-shaped retinal tissue according to [27], wherein the adhesion factor is gelatin or fibrin.
[30] A pharmaceutical composition comprising the sheet-shaped retinal tissue according to any of [25] to [29].
[31] A method for treating a disease caused by the damage of a retinal cell or retinal tissue or the injury of retinal tissue, comprising transplanting the sheet-shaped retinal tissue according to any of [25] to [29] to a subject in need of transplantation.
[32] A method for increasing the percentage of a retinal progenitor cell in a cell population, comprising the step of contacting the cell population comprising the retinal progenitor cell with a substance that binds to one or more antigens selected from the group consisting of CD9, CD24, CD29, CD39, CD47, CD49b, CD49c, CD49f, CD57, CD73, CD82, CD90, CD164, CD200, CD340 and CXCR4.
[33] The method according to [32], comprising the step of contacting the cell population comprising the retinal progenitor cell with the substance that binds to one or more antigens selected from the group consisting of CD9, CD39, CD90 and CXCR4.
[34] The method according to [32] or [33], comprising the step of contacting the cell population comprising the retinal progenitor cell with a substance that binds to one or more antigens selected from the group consisting of SSEA1, CD66b, CD69 and CD84.
[35] A cell population comprising 90% or more of a retinal progenitor cell that is positive to at least one factor selected from the group consisting of CD9, CD39, CD90 and CXCR4 and is Rx-positive with respect to the total number of cells in the cell population.
[36] The cell population according to [35], wherein the retinal progenitor cell is negative to one or more antigens selected from the group consisting of SSEA1, CD66b, CD69 and CD84.
[37] The sheet-shaped retinal tissue according to any of [25] to [29] for use in the treatment of a disease caused by the damage of a retinal cell or retinal tissue or the injury of retinal tissue.
[38] Use of the sheet-shaped retinal tissue according to any of [25] to [29] in the manufacturing of a therapeutic product for a disease caused by the damage of a retinal cell or retinal tissue or the injury of retinal tissue.

### Advantageous Effects of Invention

According to the present invention, it becomes possible to provide a method for reforming the layer structure of retinal tissue from a retinal cell and a method for manufacturing a sheet-shaped retinal tissue by applying the method, and a sheet-shaped retinal tissue.

### Brief Description of Drawings

[Figure 1] Figure 1 is bright field microscope images showing the reformed states of aggregates on Day 1 after KhES-1-derived aggregates were dispersed into single cells and seeded in Example 1.
[Figure 2] Figure 2 is bright field microscope images showing the reformed states of aggregates on Day 1 after 1231A3-derived aggregates were dispersed into single cells and seeded in Example 1.
[Figure 3] Figure 3 is bright field microscope images showing the morphology of aggregates reformed on Day 1 after KhES-1-derived aggregates were dispersed into single cells and seeded in Example 2.
[Figure 4] Figure 4 is fluorescence microscope images showing the morphology of aggregates reformed on Day 7 after KhES-1-derived aggregates were dispersed into single cells and seeded in Example 2.
[Figure 5] Figure 5 is fluorescence microscope images showing the morphology of aggregates reformed on Day 14 after KhES-1-derived aggregates were dispersed into single cells and seeded in Example 2.
[Figure 6] Figure 6 is graphs showing results of measuring, in Image J, the areas of aggregates in which KhES-1-derived single cells were reformed in Example 2. Figure 6(A) shows the areas of the aggregates on Day 1, Day 7 and Day 14 after seeding, and Figure 6(B) shows the ratios of the areas (area ratios) of the aggregates on Day 7 and Day 14 to the areas of the aggregates on Day 1 after seeding.
[Figure 7] Figure 7 is bright field microscope images showing the morphology of aggregates formed on Day 1 after 1231A3-derived aggregates were dispersed into single cells and seeded in Example 2.
[Figure 8] Figure 8 is bright field microscope images showing the morphology of aggregates formed on Day 1 after KhES-1-derived aggregates were dispersed into single cells and seeded in Example 3.
[Figure 9] Figure 9 is fluorescence microscope images showing the morphology of aggregates formed on Day 14 after KhES-1-derived aggregates were dispersed into single cells and seeded in Example 3.
[Figure 10] Figure 10 is bright field microscope and fluorescence microscope images showing the morphology of aggregates formed on Day 28 after KhES-1-derived aggregates were dispersed into single cells and seeded in Example 3.
[Figure 11] Figure 11 is images showing results of observing, under a confocal laser scanning fluorescence microscope, immunostained (DAPI, Rx::Venus, Chx10) sections of aggregates on Day 15 after KhES-1-derived aggregates were dispersed into single cells and seeded in Example 3.
[Figure 12] Figure 12 is images showing results of observing, under a confocal laser scanning fluorescence microscope, immunostained (β-catenin) sections of aggregates on Day 15 after KhES-1-derived aggregates were dispersed into single cells and seeded in Example 3.
[Figure 13] Figure 13 is images showing results of observing, under a confocal laser scanning fluorescence microscope, immunostained (collagen IV, Zo-1) sections of aggregates on Day 15 after KhES-1-derived aggregates were dispersed into single cells and seeded in Example 3.
[Figure 14] Figure 14 is images showing results of observing, under a confocal laser scanning fluorescence microscope, immunostained (DAPI, Chx10, Ki67, Pax6) sections of aggregates on Day 28 after KhES-1-derived aggregates were dispersed into single cells and seeded in Example 3.
[Figure 15] Figure 15 is images showing results of observing, under a confocal laser scanning fluorescence microscope, the fluorescence of Rx:: Venus from sections of aggregates on Day 28 after KhES-1-derived aggregates were dispersed into single cells and seeded in Example 3.
[Figure 16] Figure 16 is images showing results of observing, under a confocal laser scanning fluorescence microscope, immunostained (collagen IV, Zo-1) sections of aggregates on Day 28 after KhES-1-derived aggregates were dispersed into single cells and seeded in Example 3.
[Figure 17] Figure 17 is images showing results of observing, under a confocal laser scanning fluorescence microscope, immunostained (DAPI, Rx:: Venus, collagen IV, Zo-1) sections of aggregates on Day 28 after KhES-1-derived aggregates were dispersed into single cells and seeded in Example 3.
[Figure 18] Figure 18 is images showing results of observing, under a confocal laser scanning fluorescence microscope, immunostained (DAPI, Chx10, Ki67, Pax6) sections of aggregates on Day 28 after KhES-1-derived aggregates were dispersed into single cells and seeded in Example 3.
[Figure 19] Figure 19 is images showing results of observing, under a confocal laser scanning fluorescence microscope, immunostained (CRX, RxRg, NRL, recoverin) sections of aggregates on Day 28 after KhES-1-derived aggregates were dispersed into single cells and seeded in Example 3.
[Figure 20] Figure 20 is images showing results of observing, under a confocal laser scanning fluorescence microscope, immunostained (DAPI, Islet-1, Brn3, calretinin) sections of aggregates on Day 28 after KhES-1-derived aggregates were dispersed into single cells and seeded in Example 3.
[Figure 21] Figure 21 is bright field microscope and fluorescence microscope images showing the states of aggregates reformed on Day 3, Day 15 and Day 21 after KhES-1-derived aggregates, the number of differentiation days of which is Day 18, Day 25, Day 40, Day 61, and Day 75, were dispersed into single cells and seeded in Example 4.
[Figure 22] Figure 22 is bright field microscope and fluorescence microscope (Rx:: Venus) images showing the states of aggregates reformed on Day 3, Day 15 and Day 21 after KhES-1-derived aggregates (BMP4+/BMP4-), the number of differentiation days of which is Day 40, were dispersed into single cells and seeded in Example 5.
[Figure 23] Figure 23 is fluorescence microscope images in which the morphology of aggregates formed 1 day and 7 days after dispersed retinal cells cryopreserved in various cryopreservation solutions were seeded was observed in Example 6.
[Figure 24] Figure 24 is graphs showing viability after dispersed retinal cells cryopreserved in various cryopreservation solutions were reconstituted (A), and the areas of aggregates reformed using the reconstituted retinal cells (B) in Example 6.
[Figure 25] Figure 25 is images showing results of observing, under a confocal laser scanning fluorescence microscope, immunostained sections of basal membranes expressed in retinal tissue formed by suspension culture in Example 7.
[Figure 26] Figure 26 is histologically immunostained images showing results of examining isoforms of laminin expressed in mouse fetal neural retinal tissues in Example 7.
[Figure 27] Figure 27 is confocal laser scanning fluorescence microscope images in which the reforming of sheet-shaped retinal tissues by adhesion culture from single cell suspensions of retinal cells under Conditions 1 to 3 was confirmed in Example 8.
[Figure 28] Figure 28 is confocal laser scanning fluorescence microscope images in which the reforming of sheet-shaped retinal tissues by adhesion culture from single cell suspensions of retinal cells under Conditions 1 to 3 was confirmed in Example 8.
[Figure 29] Figure 29 is bright field microscope images and fluorescence microscope images showing results of confirming the effects of various factors on the reforming of sheet-shaped retinal tissues by adhesion culture in Example 9.
[Figure 30] Figure 30 is confocal laser scanning fluorescence microscope images showing results of confirming the effects of various factors on the reforming of sheet-shaped retinal tissues by adhesion culture in Example 9.
[Figure 31] Figure 31 is bright field microscope images, fluorescence microscope images and confocal laser scanning fluorescence microscope images showing the concentrations and addition periods of CHIR99021 in the reforming of sheet-shaped retinal tissues by adhesion culture in Example 10.
[Figure 32] Figure 32 is confocal laser scanning fluorescence microscope images showing results of studying the concentrations and addition periods of CHIR99021 in the reforming of sheet-shaped retinal tissues by adhesion culture in Example 10.
[Figure 33] Figure 33 is bright field microscope images, bright field and fluorescence microscope images, and fluorescence microscope images showing results of studying scaffolds at the time of seeding onto Transwell in the reforming of sheet-shaped retinal tissues by adhesion culture in Example 11.
[Figure 34] Figure 34 is bright field microscope images and fluorescence microscope images showing results of confirming the effect of CHIR99021 on the reforming of sheet-shaped retinal tissues by adhesion culture in Example 12.
[Figure 35] Figure 35 is confocal laser scanning fluorescence microscope images showing results of confirming the effect of CHIR99021 on the reforming of sheet-shaped retinal tissues by adhesion culture in Example 12.
[Figure 36] Figure 36 is confocal laser scanning fluorescence microscope images showing results of confirming the effect of CHIR99021 on the reforming of sheet-shaped retinal tissues by adhesion culture in Example 12.
[Figure 37] Figure 37 is confocal laser scanning fluorescence microscope images showing results of confirming the effect of CHIR99021 on the reforming of sheet-shaped retinal tissues by adhesion culture in Example 12.
[Figure 38] Figure 38 is confocal laser scanning fluorescence microscope images showing results of confirming the effect of CHIR99021 on the reforming of sheet-shaped retinal tissues by adhesion culture in Example 12.
[Figure 39] Figure 39 is confocal laser scanning fluorescence microscope images showing results of confirming the effect of CHIR99021 on the reforming of sheet-shaped retinal tissues by adhesion culture in Example 12.
[Figure 40] Figure 40 is confocal laser scanning fluorescence microscope images showing results of confirming the effect of CHIR99021 on the reforming of sheet-shaped retinal tissues by adhesion culture in Example 12.
[Figure 41] Figure 41 is confocal laser scanning fluorescence microscope images showing results of confirming the effect of CHIR99021 on the reforming of sheet-shaped retinal tissues by adhesion culture in Example 12.
[Figure 42] Figure 42 is confocal laser scanning fluorescence microscope images showing results of confirming the effect of CHIR99021 on the reforming of sheet-shaped retinal tissues by adhesion culture in Example 12.
[Figure 43] Figure 43 is confocal laser scanning fluorescence microscope images showing results of confirming the effect of CHIR99021 on the reforming of sheet-shaped retinal tissues by adhesion culture in Example 12.
[Figure 44] Figure 44 is fluorescence microscope images showing results of a study on the maintenance effects of various factors on retina cells in Example 13.
[Figure 45] Figure 45 is fluorescence microscope images showing results of a study on conditions effective for the maintenance culture of retinal progenitor cells in Example 14.
[Figure 46] Figure 46 is stereo microscope images showing results of being prepared into a sheet again on collagen in Example 15.
[Figure 47] Figure 47 is fluorescence microscope images showing grafts for transplantations prepared from retina cell sheets prepared again in Example 16.
[Figure 48] Figure 48 is fluorescence stereo microscope and fluorescence microscope images showing results of observing the eye fundus of a retina after transplantation in Example 16.
[Figure 49] Figure 49 is graphs showing results of FACS analysis for sorting Rx:: Venus-positive fractions from a cell population dispersed into single cells in Example 17. Figure 49(A) is a dot plot, and Figure 49(B) is a histogram plot.
[Figure 50] Figure 50 is stereo microscope and fluorescence stereo microscope images in which a sorted or unsorted cell population was observed in Example 17.
[Figure 51] Figure 51 is confocal laser scanning fluorescence microscope images in which a sorted or unsorted cell population was observed in Example 17.
[Figure 52] Figure 52 is confocal laser scanning fluorescence microscope images in which a sorted or unsorted cell population was observed in Example 17.
[Figure 53] Figure 53 is bright field microscope and fluorescence microscope images in which a sorted cell population was observed in Example 17.
[Figure 54] Figure 54 is fluorescence microscope images in which a Rx:: Venus-positive cell population in the case of adding various proteins was observed in Example 18.
[Figure 55] Figure 55 is fluorescence microscope images in which a Rx:: Venus-positive cell population in the case of adding various lowmolecular compounds was observed in Example 18.
[Figure 56] Figure 56 is fluorescence microscope images in which a Rx:: Venus-positive cell population in the case of adding different concentrations of FGF2, FGF4 and FGF8 was observed in Example 18.
[Figure 57] Figure 57 is bright field microscope and fluorescence microscope images showing aggregate reforming and a retinal differentiation effect by sorting and the addition of FGF8 in Example 19.
[Figure 58] Figure 58 is bright field microscope and fluorescence microscope images and FACS analysis results showing aggregate reforming and a retinal differentiation effect by sorting and the addition of FGF8 in Example 19.
[Figure 59] Figure 59 is bright field microscope and fluorescence microscope images and FACS analysis results showing aggregate reforming and a retinal differentiation effect by the addition of FGF8 in Example 19.
[Figure 60] Figure 60 is bright field microscope and fluorescence microscope images showing influence at the time of preparation into a sheet again by the addition of FGF8 in Example 20.
[Figure 61] Figure 61 is bright field microscope and fluorescence microscope images showing influence at the time of preparation into a sheet again by the addition of FGF8 in Example 20.
[Figure 62] Figure 62 is bright field microscope and fluorescence microscope images showing preparation into a sheet again and a retinal differentiation effect by the addition of FGF8, and graphs showing FACS analysis results in Example 20.
[Figure 63] Figure 63 is stereo microscope and fluorescence stereo microscope images showing time-dependent change in retina sheet prepared again in Example 21.
[Figure 64] Figure 64 is a diagram showing markers focused on in surface antigen screening in Example 22.
[Figure 65] Figure 65 is graphs showing results of cell sorting using various surface antigens in Example 22.
[Figure 66] Figure 66 is graphs showing results of cell sorting using various surface antigens in Example 22.
[Figure 67] Figure 67 is graphs showing results of cell sorting using various surface antigens in Example 22.
[Figure 68] Figure 68 is graphs showing results of cell sorting using various surface antigens in Example 22.
[Figure 69] Figure 69 is graphs showing results of cell sorting using various surface antigens in Example 22.
[Figure 70] Figure 70 is bright field microscope and fluorescence microscope images in which a state differentiated into brain organoid was observed in Example 23.
[Figure 71] Figure 71 is confocal laser scanning fluorescence microscope images in which a state differentiated into brain organoid was observed in Example 23.
[Figure 72] Figure 72 is graphs showing FACS analysis results about the expression of CD39, CD73 and CXCR4 in brain organoid in Example 23.
[Figure 73] Figure 73 is bright field microscope and fluorescence microscope images showing results of differentiation by different concentrations of SAG in Example 24.
[Figure 74] Figure 74 is FACS analysis results and a digitized graph about the expression of CD39 and CXCR4 after differentiation by different concentrations of SAG in Example 24.
[Figure 75] Figure 75 is confocal laser scanning fluorescence microscope images in which immunostained images of tissues differentiated by different concentrations of SAG were observed in Example 24.
[Figure 76] Figure 76 is confocal laser scanning fluorescence microscope images in which immunostained images of tissues differentiated by different concentrations of SAG were observed in Example 24.
[Figure 77] Figure 77 is FACS analysis results about the expression of CD39 and BV421 by different concentrations of SAG, and confocal laser scanning fluorescence microscope images in which immunostained images of tissues differentiated by different concentrations of SAG were observed in Example 24.
[Figure 78] Figure 78 is confocal laser scanning fluorescence microscope images in which immunostained images of tissues differentiated by different concentrations of SAG were observed in Example 24.
[Figure 79] Figure 79 is confocal laser scanning fluorescence microscope images in which immunostained images of tissues differentiated by different concentrations of SAG were observed in Example 24.
[Figure 80] Figure 80 is graphs showing FACS analysis results showing results of a study on substances capable of enhancing the expression of CD39 in Example 25.
[Figure 81] Figure 81 is digitized graphs of FACS analysis results showing results of a study on substances capable of enhancing the expression of CD39 in Example 25. Figure 81(A) is CD39-positive and RX::venus-positive results, and Figure 81(B) is CXCR4-negative and RX::venus-positive results.
[Figure 82] Figure 82 is bright field microscope and fluorescence microscope images showing the step of observing an Islet-1 KO hESC-retina sheet (dd74) prepared with FGF8- and FGF8+, and dissecting a graft for transplantation in Example 26.
[Figure 83] Figure 83 is FACS dot plots showing results of screening for various surface antigens in NR compared with brain organoid in Example 27.
[Figure 84] Figure 84 is FACS dot plots showing results of screening for various surface antigens in NR in Example 27.
[Figure 85] Figure 85 is FACS dot plots showing results of time-dependent change in expression of CD9 in hESC-retina in Example 28.
[Figure 86] Figure 86 is FACS dot plots showing results of analyzing the expression of CD9 and SSEA-1 in hESC-retina in Example 29.
[Figure 87] Figure 87 is FACS dot plots showing results of a purification study using CD9, CD90, CXCR4 and SSEA-1 in hESC-retina in Example 30.
[Figure 88] Figure 88 is FACS dot plots showing results of a purification study using CD9, CD90, CXCR4 and SSEA-1 in hESC-retina in Example 30.
[Figure 89] Figure 89 is a graph showing results of a purification study using CD9, CD90, CXCR4 and SSEA-1 in hESC-retina in Example 30.
[Figure 90] Figure 90 is images showing results of a purification study using CD9, CD90, CXCR4 and SSEA-1 in hESC-retina in Example 30.
[Figure 91] Figure 91 is images showing results of a purification study using CD9, CD90, CXCR4 and SSEA-1 in hESC-retina in Example 30.
[Figure 92] Figure 92 is FACS diagrams showing results of time-dependent change in expression of CD9 and SSEA-1 in hESC-retina in Example 31.
[Figure 93] Figure 93 is a schematic view showing the process of conducting a complex formation study of a RPE sheet and a retina sheet using gelatin in Example 32.
[Figure 94] Figure 94 is stereo microscope and fluorescence microscope images in which a RPE sheet and a neural retina prepared into a sheet again which were cultured on Transwell and used in complex formation using gelatin were observed in Example 32.
[Figure 95] Figure 95 is stereo microscope images showing the process of detaching a RPE sheet and a neural retina prepared into a sheet again from Transwell, and adding gelatin in Example 32.
[Figure 96] Figure 96 is stereo microscope images showing the process of adding gelatin to a RPE sheet and a neural retina prepared into a sheet again in Example 32.
[Figure 97] Figure 97 is stereo microscope and fluorescence microscope images showing the process of subjecting a RPE sheet and a neural retina prepared into a sheet again to complex formation in Example 32.
[Figure 98] Figure 98 is stereo microscope and fluorescence microscope images showing that a RPE sheet and a neural retina prepared into a sheet again were subjected to complex formation and then lifted using tweezers in Example 32.
[Figure 99] Figure 99 is stereo microscope and fluorescence microscope images showing that a RPE sheet and a neural retina prepared into a sheet again were subjected to complex formation and then dissected using scissors in Example 32.
[Figure 100] Figure 100 is stereo microscope and fluorescence microscope images in which a cross section of a dissected complex of a RPE sheet and a neural retina prepared into a sheet again was observed in Example 32.
[Figure 101] Figure 101 is stereo microscope and fluorescence microscope images in which the aspiration and discharge of a dissected complex of a RPE sheet and a neural retina prepared into a sheet again were observed in Example 32.
[Figure 102] Figure 102 is stereo microscope and fluorescence microscope images in which a RPE sheet and a neural retina prepared into a sheet again which were cultured on Transwell and used in complex formation using fibrin were observed in Example 33.
[Figure 103] Figure 103 is stereo microscope and fluorescence microscope images showing the process of a complex formation study in which a RPE sheet and a neural retina prepared into a sheet again were detached from Transwell in Example 33.
[Figure 104] Figure 104 is stereo microscope and fluorescence microscope images showing the process of a complex formation study in which Fibrinogen and Thrombin were added to a recovered RPE sheet and prepared into a sheet again in Example 33.
[Figure 105] Figure 105 is stereo microscope and fluorescence microscope images showing the process of subjecting a RPE sheet and a neural retina prepared into a sheet again to complex formation in Example 33.
[Figure 106] Figure 106 is stereo microscope and fluorescence microscope images showing the process of subjecting a RPE sheet and a neural retina prepared into a sheet again to complex formation in Example 33.
[Figure 107] Figure 107 is stereo microscope and fluorescence microscope images showing that a RPE sheet and a neural retina prepared into a sheet again were subjected to complex formation and then lifted using tweezers in Example 33.
[Figure 108] Figure 108 is stereo microscope and fluorescence microscope images showing a state in which a RPE sheet and a neural retina prepared into a sheet again were subjected to complex formation in Example 33.
[Figure 109] Figure 109 is stereo microscope and fluorescence microscope images showing the process of a complex formation study in which a RPE sheet was detached from the mesh of Transwell in Example 33.
[Figure 110] Figure 110 is a schematic view and images showing results of an ejection study of unnecessary hydrogel using CellShifter in Example 34.
[Figure 111] Figure 111 is images showing the process of preparing a planarized sheet on a temperature-responsive culture dish, and detaching it in Example 35.

### Description of Embodiments

### [Definition]

The "stem cells" refer to undifferentiated cells having differentiation potency and proliferation potency (particularly, self-renewal ability). In the stem cells, subgroups of pluripotent stem cells, multipotent stem cells and unipotent stem cells, are included according to the differentiation potency. The pluripotent stem cells refer to stem cells that can be cultured *in vitro* and has an ability (pluripotency) to be able to differentiate into three germ layers (ectoderm, mesoderm, endoderm) and/or all cell lineages belonging to the extraembryonic tissue. The multipotent stem cells refer to stem cells having an ability to differentiate into a plurality of tissues or cells, although the definition is not applied to all of them. The unipotent stem cells refer to stem cells having an ability to be able to differentiate into a predetermined tissue or cells.

The "pluripotent stem cells" can be induced from, e.g., a fertilized egg, a cloned embryo, germline stem cells, tissue stem cells and somatic cells. Examples of the pluripotent stem cells can include embryonic stem cells (ES cells), embryonic germ cells (EG cells) and induced pluripotent stem cells (iPS cells). Muse cells (Multi-lineage differentiating stress enduring cells) obtained from the mesenchymal stem cells (MSC) and GS cells prepared from germ cells (for example, testis) are included in the pluripotent stem cells.

Human embryonic stem cells were established in 1998 and have been used also for regenerative medicine. The embryonic stem cells can be produced by culturing inner cell aggregate on feeder cells or a culture medium containing bFGF. The method for producing embryonic stem cells is described, for example, in WO96/22362, WO02/101057, US5,843,780, US6,200,806, US6,280,718. The embryonic stem cells are available from a predetermined institution and also, commercially available. For example, human embryonic stem cells such as KhES-1, KhES-2 and KhES-3 are available from the Institute for Frontier Life and Medical Sciences, Kyoto University. Human embryonic stem cells such as human ES cells genetically engineered so as to have Crx:: Venus strain and Rx:: Venus strain (both are derived from KhES-1) are available from RIKEN.

The "induced pluripotent stem cells" refers to cells having pluripotency, which is induced by reprogramming somatic cells by a method known in the art.

The induced pluripotent stem cells were established in mouse cells by Yamanaka et al., in 2006 (Cell, 2006, 126 (4), pp. 663-676). The induced pluripotent stem cells were also established in human fibroblasts in 2007. The induced pluripotent stem cells have pluripotency and self-renewal ability similarly to embryonic stem cells (Cell, 2007, 131 (5), pp. 861-872; Science, 2007, 318 (5858), pp. 1917-1920; Nat. Biotechnol., 2008, 26 (1), pp. 101-106).

The induced pluripotent stem cells more specifically refer to cells which are induced to be pluripotent by reprogramming somatic cells differentiated into, for example, fibroblasts and peripheral blood mononuclear cells, by allowing any one of sets of a plurality of genes selected from a reprogramming gene group containing Oct3/4, Sox2, Klf4, Myc (c-Myc, N-Myc, L-Myc), Glis1, Nanog, Sall4, lin28 and Esrrb to express. Examples of a preferable set of reprogramming factors may include (1) Oct3/4, Sox2, Klf4, and Myc (c-Myc or L-Myc) and (2) Oct3/4, Sox2, Klf4, Lin28 and L-Myc (Stem Cells, 2013; 31: 458-466).

Other than producing induced pluripotent stem cells through direct reprogramming by gene expression, the pluripotent stem cells can be artificially induced from somatic cells, for example, by adding a chemical compound (Science, 2013, 341, pp. 651-654).

Alternatively, an induced pluripotent stem cell strain is available. For example, human induced pluripotent cell strains established by Kyoto University, such as 201B7 cell, 201B7-Ff cell, 253G1 cell, 253G4 cell, 1201C1 cell, 1205D1 cell, 1210B2 cell and 1231A3 cell, are available form Kyoto University and iPS Academia Japan, Inc. As the induced pluripotent stem cells, for example, Ff-I01 cell, Ff-I14 cell and QHJI01s04 cell established by Kyoto University, are available from Kyoto University.

In the specification, the pluripotent stem cells are preferably embryonic stem cells or induced pluripotent stem cells, more preferably induced pluripotent stem cells.

In the specification, the pluripotent stem cells are human pluripotent stem cells, preferably human induced pluripotent stem cells (iPS cells) or human embryonic stem cells (ES cells).

Pluripotent stem cells such as human iPS cells can be subjected to maintenance culture and expansion culture performed by methods known to those skilled in the art.

The "neural tissue" refers to a tissue constituted by neural cells of the cerebrum, the midbrain, the cerebellum, the spinal cord, a retina, peripheral nerve, the forebrain, the hindbrain, the telencephalon, the diencephalon, and the like of a developing stage or an adult stage. The neural tissue may form an epithelial structure (neural epithelium) having a layer structure, and the abundance of neural epithelium in a cell aggregate can be evaluated by bright field observation using an optical microscope.

The "neural cells" refer to cells other than epidermal cells in ectoderm-derived tissue. Specifically, they include cells such as neural precursor cells, neurons (neuronal cells), glia, neural stem cells, neuronal progenitor cells and glial progenitor cells. The neural cells also encompass cells constituting retinal tissue mentioned below (retina cells), retinal progenitor cells, retinal layer-specific neuronal cells, neural retina cells, and retinal pigment epithelial cells. The neural cells can be identified with nestin, TuJ1, PSA-NCAM, N-cadherin and the like as markers.

The "neurons" or the "neuronal cells" are functional cells that form a neural circuit and contribute to signal transduction, and can be identified with the expression of immature neuronal cell markers such as TuJ1, Dcx, and HuC/D and/or mature neuronal cell markers such as Map2 and NeuN as an index.

The "neural precursor cells" are a population of precursor cells including neural stem cells, neuronal progenitor cells and glial progenitor cells and have proliferation potency and the ability to produce neurons and glia. The neural precursor cells can be identified with nestin, GLAST, Sox2, Sox1, Musashi, Pax6 and the like as markers. Alternatively, neural cell marker-positive and proliferation marker (Ki67, pH3, MCM)-positive cells can also be identified as the neural precursor cells.

The "retinal tissue" means a tissue in which a single type or a plurality of types of retinal cells constituting each retinal layer in a retina *in vivo* are present according to a predetermined order. The "neural retina" is a retinal tissue and means a tissue containing an inside neural retinal layer that does not contain a retinal pigment epithelial layer among retinal layers mentioned later.

The "retinal cells" mean cells constituting each retinal layer in a retina *in vivo* or progenitor cells thereof. In the retinal cells, cells such as photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, retinal pigment epithelial (RPE) cells, ciliary body, their progenitor cells (e.g., photoreceptor progenitor cell, bipolar progenitor cell, retinal pigment epithelial progenitor cells), neural retinal progenitor cells and retinal progenitor cells are included, though not limited thereto. Among the retinal cells, examples of cells constituting a neural retinal layer (also referred to as neural retina cells or neural retina-related cells) specifically include cells such as photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, and their progenitor cells (e.g., photoreceptor progenitor cell, bipolar progenitor cell). In other words, in the neural retina-related cells, neither retinal pigment epithelial cells nor ciliary body cells are included.

The "matured retinal cells" mean cells that may be contained in the retinal tissue of a human adult, and specifically mean differentiated cells such as photoreceptor cells (rod photoreceptor cell, cone photoreceptor cell), horizontal cells, amacrine cells, intermediate neuronal cells, retinal ganglion cells (ganglion cell), bipolar cells (rod bipolar cell, cone bipolar cell), Muller glial cells, retinal pigment epithelial (RPE) cells, and ciliary body cells. The "immature retinal cells" mean progenitor cells (e.g., photoreceptor progenitor cell, bipolar progenitor cell, retinal progenitor cell) destined for differentiation into matured retinal cells.

The photoreceptor progenitor cells, the horizontal progenitor cells, the bipolar progenitor cells, the amacrine progenitor cells, the retinal ganglion progenitor cells, the Muller glial progenitor cells, and the retinal pigment epithelial progenitor cells refer to progenitor cells destined for differentiation into photoreceptor cells, horizontal cells, bipolar cells, amacrine cells, retinal ganglion cells, Muller glial cells, and retinal pigment epithelial cells, respectively.

The "retinal progenitor cells" are progenitor cells capable of differentiating into any one of the immature retinal cells such as photoreceptor progenitor cells, horizontal progenitor cells, bipolar progenitor cells, amacrine progenitor cells, retinal ganglion progenitor cells, Muller glial cells, and retinal pigment epithelial progenitor cells, and refer to progenitor cells also capable of eventually differentiating into any one of the matured retinal cells such as photoreceptor cells, rod photoreceptor cells, cone photoreceptor cells, horizontal cells, bipolar cells, amacrine cells, retinal ganglion cells, and retinal pigment epithelial cells. The "neural retinal progenitor cell" are progenitor cells capable of differentiating into any one of the immature neural retinal cells such as photoreceptor progenitor cells, horizontal progenitor cells, bipolar progenitor cells, amacrine progenitor cells, retinal ganglion progenitor cells, and Muller glial cells, and refer to progenitor cells also capable of eventually differentiating into any one of the matured neural retinal cells such as photoreceptor cells, rod photoreceptor cells, cone photoreceptor cells, horizontal cells, bipolar cells, amacrine cells, and retinal ganglion cells. The neural retinal progenitor cells have no differentiation capacity into retinal pigment epithelial cells.

The "photoreceptor cells" are present in the photoreceptor layer of a retina *in vivo* and plays a role in absorbing light stimuli and converting them to electrical signals. The photoreceptor cells have two types, cones which function in the light and rods which function in the dark (referred to as cone photoreceptor cells and rod photoreceptor cells, respectively). Examples of the cone photoreceptor cells can include S cone photoreceptor cells which express S-opsin and receive blue light, L cone photoreceptor cells which express L-opsin and receive red light, and M cone photoreceptor cells which express M-opsin and receive green light. The photoreceptor cells are matured after differentiation from photoreceptor progenitor cells. Whether or not cells are photoreceptor cells or photoreceptor progenitor cells can be readily confirmed by those skilled in the art, for example, through the expression of cell markers (Crx and Blimp1 expressed in photoreceptor progenitor cells, recoverin expressed in photoreceptor cells, rhodopsin, S-opsin and M/L-opsin expressed in mature photoreceptor cells, etc.) mentioned later or the formation of an outer segment structure. In an embodiment, the photoreceptor progenitor cells are Crx-positive cells, and the photoreceptor cells are rhodopsin-, S-opsin- and M/L-opsin-positive cells. In an embodiment, the rod photoreceptor cells are NRL- and rhodopsin-positive cells. In an embodiment, the S cone photoreceptor cells are S-opsin-positive cells, the L cone photoreceptor cells are L-opsin-positive cells, and the M cone photoreceptor cells are M-opsin-positive cells.

The presence of neural retina-related cells can be confirmed from the presence or absence of expression of a neural retina-related cellrelated gene (hereinafter, also referred to as "neural retina-related cell marker" or "neural retina marker"). The presence or absence of expression of the neural retina-related cell marker, or the percentage of neural retina-related cell marker-positive cells in a cell population or a tissue can be readily confirmed by those skilled in the art. Examples thereof include an approach using an antibody, an approach using nucleic acid primers, and an approach using sequencing reaction. As the approach using an antibody, the expression of a protein of the neural retina-related cell marker can be confirmed, for example, by dividing the number of predetermined neural retina-related cell marker-positive cells by the total number of cells in accordance with an approach such as flow cytometry (FACS) or immunostaining using a commercially available antibody. As the approach using nucleic acid primers, the expression of RNA of the neural retina-related cell marker can be confirmed by, for example, PCR, semiquantitative PCR, or quantitative PCR (e.g., realtime PCR). As the approach using sequencing reaction, the expression of RNA of the neural retina-related cell marker can be confirmed using, for example, a nucleic acid sequencer (e.g., next-generation sequencer).

Examples of the neural retina-related cell marker include Rx (also referred to as Rax) and PAX6 expressed in retinal progenitor cells, Rx, PAX6 and Chx10 (also referred to as Vsx2) expressed in neural retinal progenitor cells, and Crx and Blimp1 expressed in photoreceptor progenitor cells. Examples thereof also include Chx10 strongly expressed in bipolar cells, PKCa, Goα, VSX1 and L7 expressed in bipolar cells, TuJ1 and Brn3 expressed in retinal ganglion cells, calretinin and HPC-1 expressed in amacrine cells, calbindin expressed in horizontal cells, recoverin expressed in photoreceptor cells and photoreceptor progenitor cells, rhodopsin expressed in rod cells, Nrl expressed in rod photoreceptor cells and rod photoreceptor progenitor cells, S-opsin and LM-opsin expressed in cone photoreceptor cells, RXR-γ expressed in cone cells, cone photoreceptor progenitor cells and ganglion cells, TKβ2, OTX2 and OC2 expressed in cone photoreceptor cells that appear at the early phase of differentiation among cone photoreceptor cells, or progenitor cells thereof, and Pax6 commonly expressed in horizontal cells, amacrine cells and ganglion cells. Surface antigens of retinal progenitor cells or neural retinal progenitor cells identified in Examples of the present application can also be used as markers of retinal progenitor cells or neural retinal progenitor cells. The details will be mentioned later.

The "positive cells" mean cells expressing a predetermined marker on the cell surfaces or within the cells. For example, the "Chx10-positive cells" mean cells expressing Chx10 protein.

The "retinal pigment epithelial cells" mean epithelial cells present outside the neural retina in a retina *in vivo.* Whether or not cells are retinal pigment epithelial cells can be readily confirmed by those skilled in the art, for example, through the expression of cell markers (MITF, Pax6, PMEL17, TYRP1, TRPM1, ALDH1A3, GPNMB, RPE65, CRALBP, MERTK, BEST1, TTR, etc.), the presence of melanin granules (brown-black), intercellular tight junctions, or polygonal/flagstone-like characteristic cell morphology. Whether or not cells have a function of retinal pigment epithelial cells can be readily confirmed from the ability to secrete cytokines such as VEGF and PEDF, phagocytic capacity of photoreceptor cell outer segment, or the like. In an embodiment, the retinal pigment epithelial cells are RPE65-positive cells, MITF-positive cells, or RPE65-positive and MITF-positive cells.

The "retinal pigment epithelial cell sheet" refers to a single-layer or multilayer sheet-shaped structure constituted from single or a plurality of cells in which retinal pigment epithelial cells adhere to each other through a biological bond at least in the two-dimensional direction.

The "retinal layer" means individual layers constituting the retina, and examples thereof can specifically include retinal pigment epithelial layer, photoreceptor layer, outer limiting membrane, outer nuclear layer, outer plexiform layer, inner nuclear layer, inner plexiform layer, ganglion cell layer, nerve fiber layer and inner limiting membrane.

The "neural retinal layer" means individual layers constituting the neural retina, and examples thereof can specifically include photoreceptor layer, outer limiting membrane, outer nuclear layer, outer plexiform layer, inner nuclear layer, inner plexiform layer, ganglion cell layer, nerve fiber layer and inner limiting membrane. The "photoreceptor layer" means a retinal layer that is formed in the outermost of the neural retina and is rich in one or more cells selected from the group consisting of a photoreceptor cell (rod photoreceptor cell, cone photoreceptor cell), a photoreceptor progenitor cell and a retinal progenitor cell. Each layer other than the photoreceptor layer is referred to as an inner layer. Which retinal layer the individual cells constitute can be confirmed by a known method, for example, by determining the presence or absence of expression or expression level of a cell marker.

In the case of retinal tissue at a stage where the appearance percentage of photoreceptor cells or photoreceptor progenitor cells is low, a layer containing proliferating neural retinal progenitor cells is referred to as "neuroblastic layer" and includes inner neuroblatic layer and outer neuroblastic layer. Those skilled in the art can make a judgment from the shade of color (the outer neuroblastic layer is light, and the inner neuroblatic layer is dark) by a known method, for example, under a bright field microscope.

The "ciliary body" includes "ciliary body" and "ciliarymarginal zone" in the process of development and of an adult. Examples of a marker of the "ciliary body" include Zic1, MAL, HNF1beta, FoxQ1, CLDN2, CLDN1, GPR177, AQP1 and AQP4. Examples of the "ciliarymarginal zone (CMZ)" can include a tissue that is present in a boundary region between the neural retina and the retinal pigment epithelium in a retina *in vivo,* and is a region containing tissue stem cells of the retina (retinal stem cells). The ciliarymarginal zone is also called ciliarymargin or retinalmargin, and the ciliarymarginal zone, the ciliarymargin and the retinalmargin are equivalent tissues. The ciliarymarginal zone is known to play an important role in the supply of retinal progenitor cells or differentiated cells to retinal tissue, the maintenance of a retinal tissue structure, etc. Examples of a marker gene of the ciliarymarginal zone can include *Rdh10* gene (positive), *Otx1* gene (positive) and *Zic1* (positive). The "ciliarymarginal zone-like structure" is a structure similar to the ciliarymarginal zone.

The "cerebral tissue" is a tissue in which one type or at least a plurality of types of cells constituting the cerebrum of a fetal stage or an adult (e.g., cortical neural precursor cells, dorsal cerebral neural precursor cells, ventral cerebral neural precursor cells, cerebral layer structurespecific neuronal cells (neurons), layer 1 neurons, layer 2 neurons, layer 3 neurons, layer 4 neurons, layer 5 neurons, layer 6 neurons, glial cells (astrocytes and oligodendrocytes), and their progenitor cells) are threedimensionally arranged in layers. The cerebrum of a fetal stage is also called forebrain or telencephalon. The presence of each cell can be confirmed by a known method, for example, the presence or absence of expression of a cell marker or the level thereof.

The "cerebral layer" refers to each layer constituting the adult cerebrum or the fetal stage cerebrum, and examples can specifically include molecular layer, outer nuclear layer, external pyramidal layer, inner nuclear layer, neuronal cell layer (internal pyramidal layer), polymorphic cell layer, layer 1, layer 2, layer 3, layer 4, layer 5, layer 6, cortex zone, intermediate zone, subventricular zone, and ventricular zone.

Examples of the "cortical neural precursor cells" can include neuronal progenitor cells, layer 1 neuronal progenitor cells, layer 2 neuronal progenitor cells, layer 3 neuronal progenitor cells, layer 4 neuronal progenitor cells, layer 5 neuronal progenitor cells, layer 6 neuronal progenitor cells, astrocyte progenitor cells, and oligodendrocyte progenitor cells. These cells are progenitor cells committed to differentiate into layer 1 neurons, layer 2 neurons, layer 3 neurons, layer 4 neurons, layer 5 neurons, layer 6 neurons, astrocytes, and oligodendrocytes, respectively.

The "cortical neural precursor cells" include multipotent stem cells (multipotent neural stem cells) having differentiation capacity (multipotency) into at least a plurality of differentiation lineages among layer 1 neurons, layer 2 neurons, layer 3 neurons, layer 4 neurons, layer 5 neurons, layer 6 neurons, astrocytes, and oligodendrocytes.

The "cerebral layer-specific neuronal cells" are cells constituting the cerebral layer and refer to neuronal cells specific for the cerebral layer. Examples of the cerebral layer-specific neuronal cells can include layer 1 neurons, layer 2 neurons, layer 3 neurons, layer 4 neurons, layer 5 neurons, layer 6 neurons, cerebral excitatory neurons, and cerebral inhibitory neurons.

Examples of the cerebral cell marker include FoxG1 (also called Bf1) expressed in cerebral cells, Sox2 and nestin expressed in cortical neural precursor cells, Pax6 and Emx2 expressed in dorsal cortical neural precursor cells, Dlx1, Dlx2 and Nkx2.1 expressed in ventral cortical neural precursor cells, Tbr2, Nex, and Svet1 expressed in neuronal progenitor cells, Tbr1 expressed in layer 6 neurons, Ctip2 expressed in layer 5 neurons, RORβ expressed in layer 4 neurons, Cux1 or Brn2 expressed in layer 3 neurons or layer 2 neurons, and reelin expressed in layer 1 neurons.

The "cell aggregate" is not particularly limited as long as a plurality of cells mutually adhere to form a three-dimensional structure, and refers to, for example, a mass formed by the aggregation of cells dispersed in a vehicle such as a culture medium, or a mass of cells formed through cell division. In the cell aggregate, the case of forming a predetermined tissue is also included.

The "sphere-like cell aggregate" means a cell aggregate having a stereoscopic shape close to a spherical shape. The stereoscopic shape close to a spherical shape is a shape having a three-dimensional structure, and examples thereof include a spherical shape that exhibits a circle or an ellipse when projected onto a two-dimensional surface, and a shape formed by fusing a plurality of spherical shapes (e.g., which exhibits a shape formed by 2 to 4 circles or ellipses overlapping when twodimensionally projected). In an embodiment, the core part of the aggregate has a vesicular lamellar structure and is characterized in that the central part is observed to be dark and the outer edge portion is observed to be bright under a bright field microscope.

The "epithelial tissue" is a tissue formed by covering the body surface or the surface of a lumen (digestive tract, etc.), body cavity (pericardial cavity, etc.) or the like with cells without any space. The cells forming the epithelial tissue are referred to as epithelial cells. The epithelial cells have a polarity in the apical-basal direction. The epithelial cells can mutually and firmly join via adherence junction and/or tight junction to form a layer of the cells. A tissue formed from a single layer or dozen layers overlapping of this layer of the cells is the epithelial tissue. In a tissue capable of forming the epithelial tissue, retinal tissue, brain and spinal cord tissue, eyeball tissue, neural tissue or the like of a fetal stage and/or an adult is also included. In the specification, the neural retina is also the epithelial tissue. The "epithelial structure" means a structure characteristic of the epithelial tissue (e.g., having polarities of a basal surface and an apical surface).

The "continuous epithelial tissue" is a tissue having a continuous epithelium structure. The continuous epithelium structure is a structure where the epithelial tissue is continuously formed. The epithelium tissue continuously formed is a state in which 10 cells to 10⁷ cells, for example, in the tangent direction of the epithelial tissue, preferably 30 cells to 10⁷ cells, further preferably 10² cells to 10⁷ cells, in the tangent direction, are aligned.

For example, in the continuous epithelium structure formed in retinal tissue, the retinal tissue has an apical surface intrinsic to the epithelial tissue. The apical surface is formed almost in parallel to, for example, at least photoreceptor layer (outer nuclear layer) among the layers forming a neural retinal layer and continuously on the surface of the retinal tissue. For example, in the case of a cell aggregate containing retinal tissue prepared from pluripotent stem cells, the apical surface is formed on the surface of the aggregate, and 10 cells or more, preferably 30 cells or more, more preferably 100 cells or more, further preferably 400 cells or more of photoreceptor cells or photoreceptor progenitor cells are regularly and continuously arranged in a row in the tangent direction of the surface.

In an embodiment, epithelial tissue is polarized so that "apical surface" and "basal surface" and "basal membrane" are formed. The "basal membrane" refers to a basal side layer (basal membrane) produced by epithelial cells, is rich in laminin and IV-type collagen, and has a thickness of 50 to 100 nm. The "basal surface" refers to the surface (upper surface layer) formed on the "basal membrane" side. The "apical surface" refers to the surface (upper surface layer) formed on the opposite side to the "basal membrane". In an embodiment, in the retinal tissue developed to the extent that photoreceptor cells or photoreceptor progenitor cells are observed, the "apical surface" refers to a surface in contact with photoreceptor layer (outer nuclear layer) in which outer limiting membrane is formed and photoreceptor cells and photoreceptor progenitor cells are present. Such an apical surface can be identified by, for example, immunostaining (known to those skilled in the art) using an antibody against an apical surface marker (e.g., atypical PKC (hereinafter, abbreviated to "aPKC"), tight junction marker (Zo-1), ERM protein ezrin, E-cadherin, N-cadherin).

### [Method for manufacturing cell aggregate]

An aspect of the present invention is a manufacturing method for manufacturing a cell aggregate comprising retinal tissue having an epithelial structure (or a multilayered structure) (in the present specification, the "cell aggregate comprising retinal tissue" is also simply referred to as "retinal tissue") from a dispersed retinal cell population. The method comprises suspension-culturing or adhesion-culturing a dispersed retinal cell population in a culture medium containing a Wnt signaling pathway agonist. In this context, the dispersed retinal cell population comprises one or more cells selected from the group consisting of a retinal progenitor cell and a photoreceptor progenitor cell. The retinal tissue is as defined above. In an embodiment, the retinal tissue includes neural retinal tissue and may include both neural retinal tissue and retinal pigment epithelial tissue.

The retinal cell is as defined above, and in an embodiment, the retinal cell includes one or more cells selected from the group consisting of a retinal progenitor cell and a photoreceptor progenitor cell and may additionally include cells such as photoreceptor cells (rod photoreceptor cell, cone photoreceptor cell), horizontal cells, amacrine cells, retinal ganglion cells (ganglion cell), bipolar cells (rod bipolar cell, cone bipolar cell), and Muller glial cells. It is preferable that the retinal cell should be a neural retinal cell. The retinal cell population used may be derived from living tissue or derived from pluripotent stem cells, and it is preferable to be obtained by differentiating pluripotent stem cells.

The retinal cell derived from living tissue can be prepared by those skilled in the art from retinal tissue derived from a living body through a well-known technique. For example, there are a method of harvesting the eye, followed by detachment from RPE and recovery and a method of directly harvesting a neural retina.

An aspect of the present invention is a method for manufacturing a cell aggregate comprising neural tissue (e.g., cerebral (telencephalic) tissue) having an epithelial structure (or a multilayered structure) from a dispersed neural cell population. The method comprises adhesion-culturing or suspension-culturing a dispersed neural cell population in a culture medium containing a Wnt signaling pathway agonist. It is preferable that the neural cell should include a neural precursor cell (e.g., cortical neural precursor cell). The neural cell population used may be derived from living tissue or derived from pluripotent stem cells, and it is preferable to be obtained by differentiating pluripotent stem cells.

### (Method for differentiating retinal cell population as starting cell)

Pluripotent stem cell-derived retinal cells as starting cells in the manufacturing method of the present invention can be obtained by differentiating pluripotent stem cells. In an embodiment, an aggregate of retinal cells can be obtained by differentiating pluripotent stem cells using a differentiation factor. Examples of the differentiation factor include basal membrane preparations, BMP signaling pathway agonists, Wnt signaling pathway inhibitors, and IGF signaling pathway agonists. An embodiment includes a method for manufacturing an aggregate of retinal cells by self-organization. The self-organization refers to a mechanism in which a population of cells autonomously yields a complicated structure. The self-organization can be performed by, for example, SFEB (serum-free floating culture of embryoid bodies-like aggregates) (WO2005/12390) or SFEBq (WO2009/148170).

Specific examples of the differentiation method include, but are not particularly limited to, methods disclosed in WO2011/055855, WO2013/077425, WO2015/025967, WO2016/063985, WO2016/063986, WO2017/183732, PLoS One. 2010 Jan 20; 5 (1): e8763, Stem Cells. 2011 Aug; 29 (8): 1206-18, Proc Natl Acad Sci USA. 2014 Jun 10; 111 (23): 8518-23, or Nat Commun. 2014 Jun 10; 5: 4047.

In a specific embodiment, an aggregate of retinal cells can be prepared by a method comprising the following steps (A), (B) and (C):
(A) culturing pluripotent stem cells in a culture medium containing a factor for maintaining undifferentiated state in the absence of feeder cells;
(B) forming a cell aggregate by suspension-culturing the cells obtained in the step (A); and
(C) further suspension-culturing the cell aggregate obtained in the step (B) in a culture medium containing a BMP signaling pathway agonist.

The step (A) may further involve a TGFβ family signaling pathway inhibitor and/or a sonic hedgehog signaling pathway agonist.

Also, the step (B) may involve a sonic hedgehog signaling pathway agonist and/or a Wnt signaling inhibitor, as mentioned later.

This method is also disclosed in, for example, WO2015/025967, WO2016/063985, and WO2017/183732. For more details, see WO2015/025967, WO2016/063985, WO2017/183732, etc.

The culture medium that is used in the preparation of the aggregate of retinal cells can employ a basal medium for cell proliferation (also referred to as a basal medium), unless otherwise specified. The basal medium for cell proliferation is not particularly limited as long as the culture of cells is possible. A basal medium commercially available as a culture medium for cell proliferation can be appropriately used. Specifically, examples thereof can include culture media that can be used in the culture of animal cells, such as BME medium, BGJb medium, CMRL 1066 medium, Glasgow MEM(GMEM) medium, Improved MEM Zinc Option medium, IMDM medium, Medium 199 medium, MEM medium, Eagle MEM medium, αMEM medium, DMEM medium, F-12 medium, DMEM/F12 medium, IMDM/F12 medium, Ham's medium, RPMI 1640 medium, Fischer's medium, Leibovitz's L-15 medium and mixtures of these media. Alternatively, a culture medium supplemented with N2 medium which is an assisted culture medium may be used.

The TGFβ family signaling pathway inhibitor refers to a substance inhibiting the TGFβ family signaling pathway, i.e., the signaling pathway transduced by the Smad family. Specifically, examples thereof can include TGFβ signaling pathway inhibitors (e.g., SB431542, LY-364947, SB505124, A-83-01), Nodal/activin signaling pathway inhibitors (e.g., SB431542, A-83-01) and BMP signaling pathway inhibitors (e.g., LDN193189, dorsomorphin). These substances are commercially available and can be obtained.

The sonic hedgehog (hereinafter, also referred to as "Shh" or "SHH") signaling pathway agonist is a substance capable of enhancing signal transduction mediated by Shh. Examples of the Shh signaling pathway agonist include SHH, partial peptides of SHH, PMA (purmorphamine), and SAG (smoothened agonist).

The concentrations of the TGFβ family signaling pathway inhibitor and the sonic hedgehog signaling pathway agonist can be concentrations capable of inducting differentiation into retinal cells. For example, SB431542 is used at a concentration of usually 0.1 to 200 µM, preferably 2 to 50 µM. A-83-01 is used at a concentration of usually 0.05 to 50 µM, preferably 0.5 to 5 µM. LDN193189 is used at a concentration of usually 1 to 2000 nM, preferably 10 to 300 nM. SAG is used at a concentration of usually 1 to 2000 nM, preferably 10 to 700 nM. PMA is used at a concentration of usually 0.002 to 20 µM, preferably 0.02 to 2 µM.

The factor for maintaining undifferentiated state is not particularly limited as long as it is a substance having an action of suppressing the differentiation of pluripotent stem cells. Examples of the factor for maintaining undifferentiated state that is generally used by those skilled in the art can include FGF signaling pathway agonists, TGFβ family signaling pathway agonists, and insulin. Examples of the FGF signaling pathway agonist specifically include fibroblast growth factors (e.g., bFGF, FGF4, FGF8). Examples of the TGFβ family signaling pathway agonist include TGFβ signaling pathway agonists and Nodal/activin signaling pathway agonists. Examples of the TGFβ signaling pathway agonist include TGFβ1 and TGFβ2. Examples of the Nodal/activin signaling pathway agonist include Nodal, activin A, and activin B. In the case of culturing human pluripotent stem cells (human ES cells, human iPS cells), the culture medium in the step (A) preferably contains bFGF as the factor for maintaining undifferentiated state.

The concentration of the factor for maintaining undifferentiated state in the culture medium that is used in the step (A) is a concentration capable of maintaining the undifferentiated state of the pluripotent stem cells to be cultured, and can be appropriately set by those skilled in the art. Specifically, for example, in the case of using bFGF as a factor for maintaining undifferentiated state in the absence of feeder cells, its concentration is usually on the order of 4 to 500 ng/mL, preferably on the order of 10 to 200 ng/mL, more preferably on the order of 30 to 150 ng/mL.

Many synthetic media have been developed or are commercially available as feeder-free media that contain a factor for maintaining undifferentiated state and may be used for culturing pluripotent stem cells. Examples thereof include Essential 8 medium (manufactured by Life Technologies Corp.). The Essential 8 medium contains L-ascorbic acid-2-phosphate magnesium (64 mg/L), sodium selenium (14 µg/L), insulin (19.4 mg/L), NaHCO₃ (543 mg/L), transferrin (10.7 mg/L), bFGF (100 ng/mL), and the TGFβ family signaling pathway agonist (TGFP 1 (2 ng/mL) or Nodal (100 ng/mL)) as additives in DMEM/F12 medium (Nature Methods, 8, 424-429 (2011)). Examples of other commercially available feeder-free media include S-medium (manufactured by DS Pharma Biomedical Co., Ltd.), StemPro (manufactured by Life Technologies Corp.), hESF9 (Proc. Natl. Acad. Sci. USA. 2008 Sep 9; 105 (36): 13409-14), mTeSR1 (manufactured by STEMCELL Technologies Inc.), mTeSR2 (manufactured by STEMCELL Technologies Inc.), TeSR-E8 (manufactured by STEMCELL Technologies Inc.), and StemFit (manufactured by Ajinomoto Co., Inc.). In the step (A), the present invention can be conveniently carried out by using these. By using these culture media, it is possible to perform the culture of pluripotent stem cells under feeder-free conditions. The culture medium that is used in the step (A) is, as one example, a serum-free medium that is not supplemented with any of the BMP signaling pathway agonist, the Wnt signaling pathway agonist and the Wnt signaling pathway inhibitor.

In the culture of pluripotent stem cells under feeder-free conditions in the step (A), a suitable matrix may be used as a scaffold in order to provide a scaffold as a replacement for feeder cells to the pluripotent stem cells. Examples of the matrix that can be used as a scaffold include laminin (Nat Biotechnol 28, 611-615, (2010)), laminin fragments (Nat Commun 3, 1236, (2012)), basal membrane preparations (Nat Biotechnol 19, 971-974, (2001)), gelatin, collagen, heparan sulfate proteoglycan, entactin, and vitronectin.

The culture time of the pluripotent stem cells in the step (A) is not particularly limited within a range in which an effect of improving the quality of the cell aggregate to be formed in the step (B) can be achieved in the case of culture in the presence of the TGFβ family signaling pathway inhibitor and/or the sonic hedgehog signaling pathway agonist (e.g., from 100 nM to 700 nM), and is usually from 0.5 to 144 hours. In an embodiment, it is preferably from 2 to 96 hours, more preferably from 6 to 48 hours, further preferably from 12 to 48 hours, still further preferably from 18 to 28 hours (e.g., 24 hours).

The culture medium that is used in the step (B) may be a serumcontaining medium or a serum-free medium. A serum-free medium is suitably used from the viewpoint of circumventing contamination with chemically undetermined components. In order to circumvent the complication of preparation, examples thereof include serum-free media supplemented with an appropriate amount of a serum replacement such as commercially available KSR. The amount of KSR added to the serum-free medium is usually from about 1% to about 30%, preferably from about 2% to about 20%.

For the formation of the aggregate, first, dispersed cells are prepared by the dispersion operation of the cells obtained in the step (A). The "dispersed cells" obtained by dispersion operation include a state in which 70% (preferably 80% or more) or more are single cells and 30% or less (preferably 20% or less) of 2- to 50-cell masses are present. The dispersed cells include a state in which the mutual adhesion (e.g., surface adhesion) of cells has been mostly lost.

A suspension of the dispersed cells is seeded into an incubator, and the dispersed cells are cultured under conditions of non-adhesive to the incubator, thereby causing the aggregation of a plurality of cells to form an aggregate. In an embodiment, when a predetermined number of dispersed stem cells is placed in each well of a multi-well plate (U-bottom, V-bottom) such as a 96-well plate and this is statically cultured, the cells aggregate rapidly, thereby forming one aggregate in each well (SFEBq). In the case of suspension-culturing cells using a 96-well plate, a liquid prepared so as to attain about 1 × 10³ to about 1 × 10⁵ cells (preferably about 3 × 10³ to about 5 × 10⁴ cells or about 4 × 10³ to about 2 × 10⁴ cells) per well is added to the wells, and the plate is left standing to form aggregates.

In an embodiment, the culture medium that is used in the step (B) contains a sonic hedgehog signaling pathway agonist.

In other words, in a specific embodiment, the aggregate of the retinal cell containing a neural retina can be prepared by a method comprising the following steps (A), (B) and (C):
(A) culturing pluripotent stem cells in a culture medium containing a factor for maintaining undifferentiated state and optionally containing a TGFβ family signaling pathway inhibitor and/or a sonic hedgehog signaling pathway agonist in the absence of feeder cells;
(B) forming a cell aggregate by suspension-culturing the cells obtained in the step (A) in a culture medium containing a sonic hedgehog signaling pathway agonist; and
(C) further suspension-culturing the cell aggregate obtained in the step
(B) in a culture medium containing a BMP signaling pathway agonist.

As the sonic hedgehog signaling pathway agonist in the step (B), the one mentioned above can be used at the concentration mentioned above (e.g., from 10 nM to 300 nM). The sonic hedgehog signaling pathway agonist is preferably contained in the culture medium from the start of suspension culture. A ROCK inhibitor (e.g., Y-27632) may be added to the culture medium. The culture time is, for example, from 12 hours to 6 days. The culture medium that is used in the step (B) is, as one example, a culture medium that is not supplemented with one or more (preferably all) selected from the group consisting of a BMP signaling pathway agonist, a Wnt signaling pathway agonist, a TGFβ family signaling pathway inhibitor and a TGFβ family signaling pathway agonist.

In the step (B), the expression of a cell surface marker CD39 for retinal progenitor cells or neural retinal progenitor cells (Rx-positive cells) mentioned later increases by performing culture in a culture medium containing a sonic hedgehog signaling pathway agonist. In an embodiment, the expression of CD39 increases by adding a high concentration (100 nM to 1000 nM, for example, 300 nM) of SAG from the start of suspension culture (Day 0) in the step (B). In the case of performing a purification step for retinal progenitor cells or neural retinal progenitor cells using CD39, more CD39-positive cells can be purified by using the method, and manufacturing efficiency is improved. A tissue containing CD39-positive cells obtained by adding 300 nM SAG is Chx10-positive, Lhx2-positive, and NKX2.1-negative and is therefore retinal tissue. Particularly, ventral (CoupTF1-positive) retinal tissue is induced. Retinal tissue obtained without adding SAG in the step (B) is induced as dorsal (ALDH1A1-positive) retinal tissue.

In the step (B), the percentage of CD39-positive cells in a tissue to be induced also increases by performing culture in a culture medium containing one or more selected from the group consisting of ATP, an ATP analog (AMP-PNP, etc.) and an A2A receptor agonist (adenosine, CGS 21680 (3-[4-[2-[[6-amino-9-[(2R,3R,4S,SS)-5-(ethylcarbamoyl)-3,4-dihydroxy-oxolan-2-yl]purin-2-yl]amino]ethyl]phenyl]propanoicacid), etc.).

The BMP signaling pathway agonist is a substance capable of enhancing the signaling pathway mediated by BMP. Examples of the BMP signaling pathway agonist include BMP protein such as BMP2, BMP4 and BMP7, GDF protein such as GDF7, anti-BMP receptor antibodies, and BMP partial peptides. The BMP2 protein, the BMP4 protein and the BMP7 protein are available from, for example, R&D Systems, Inc., and the GDF7 protein is available from, for example, Wako Pure Chemical Industries, Ltd.

Examples of the culture medium that is used in the step (C) include serum-free media and serum media (preferably serum-free media) supplemented with a BMP signaling pathway agonist. The serum-free medium and the serum medium can be provided as mentioned above. The culture medium that is used in the step (C) is, as one example, a culture medium that is not supplemented with one or more (preferably all) selected from the group consisting of a Wnt signaling pathway agonist, a TGFβ family signaling pathway inhibitor and a TGFβ family signaling pathway agonist. Alternatively, the culture medium that is used in the step (C) is, as one example, a culture medium that is not supplemented with a sonic hedgehog signaling pathway agonist. Alternatively, the culture medium that is used in the step (C) is a culture medium that may be supplemented with a Wnt signaling pathway agonist.

The concentration of the BMP signaling pathway agonist can be a concentration capable of inducing differentiation into retinal cells. For example, human BMP4 protein is added to the culture medium so as to attain a concentration of about 0.01 nM to about 1 µM, preferably about 0.1 nM to about 100 nM, more preferably about 1 nM to about 10 nM, further preferably about 1.5 nM (55 ng/mL).

The BMP signaling pathway agonist can be added about 24 hours or later after the start of suspension culture in the step (A), and may be added to the culture medium within several days (e.g., within 15 days) after the start of suspension culture. Preferably, the BMP signaling pathway agonist is added to the culture medium between Day 1 and Day 15, more preferably between Day 1 and Day 9, most preferably on Day 3, after the start of suspension culture.

In a specific embodiment, a part or the whole of the culture medium is exchanged with a culture medium containing BMP4, for example, on Days 1 to 9, preferably Days 1 to 3, after the start of suspension culture in the step (B), and the final concentration of BMP4 is prepared to about 1 to 10 nM. Culture can be performed for, for example, 1 to 12 days, preferably 2 to 9 days, further preferably 2 to 5 days, in the presence of BMP4. In this context, in order to maintain the concentration of BMP4 at the same concentration, a part or the whole of the culture medium can be exchanged with a culture medium containing BMP4 once or about twice. Alternatively, the concentration of BMP4 may be decreased in stages. For example, the concentration of the BMP signaling pathway agonist (BMP4) is maintained from Days 2 to 10 after the start of suspension culture in the step (B), and then, the concentration of the BMP signaling pathway agonist (BMP4) may be decreased in stages from Days 6 to 20 after the start of suspension culture in the step (B).

Culture conditions such as culture temperature and CO₂ concentration in the step (A) to the step (C) can be appropriately set. The culture temperature is, for example, from about 30°C to about 40°C, preferably about 37°C. The CO₂ concentration is, for example, from about 1% to about 10%, preferably about 5%.

Retinal cells at various stages of differentiation can be produced as retinal cells contained in the cell aggregate by varying the culture period in the step (C). In other words, retinal cells in the cell aggregate containing immature retinal cells (e.g., retinal progenitor cell, photoreceptor progenitor cell) and matured retinal cells (e.g., photoreceptor cell) at various percentages can be produced. The percentage of matured retinal cells can be increased by extending the culture period in the step (C).

The step (B) and/or the step (C) may employ a method disclosed in WO2017/183732. Specifically, in the step (B) and/or the step (C), the cell aggregate can be formed by suspension culture in a culture medium further containing a Wnt signaling pathway inhibitor.

The Wnt signaling pathway inhibitor that is used in the step (B) and/or the step (C) is not particularly limited as long as it is capable of suppressing signal transduction mediated by Wnt, and may be any of a protein, a nucleic acid, a low-molecular compound, and the like. Signals mediated by Wnt are transduced via Wnt receptor present as a heterodimer of frizzled (Fz) and LRP5/6 (low-density lipoprotein receptor-related protein 5/6). Examples of the Wnt signaling pathway inhibitor include, but are not limited to, substances acting directly on Wnt or Wnt receptor (anti-Wnt neutralizing antibody, anti-Wnt receptor neutralizing antibody, etc.), substances suppressing the expression of a gene encoding Wnt or Wnt receptor (e.g., antisense oligonucleotide, siRNA), substances inhibiting the binding of Wnt to Wnt receptor (soluble Wnt receptor, dominant negative Wnt receptor, etc., Wnt antagonist, Dkk1, Cerberus protein, etc.), and substances inhibiting bioactivity caused by signal transduction ascribable to Wnt receptor [e.g., low-molecular compounds such as CKI-7 (N-(2-aminoethyl)-5-chloroisoquinoline-8-sulfonamide), D4476 (4-[4-(2,3-dihydro-1,4-benzodioxin-6-yl)-5-(2-pyridinyl)-1H-imidazol-2-yl]benzamide), IWR-1-endo (IWR1e) (4-[(3aR,4S,7R,7aS)-1,3,3a,4,7,7a-hexahydro-1,3-dioxo-4,7-methano-2H-isoindol-2-yl]-N-8-quinolinyl-benzamide), and IWP-2 (N-(6-methyl-2-benzothiazolyl)-2-[(3,4,6,7-tetrahydro-4-oxo-3-phenylthieno[3,2-d]pyrimidin-2-yl)thio]acetamide)]. One or two or more of these may be contained as the Wnt signaling pathway inhibitor. CKI-7, D4476, IWR-1-endo (IWR1e), IWP-2, and the like are known Wnt signaling pathway inhibitors, and commercially available products, etc. can be appropriately obtained. IWR1e is preferably used as the Wnt signaling pathway inhibitor.

The concentration of the Wnt signaling pathway inhibitor in the step (B) can be a concentration capable of inducing the favorable formation of the cell aggregate. For example, IWR-1-endo is added to the culture medium so as to attain a concentration of about 0.1 µM to about 100 µM, preferably about 0.3 µM to about 30 µM, more preferably about 1 µM to about 10 µM, further preferably about 3 µM. In the case of using a Wnt signaling pathway inhibitor other than IWR-1-endo, it is desirable to be used at a concentration that exhibits Wnt signaling pathway inhibitory activity equivalent to the concentration of IWR-1-endo.

In the step (B), the timing of adding the Wnt signaling pathway inhibitor to the culture medium is preferably earlier. The Wnt signaling pathway inhibitor is added to the culture medium usually within 6 days, preferably within 3 days, more preferably within 1 day, more preferably within 12 hours, from the start of suspension culture in the step (B), further preferably at the start of suspension culture in the step (B). Specifically, for example, the addition of a basal medium supplemented with the Wnt signaling pathway inhibitor, or the exchange of a part or the whole of the culture medium with the basal medium can be performed. Although a period for which the Wnt signaling pathway inhibitor is allowed to act on the cells obtained in the step (A) in the step (B) is not particularly limited, preferably, it is added to the culture medium at the start of suspension culture in the step (B) and then allowed to act until the completion of the step (B) (immediately before addition of a BMP signaling pathway agonist). Further preferably, as mentioned later, exposure to the Wnt signaling pathway inhibitor is continued even after the completion of the step (B) (i.e., during the period of the step (C)). In an embodiment, as mentioned later, the action of the Wnt signaling pathway inhibitor is continued even after the completion of the step (B) (i.e., during the period of the step (C)), and the action may be performed until retinal tissue is formed.

In the step (C), as the Wnt signaling pathway inhibitor, any of the Wnt signaling pathway inhibitors mentioned above can be used. Preferably, the same type as the Wnt signaling pathway inhibitor used in the step (B) is used in the step (C).

The concentration of the Wnt signaling pathway inhibitor in the step (C) can be a concentration capable of inducing retinal progenitor cells and retinal tissue. For example, IWR-1-endo is added to the culture medium so as to attain a concentration of about 0.1 µM to about 100 µM, preferably about 0.3 µM to about 30 µM, more preferably about 1 µM to about 10 µM, further preferably about 3 µM. In the case of using a Wnt signaling pathway inhibitor other than IWR-1-endo, it is desirable to be used at a concentration that exhibits Wnt signaling pathway inhibitory activity equivalent to the concentration of IWR-1-endo. The concentration of the Wnt signaling pathway inhibitor in the culture medium in the step (C) is preferably 50 to 150, more preferably 80 to 120, further preferably 90 to 110, when the concentration of the Wnt signaling pathway inhibitor in the culture medium in the step (B) is defined as 100. It is more preferable to be equivalent to the concentration of the Wnt signaling pathway inhibitor in the culture medium in the step (B).

The timing of addition of the Wnt signaling pathway inhibitor to the culture medium is not particularly limited within a range that can achieve the formation of an aggregate containing retinal cells or retinal tissue, and is preferably earlier. Preferably, the Wnt signaling pathway inhibitor is added to the culture medium at the start of the step (C). More preferably, the Wnt signaling pathway inhibitor is added in the step (B) and then also continuously (i.e., from the start of the step (B)) contained in the culture medium in the step (C). Further preferably, the Wnt signaling pathway inhibitor is added at the start of suspension culture in the step (B) and then also continuously contained in the culture medium in the step (C). For example, a BMP signaling agonist (e.g., BMP4) can be added to the cultures (suspension of aggregates in a culture medium containing a Wnt signaling pathway inhibitor) obtained in the step (B).

A period for which the Wnt signaling pathway inhibitor is allowed to act is not particularly limited, but is preferably from 2 days to 30 days, more preferably from 6 days to 20 days, from 8 days to 18 days, from 10 days to 18 days, or from 10 days to 17 days (e.g., 10 days), with the start of suspension culture in the step (B) as a commencement when the Wnt signaling pathway inhibitor is added at the start of suspension culture in the step (B). In another embodiment, the period for which the Wnt signaling pathway inhibitor is allowed to act is preferably from 3 days to 15 days (e.g., 5 days, 6 days, 7 days), more preferably from 6 days to 10 days (e.g., 6 days), with the start of suspension culture in the step (B) as a commencement when the Wnt signaling pathway inhibitor is added at the start of suspension culture in the step (B).

A neural retina having a ciliarymarginal zone-like structure can also be produced by culturing the cell aggregate obtained by the method mentioned above in a serum-free medium or a serum medium containing a Wnt signaling pathway agonist and/or an FGF signaling pathway inhibitor for a period on the order of 2 days to 4 days (step (D)), followed by culture in a serum-free medium or a serum medium containing neither a Wnt signaling pathway agonist nor an FGF signaling pathway inhibitor for about 30 days to about 200 days (from 30 days to 150 days, from 50 days to 120 days, from 60 days to 90 days) (step (E)).

In an embodiment, a neural retina having a ciliarymarginal zone-like structure can be produced by the step (D) and the step (E) from the cell aggregate obtained in the steps (A) to (C), the cell aggregate being of Days 6 to 30 or Days 10 to 20 (Day 10, Day 11, Day 12, Day 13, Day 14, Day 15, Day 16, Day 17, Day 18, Day 19 or Day 20) after the start of suspension culture in the step (B).

The Wnt signaling pathway agonist is not particularly limited as long as it is capable of enhancing signal transduction mediated by Wnt. Examples of a specific Wnt signaling pathway agonist can include GSK3β inhibitors (e.g., 6-bromoindirubin-3'-oxime (BIO), CHIR99021, kenpaullone). For example, in the case of CHIR99021, the range of about 0.1 µM to about 100 µM, preferably about 1 µM to about 30 µM, can be included.

The FGF signaling pathway inhibitor is not particularly limited as long as it can inhibit signal transduction mediated by FGF. Examples of the FGF signaling pathway inhibitor include SU-5402, AZD4547, and BGJ398. For example, SU-5402 is added at a concentration of about 0.1 µM to about 100 µM, preferably about 1 µM to about 30 µM, more preferably about 5 µM.

The culture medium that is used in the step (D) is, as one example, a culture medium that is not supplemented with one or more (preferably all) selected from the group consisting of a BMP signaling pathway agonist, a Wnt signaling pathway inhibitor, a SHH signaling pathway agonist, a TGFβ family signaling pathway inhibitor and a TGFβ family signaling pathway agonist.

Apart of the step (E) or the whole step can perform culture using a culture medium for continuous epithelial tissue maintenance disclosed in WO2019/017492. Specifically, the continuous epithelium structure of the neural retina can be maintained by culture using a culture medium for continuous epithelial tissue maintenance. One example of the culture medium for continuous epithelial tissue maintenance can include a medium in which Neurobasal medium (e.g., manufactured by Thermo Fisher Scientific Inc., 21103049) is blended with B27 supplement (e.g., Thermo Fisher Scientific Inc., 12587010).

For the culture in the step (E), exchange with the culture medium for continuous epithelial tissue maintenance in stages is preferable for achieving both the differentiation and/or maturation of retinal cells (particularly, photoreceptor cell) and the maintenance of the continuous epithelium structure. For example, culture can be performed using a basal medium for cell proliferation (e.g., a culture medium in which DNMMIF12 medium is supplemented with 10% fetal bovine serum, 1% N2 supplement, and 100 µM taurine) for first 10 days to 30 days, a mixture of a basal medium for cell proliferation and a culture medium for continuous epithelial tissue maintenance (culture medium in which a medium in which DMEM/F12 medium is supplemented with 10% fetal bovine serum, 1% N2 supplement, and 100 µM taurine, and a medium in which Neurobasal medium is supplemented with 10% fetal bovine serum, 2% B27 supplement, 2 mM glutamine, and 100 µM taurine, are mixed at a ratio of 1:3) for next 10 days to 40 days, and a culture medium for continuous epithelial tissue maintenance (e.g., a culture medium in which Neurobasal medium is supplemented with 10% fetal bovine serum, 2% B27 supplement, 2 mM glutamine, and 100 µM taurine) for next 20 days to 140 days.

In a part of the step (E) or the whole step, in the case of using any medium of the basal medium for cell proliferation, the culture medium for continuous epithelial tissue maintenance or a mixture of these media, a thyroid hormone signaling pathway agonist may be further contained. By culture in a culture medium containing a thyroid hormone signaling pathway agonist, the production of a aggregate of the retinal cell becomes possible in which the percentage of bipolar cells, amacrine cells, ganglion cells or horizontal cells, etc. contained in the neural retina is low and the percentage of photoreceptor progenitor cells has been increased.

In the specification, the thyroid hormone signaling pathway agonist is a substance capable of enhancing signal transduction mediated by thyroid hormone, and is not particularly limited as long as it is capable of enhancing the thyroid hormone signaling pathway. Examples of the thyroid hormone signaling pathway agonist include triiodothyronine (hereinafter, also abbreviated to T3), thyroxin (hereinafter, also abbreviated to T4), and thyroid hormone receptor (preferably TRβ receptor) agonists.

Examples of the thyroid hormone receptor agonist known to those skilled in the art can include compounds such as diphenylmethane derivatives, diaryl ether derivatives, pyridazine derivatives, pyridine derivatives and indole derivatives described in International Publication No. WO 97/21993, International Publication No. WO 2004/066929, International Publication No. WO 2004/093799, International Publication No. WO 2000/039077, International Publication No. WO 2001/098256, International Publication No. WO 2003/018515, International Publication No. WO 2003/084915, International Publication No. WO 2002/094319, International Publication No. WO 2003/064369, Japanese Unexamined Patent Publication No. 2002-053564, Japanese Unexamined Patent Publication No. 2002-370978, Japanese Unexamined Patent Publication No. 2000-256190, International Publication No. WO 2007/132475, International Publication No. WO 2007/009913, International Publication No. WO 2003/094845, International Publication No. WO 2002/051805 or International Publication No. WO 2010/122980.

In the case of using T3 as the thyroid hormone signaling pathway agonist, it can be added to the culture medium so as to attain, for example, the range of 0.1 to 1000 nM. Preferably, examples thereof include concentrations having thyroid hormone signaling enhancing activity that corresponds to T3 with a concentration of 1 to 500 nM; more preferably 10 to 100 nM; further preferably 30 to 90 nM; still more preferably around 60 nM. In the case of using T4 as the thyroid hormone signaling pathway agonist, it can be added to the culture medium so as to attain, for example, the range of 1 nM to 500 µM. Preferably, it is the range of 50 nM to 50 µM; more preferably 500 nM to 5 µM. In the case of using other thyroid hormone receptor agonists, the concentration can exhibit activity equivalent to the agonist activity exhibited by T3 or T4 with the concentration mentioned above.

The culture medium that is used in the step (E) may appropriately contain L-glutamine, taurine, serum, or the like. The culture medium that is used in the step (E) is, as one example, a culture medium that is not supplemented with one or more (preferably all) selected from the group consisting of a BMP signaling pathway agonist, an FGF signaling pathway inhibitor, a Wnt signaling pathway agonist, a Wnt signaling pathway inhibitor, a SHH signaling pathway agonist, a TGFβ family signaling pathway inhibitor and a TGFβ family signaling pathway agonist.

In a specific embodiment, the aggregate of the retinal cell can be prepared by a method comprising the following steps (A) to (E):
(A) culturing pluripotent stem cells in a culture medium containing a factor for maintaining undifferentiated state and optionally containing a TGFβ family signaling pathway inhibitor and/or a sonic hedgehog signaling pathway agonist in the absence of feeder cells;
(B) forming a cell aggregate by suspension-culturing the cells obtained in the step (A) in a culture medium optionally containing a Wnt signaling pathway inhibitor and/or a sonic hedgehog signaling pathway agonist;
(C) further suspension-culturing the cell aggregate obtained in the step (B) in a culture medium containing a BMP signaling pathway agonist;
(D) culturing the cell aggregate obtained in the step (C) in a serum-free medium or a serum medium containing a Wnt signaling pathway agonist and/or an FGF signaling pathway inhibitor for a period on the order of 2 days to 4 days; and
(E) culturing the cell aggregate obtained in the step (D) in a serum-free medium or a serum medium containing neither a Wnt signaling pathway agonist nor an FGF signaling pathway inhibitor and optionally containing a thyroid hormone signaling pathway agonist for about 30 days to about 200 days.

In a specific embodiment, the aggregate of the retinal cell can be prepared by a method comprising the following steps (A') to (E'):
(A') culturing pluripotent stem cells in a culture medium containing a factor for maintaining undifferentiated state and containing a TGFβ family signaling pathway inhibitor and/or a sonic hedgehog signaling pathway agonist in the absence of feeder cells for 12 hours to 48 hours;
(B') forming a cell aggregate by suspension-culturing the cells obtained in the step (A') in a culture medium containing a Wnt signaling pathway inhibitor and/or a sonic hedgehog signaling pathway agonist for 12 hours to 72 days (24 hours to 48 hours);
(C') further suspension-culturing the cell aggregate obtained in the step (B') in a culture medium containing a BMP signaling pathway agonist for 8 days to 15 days (10 days to 13 days);
(D') culturing the cell aggregate obtained in the step (C') in a serum-free medium or a serum medium containing a Wnt signaling pathway agonist and/or an FGF signaling pathway inhibitor for 2 days to 4 days; and
(E') culturing the cell aggregate obtained in the step (D') in a serum-free medium or a serum medium containing neither a Wnt signaling pathway agonist nor an FGF signaling pathway inhibitor and optionally containing a thyroid hormone signaling pathway agonist for about 10 days to about 200 days.

In this context, the step (E') may comprise the step of performing culture in a basal medium for cell proliferation for 10 days to 30 days, subsequently performing culture in a mixture of a basal medium for cell proliferation and a culture medium for continuous epithelial tissue maintenance containing a thyroid hormone signaling pathway agonist for 10 days to 40 days, and further performing culture in a culture medium for continuous epithelial tissue maintenance containing a thyroid hormone signaling pathway agonist for 20 days to 140 days.

In an embodiment, the step (E') comprises performing culture for 20 days to 60 days (30 days to 50 days) in the presence of a thyroid hormone signaling pathway agonist.

In an embodiment, the culture period from the step (B') to the step (E') is 70 days to 100 days (80 days to 90 days).

The timing of recovery of retinal tissue containing retinal cells as starting cells is not particularly limited as long as it is timing involving one or more cells selected from the group consisting of a retinal progenitor cell, a neural retinal progenitor cell and a photoreceptor progenitor cell in the retinal tissue. Timing abundantly involving a retinal progenitor cell and/or a neural retinal progenitor cell in the retinal tissue is preferable. In the method mentioned above, the dispersed retinal cell population as starting cells can be prepared as mentioned later from, for example, the retinal tissue 10 days to 100 days after (preferably 9 days to 45 days after) the start of suspension culture.

### (Method for differentiating neural cell population as starting cell)

Pluripotent stem cell-derived neural cells as starting cells in the manufacturing method of the present invention can be induced by differentiating pluripotent stem cells. Many methods have been reported as methods for inducing neural cells or neural tissue from an aggregate of pluripotent stem cells by suspension culture. For example, methods described in WO2005/123902, WO2009/148170, WO2008/035110, WO2011/055855, WO2016/063985, Cell Stem Cell, 3, 519-32 (2008), Nature, 472, 51-56 (2011), Cell Stem Cell, 10 (6), 771-775 (2012), Nature Biotechnology, 27 (3), 275-80 (2009), or Proc Natl Acad Sci USA, 110 (50), 20284-9 (2013) are known, though not limited thereto. An aggregate containing neural cells or neural tissue can be manufactured by applying such various methods for inducing neural cells or neural tissue to the aggregate obtained in the step (B), and culturing the aggregate obtained in the step (B) under appropriate neural differentiation conditions.

One example of a method for manufacturing neuronal cells (e.g., cerebral cells (cerebral tissue)) as starting cells in the manufacturing method of the present invention will be given below.

A method for manufacturing cerebral tissue, comprising the following steps (A") to (C"):
(A") culturing pluripotent stem cells in a culture medium containing 1) a TGFβ family signaling pathway inhibitor and/or a sonic hedgehog signaling pathway agonist and 2) a factor for maintaining undifferentiated state in the absence of feeder cells;
(B") forming an aggregate of cells by suspension-culturing the cells obtained in the step (A"); and
(C") suspension-culturing the aggregate obtained in the step (B") in the absence of a differentiating agent (e.g., BMP signaling pathway agonist) or in the presence of a differentiating agent (e.g., TGF(3 family signaling pathway inhibitor and/or Wnt signaling pathway inhibitor) to obtain an aggregate containing neural tissue (cerebral tissue).

The steps (A") and (B") can be carried out in the same manner as in the steps (A) and (B) in the method for manufacturing retinal tissue mentioned above.

In the step (C"), culture may be performed in a culture medium that is not supplemented with a differentiating agent, for example, a substance that activates or inhibits specific signal transduction, such as a BMP signaling pathway agonist. The cell aggregate is committed to differentiate into nerve by the steps (A") and (B") and therefore spontaneously differentiates into nerve (e.g., the cerebrum) by performing culture in a basal medium.

In the step (C"), a TGFβ family signaling pathway inhibitor or a Wnt signaling pathway inhibitor may be added alone as a differentiating agent to the culture medium. It is more preferable to combine both. These differentiation factors may be added from the step (B").

Examples of the TGFβ family signaling pathway inhibitor that is used in the steps (B") and (C") can include TGFβ signaling pathway inhibitors, nodal/activin signaling pathway inhibitors and BMP signaling pathway inhibitors. These factors are the same as the factors mentioned above. The TGFβ family signaling pathway inhibitor is preferably SB431542, A-83-01 or LDN193189.

The Wnt signaling pathway inhibitor that is used in the steps (B") and (C") is not particularly limited as long as it is capable of suppressing signal transduction mediated by Wnt, and may be any of a protein, a nucleic acid, a low-molecular compound, and the like. Signals mediated by Wnt are transduced via Wnt receptor present as a heterodimer of frizzled (Fz) and LRP5/6 (low-density lipoprotein receptor-related protein 5/6). Examples of the Wnt signaling pathway inhibitor include, but are not limited to, substances acting directly on Wnt or Wnt receptor (anti-Wnt antibody, anti-Wnt receptor antibody, etc.), substances suppressing the expression of a gene encoding Wnt or Wnt receptor (e.g., antisense oligonucleotide, siRNA), substances inhibiting the binding of Wnt to Wnt receptor (soluble Wnt receptor, dominant negative Wnt receptor, etc., Wnt antagonist, Dkk1, Cerberus protein, etc.), and substances inhibiting bioactivity caused by signal transduction ascribable to Wnt receptor [e.g., low-molecular compounds such as CKI-7 (N-(2-aminoethyl)-5-chloroisoquinoline-8-sulfonamide), D4476 (4-[4-(2,3-dihydro-1,4-benzodioxin-6-yl)-5-(2-pyridinyl)-1H-imidazol-2-yl]benzamide), IWR-1-endo (IWR1e) (4-[(3aR,4S,7R,7aS)-1,3,3a,4,7,7a-hexahydro-1,3-dioxo-4,7-methano-2H-isoindol-2-yl]-N-8-quinolinyl-benzamide), and IWP-2 (N-(6-methyl-2-benzothiazolyl)-2-[(3,4,6,7-tetrahydro-4-oxo-3-phenylthieno[3,2-d]pyrimidin-2-yl)thio]acetamide)]. CKI-7, D4476, IWR-1-endo (IWR1e), IWP-2, and the like are known Wnt signaling pathway inhibitors, and commercially available products, etc. can be appropriately obtained. Examples of the Wnt signaling pathway inhibitor preferably include IWR1e.

The concentration of the Wnt signaling pathway inhibitor can be a concentration capable of inducing the differentiation of cells that form an aggregate of pluripotent stem cells into cerebral cells. For example, IWR-1-endo is added to the culture medium so as to attain a concentration of about 0.1 µM to about 100 µM, preferably about 0.3 µM to about 30 µM, more preferably about 1 µM to about 10 µM, further preferably about 3 µM. In the case of using a Wnt signaling pathway inhibitor other than IWR-1-endo, it is desirable to be used at a concentration that exhibits Wnt signaling pathway inhibitory activity equivalent to the concentration of IWR1e.

By this culture, the differentiation of the cells that form the aggregate obtained in the step (B") into cortical neural precursor cells is induced, and an aggregate containing cortical neural precursor cells can be obtained. It can be confirmed that the aggregate containing cortical neural precursor cells has been obtained, for example, by detecting cells expressing a cortical neural precursor cell marker FoxG1, Lhx2, PAX6, Emx2, or the like contained in the aggregate. Examples of an embodiment of the step (C") can include the step of suspension-culturing the aggregate formed in the step (B") in a serum-free medium or a serum medium until cells expressing FoxG1 gene start to appear. In an embodiment, the culture of the step (C") is carried out up to a state in which 20% or more (preferably 30% or more, 40% or more, 50% or more, 60% or more) of the cells contained in the aggregate express FoxG1.

The timing of recovery of neural tissue containing neural cells as starting cells is not particularly limited as long as it is timing involving a neural precursor cell or a cortical neural precursor cell in the neural tissue (cerebral tissue). Timing abundantly involving these cells in the tissue is preferable. In the method mentioned above, the dispersed neural cell population as starting cells can be prepared as mentioned later from, for example, the neural tissue (cerebral tissue) 10 days to 100 days after (preferably 15 days to 40 days after) the start of suspension culture.

### (Dispersed retinal or neural cell population)

The "dispersion" refers to separating cells or tissues into a small cell clot or cell clump (2 cells or more and 100 cells or less, preferably 50 cells or less, 30 cells or less, 20 cells or less, 10 cells or less, 5 cells or less; for example, a clump of 2 to 5 cells) or single cells by dispersion treatment such as enzymatic treatment or physical treatment. The dispersed cell population refers to a cell clot or a cell clump, a collection of a given number of single cells. The "dispersed retinal cell population" refers to a cell population in a dispersed state and can be obtained by dispersing living tissue or a cell clump such as a cell aggregate. It is preferable that the dispersed retinal cell population should be obtained by dispersing the aggregate of retinal cells described above.

In an embodiment, a dispersion method can be a method that can disperse cells alive. Examples thereof include mechanical dispersion treatment, cell dispersion solution treatment, and cytoprotective agent addition treatment. These treatments may be performed in combination. Preferably, cell dispersion solution treatment is performed, and subsequently, mechanical dispersion treatment can be performed.

Examples of a method of the mechanical dispersion treatment include pipetting treatment and scraping operation with a scraper.

Examples of the cell dispersion solution that is used in the cell dispersion solution treatment can include solutions containing any of enzymes such as trypsin, collagenase, hyaluronidase, elastase, pronase, DNase, and papain, and chelating agents such as ethylenediaminetetraacetic acid. A commercially available cell dispersion solution, for example, TrypLE Select (manufactured by Life Technologies Corp.), TrypLE Express (manufactured by Life Technologies Corp.), or a neuronal cell dispersion solution (FUJIFILM Holdings Corp.) can also be used.

When cells are dispersed, cell death of the cells may be suppressed by treating with a cytoprotective agent. Examples of the cytoprotective agent that is used in the cytoprotective agent treatment can include FGF signaling pathway agonists, heparin, IGF signaling pathway agonists, serum, and serum replacements. In order to suppress cell death induced by dispersion (particularly, cell death of human pluripotent stem cells), an inhibitor of Rho-associated coiled-coil containing protein kinase ("ROCK" or "Rho kinase") (ROCK inhibitor) or an inhibitor of myosin may be added during dispersion. Examples of the ROCK inhibitor can include Y-27632, fasudil (HA1077), and H-1152. Examples of the inhibitor of myosin can include blebbistatin. Preferable examples of the cytoprotective agent include ROCK inhibitors.

The dispersed retinal cell population (or neural cell population) includes a state in which single cells are included, for example, 70% or more, preferably 80% or more, with respect to the total number of cells of the cell population is single cells, and a mass of 2 to 50 cells is present at 30% or less, preferably 20% or less, with respect to the total number of cells of the cell population. In the dispersed retinal cell population (or neural cell population), the mutual adhesion (e.g., surface adhesion) of cells has been mostly lost. It is more preferable that the dispersed retinal cell population (or neural cell population) should consist of single cells as much as possible. Such a dispersed retinal cell population (or neural cell population) can be obtained by removing a mass of cells that are not single cells after dispersion treatment. Examples of the method for removing a mass of cells include, but are not particularly limited to, the removal of the mass through a membrane filter (cell strainer). In an embodiment, the dispersed cells include a state cell-cell junction (e.g., adherence junction) has been mostly lost.

In the case of preparing a retinal cell population as starting cells from retinal tissue, unnecessary cells such as retinal pigment epithelial cells may be contained in the retinal tissue. Examples thereof include the case where the retinal tissue has been collected from a living body, and the case where the step (D) and the step (E) in the aforementioned method for manufacturing retinal tissue from pluripotent stem cells have been carried out. In this case, the retinal cell population can be dispersed after a region where unnecessary cells such as retinal pigment epithelial cells are present are isolated. Retinal pigment epithelial cells are identifiable from morphology or a pigment and can be readily resected by those skilled in the art.

Before culture in a culture medium containing a Wnt signaling pathway agonist mentioned later is started, the step of increasing the purity of a retinal progenitor cell or a neural retinal progenitor cell (purification step) as mentioned later may be carried out, and the maintenance and/or expansion culture of the dispersed retinal cell population (or neural cell population) may be further carried out. The culture medium is not particularly limited as long as it is a culture medium (DMEM medium, etc.) in which retinal cells (or neural cells) are capable of surviving and proliferating. Since a frozen and thawed retinal cell population can also be used as starting cells in the manufacturing method of the present invention, the dispersed retinal cell population may be cryopreserved. A cryopreservation solution is not particularly limited, and a commercially available cryopreservation solution can be used.

### [Purification of retinal progenitor cell]

Before the culture of the dispersed retinal cell population mentioned above in a culture medium containing a Wnt signaling pathway agonist is started, the step of increasing the percentage (purity) of a retinal progenitor cell, preferably the percentage (purity) of a neural retinal progenitor cell (purification step) may be carried out for the dispersed retinal cell population. In the purification step, an operation such as cell sorting using a specific marker expressed in the retinal progenitor cell and/or the neural retinal progenitor cell may be carried out. The cell sorting can be carried out using a well-known technique such as FACS or MACS. By carrying out the purification step for a retinal progenitor cell and/or a neural retinal progenitor cell, it is possible to reduce contamination with RPE cells or non-target cells in the manufacturing method described in the present specification. By the step of increasing the percentage of a retinal progenitor cell contained in the dispersed retinal cell population, the production of retinal pigment epithelial progenitor cells and/or retinal pigment epithelial cells is suppressed. Whether or not the production of retinal pigment epithelial progenitor cells and/or retinal pigment epithelial cells has been suppressed can be determined from whether or not the retinal pigment epithelial cells have been produced on the basis of a marker, the morphology, the properties, etc. of the retinal pigment epithelial cells mentioned above after the dispersed retinal cell population is cultured by a culture method mentioned later. "The production of retinal pigment epithelial progenitor cells and/or retinal pigment epithelial cells has been suppressed" can mean that the percentage of the retinal pigment epithelial cells to the total number of cells after the culture is suppressed as compared with the case where the step of increasing the percentage of a retinal progenitor cell has not been carried out. Provided that the percentage of the retinal pigment epithelial cells is a level described in the paragraph 0175, it can be determined that the production of retinal pigment epithelial progenitor cells and/or retinal pigment epithelial cells has been suppressed.

Rx, Chx10, and the like are well known as cell markers for retinal progenitor cells (positive markers). However, since these genes are intracellularly expressed, for example, a device such as use of cells in which such a gene is linked to a fluorescent protein by a gene recombination technique, or cells in which the gene is replaced with a fluorescent protein is necessary (e.g., Rx::Venus cells). Accordingly, positive markers for retinal progenitor cells and/or neural retinal progenitor cells have been searched for, and it has been found that CD9 (GenBank ID: NM_001769.4, NM_001330312.2), CD24 (GenBank ID: NM_001291737.1, NM_001291738.1, NM_001291739.1, NM_001359084.1, NM_013230.3), CD29 (GenBank ID: NM_002211.4, NM_033668.2, NM_133376.2), CD39 (GenBank ID: NM_001098175.2, NM_001164178.1, NM_001164179.2, NM_001164181.1, NM_001164182.2, NM_001164183.2, NM_001312654.1, NM_001320916.1, NM_001776.6), CD47 (GenBank ID: NM_001777.3, NM_198793.2), CD49b (GenBank ID: NM_002203.4), CD49c (GenBank ID: NM_002204.4), CD49f (GenBank ID: NM_000210.4, NM_001079818.3, NM_001316306.2, NM_001365529.2, NM_001365530.2), CD57 (GenBank ID: NM_001367973.1, NM_018644.3, NM_054025.3), CD73 (GenBank ID: NM_001204813.1, NM_002526.4), CD82 (GenBank ID: NM_001024844.2, NM_002231.4), CD90 (GenBank ID: NM_001311160.2, NM_001311162.2, NM_001372050.1, NM_006288.5), CD164 (GenBank ID: NM_001142401.2, NM_001142402.2, NM_001142403.3, NM_001142404.2, NM_001346500.2, NM_006016.6), CD200 (GenBank ID: NM_001004196.3, NM_001318826.1, NM_001318828.1, NM_001318830.1, NM_001365851.2, NM_001365852.1, NM_001365853.1, NM 001365854.1, NM_001365855.1, NM_005944.7), CD340 (GenBank ID: NM_001005862.2, NM_001289936.1, NM 001289937.1, NM_001289938.1, NM_004448.3) and CXCR4 (GenBank ID: NM_001008540.2, NM_001348056.2, NM_001348059.2, NM_001348060.2, NM_003467.3) are expressed on the cell surface of these cells. Preferable examples of the cell surface marker include CD9, CD39, CD90 and CXCR4. In an embodiment, a method for increasing the percentage of a retinal progenitor cell in a cell population, comprising the step of contacting the cell population containing the retinal progenitor cell with substance(s) (e.g., antibody or peptide) that binds to one or more antigens selected from the group consisting of CD9, CD39, CD90 and CXCR4 to separate positive fractions may be carried out before the aforementioned culture in a culture medium containing a Wnt signaling pathway agonist is started. By the method, it is possible to reduce contamination with RPE cells in the manufacturing method described in the present specification.

Further, SSEA1 (GenBank ID: NM_002033.3), CD66b (GenBank ID: NM_001816.4), CD69 (GenBank ID: NM_001781.2) and CD84 (GenBank ID: NM_001184879.2, NM_001184881.2, NM_001184882.1, NM_001330742.2, NM_003874.4), etc. have been found as negative markers for these cells, i.e., markers whose expression is not found in these cells. By combining them with the positive markers mentioned above, it is possible to more increase the purity of a retinal progenitor cell and/or a neural retinal progenitor cell. In an embodiment, a method for increasing the percentage of a retinal progenitor cell in a cell population, comprising the step of contacting the cell population containing the retinal progenitor cell with substance(s) (e.g., antibody) that binds to one or more antigens selected from the group consisting of CD9, CD39, CD90 and CXCR4, preferably, further with substance(s) (e.g., antibody) that binds to one or more antigens selected from the group consisting of SSEA1, CD66b, CD69 and CD84, to separate positive fractions of the former markers and negative fractions of the latter markers may be carried out before the culture in a culture medium containing a Wnt signaling pathway agonist is started. The step of contacting the dispersed retinal cell population with substance(s) (e.g., antibody) that binds to one or more antigens selected from the group consisting of SSEA1, CD66b, CD69 and CD84 to separate negative fractions of these markers is the step of obtaining a cell population having expression levels of the antigens that are equal to or less than a reference. In this context, the reference can be arbitrarily set by those skilled in the art. For example, negative fractions can be separated by obtaining a cell population treated with fluorescently labeled antibodies against the target antigens having intensity equivalent to fluorescence intensity obtained when the cell population is treated with a fluorescently labeled isotype control antibody.

The method for increasing the percentage of a retinal progenitor cell and/or a neural retinal progenitor cell in a cell population using substances (e.g., antibodies) that bind to the positive markers and the negative markers mentioned above is not limited to a method as one step of the manufacturing method described in the present specification. The method for increasing the percentage of a retinal progenitor cell and/or a neural retinal progenitor cell in a cell population using the cell surface markers can also be used as, for example, one step of other methods for manufacturing retinal tissue.

In an embodiment, in the dispersed retinal cell population, the retinal progenitor cell and/or the neural retinal progenitor cell can occupy, for example, 30% or more of the total number of cells. It is preferable to occupy 50% or more of the total number of cells, it is more preferable to occupy 80% or more of the total number of cells, and it is further preferable to occupy 90% or more of the total number of cells. The dispersed retinal cell population comprises, for example, 50% or more of a cell (retinal progenitor cell or neural retinal progenitor cell) that is positive to at least one marker selected from the group consisting of CD9, CD39, CD90 and CXCR4 and is Rx- and/or Chx10-positive with respect to the total number of cells in the cell population, and preferably comprises 80% or more, 85% or more, 90% or more, or 95% or more thereof. It is preferable that the cell population should be negative to one or more, preferably two or more, three or more or all antigens selected from the group consisting of SSEA1, CD66b, CD69 and CD84. In this context, the negativity can be equal to or less than the reference mentioned above.

In an embodiment, the step of increasing the percentage of a retinal progenitor cell (purification step) comprises the following steps:
(1) culturing pluripotent stem cells in the presence of one or more selected from the group consisting of a Shh signaling pathway agonist, ATP and an A2A receptor agonist to manufacture a cell aggregate;
(2) differentiating the cell aggregate into a retinal progenitor cell; and
(3) dispersing the cell aggregate and contacting it with a substance (e.g., antibody) that binds to CD39.

The details of the steps (1) and (2) are as described in "Method for differentiating retinal cell population as starting cell".

### (Reforming of layer structure by culture of dispersed retinal cell population or neural cell population)

The method for manufacturing retinal tissue according to the present invention comprises adhesion-culturing or suspension-culturing a dispersed retinal cell population in a culture medium containing a Wnt signaling pathway agonist. By any of the culture methods, it is possible to reform an epithelial structure (multilayered structure) from the dispersed retinal cell population. Adhesion culture is preferable for manufacturing a sheet-shaped retinal tissue having an epithelial structure (or a multilayered structure). The manufacturing method according to the present invention can also be similarly carried out with a dispersed neural cell population as starting cells. Hereinafter, the case of using a dispersed retinal cell population as starting cells will be described. However, the same holds true for the case of using a dispersed neural cell population as starting cells, unless otherwise specified.

The culture medium that is used in the culture of the dispersed retinal cell population is not particularly limited as long as it is a culture medium in which retinal cells are capable of surviving and proliferating. In an embodiment, examples of the culture medium that is used in the culture of the dispersed retinal cell population include culture media for continuous epithelial tissue maintenance. One example of the culture medium for continuous epithelial tissue maintenance can include a medium in which Neurobasal medium (e.g., manufactured by Thermo Fisher Scientific Inc., 21103049) is blended with B27 supplement (e.g., Thermo Fisher Scientific Inc., 12587010).

The Wnt signaling pathway agonist contained in a culture medium is not particularly limited as long as it is capable of enhancing signal transduction mediated by Wnt. Examples of a specific Wnt signaling pathway agonist can include GSK3β inhibitors (e.g., 6-bromoindirubin-3'-oxime (BIO), CHIR99021, kenpaullone), Wnt proteins Wnt2b and Wnt3a, and partial peptides thereof. One substance may be used, or two or more substances may be used. The Wnt signaling pathway agonist is preferably one or more substances, two or more substances, or three or more substances selected from the group consisting of CHIR99021, BIO, Wnt2b and Wnt3a. The Wnt signaling pathway agonist can reform an epithelial structure (or a multilayered structure) having a polarity of apical surface/basal membrane while increasing the size of an aggregate in the process of reaggregating the dispersed retinal cell population. By the Wnt signaling pathway agonist, rosette-like structure formation can also be suppressed.

The concentration of the Wnt signaling pathway agonist can be a concentration capable of inducing the formation of the desired cell aggregate (e.g., the reforming of an epithelial structure (or a multilayered structure)). For example, in the case of using CHIR99021, the concentration of the Wnt signaling pathway agonist can be 0.01 µM to 100 µM and is preferably 0.1 µM to 10 µM, more preferably 1 µM to 10 µM. In the case of using other Wnt signaling pathway agonists, the concentration can exhibit a Wnt signal-activating effect equivalent to that of CHIR99021 with the concentration mentioned above. The Wnt signal-activating effect is measurable by those skilled in the art through a method, for example, the confirmation of expression of β-catenin.

The timing of adding the Wnt signaling pathway agonist is not particularly limited. It is preferable to add it at as early timing as possible after the start of suspension culture or adhesion culture for preparation into a sheet again. In an embodiment, it is preferable to perform culture in the culture medium containing the Wnt signaling pathway agonist from the start of the culture. The number of culture days in the culture medium containing the Wnt signaling pathway agonist is not particularly limited within a range in which an effect of reforming an epithelial structure (or a multilayered structure) having a polarity of apical surface/basal membrane is found, and is, for example, 1 day to 14 days.

In an embodiment, the culture medium may further contain one or more substances selected from the group consisting of a ROCK inhibitor, a SHH (sonic hedgehog) signaling pathway agonist and a fibroblast growth factors (FGF) signaling pathway agonist. For example, by the addition of the ROCK inhibitor, an effect of promoting the reaggregation of a dispersed retinal progenitor cell population is found. By the addition of the SHH signaling pathway agonist, an effect of promoting the proliferation of a cell aggregate and increasing the size of the cell aggregate is found. By the addition of the FGF signaling pathway agonist (e.g., FGF2, FGF8), an effect of reducing damage on cells and suppressing differentiation into RPE cells is found.

The ROCK inhibitor is not particularly limited as long as a function of Rho kinase (ROCK) can be suppressed. Examples thereof include Y-27632 (see e.g., Ishizaki et al., Mol. Pharmacol. 57, 976-983 (2000); Narumiya et al., Methods Enzymol. 325, 273-284 (2000)), fasudil/HA1077 (see e.g., Uenata et al., Nature 389: 990-994 (1997)), H-1152 (see e.g., Sasaki et al., Pharmacol. Ther. 93: 225-232 (2002)), Wf-536 (see e.g., Nakajima et al., Cancer Chemother Pharmacol. 52 (4): 319-324 (2003)) and their derivatives, antisense nucleic acids against ROCK, RNA interference-inducing nucleic acids (e.g., siRNA), dominant negative mutants, and their expression vectors. Since other low-molecular compounds are also known as ROCK inhibitors, such compounds or their derivatives can also be used in the present invention (see e.g., U.S. Patent Application Publication Nos. 20050209261, 20050192304, 20040014755, 20040002508, 20040002507, 20030125344, and 20030087919, and International Publication Nos. WO 2003/062227, WO 2003/059913, WO 2003/062225, WO 2002/076976, and WO 2004/039796). The ROCK inhibitor can employ one or two or more ROCK inhibitors. The ROCK inhibitor preferably includes one or more substances selected from the group consisting of Y-27632, fasudil (HA1077), and H-1152.

Those skilled in the art are capable of appropriately setting the concentration of the ROCK inhibitor according to experimental conditions. In an embodiment, the concentration can be capable of promoting the reaggregation of a dispersed retinal progenitor cell population. For example, in the case of using Y-27632, it may be 0.1 µM to 1 mM and is preferably 1 µM to 100 µM, more preferably 5 µM to 20 µM. In the case of using a ROCK inhibitor other than Y-27632, the concentration can exhibit a ROCK inhibitory effect equivalent to that of Y-27632 with the concentration mentioned above. The ROCK inhibitory effect is measurable by those skilled in the art through a method, for example, expression analysis on the phosphorylation of MLC2.

The SHH (sonic hedgehog) signaling pathway agonist is a substance capable of enhancing signal transduction mediated by SHH (also referred to as Shh). Examples of the SHH signaling pathway agonist include proteins belonging to the hedgehog family (e.g., Shh and Ihh), SHH receptor, Shh receptor agonists, PMA (purmorphamine; 9-cyclohexyl-N-[4-(4-morpholinyl)phenyl]-2-(1-naphthalenyloxy)-9H-purin-6-amine) and SAG (smoothened agonist; N-methyl-N'-(3-pyridinylbenzyl)-N'-(3-chlorobenzo[b]thiophene-2-carbonyl)-1,4-diaminocyclohexane). One or two or more of these may be contained as the Shh signaling pathway agonist. The Shh signaling pathway agonist is preferably one or more substances selected from the group consisting of Shh (GenBank accession No: NM_000193, NP_000184), SAG and PMA.

Those skilled in the art are capable of appropriately setting the concentration of the SHH signaling pathway agonist according to experimental conditions. In an embodiment, the concentration can be within a range in which an effect of increasing the size of a cell aggregate is found. For example, SAG is used at a concentration of usually 1 to 2000 nM, preferably 10 to 700 nM. PMA is used at a concentration of usually 0.002 to 20 µM, preferably 0.02 to 2 µM. SHH is used at a concentration of usually 4 to 500 ng/mL, preferably 10 to 200 ng/mL. In the case of using other SHH signaling pathway agonists, the concentration can exhibit a SHH signal-activating effect equivalent to that of SAG with the concentration mentioned above. The SHH signal-activating effect is measurable by those skilled in the art through a method, for example, the expression analysis of a downstream signal (SMO, GLI).

The FGF signaling pathway agonist is not particularly limited as long as it is a substance capable of enhancing signal transduction mediated by FGF. Examples of the FGF signaling pathway agonist specifically include fibroblast growth factors (e.g., bFGF, FGF4, FGF8 and FGF9). The FGF signaling pathway agonist is preferably one or more fibroblast growth factors selected from the group consisting of FGF2, FGF4 and FGF8.

Those skilled in the art are capable of appropriately setting the concentration of the FGF signaling pathway agonist according to experimental conditions. In an embodiment, the concentration can be within a range in which an effect of suppressing differentiation into RPE cells is found. For example, in the case of using FGF2, FGF4 or FGF8, it is on the order of 4 to 500 ng/mL, preferably on the order of 10 to 200 ng/mL, more preferably on the order of 25 to 100 ng/mL. In the case of using other FGF signaling pathway agonists, the concentration can exhibit an FGF signal-activating effect equivalent to that of FGF8 or the like with the concentration mentioned above. The FGF signal-activating effect is measurable by those skilled in the art through a method, for example, the expression analysis of a downstream signal (Akt, MEK).

The timing of adding the ROCK inhibitor, the SHH (sonic hedgehog) signaling pathway agonist and/or the FGF signaling pathway agonist is not particularly limited. It is preferable to perform culture in the culture medium containing the ROCK inhibitor, the SHH (sonic hedgehog) signaling pathway agonist and/or the FGF signaling pathway agonist from the start of the culture. It is preferable that these substances should be simultaneously added to the culture medium, and further, it is preferable to add them to the culture medium simultaneously with the Wnt signaling pathway agonist. In an embodiment, the dispersed retinal cells can be cultured in the culture medium containing these substances for 1 day to 14 days.

The suspension culture refers to culturing cells in a state nonadhesive to a culture container and is not particularly limited. It can be performed using a culture container that has not undergone artificial treatment (e.g., coating treatment with an extracellular matrix or the like) for the purpose of improving adhesiveness to cells, or a culture container treated by coating using a treatment that artificially suppresses adhesion (e.g., polyhydroxyethyl methacrylate (poly-HEMA), nonionic surface activating polyol (Pluronic F-127, etc.) or a phospholipid-analogous substance (e.g., water-soluble polymer (Lipidure) having 2-methacryloyloxyethyl phosphorylcholine as a constituent unit).

The suspension culture can be performed with dispersed retinal cells as starting cells, for example, by using SFEB (serum-free floating culture of embryoid bodies-like aggregates) (WO2005/12390) or SFEBq (WO2009/148170).

The adhesion culture refers to culturing cells in a state of adhesion to a culture container and is not particularly limited. It can be performed using a culture container or the like artificially treated for the purpose of improving adhesiveness to cells. It is preferable that the adhesion culture should be performed using a culture container or the like coated with an extracellular matrix and/or a temperature-responsive polymer. The method for manufacturing retinal tissue of an embodiment comprises the step of exposing a culture container coated with a temperature-responsive polymer to a temperature that changes the properties of the temperature-responsive polymer to thereby detach a sheet-shaped retinal tissue from the culture container.

A sheet-shaped retinal tissue mentioned later can be manufactured by performing adhesion culture on a culture container coated with an extracellular matrix.

By performing culture in the presence of an extracellular matrix, an apical surface is easily formed because cells can recognize the basal membrane side. Besides, cells face a direction roughly perpendicular to the layer direction, and retinal tissue having a more favorable layer structure is obtained. By using a culture container or the like coated with a temperature-responsive polymer, retinal tissue having the formed epithelial structure (or multilayered structure) can be easily detached from the culture container or the like through mere change in temperature, and enzymatic treatment is unnecessary. Therefore, a tough sheet-shaped retinal tissue can be recovered without weakening intercellular junction due to enzymatic treatment. Hence, it is preferable to use a culture container or the like coated with an extracellular matrix and/or a temperature-responsive polymer, particularly, both an extracellular matrix and a temperature-responsive polymer. Examples thereof include the case where a culture surface of the culture container is coated with the temperature-responsive polymer, and an upper surface of the polymer is coated with the extracellular matrix. In this context, the culture surface refers to a surface to which cells adhere in the culture container, and the upper surface of the polymer refers to a surface opposite to a surface in contact with the polymer-coated culture surface.

The extracellular matrix refers to a biopolymer constituting the external space of a cell. Examples thereof include cell-adhesive proteins such as fibronectin, vitronectin, and laminin, fibrous proteins such as collagen and elastin, fragments of these proteins, hyaluronic acid, glycosaminoglycan or proteoglycan such as chondroitin sulfate, and Matrigel. The extracellular matrix is preferably one or more substances selected from the group consisting of collagen, laminin, fibronectin, Matrigel, vitronectin and fragments of these proteins. Examples of the laminin fragment include commercially available products such as iMatrix-511, iMatrix-411, and iMatrix-221.

Matrigel is a basal membrane preparation derived from Engelbreth Holm Swarn (EHS) mouse sarcoma. Matrigel can be prepared by, for example, a method disclosed in US Patent No. 4829000, and a commercially available product can also be purchased. Main components of Matrigel are laminin, IV-type collagen, heparan sulfate proteoglycan and entactin.

The temperature-responsive polymer is a polymer in which the properties of the polymers vary depending on change in temperature. Specifically, it has a lower critical solution temperature (LCST) in water and exhibits phase transition behavior in which, with a certain temperature as a threshold, an intramolecular or intermolecular hydrophobic bond thereof is strengthened at a temperature higher than it so that the polymer chain aggregates, whereas the polymer chain is hydrated by binding to a water molecule at a lower temperature. Specifically, examples thereof include temperature-responsive polymers that maintain the hydrophobic state of equipment surface at a culture temperature (about 37°C) while the equipment surface shifts to hydrophilicity at a temperature lower than the culture temperature, for example, on the order of 20 to 30°C, so that cultured cells become easy to detach. Thus, enzymatic treatment is not necessary for cell detachment, and it is also possible to recover one large sheet without disrupting proteins present between cells due to enzymatic treatment. For example, poly-N-isopropylacrylamide (PIPAAm) (LCST is 32°C) is preferably used as such a temperature-responsive polymer. Usually, cell culture is performed under a condition of about 37°C, and a temperature-responsive polymer whose LCST is in the range of about 20°C to 35°C is preferable with consideration also given to the damage of a low temperature on cells. Temperature-responsive cell culture equipment for cell sheet recovery (CellSeed Inc.: UpCell(R)) or the like in which the temperature-responsive polymer is fixed to the surface is also obtainable as a commercially available product.

As for culture conditions, the culture temperature is not particularly limited and is about 30 to 40°C, preferably about 37°C. Culture is performed in an atmosphere of CO₂-containing air, and the CO₂ concentration is preferably about 2 to 5%.

A Wnt signaling pathway agonist is necessary for the formation of a layer structure and, particularly, apical surface/basal surface (apical/basal polarity), in a sheet-shaped retinal tissue.

The concentration of the Wnt signaling pathway agonist can be a concentration capable of inducing a sheet-shaped retinal tissue having a layer structure and an apical surface. For example, in the case of using CHIR99021, the concentration of the Wnt signaling pathway agonist can be 0.01 µM to 100 µM and is preferably 0.1 µM to 10 µM, more preferably 1 µM to 10 µM. In the case of using other Wnt signaling pathway agonists, the concentration can exhibit a Wnt signal-activating effect equivalent to that of CHIR99021 with the concentration mentioned above. Whether or not the layer structure has been formed can be readily determined by those skilled in the art, for example, by observation under a microscope or measuring a thickness using an apparatus such as OCT. Whether the apical surface has been formed can be confirmed, for example, by staining using an anti-Zo-1 antibody, an anti-ezrin antibody, or an anti-atypical-PKC antibody.

The timing of adding the Wnt signaling pathway agonist is not particularly limited. It is preferable to add it at as early timing as possible after the start of adhesion culture for preparation into a sheet again. In an embodiment, it is preferable to perform culture in the culture medium containing the Wnt signaling pathway agonist from the start of the culture. The number of culture days in the culture medium containing the Wnt signaling pathway agonist is not particularly limited within a range in which an effect of reforming an epithelial structure (or a multilayered structure) having a polarity of apical surface/basal membrane is found, and is, for example, 1 day to 15 days, preferably 1 day to 9 days.

Culture may be continued in a culture medium from which the Wnt signaling pathway agonist or the like has been removed. By continuing culture, the layer structure is thickened, and the differentiation of retinal cells proceeds. The culture period is not particularly limited and can be a period for which a seeded dispersed retinal cell population proliferates and at least an epithelial structure (or a multilayered structure) is formed. Culture can be performed until a sheet-shaped retinal tissue at a targeted stage of differentiation is manufactured. It is desirable that culture should be performed for at least 7 days from the viewpoint of the formation of an epithelial structure (or a multilayered structure). The culture period can be, for example, 7 days to 60 days or less and may be 40 days or less, 30 days or less, 20 days or less, or 16 days or less (e.g., 16 days).

Even after an epithelial structure (or a multilayered structure) is formed, culture may be further continued in order to allow cells to proliferate, differentiate or mature. The culture medium that is used in the further culture may or may not contain a Wnt signaling pathway agonist, a ROCK inhibitor, a SHH (sonic hedgehog) signaling pathway agonist and/or an integrin signaling pathway agonist.

The "multilayered structure" is a structure where two or more cell layers in which cells are arranged in the same direction are stacked in a tissue having polarities on the basal membrane side and the apical side (i.e., having an epithelial structure), and is a structure where the tangent directions of the respective surfaces of different layers are roughly in parallel. It is preferable that the multilayered structure should have polarities of a basal surface and an apical surface. Retinal tissue having a basal membrane and in an embodiment, the multilayered structure can be a sheet-shaped retinal tissue, and a cell aggregate comprising retinal tissue having the multilayered structure can be a sheet-shaped cell aggregate. In an embodiment, the cell layers can be neural retinal progenitor cell layers, ganglion cell layers, or photoreceptor cell layers.

In an embodiment of the present invention, the orientation of cells in the multilayered structure can be a direction roughly perpendicular to the layer direction. In this context, the "orientation of cells" refers to a direction in which the shape of nuclei and the orientation of cell bodies extend on the basal membrane side and the apical side. The direction roughly perpendicular to the layer direction refers to a direction orthogonal to a direction in which individual cells are arranged in contact in layers of the multilayered structure (i.e., the tangent directions of the surfaces of layers), and refers to a perpendicular direction or a longitudinal direction with respect to the layers.

In an embodiment of the present invention, the step of dissecting a size necessary for transplantation from the retinal tissue (particularly, sheet-shaped retinal tissue) having an epithelial structure (or a multilayered structure) obtained by the suspension culture or the adhesion culture may be further included. Dissection can be performed using, for example, tweezers, a knife, or scissors.

Meanwhile, the problem has been found that in the case of manufacturing a sheet-shaped retinal tissue by the method mentioned above, RPE cells are manufactured at a given percentage and cause contamination. These RPE cells can be visually confirmed and can also be removed. It is preferable that the RPE cells should not be contained from the beginning from the viewpoint of quality and manufacturing efficiency. Accordingly, as a result of conducting diligent studies to solve the problem, it has become possible to drastically reduce contamination with RPE cells by using a method disclosed below.

### [Sheet-shaped retinal tissue]

An aspect of the present invention is a sheet-shaped retinal (neural retinal) tissue having an epithelial structure. An embodiment of the sheet-shaped retinal tissue consists of a retinal cell layer having a multilayered structure, wherein the multilayered structure has polarities of a basal surface and an apical surface, the retinal cell layer having a multilayered structure comprises one or more cells selected from the group consisting of a retinal progenitor cell, a photoreceptor progenitor cell and a photoreceptor cell, and in the retinal cell layer, the orientation of cells is a direction roughly perpendicular to the layer direction. In this context, the "sheet shape" refers to a single-layer or multilayer structure constituted by single or a plurality of cells having a biological bond at least in the two-dimensional direction.

One of the advantages of the present invention is that a sheet-shaped retinal tissue having an arbitrary size can be manufactured according to the culture equipment used. Specifically, it is possible to manufacture a sheet-shaped retinal tissue having a size that has been impossible to manufacture so far. When a disease spans a wide range, treatment becomes possible by the transplantation of one sheet.

In an embodiment, the major axis (also referred to as diameter) of the sheet-shaped retinal tissue according to the present invention is, for example, 2 mm or more, 4 mm or more, 5 mm or more, 7.5 mm or more, or 10 mm or more. In an embodiment, the minor axis of the sheet-shaped retinal tissue according to the present invention is, for example, 2 mm or more, 3 mm or more, 4 mm or more, or 5 mm or more. In principle, the major axis and the minor axis have no upper limit and are limited by the size of a dish or the like that is used in culture. In an embodiment, the major axis can be 10 cm or less, 5 cm or less, 4 cm or less, 3 cm or less, 2 cm or less, or 1 cm or less. In an embodiment, the height of the sheet-shaped retinal tissue according to the present invention can be, for example, 50 µm to 1500 µm, and is preferably 200 µm to 700 µm.

Methods for measuring the major axis, minor axis and height of the sheet-shaped retinal tissue are not particularly limited, and they can be measured, for example, from an image taken under a microscope. For example, a front image taken with an apical surface turned to an objective lens side, and a side image taken with the cut surface inclined so as to be perpendicular to an objective lens are taken under a stereo microscope as to the sheet-shaped retinal tissue, and they can be measured from the taken images. In this context, the major axis means the longest line segment among line segments connecting two end points on the sheet cross section in the front image, and the length thereof. The minor axis means the longest line segment among line segments connecting two end points on the sheet cross section in the front image and orthogonal to the major axis, and the length thereof. The height means the longest line segment among line segments orthogonal to the sheet cross section and having a point intersecting the sheet cross section and the apex of the retina sheet as end points, and the length thereof.

The basal surface and the apical surface are as described in the definitions mentioned above. The "multilayered structure has polarities of a basal surface and an apical surface" means that the basal surface is present on one side of the multilayered structure and the apical surface is present on the other side. A basal membrane may be present on the basal surface.

The "orientation of cells is a direction roughly perpendicular to the layer direction" means that the major axis of cells present in each layer of the retinal cell layer faces a direction roughly perpendicular to the layer direction. As for the roughly perpendicular direction, an acute angle formed by the layer direction and the major axis of cells is roughly 75° (or 80°) or more and 90° or less. In the present specification, provided that about 50% or more, preferably 60% or more, 70% or more, 80% or more, 85% or more, 90% or more, or 95% or more of cells present in each layer face a direction roughly perpendicular to the layer direction, it is determined that "the orientation of cells is a direction roughly perpendicular to the layer direction".

It is preferable that the sheet-shaped retinal tissue should not contain RPE cells. In an embodiment, the percentage of the number of RPE cells to the total number of cells in the sheet-shaped retinal tissue is 10% or less, preferably 9% or less, 8% or less, 7% or less, 6% or less, 5% or less, 4% or less, 3% or less, 2% or less, or 1% or less. For the percentage of the number of RPE cells to the total number of cells in the sheet-shaped retinal tissue, it is possible to measure, for example, the percentage of cells expressing the markers for RPE cells mentioned above using flow cytometry (FACS) or the like. In an embodiment, the percentage of the area of RPE cells to the total area of the sheet-shaped retinal tissue is 10% or less, preferably 9% or less, 8% or less, 7% or less, 6% or less, 5% or less, 4% or less, 3% or less, 2% or less, or 1% or less. Since RPE cells assume black color, the percentage of the area of RPE cells to the total area of the sheet-shaped retinal tissue is calculable from the percentage of an area that exhibits black color under a microscope. It is also possible to detect cells expressing the markers for RPE cells by PCR or the like. A method for decreasing the percentage of RPE cells in the sheet-shaped retinal (neural retinal) tissue is as mentioned above.

In the sheet-shaped retinal tissue consisting of a retinal cell layer having a multilayered structure of an embodiment,
(1) the retinal cell layer having a multilayered structure has polarities of a basal surface and an apical surface,
(2) the retinal cell layer having a multilayered structure comprises one or more cells selected from the group consisting of a retinal progenitor cell, a photoreceptor progenitor cell and a photoreceptor cell,
(3) in each layer of the retinal cell layer, the orientation of cells is a direction roughly perpendicular to the layer direction, and
(4) a diameter is 8 mm or more.

### (Complex (complex sheet) of sheet-shaped retinal cell layer (neural retina)-retinal pigment epithelial cell)

In an embodiment of the present invention, the retinal cell layer having a multilayered structure may further comprise a sheet-shaped retinal pigment epithelial cell joined to the retinal cell layer, and a complex (complex sheet) is included in which the tangent directions of the respective surfaces of the retinal cell layer and the sheet-shaped retinal pigment epithelial cell are roughly in parallel, the apical surface of the retinal cell layer and the apical surface of the sheet-shaped retinal pigment epithelial cell face each other, and the retinal cell layer and the sheet-shaped retinal pigment epithelial cell are joined through an adhesion factor present therebetween. A sheet-shaped neural retina is also referred to as a neural retina sheet or a NR sheet, and the sheet-shaped retinal pigment epithelial cell is also referred to as a retinal pigment epithelial cell sheet or an RPE cell sheet (RPE sheet). Specifically, an aspect of the present invention also provides a complex (complex sheet) of a neural retina and RPE cells. Another aspect of the present invention also includes a complex (complex sheet) in which dispersed retinal pigment epithelial cells are joined to the apical surface side of a sheet-shaped retinal cell layer (sheet-shaped neural retina) through an adhesion factor.

In the complex of the present specification, the respective tangent directions of the neural retina sheet and the retinal pigment epithelial cell sheet are roughly in parallel. The "tangent directions are roughly in parallel" means that the tangent directions of the facing surfaces of the neural retina and the retinal pigment epithelial cell sheet are in parallel. In the complex according to the present invention, the apical surface of the neural retina and the apical surface of the retinal pigment epithelial cell face each other. Specifically, the apical surface of the neural retina and the apical surface of the retinal pigment epithelial cell are adjacently present.

The complex (complex sheet) of a neural retina and RPE cells can be prepared by joining a sheet-shaped retinal cell layer (sheet-shaped neural retina) to an RPE cell sheet or dispersed RPE cells in the presence of an adhesion factor, as mentioned later. The sheet-shaped retinal cell layer (sheet-shaped neural retina) can be manufactured by the aforementioned method for manufacturing a cell aggregate comprising retinal tissue having an epithelial structure (or a multilayered structure). The RPE cell sheet can be manufactured by a method for manufacturing a retinal pigment epithelial cell sheet mentioned later.

### (Method for manufacturing retinal pigment epithelial cell sheet)

The retinal pigment epithelial (RPE) cells are derived from pluripotent stem cells and, specifically, can be obtained by differentiating pluripotent stem cells. Examples of the method for manufacturing retinal pigment epithelial cells include, but are not particularly limited to, methods disclosed in WO2005/070011, WO2006/080952, WO2011/063005, WO2012/173207, WO2015/053375, WO2015/053376, WO2015/068505, WO2017/043605, Stem Cell Reports, 2 (2), 205-218 (2014) and Cell Stem Cell, 10 (6), 771-785 (2012). It is also possible to prepare a retinal pigment epithelial (RPE) cell sheet by modifying a method described in WO2016/063985 mentioned above. The retinal pigment epithelial cells may be manufactured as a cell sheet or a sphere-like cell aggregate. When manufactured as a sphere-like cell aggregate, the RPE cell sheet is preparable by cutting open the cell aggregate using, for example, tweezers, a knife, or scissors.

As the modified version of the method described in WO2016/063985, pluripotent stem cells are cultured in the absence of feeder cells under conditions involving 1) performing a treatment with a TGFβ family signaling pathway inhibitor and a sonic hedgehog signaling pathway agonist 1 day before differentiation and 2) performing no treatment with a sonic hedgehog signaling pathway agonist at the start of differentiation in the method mentioned above. Then, the steps (B) and (C) mentioned above are performed. Further, it is preferable to accelerate the timing of start of the step (D). Specifically, the step (D) is started around 9 days after (e.g., 7 days, 8 days, 9 days, 10 days, or 11 days after) the start of suspension culture of the step (B). Then, the step (E) mentioned above can be carried out. By this method, a sphere-like cell aggregate of RPE cells is obtained. The cell aggregate may be dispersed to prepare a cell suspension, and the RPE cell sheet can also be prepared by cutting open the cell aggregate using tweezers, a knife, scissors, or the like. The RPE cell sheet can also be prepared by culturing the dispersed cell suspension by adhesion culture. Dispersed RPE cells can also be obtained by the dispersion of the RPE cell sheet or the aggregate of RPE cells.

The retinal pigment epithelial cell sheet may be further cultured until having polygonal/flagstone-like cell morphology before being contacted with a cell aggregate of a neural retina. The culture medium in this case is not particularly limited, and culture can also be further performed by replacement with a maintenance medium for retinal pigment epithelial cells (hereinafter, also referred to as a RPE maintenance medium). A melamine pigmented cell group or a cell group having polygonal flat morphology adhering to a basal membrane can thereby be observed further clearly. Culture in a RPE maintenance medium is not limited as long as a colony that proliferates is formed while the properties of retinal pigment epithelial cells are maintained. Culture is performed for 5 days or more (e.g., on the order of 5 to 20 days) while medium replacement in the whole amount is performed at a frequency of, for example, once or more per three days. Those skilled in the art can readily set a culture period while confirming the morphology. The maintenance medium for retinal pigment epithelial cells can employ one described in, for example, IOVS, March 2004, Vol. 45, No. 3, Masatoshi Haruta et al., IOVS, November 2011, Vol. 52, No. 12, Okamoto et al., Cell Science 122 (17), Fumitaka Osakadar et al., or February 2008, Vol. 49, No. 2, Gamm et al.

The major axis of the retinal pigment epithelial cell sheet may be the same as the major axis of the sheet-shaped retinal tissue (neural retina sheet). In an embodiment, the major axis of the retinal pigment epithelial cell sheet can be in the range of, for example, 3 mm to 50 mm, 5 mm to 30 mm, or 10 mm to 20 mm.

The minor axis of the retinal pigment epithelial cell sheet may be the same as the minor axis of the sheet-shaped retinal tissue (neural retina sheet). In an embodiment, the minor axis of the retinal pigment epithelial cell sheet can be in the range of, for example, 2 mm to 40 mm, 5 mm to 30 mm, or 10 mm to 20 mm.

The degree of melanin pigmentation of the retinal pigment epithelial cell sheet is not particularly limited. It is preferable that the degree of melanin pigmentation of retinal pigment epithelial cells contained in the retinal pigment epithelial cell sheet should be the same level among the cells. In an embodiment, the average melanin content of the retinal pigment epithelial cell sheet can be less than 20 pg/cell, less than 15 pg/cell, less than 10 pg/cell, less than 8 pg/cell, less than 7 pg/cell, less than 6 pg/cell, less than 5 pg/cell, less than 4 pg/cell, less than 3 pg/cell, less than 2 pg/cell, or less than 1 pg/cell. The average melanin content of the retinal pigment epithelial cell sheet may be 0.1 pg/cell or more, 0.5 pg/cell or more, 1 pg/cell or more, 2 pg/cell or more, or 5 pg/cell or more.

The melanin content of the retinal pigment epithelial cell sheet can be measured using, for example, cell extracts obtained with NaOH or the like after dispersing the retinal pigment epithelial cell sheet, and using a spectrophotometer or the like. The average melanin content can be determined by dividing the melanin content by the total number of cells contained in the retinal pigment epithelial cell sheet.

The complex mentioned above can be manufactured by joining a neural retina sheet to dispersed RPE cells or a RPE cell sheet. A complex sheet in which a neural retina sheet is joined to a RPE cell sheet is preferable. The neural retina sheet and the RPE cell sheet mentioned above can be easily taken out of culture equipment by using tweezers, a knife, scissors, or the like. Both the sheets taken out may be transferred to a fresh container (culture equipment, etc.). While one of the sheets is allowed to remain in the culture equipment, the other sheet may be transferred into the culture equipment. The sizes of the sheet-shaped tissues to be joined can be uniformed by manufacturing them in culture equipment having the same size. In the case of using a neural retina sheet and retinal pigment epithelial cells with different numbers of culture days in the manufacturing method mentioned above as to the neural retina sheet and the retinal pigment epithelial cells to be contacted, the days when manufacturing is started can be changed.

It is preferable that the neural retina sheet and the retinal pigment epithelial cells mentioned above should be contacted in the presence of an adhesion factor. The adhesion factor refers to a substance having the action of allowing cells to adhere to each other. Examples thereof include, but are not particularly limited to, the extracellular matrixes mentioned above and artificial hydrogels. The adhesion factor does not have to be an isolated single substance and also includes, for example, Matrigel, an inter-photoreceptor cell matrix, and a preparation from a living body or cells, such as serum. Matrigel is a basal membrane preparation derived from Engelbreth Holm Swam (EHS) mouse sarcoma. Matrigel can be prepared by, for example, a method disclosed in US Patent No. 4829000, and a commercially available product can also be purchased. Main components of Matrigel are laminin, IV-type collagen, heparan sulfate proteoglycan and entactin. The inter-photoreceptor cell matrix is a generic name for extracellular matrixes present between retinal cells such as photoreceptor cells in a retina *in vivo,* and, for example, hyaluronic acid is included. Those skilled in the art are capable of collecting the inter-photoreceptor cell matrix from a retina *in vivo* by, for example, a method of placing the retina in distilled water so as to swell, followed by separation, and a commercially available product can also be purchased. An extracellular matrix or a hydrogel is preferable as the adhesion factor. One or more extracellular matrixes selected from the group consisting of hyaluronic acid, fibrin, laminin, IV-type collagen, heparan sulfate proteoglycan, and entactin are preferable as the extracellular matrix. One or more hydrogels selected from the group consisting of gelatin, fibrin, collagen, pectin, hyaluronic acid, and alginic acid are preferable as the hydrogel. A substance that is classified into both an extracellular matrix and a hydrogel is present and is treated as a hydrogel in the present specification when used as a gel-like adhesion factor. Examples of the commercially available extracellular matrix include Corning(R) Matrigel Basal Membrane Matrix and iMatrix511. The adhesion factor can be one or more substances selected from gelatin, fibrin, fibronectin, hyaluronic acid, laminin, IV-type collagen, heparan sulfate proteoglycan and entactin, and it is particularly preferable to be gelatin or fibrin.

Fibrin gel is gel-like fibrin that is obtained by reacting a fibrinogen solution with a thrombin solution. Commercially available Bolheal(R) for tissue adhesion can also be used. The fibrinogen solution and the thrombin solution are contacted or mixed and both can thereby be reacted.

The "gelatin" is a solubilized form of water-insoluble collagen, for example, by pretreatment with an acid or an alkali and thermal hydrolysis. It is also possible to obtain commercially available gelatin. Examples thereof include gelatin LS-H (Nitta Gelatin Inc., alkali-treated porcine skin gelatin, no-heat-treated gelatin, high jelly strength) and gelatin LS-W (Nitta Gelatin Inc., alkali-treated porcine skin gelatin, heat-treated gelatin, low jelly strength). Alkali-treated (limed) gelatin (B-type gelatin) is preferable, and heat-treated gelatin is preferable.

The hydrogel is heated or cooled so that the solution changes a phase from gel to sol or from sol to gel. The hydrogel is converted to gel (jelly) by losing fluidity through cooling and converted to sol (aqueous solution) by acquiring fluidity through heating. In this context, the "melting point" means a temperature at which solation occurs under a predetermined pressure, and the "freezing point" means a temperature at which gelation occurs under a predetermined pressure. It is preferable that the hydrogel should be biodegradable. In an embodiment, a hydrogel (e.g., gelatin) whose melting point is a temperature (25°C to 40°C) near a body temperature is preferable. The hydrogel in the present specification may have a melting point of 20°C to 40°C (e.g., 20°C to 35°C, 25°C to 35°C, 30°C to 40°C, or 35°C to 40°C). In general, the melting point of gel is a measure for the strength of a network. The melting point of the hydrogel (e.g., gelatin) is elevated with the elevation of the concentration and molecular weight of the hydrogel. For example, the melting point and the freezing point tend to be elevated as a solid content is increased with a saccharide. Thus, it is possible to vary the melting point and the freezing point within predetermined ranges. A method for measuring the melting point of the hydrogel is not particularly limited, and it can be measured by, for example, a method prescribed in JIS K6503.

The strength of the hydrogel can be to an extent that the hydrogel does not collapse in operation for transplantation. The "jelly strength" serves as an index for the strength of the hydrogel. The "jelly strength" of the hydrogel means the mechanical strength of an object that has formed gel. It is usually expressed as force required for deforming gel in a predetermined shape or force required for breaking gel (unit: g, dyne(s)/cm² or g/cm²), and is typically a measure for the hardness of gel. 1 dyne is defined as force which when acting on a body of mass 1 g produces an acceleration of 1 cm/s² in that direction. The jelly strength of gelatin can be measured by, for example, a method prescribed in JIS K6503. In the present specification, the jelly strength of the hydrogel (e.g., gelatin) gel can be, for example, 50 g or more, 100 g or more, 200 g or more, 500 g or more, 1000 g or more, 1200 g or more, 1300 g or more, 1400 g or more, or 1500 g or more. The jelly strength of the hydrogel (gelatin) may be 3000 g or less, 2500 g or less or 2000 g or less.

The concentration of the adhesion factor (extracellular matrix) differs depending on the size of the neural retina sheet or the retinal pigment epithelial cell sheet or the number of cells of retinal pigment epithelial cells. Those skilled in the art are capable of readily setting it by confirming the state of adhesion of RPE cells. For example, it is preferable to add Matrigel at a concentration of a ready-made (Corning, Inc.) diluted 200- to 10000-fold or iMatrix511 at a concentration of 0.1 to 5 µg/mL.

In an embodiment of the method for manufacturing the complex using an extracellular matrix, culture for allowing the neural retina sheet to adhere to the retinal pigment epithelial cells or the retinal pigment epithelial cell sheet can be performed in a vehicle containing the adhesion factor (extracellular matrix). Examples of the vehicle used include, but are not particularly limited to, culture media that are used in the culture of retinal pigment epithelial cells or neural retinas (e.g., DMEM/F12 medium, neurobasal medium, a mixture of these culture media, RPE maintenance medium, etc.). Culture for adhesion may be performed in the presence of other components such as a growth factor (e.g., EGF) together with the extracellular matrix. For a culture period for allowing the neural retina sheet mentioned above to adhere to the retinal pigment epithelial cells or the retinal pigment epithelial cell sheet, culture may be continuously performed in the vehicle containing the adhesion factor, or culture may be continuously performed by culture in the vehicle containing the adhesion factor for a given period (e.g., 1 day to 10 days), followed by replacement with a vehicle containing no adhesion factor.

Before culture for allowing the neural retina sheet to adhere to the retinal pigment epithelial cells or the retinal pigment epithelial cell sheet is performed, the neural retina sheet or the retinal pigment epithelial cells or the retinal pigment epithelial cell sheet may be coated with the adhesion factor. Specifically, the neural retina sheet or the retinal pigment epithelial cells or the retinal pigment epithelial cell sheet can be cultured in the vehicle containing the adhesion factor. Those skilled in the art are appropriately capable of setting a culture time. Culture can be performed for a time on the order of 10 minutes to 5 hours (e.g., 10 minutes to 60 minutes). After culture, washing may be performed with a vehicle such as PBS.

The adhesion factor, the hydrogel or the matrix gel is preferably fibrin gel. The fibrin gel is gel-like fibrin that is obtained by reacting a fibrinogen solution with a thrombin solution. In the specification, a substance having a property of forming gel through reaction is referred to as a matrix precursor, and, for example, thrombin and fibrinogen which react to form fibrin gel are one example of the matrix precursor. The fibrinogen solution can be prepared by dissolving a fibrinogen powder or the like in a dissolving liquid that contains aprotinin and can dissolve fibrinogen, and its concentration is not particularly limited, but is, for example, from 40 to 480 mg/ml, preferably from 80 mg/mL to 320 mg/mL (e.g., 160 mg/mL). When the activity of blood coagulation factor VIII contained in 1 ml of normal human plasma is defined as 1 unit, it can be from 37.5 units/mL to 225 units/mL (e.g., 75 units/mL). The thrombin solution can be prepared by dissolving a thrombin powder or the like in a thrombin-dissolving liquid containing calcium chloride hydrate, and its concentration is not particularly limited, but is, for example, from 125 units/mL to 750 units/mL (e.g., 75 units/mL). The fibrinogen solution and the thrombin solution are contacted or mixed so that they can be reacted with each other. In this respect, it is preferable that the fibrinogen solution and the thrombin solution should be used in the range of 1:1 to 1:9, preferably 1:3 to 1:4, in terms of an activity ratio.

A method for manufacturing the complex using fibrin gel will be given below as an embodiment of the method for manufacturing the complex using a hydrogel. Specifically, the neural retina sheet is allowed to adhere to the retinal pigment epithelial cells or the retinal pigment epithelial cell sheet through fibrin gel that is produced by reacting fibrinogen and thrombin.

In an embodiment, of the two tissues described above, one tissue (neural retina or retinal pigment epithelial cells) contacted with a fibrinogen solution and the other tissue (retinal pigment epithelial cells or neural retina) contacted with a thrombin solution are contacted with each other so that fibrinogen and thrombin may be reacted for gelation. In this context, the contact with a solution means that at least an adhesion surface (a surface that faces another tissue when adhering to another tissue through fibrin gel) of a tissue is contacted with a fibrinogen solution or a thrombin solution to the extent that attachment to the adhesion surface of the tissue is caused by the solution.

More specifically, for example, in the case of allowing a neural retina sheet to adhere to a retinal pigment epithelial cell sheet, the neural retina sheet can be added in a fibrinogen solution, and the retinal pigment epithelial cell sheet can be added in a thrombin solution (the solutions may be reversed). It is preferable to use the fibrinogen solution and the thrombin solution in the range of 3:1 to 1:3 in terms of a volume ratio. Before adhesion, extra fibrinogen or thrombin attached to the tissue may be removed. By this operation, the thickness of the fibrin gel can be adjusted.

In order to allow the neural retina sheet contacted with the fibrinogen solution to adhere to the retinal pigment epithelial cell sheet contacted with the thrombin solution, both may be contacted. In this context, contacting the tissues refers to contacting, i.e., overlapping, the surface attached to the fibrinogen solution and the surface attached to the thrombin solution. It is preferable to allow the retinal pigment epithelial cell sheet to adhere to the neural retina such that the tangent directions of their respective surfaces are roughly in parallel and the apical surface of the neural retina and the apical surface of the retinal pigment epithelial cell sheet face each other.

In an embodiment, fibrinogen and thrombin may be reacted by contacting the tissues described above with a fibrinogen solution, and subsequently adding a thrombin solution to the fibrinogen solution. In this case, the tissues are embedded in fibrin gel.

In an embodiment, the manufacturing method of the present invention may further comprise the step of dissecting a complex having a size necessary for transplantation from the complex. Since two or more tissues have been allowed to adhere firmly through fibrin gel, a graft, when dissected from the complex, can be easily dissected into the desired size without the tissues coming off from the complex. It is possible to use tweezers, a knife, scissors, or the like in dissection.

### [Pharmaceutical composition, treatment method, therapeutic product and use]

An aspect of the present invention includes a pharmaceutical composition comprising a sheet-shaped retinal tissue. The pharmaceutical composition preferably comprises a pharmaceutically acceptable carrier, in addition to the sheet-shaped retinal tissue of the present invention. The pharmaceutical composition can be used in the treatment of a disease caused by the damage of a neural retinal cell or a neural retina or the injury of a neural retina. Examples of the disease caused by the damage of a neural retinal cell or neural retina include ophthalmic diseases such as retinal degenerative diseases, macular degeneration, age-related macular degeneration, retinitis pigmentosa, glaucoma, corneal diseases, retinal detachment, central serous chorioretinopathy, cone dystrophy, and cone rod dystrophy. Examples of the injury state of neural retina include a state in which photoreceptor cells die of degeneration.

As the pharmaceutically acceptable carrier, a physiological aqueous solvent (physiological saline, buffer, serum-free medium, etc.) can be used. If necessary, the pharmaceutical composition may be blended with a preservative, a stabilizer, a reducing agent, a tonicity agent, and the like which are usually used in a medicine containing tissues or cells to be transplanted in medical transplantation.

An aspect of the present invention provides a therapeutic product for a disease caused by the damage of a neural retina, comprising a sheet-shaped retinal tissue obtained in the present invention. An aspect of the present invention includes a method for treating a disease caused by the damage of a neural retinal cell or a neural retina or the injury of a neural retina, comprising transplanting a sheet-shaped retinal tissue obtained in the present invention to a subject in need of transplantation (e.g., subretinally to an eye having the ophthalmic disease). As the therapeutic product for a disease caused by the damage of a neural retina, or in order to make up for a corresponding injured site in the injury state of the neural retina, the sheet-shaped retinal tissue of the present invention can be used. The disease caused by the damage of a neural retinal cell or a neural retina or the injury state of a neural retina can be treated by transplanting the sheet-shaped retinal tissue of the present invention to a patient having the disease caused by the damage of a neural retinal cell or a neural retina, or a patient with the injury state of a neural retina, in need of transplantation, and making up for the neural retinal cell or the damaged neural retina. Examples of a transplantation method include a method of subretinally transplanting the sheet-shaped retinal tissue to an injured site through an incision to an eyeball. Examples of a method for transplantation include a method of performing infusion using a thin tube, and a method of performing transplantation by sandwiching between tweezers, and examples of the thin tube include injection needles.

An aspect of the present invention provides a sheet-shaped retinal tissue obtained in the present invention for use in the treatment of a disease caused by the damage of a retinal cell or retinal tissue or the injury of retinal tissue. An aspect of the present invention provides use of a sheet-shaped retinal tissue obtained in the present invention in the manufacturing of a therapeutic product for a disease caused by the damage of a retinal cell or retinal tissue or the injury of retinal tissue.

### Examples

Hereinafter, the present invention will be described in detail with reference to Examples. However, the present invention is not limited by these by any means.

### <Example 1 Search of aggregate reforming factor>

Human ES cells (KhES-1 strain (Non Patent Literature 3)) and human iPS cells (1231A3 strain, Kyoto University) genetically engineered so as to have Rx:: Venus reporter gene were cultured under feeder-free conditions in accordance with the method described in "Scientific Reports, 4, 3594 (2014)". As a feeder-free medium, StemFit medium (trade name: AK03N, manufactured by Ajinomoto Co., Inc.) was used, and as a scaffold as an alternative to feeder cells, Laminin511-E8 (trade name, manufactured by Nippi, Inc.) was used.

Specific operation of maintenance culture for human ES and human iPS cells (human ES/iPS cells) was performed as follows: First, human ES/iPS cells that reached sub-confluency (state where about 60% of the culture area was covered by cells) were washed with PBS and separated into single cells by use of TrypLE Select (trade name, manufactured by Life Technologies). In this context, the dispersion into single cells refers to performing dispersion so as to become single cells, and a cell population dispersed into single cells is capable of containing a mass of 2 to 50 cells, in addition to single cells. Then, the separated human ES single cells were seeded in plastic culture dishes coated with Laminin 511-E8 and cultured under feeder-free conditions in StemFit medium in the presence of Y-27632 (ROCK inhibitor, 10 µM). When 6-well plates (for cell culture, culture area: 9.4 cm², manufactured by AGC TECHNO GLASS., LTD) were used as the plastic culture dishes, the number of separated human ES/iPS single cells to be seeded was specified as 0.4 to 1.2 × 10⁴ cells per well. One day after seeding, the medium was exchanged with StemFit medium not containing Y-27632. Thereafter, the medium was exchanged with Y27632-free StemFit medium once every 1 to 2 days. Thereafter, the cells were cultured under feeder-free conditions until 1 day before reaching sub-confluency. The human ES cells the 1 day before the sub-confluency were cultured for 1 day (preconditioning treatment) under feeder-free conditions in the presence of SB431542 (TGFβ signaling pathway inhibitor, 5 µM) and SAG (Shh signaling pathway agonist, 300 nM).

The human ES/iPS cells were washed with PBS, then treated for cell dispersions using TrypLE Select, and further separated into single cells by pipetting, and then the separated human ES single cells were suspended in 100 µL of a serum-free medium such that the density of cells per well of a non-cell adhesive 96-well culture plate (trade name: PrimeSurface, 96-well V-bottom plate, manufactured by Sumitomo Bakelite Co., Ltd.) was 1.2 × 10⁴ cells, and subjected to suspension culture in the conditions of 37°C and 5% CO₂. The serum-free medium (gfCDM + KSR) used herein is a serum-free medium prepared by adding 10% KSR and 450 µM 1-monothioglycerol and 1X Chemically defined lipid concentrate to a mixture of culture fluids containing F-12 medium and IMDM medium in a ratio of 1:1.

At the initiation time of the suspension culture (at the initiation time of differentiation, Day 0), Y-27632 (ROCK inhibitor, final concentration: 10 µM or 20 µM) and SAG (Shh signaling pathway agonist, 300 nM, 30 nM or 0 nM) were added to the serum-free medium. Day 3 from initiation of the suspension culture, 50 µL of a medium was used which did not contain Y-27632 or SAG and contained exogenous human recombinant BMP4 (trade name: Recombinant Human BMP-4, manufactured by R&D Systems, Inc.) at a final concentration of 1.5 nM. Day 6 or later from initiation of the suspension culture, a half of the medium was exchanged with a culture medium, which did not contain Y-27632, SAG or human recombinant BMP4, once every 3 days.

As for the KhES-1-derived cell aggregates, the aggregates of Day 14 to Day 18 from initiation of the suspension culture were transferred to a 90-mm low adhesive plate (petri dish 90φ (deep type) for suspension culture, manufactured by Sumitomo Bakelite Co., Ltd.) and cultured at 37°C and 5% CO₂ for 3 to 4 days in a serum-free medium (DMEM/F12 medium supplemented with 1% N2 Supplement) containing a Wnt signaling pathway agonist (CHIR99021, 3 µM) and a FGF signaling pathway inhibitor (SU5402, 5 µM). Thereafter, long-term culture was performed using a serum medium (NucTO medium), which did not contain the Wnt signaling pathway agonist or the FGF signaling pathway inhibitor, in a 90-mm low adhesive plate (petri dish 90φ (deep type) for suspension culture, manufactured by Sumitomo Bakelite Co., Ltd.).

The aggregates of Day 16 (1231A3-derived) or Day 27 (KhES-1-derived) from initiation of the suspension culture were washed with PBS, and a neuronal cell dispersion solution (manufactured by FUJIFILM Wako Pure Chemical Corp.) was added. After incubation at 37°C for 20 to 30 minutes, they were dispersed into single cells by pipetting. These cells were suspended in 100 µL of a serum-free medium so as to become 2 × 10⁵ cells (KhES-1-derived) or 5 × 10⁵ cells (1231A3-derived) per well of a non-cell adhesive 96-well culture plate (trade name: PrimeSurface 96-well V-bottom plate, manufactured by Sumitomo Bakelite Co., Ltd.), and suspension-cultured at 37°C and 5% CO₂. Simultaneously with the cell addition, (1) none (control), (2) 10 µM or 20 µM (1231A3-derived) Y-27632 (FUJIFILM Wako Pure Chemical Corp.), (3) 300 nM SAG (Enzo Life Sciences, Inc.), (4) 10 µg/mL bFGF (FUJIFILM Wako Pure Chemical Corp.), (5) 1 µM LDN193189 (Stemgent), (6) 3 µM CHIR99021 (FUJIFILM Wako Pure Chemical Corp.), (7) 10 µM SB431542 (FUJIFILM Wako Pure Chemical Corp.), and (8) 3 µM IWR-1 (FUJIFILM Wako Pure Chemical Corp.) were each added.

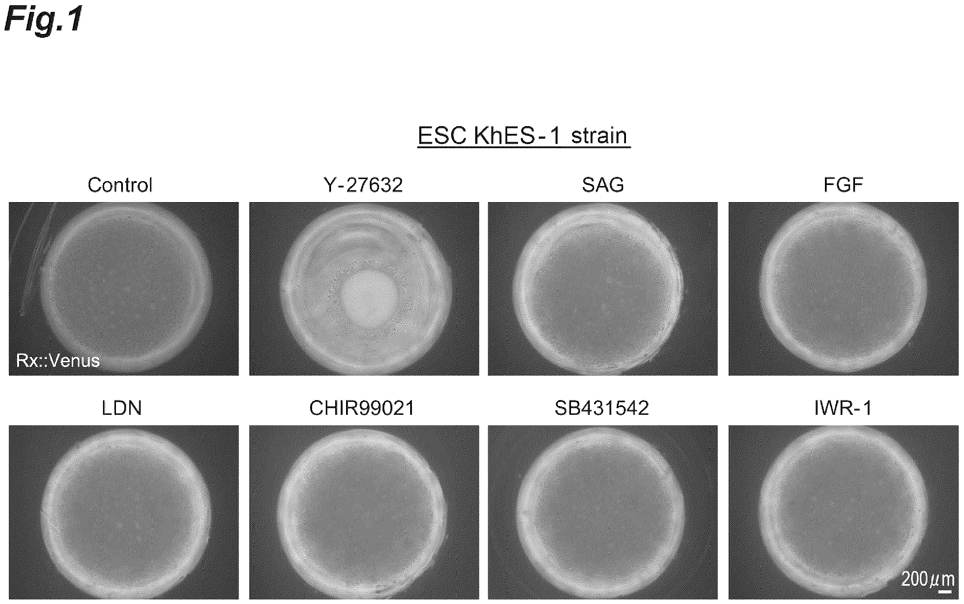

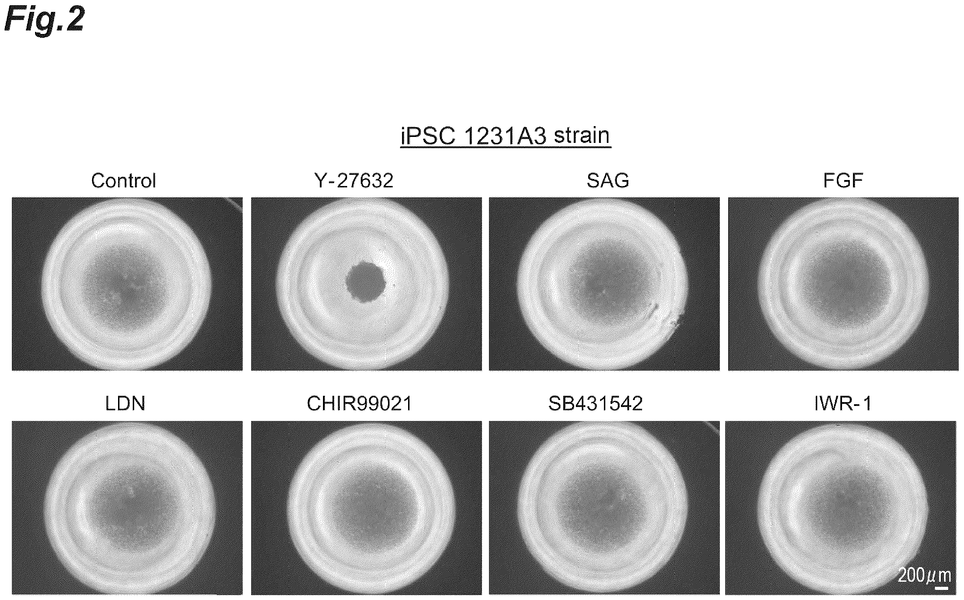

Under all of the conditions (1) to (8), it was confirmed that aggregates were reformed by culture for about 2 weeks (see results of Example 3). In the case of adding Y-27632, it was confirmed that aggregates were reformed from Day 1 to Day 2 after suspension culture for aggregate reforming (Figure 1: KhES-1, Figure 2: 1231A3). On the other hand, an early aggregate reforming-promoting effect was not found in the compounds other than Y-27632 (Figures 1 and 2). Accordingly, it was found that the reforming of aggregates is promoted from early on by adding Y-27632 to retinal progenitor cells dispersed into single cells.

### <Example 2 Search of layer structure formation-promoting factor and aggregate proliferation-promoting factor>

Human ES cells (KhES-1 strain (Non Patent Literature 3)) genetically engineered so as to have Rx:: Venus reporter gene were cultured under feeder-free conditions in accordance with the method described in "Scientific Reports, 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of Day 20 (1231A3) and Day 27 (KhES-1) from initiation of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting. These cells were suspended in 100 µL of a serum-free medium containing 10 µM Y-27632 (FUJIFILM Wako Pure Chemical Corp.) so as to become 2 to 5 × 10⁵ cells per well of a non-cell adhesive 96-well culture plate (trade name: PrimeSurface 96-well V-bottom plate, manufactured by Sumitomo Bakelite Co., Ltd.), and suspension-cultured at 37°C and 5% CO₂. Simultaneously with the cell addition, (1) none (only 10 µM Y-27632), (2) 30 nM SAG (Enzo Life Sciences, Inc.), (3) 300 nM SAG (Enzo Life Sciences, Inc.), (4) 10 ng/mL bFGF (FUJIFILM Wako Pure Chemical Corp.), (5) 1 µM LDN193189 (Stemgent), (6) 3 µM CHIR99021 (FUJIFILM Wako Pure Chemical Corp.), (7) 10 µM SB431542 (FUJIFILM Wako Pure Chemical Corp.), or (8) 3 µM IWR-1(KhES-1-derived) or 10 ng/mL EGF (1231A3-derived) were added.

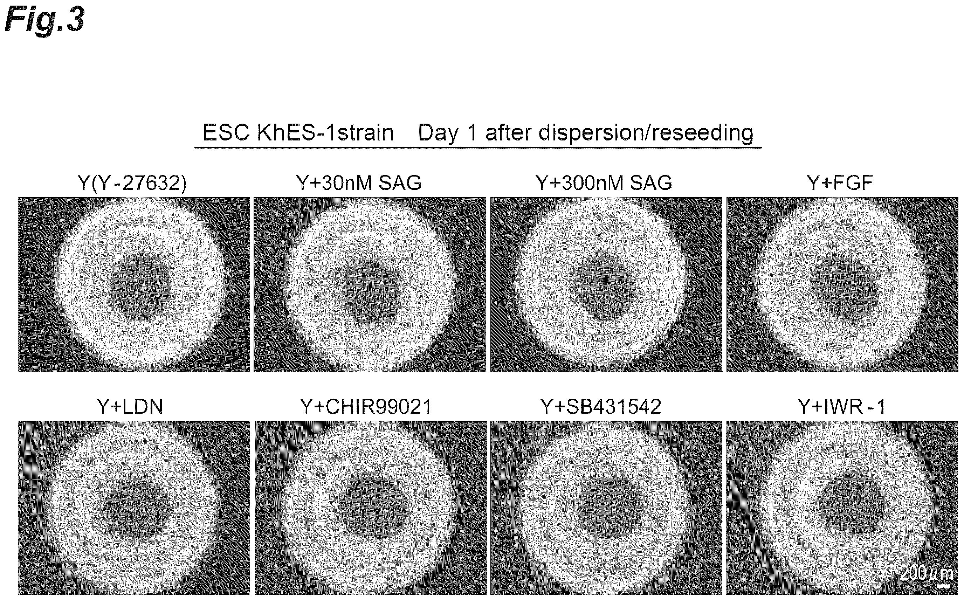

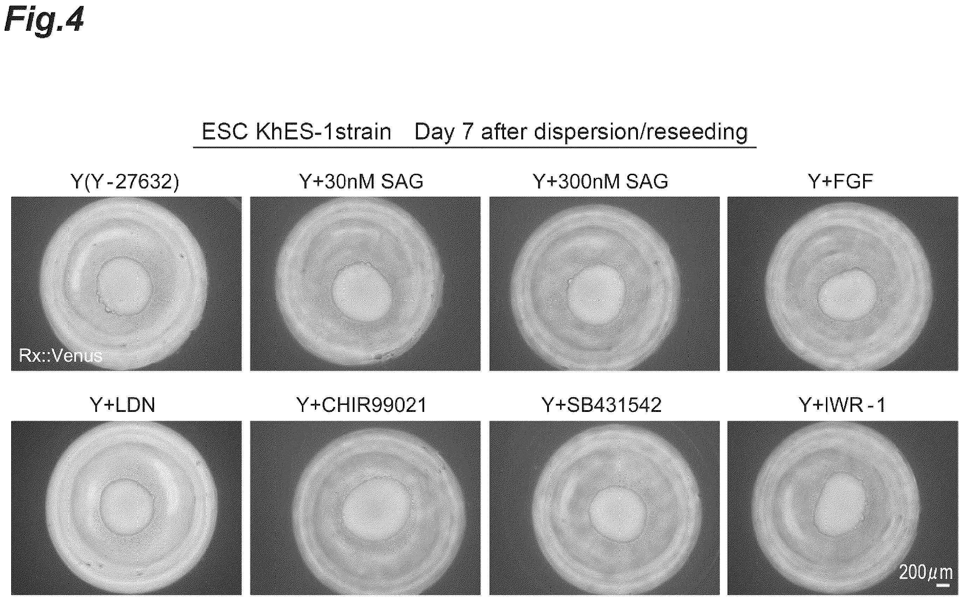

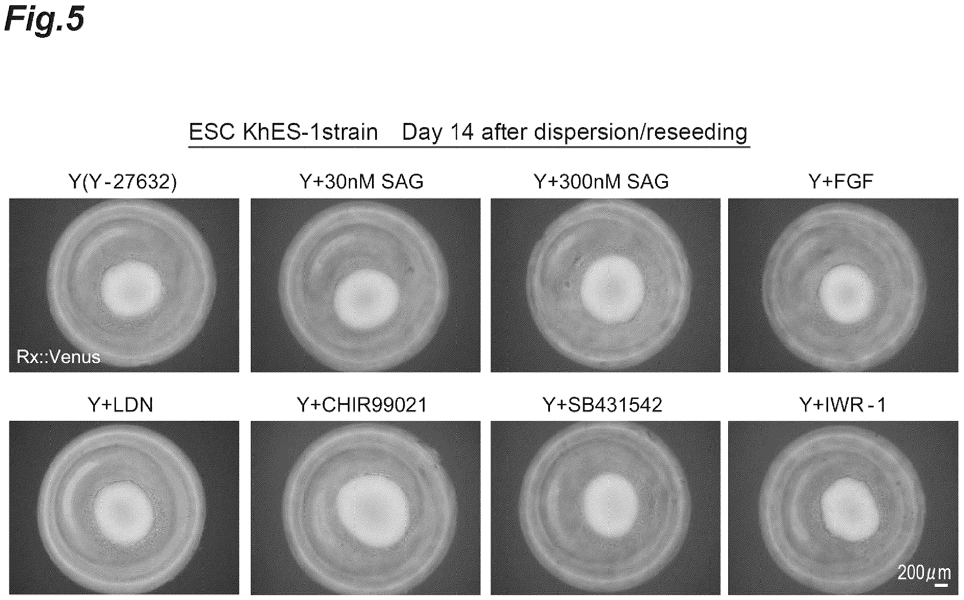

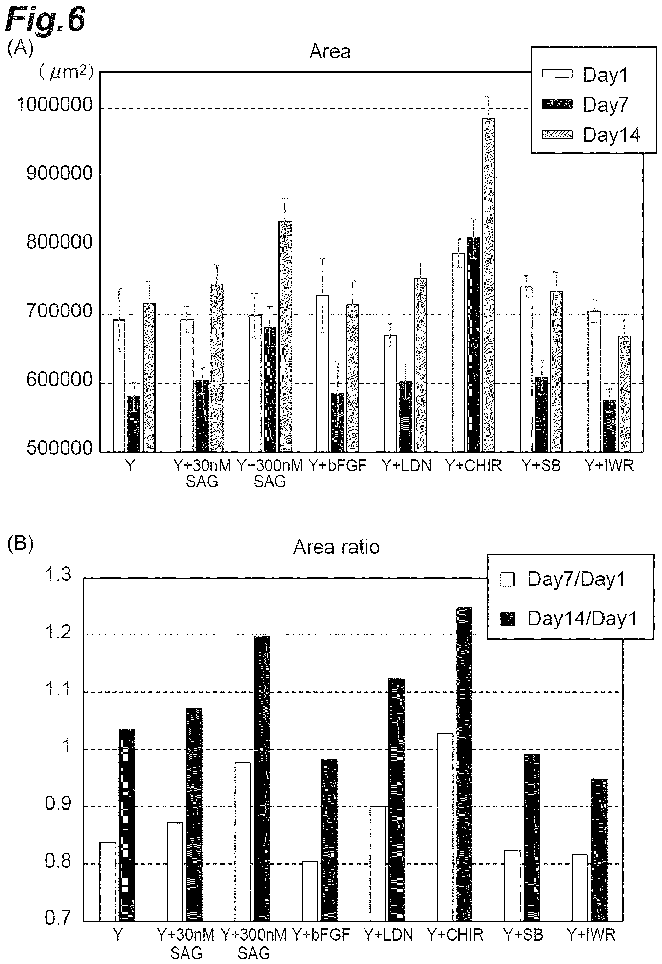

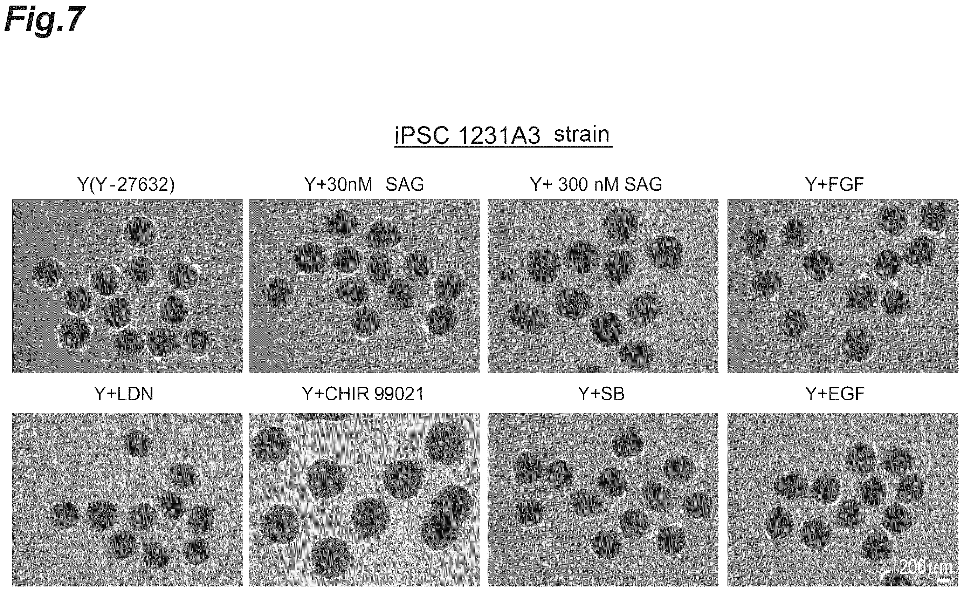

The reformed states of the aggregates were observed under a bright field microscope and a fluorescence microscope (BZ-X810 manufactured by Keyence Corp.) on Day 1, Day 7, and Day 14 after suspension culture for aggregate reforming. The results of the KhES-1-derived cells are shown in Figures 3 to 5. The areas of the aggregates were measured in Image J. The results of the KhES-1-derived cells are shown in Figure 6 (an average value from 12 cells measured). The results of the 1231A3-derived cells in which the reformed states of the aggregates of Day 1 after suspension culture were observed are shown in Figure 7. From Figures 3 to 7, it was confirmed that larger aggregates were reformed in the group supplemented with SAG (300 nM) or CHIR99021 (3 µM) in addition to Y-27632 than in the group supplemented with only Y-27632. Accordingly, SAG and CHIR99021 were confirmed to have an effect of promoting the proliferation and reforming of an aggregate.

### <Example 3 Search of layer structure formation-promoting factor and aggregate proliferation-promoting factor>

Human ES cells (KhES-1 strain (Non Patent Literature 3)) genetically engineered so as to have Rx:: Venus reporter gene were cultured under feeder-free conditions in accordance with the method described in "Scientific Reports, 4, 3594 (2014)". As a feeder-free medium, StemFit medium (trade name: AK03N, manufactured by Ajinomoto Co., Inc.) was used, and as a scaffold as an alternative to feeder cells, Laminin511-E8 (trade name, manufactured by Nippi, Inc.) was used.

The aggregates of Day 26 from initiation of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting. These cells were suspended in 100 µL of a serum-free medium so as to become 3.0 × 10⁴ cells per well of a non-cell adhesive 96-well culture plate (trade name: PrimeSurface 96-well V-bottom plate, manufactured by Sumitomo Bakelite Co., Ltd.), and suspension-cultured at 37°C and 5% CO₂. Simultaneously with the cell addition, (1) none (control), (2) 10 µM Y-27632 (FUJIFILM Wako Pure Chemical Corp.), (3) 300 nM SAG (Enzo Life Sciences, Inc.), (4) 10 µM Y-27632 (FUJIFILM Wako Pure Chemical Corp.) and 300 nM SAG (Enzo Life Sciences, Inc.), (5) 3 µM CHIR99021 (FUJIFILM Wako Pure Chemical Corp.), (6) 10 µM Y-27632 (FUJIFILM Wako Pure Chemical Corp.) and 3 µM CHIR99021 (FUJIFILM Wako Pure Chemical Corp.), (7) 300 nM SAG (Enzo Life Sciences, Inc.) and 3 µM CHIR99021 (FUJIFILM Wako Pure Chemical Corp.), or (8) 10 µM Y-27632 (FUJIFILM Wako Pure Chemical Corp.), 300 nM SAG (Enzo Life Sciences, Inc.) and 3 µM CHIR99021 (FUJIFILM Wako Pure Chemical Corp.) were added.

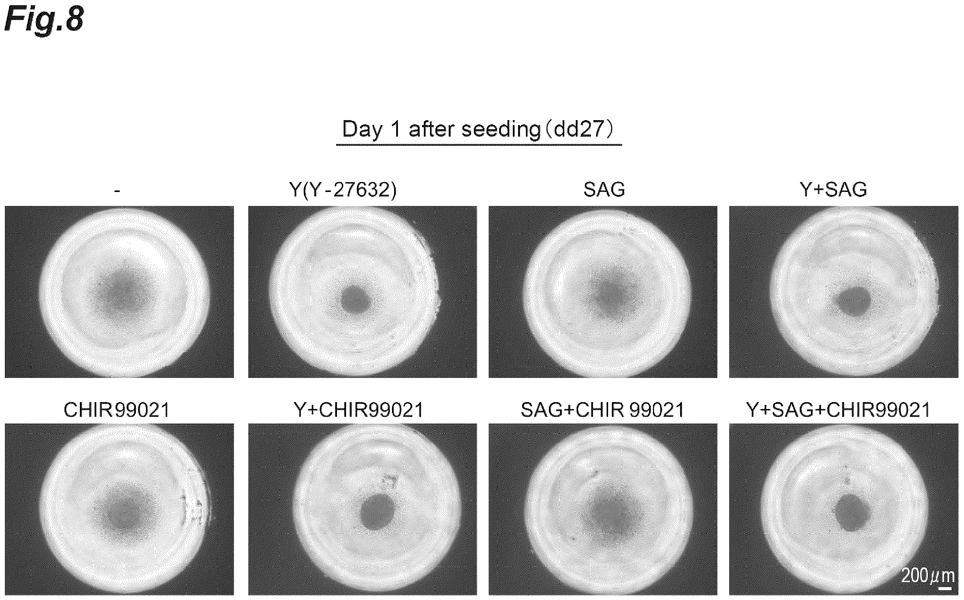

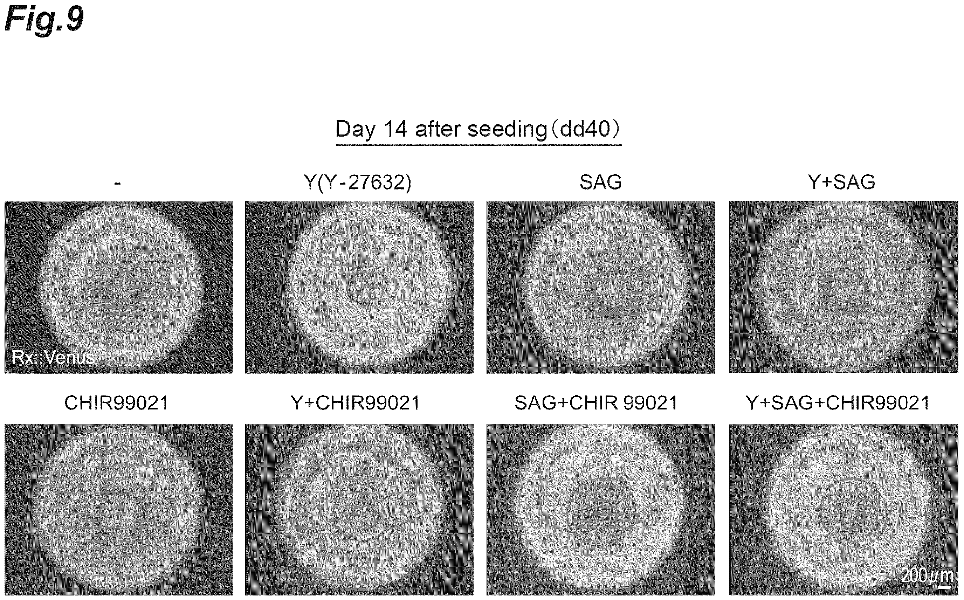

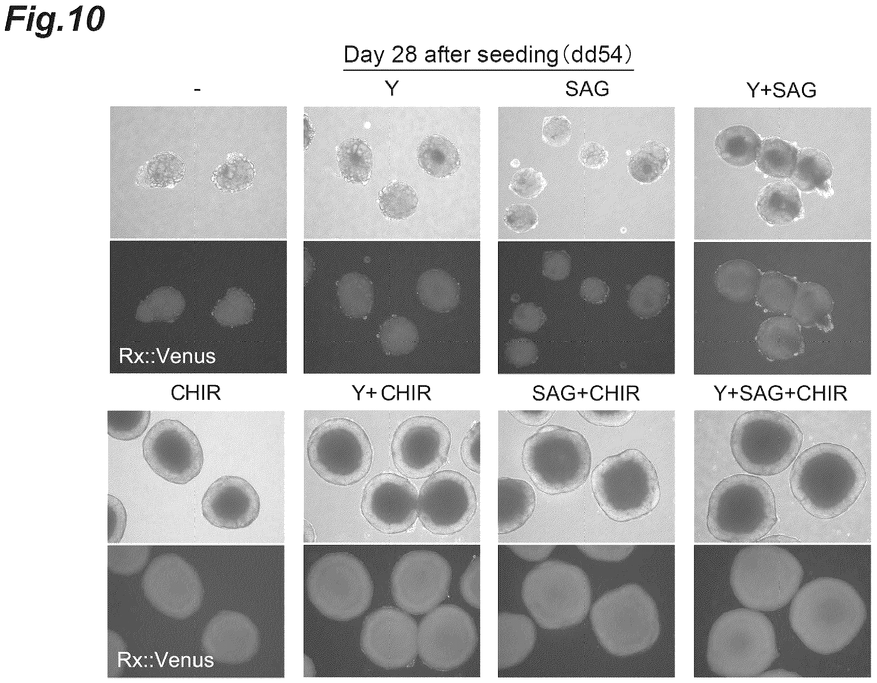

Results of observing the reformed states of the aggregates on Day 1, Day 14 and Day 28 after suspension culture for aggregate reforming, i.e., on Day 27, Day 40 and Day 54 after the start of suspension culture for differentiation (dd27, dd40, dd54) are shown in Figures 8, 9, and 10. From Figures 8, 9, and 10, it was confirmed that aggregates were reformed when CHIR99021 was added. It was also confirmed that aggregates were larger by adding SAG in addition to CHIR99021.

The aggregates of Day 15 (corresponding to Differentiation Day 41) and Day 28 (corresponding to Differentiation Day 54) after suspension culture for aggregate reforming were fixed in 4% PFA, and frozen sections were prepared. These frozen sections were subjected to immunostaining using DAPI and anti-Chx10 antibody (trade name: Anti CHX10 Antibody, Exalpha Biologicals Inc.), anti-β-catenin antibody (R&D Systems, Inc.), anti-collagen IV antibody (Abeam plc), anti-Zo-1 antibody (Invitrogen Corp.), anti-Ki67 antibody (Becton, Dickinson and Company), anti-Pax6 antibody (BioLegend, Inc.), anti-RxR-γ (RxRg) antibody (Spring Bioscience Corp.), anti-CRX antibody (Abnova Corp.), anti-NRL antibody (R&D Systems, Inc.), anti-recoverin antibody (manufactured by Proteintech Group, Inc.), anti-Islet-1 antibody (Developmental Studies Hybridoma Bank (DSHB)), anti-GS antibody (Sigma-Aldrich Co., LLC), anti-Brn3 antibody (Santa Cruz Biotechnology), and anti-calretinin antibody (R&D Systems, Inc.).

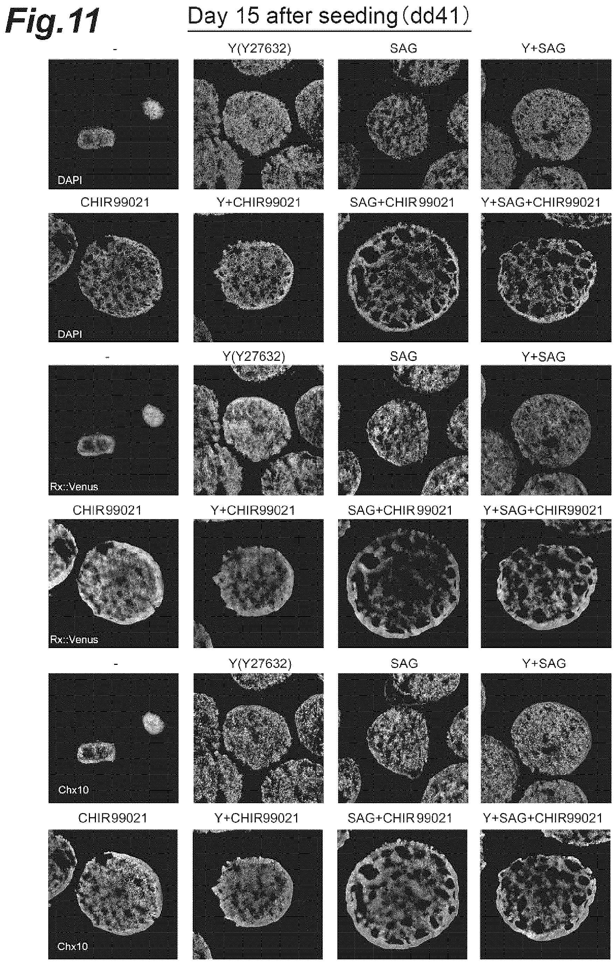

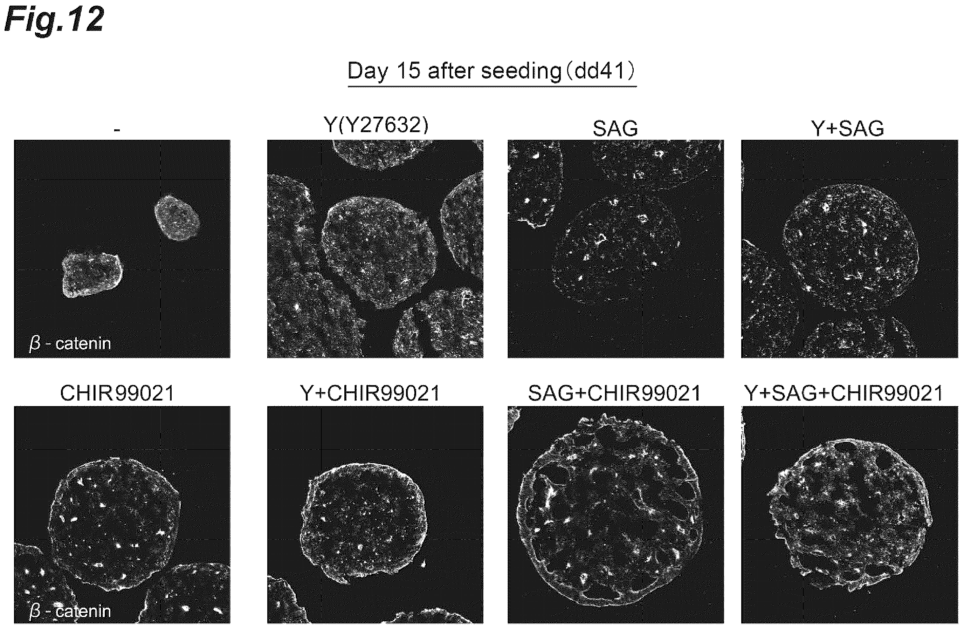

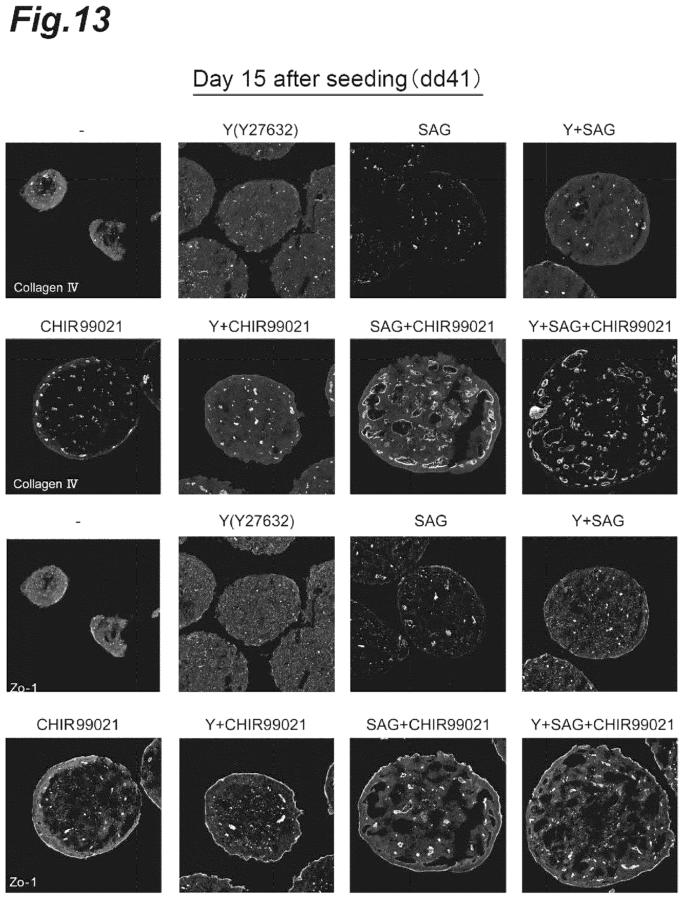

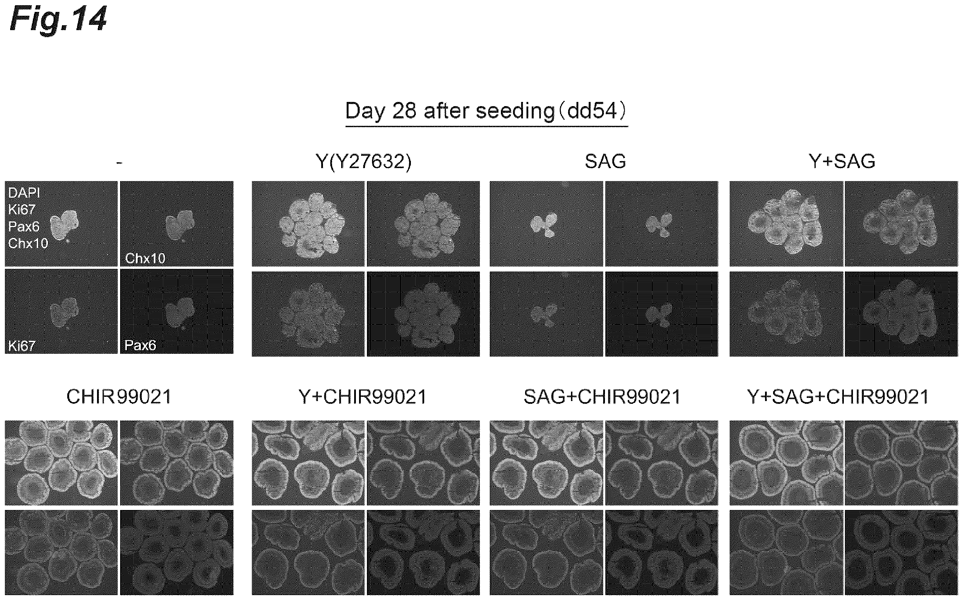

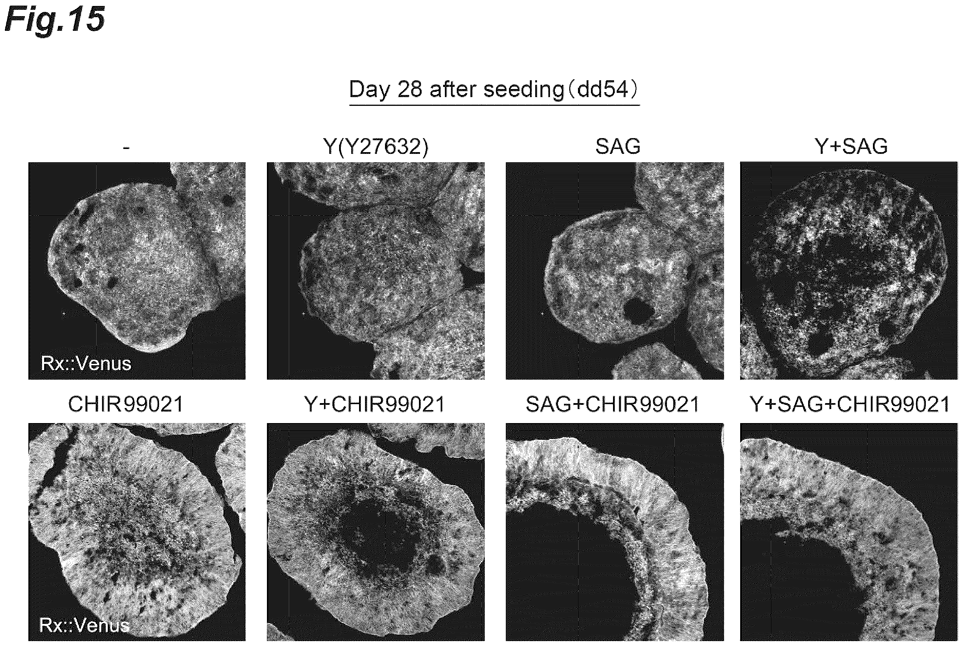

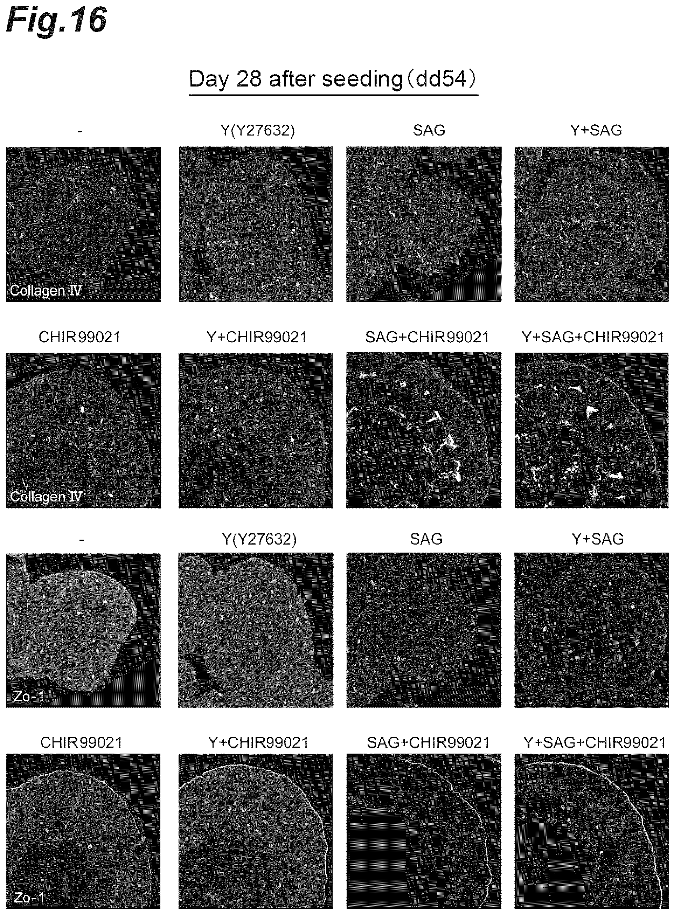

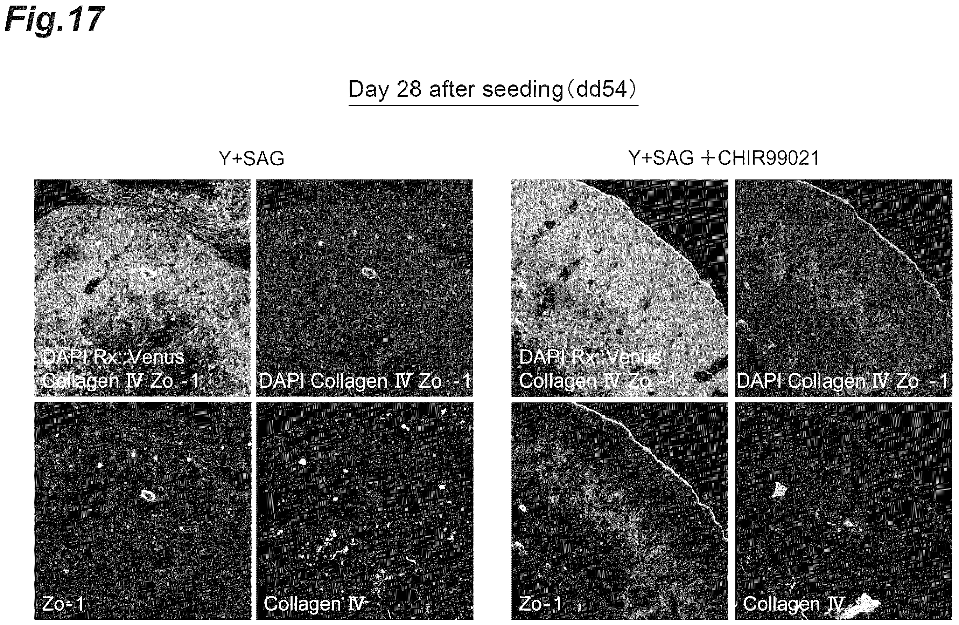

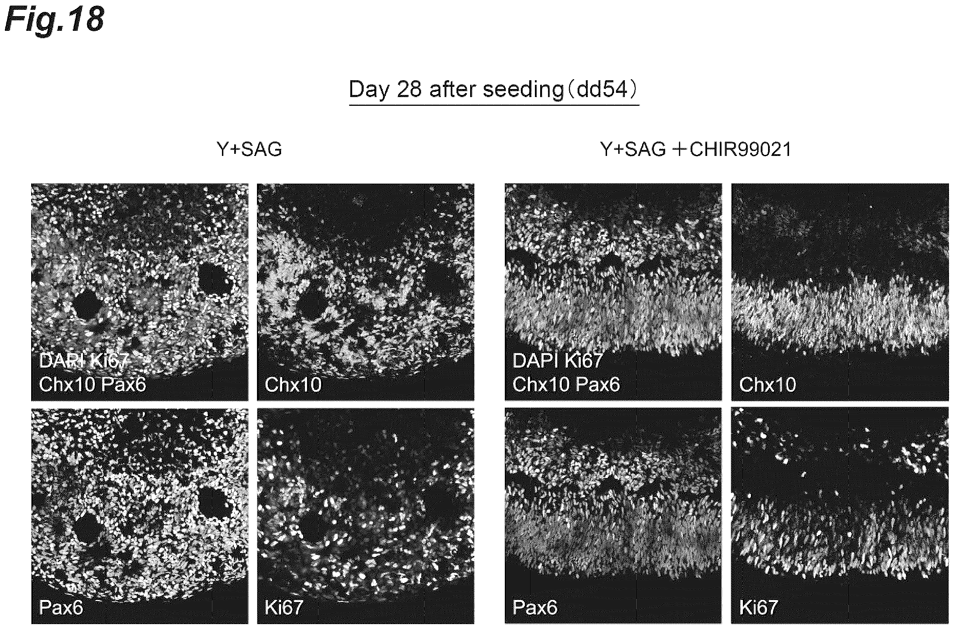

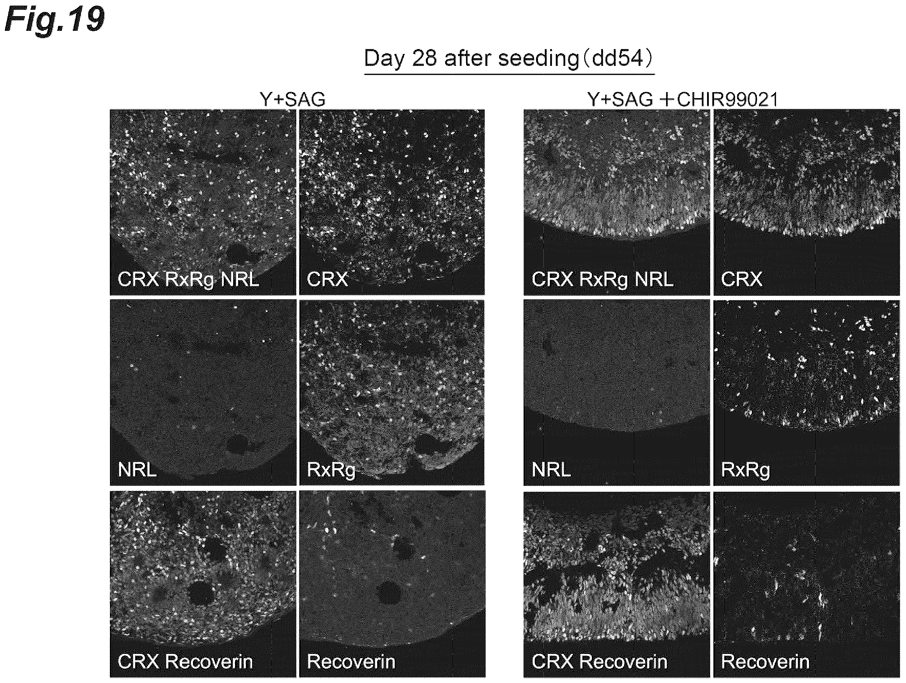

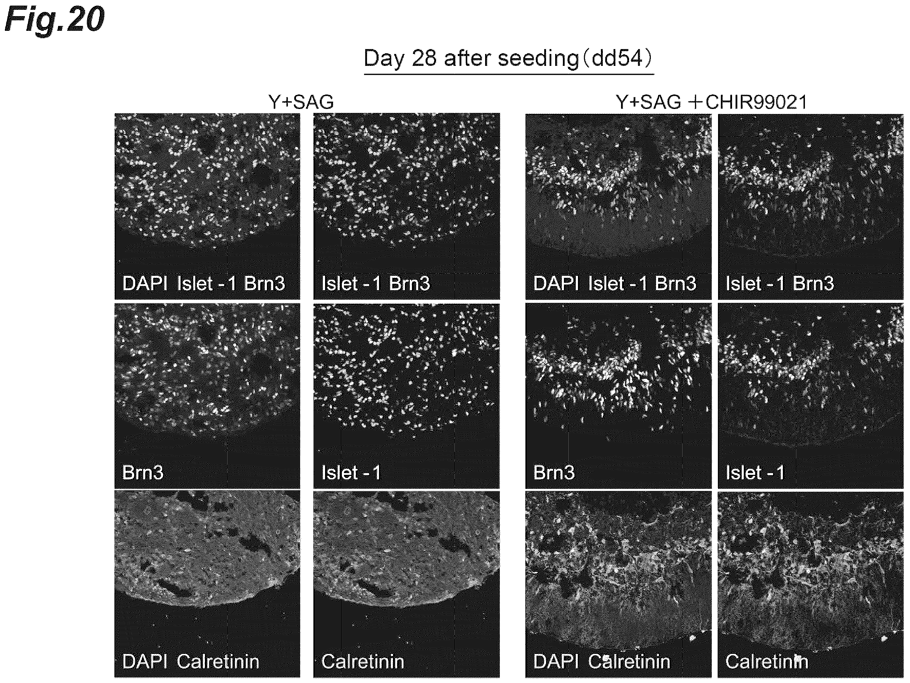

These immunostained sections were observed under a bright field microscope and a fluorescence microscope (BZ-X810 manufactured by Keyence Corp.) and a confocal laser scanning fluorescence microscope (SP-8 manufactured by Leica), and the results are shown in Figures 11 to 20. From Figures 11, 14, 15, and 18, it was confirmed that in the case of adding CHIR99021, a layer structure where Rx:: Venus-positive and Chx10-positive neural retinal progenitor cells were contained was formed on the outermost side of the aggregates. From Figures 12, 16, and 17, it was confirmed that in the case of adding CHIR99021, a Zo-1-positive apical surface and a collagen-positive basal surface were formed on the outermost side and in the inside, respectively, of the aggregates and epithelial tissue having apical and basal polarities was formed. On the other hand, the group that was not supplemented with CHIR99021, particularly, as in Figure 17, was observed to lack a polarity of cells or to exhibit a rosette-like structure. From Figures 19 and 20, it was confirmed that RxR-γ (RxRg)-positive and CRX-positive cone progenitor cells and recoverin-positive and CRX-positive photoreceptor progenitor cells were differentiated, and the differentiation of Islet-1-positive and Brn3-positive retinal ganglion cells and calretinin-positive amacrine cells was also confirmed.

From these results, it was found that retinal tissue as epithelial tissue having an apical-basal polarity can be reorganized and differentiated into a retina by once adding CHIR99021 to a single cell suspension of dispersed retinal cells.

### <Example 4 Timing of differentiation that causes aggregate reforming>

Human ES cells (KhES-1 strain (Non Patent Literature 3)) genetically engineered so as to have Rx:: Venus reporter gene were cultured under feeder-free conditions in accordance with the method described in "Scientific Reports, 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of Day 18, Day 25, Day 40, Day 61, and Day 75 (dd18, dd25, dd40, dd61, dd75) from initiation of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting. These cells were suspended in 100 µL of a serum-free medium containing 10 µM Y-27632 (FUJIFILM Wako Pure Chemical Corp.), 300 nM SAG (Enzo Life Sciences, Inc.) and 3 µM CHIR99021 (FUJIFILM Wako Pure Chemical Corp.) so as to become 2 to 5 × 10⁵ cells per well of a non-cell adhesive 96-well culture plate (trade name: PrimeSurface 96-well V-bottom plate, manufactured by Sumitomo Bakelite Co., Ltd.), and suspension-cultured at 37°C and 5% CO₂.

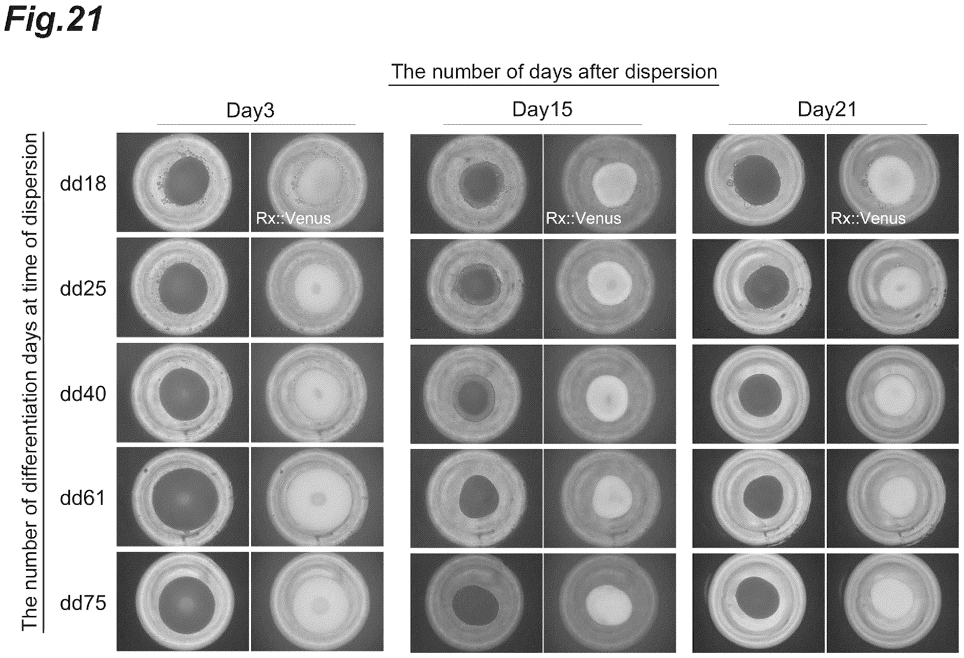

Results of observing the reformed states of the aggregates on Culture Day 3, Day 15, and Day 21 for aggregate reforming under a bright field microscope and a fluorescence microscope are shown in Figure 21. From Figure 21, it was confirmed that aggregates were also reformed and a layer structure were formed, in the cell aggregates at any number of differentiation days of dd18, dd25, dd40, dd61, and dd75. The characteristics of Rx:: Venus-positive retinal tissue were observed to be maintained (Figure 21).

From these results, it was found that aggregates can be reformed by adding Y-27632, SAG and CHIR99021 to retinal tissue at any stage of differentiation.

### <Example 5 Reaggregate formation of brain organoid>

Human ES cells (KhES-1 strain (Non Patent Literature 3)) genetically engineered so as to have Rx:: Venus reporter gene were cultured under feeder-free conditions in accordance with the method described in "Scientific Reports, 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

Day 3 from initiation of the suspension culture, 50 µL of a medium was used which did not contain Y-27632 or SAG and contained exogenous human recombinant BMP4 (trade name: Recombinant Human BMP-4, manufactured by R&D Systems, Inc.) at a final concentration of 1.5 nM. In order to prepare brain organoid, a group that was not supplemented with BMP4 was established. Day 6 or later from initiation of the suspension culture, a half of the medium was exchanged with a culture medium, which did not contain Y27632, SAG or human recombinant BMP4, once every 3 days.

The aggregates of Day 40 from initiation of the suspension culture prepared in this way were washed with PBS, and a neuronal cell dispersion solution (manufactured by FUJIFILM Wako Pure Chemical Corp.) was added. After incubation at 37°C, they were dispersed into single cells by pipetting. These cells were suspended in 100 µL of a serum-free medium containing 10 mM Y-27632 (FUJIFILM Wako Pure Chemical Corp.), 300 nM SAG (Enzo Life Sciences, Inc.) and 3 µM CHIR99021 (FUJIFILM Wako Pure Chemical Corp.) so as to become 5 × 10⁵ cells per well of a non-cell adhesive 96-well culture plate (trade name: PrimeSurface 96-well V-bottom plate, manufactured by Sumitomo Bakelite Co., Ltd.), and suspension-cultured at 37°C and 5% CO₂.

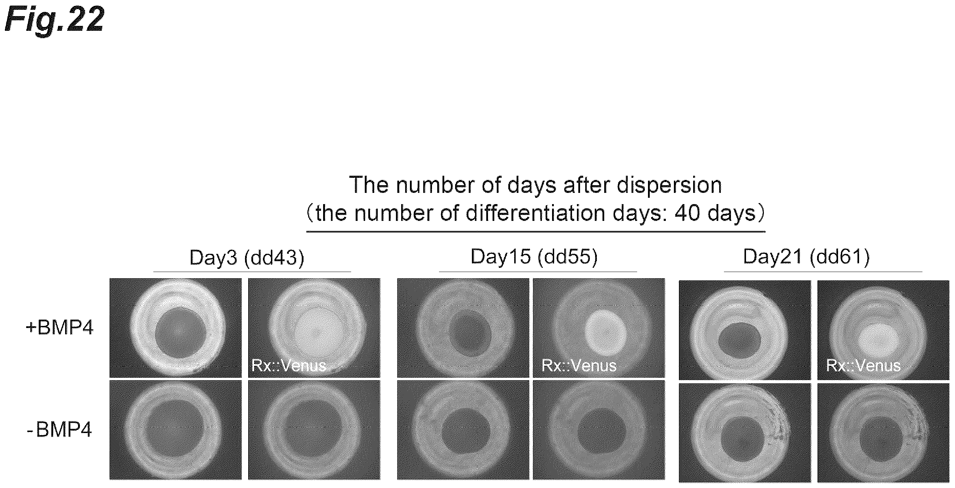

Results of observing the reformed states of the aggregates on Culture Day 3, Day 15, and Day 21 for aggregate reforming under a bright field microscope and a fluorescence microscope are shown in Figure 22. From Figure 22, it was confirmed that aggregates were also reformed and a layer structure were formed, in the Rx:: Venus-negative telencephalon organoid, as in retina organoid.

From these results, it was found that aggregates can be reformed not only in retina organoid but in neuroepithelial tissue such as telencephalon organoid.

### <Example 6 Cryopreservation study>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of Day 31 from initiation of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting.

After dispersion, a non-frozen group was suspended in 100 µL of a serum-free medium containing 10 µM Y-27632 (FUJIFILM Wako Pure Chemical Corp.), 300 nM SAG (Enzo Life Sciences, Inc.) and 3 µM CHIR99021 (FUJIFILM Wako Pure Chemical Corp.) so as to become 2.0 × 10⁵ cells per well of a non-cell adhesive 96-well culture plate (trade name: PrimeSurface 96-well V-bottom plate, manufactured by Sumitomo Bakelite Co., Ltd.), and suspension-cultured at 37°C and 5% CO₂.

After dispersion, as a frozen group, the single cells were suspended at 1.0 × 10⁶ cells/mL using a negative control PBS (Gibco) and the following cryopreservation solutions, Cell Banker 1 (TakaraBio Inc.), Stem Cell Banker (Takara Bio Inc.), CultureSure(R) cryopreserved solution (CultureSure(R) Freezing Medium; FUJIFILM Wako Pure Chemical Corp.), STEMdiff(TM) Neural Progenitor Freezing Medium (STEMCELL Technologies Inc.), StemSure(R) cryopreserved solution (FUJIFILM Wako Pure Chemical Corp.), and Bambanker (Nippon Genetics Co., Ltd.), and cryopreserved at -80°C using BiCell.

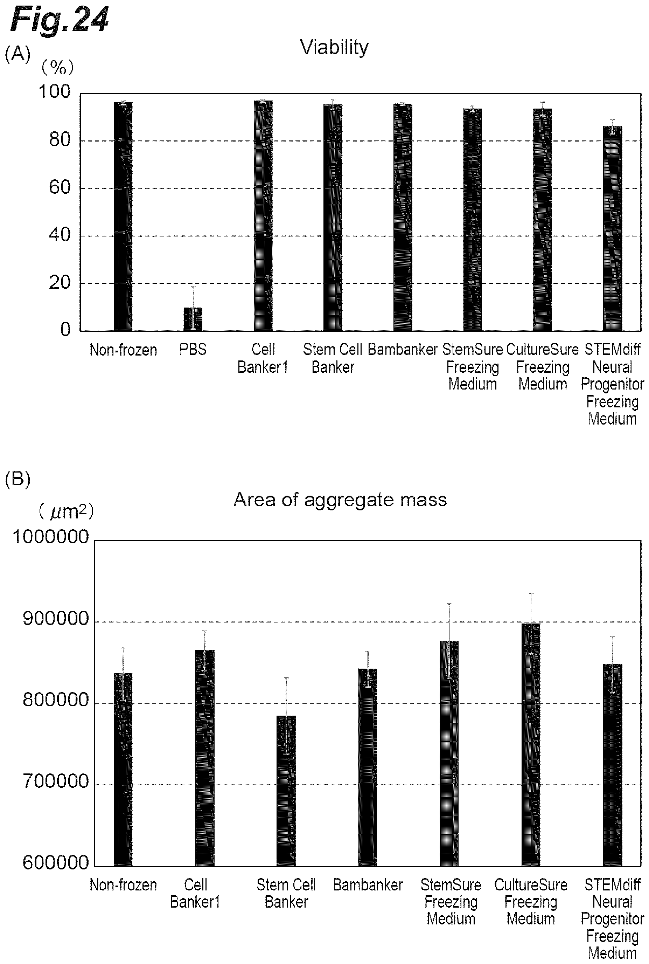

One day after cryopreservation, reconstitution was performed, and viability was confirmed. The viability immediately after reconstitution was drastically reduced in the PBS group, whereas the viability was high in all the cryopreservation solutions and exhibited 90% or more in no way inferior to the non-frozen group, in the cryopreservation solutions other than STEMdiff (Cell Banker 1, Stem Cell Banker, CultureSure, StemSure, Bambanker) (Figure 24(A)).

These cells were suspended in 100 µL of NucTO medium containing 10 µM Y-27632 (FUJIFILM Wako Pure Chemical Corp.), 300 nM SAG (Enzo Life Sciences, Inc.) and 3 µM CHIR99021 (FUJIFILM Wako Pure Chemical Corp.) so as to become 2 × 10⁵ cells per well of a non-cell adhesive 96-well culture plate (trade name: PrimeSurface 96-well V-bottom plate, manufactured by Sumitomo Bakelite Co., Ltd.), and suspension-cultured at 37°C and 5% CO₂.

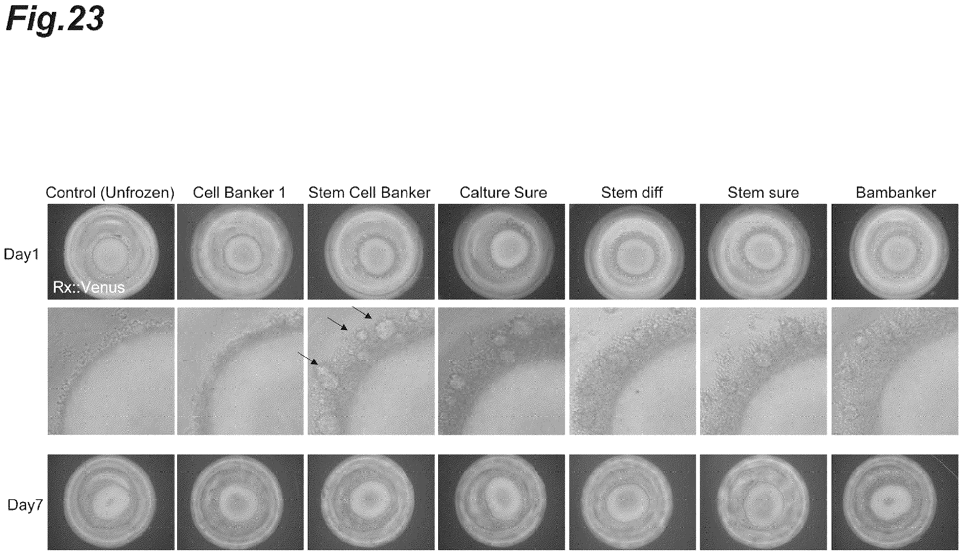

One day after seeding, it was confirmed that aggregates were reformed in no way inferior to the non-frozen group, in all the cryopreservation solutions (Cell Banker 1, Stem Cell Banker, CultureSure, STEMdiff, StemSure, Bambanker) (Figures 23 and 24(B)). Particularly, in Cell Banker 1, small Rx:: Venus-positive cell aggregates present in a neighboring part of the aggregates, which were seen in the other cryopreservation solutions, were not confirmed, and an observation image similar to that of the non-frozen group was obtained (Figure 23).

After culture for aggregate reforming for 7 days, it was found that aggregates were reformed in no way inferior to the non-frozen group, in all the cryopreservation solutions.

From these results, it was found that even if a single cell suspension of retinal cells obtained by once performing dispersion is cryopreserved, aggregates can be reformed by adding Y-27632, SAG, and CHIR99021 after reconstitution, and performing culture.

### <Example 7 Scaffold protein expressed by retina aggregate>

In the studies of Examples 1 to 6, it was found that retinal tissue having a layer structure and having a polarity can be reorganized by dispersing retinal cells, differentiating pluripotent stem cells into retinas to obtain cell aggregates containing retinal cells, dispersing the cell aggregates to obtain a single cell suspension, adding Y-27632 (FUJIFILM Wako Pure Chemical Corp.), SAG (Enzo Life Sciences, Inc.) and CHIR99021 (FUJIFILM Wako Pure Chemical Corp.) at the time of seeding of the single cell suspension, and performing suspension culture. Next, as a study for preparing a wide sheet-shaped retinal tissue, the concept to perform adhesion culture was made. Thus, an extracellular matrix for adhesion culture was studied.

Human ES cells (KhES-1 strain (Non Patent Literature 3)) genetically engineered so as to have Rx:: Venus reporter gene were cultured under feeder-free conditions in accordance with the method described in "Scientific Reports, 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

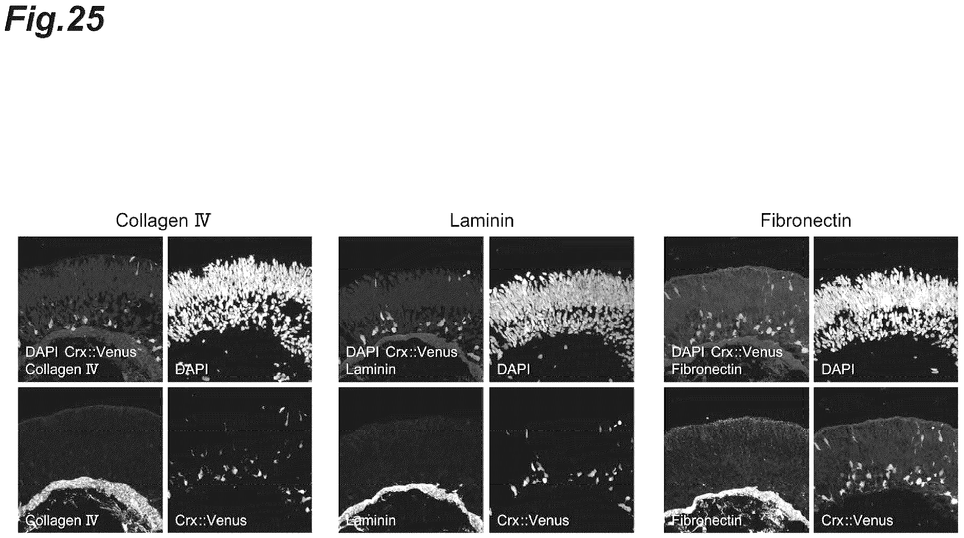

The aggregates of 25 to 35 days after the start of the suspension culture prepared as in Example 1 were fixed in 4% PFA, and frozen sections were prepared. These frozen sections were subjected to immunostaining using DAPI and anti-laminin antibody (trade name: Anti Laminin Antibody, Abeam plc), anti-fibronectin antibody (R&D systems, Inc.), and anti-collagen IV antibody (Abeam plc). These immunostained sections were observed under a confocal laser microscope. As a result, it was confirmed that the aggregates expressed at least laminin, fibronectin, and collagen IV, which are constituents of a basal membrane (Figure 25). Specifically, it was found that in this self-organization culture system, human retinal tissue itself forms a basal membrane and expresses laminin, fibronectin, and collagen IV

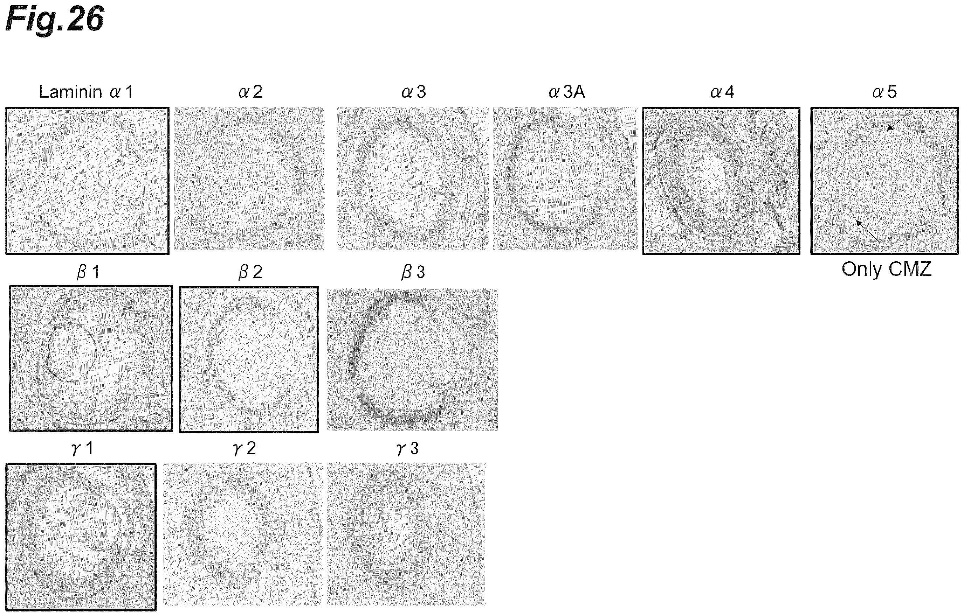

From information of a database on mouse fetal tissue staining dedicated to known basal membranes (MOUSE BASEMENT MEMBRANE BODYMAP; http://dbarchive.biosciencedbc.jp/archive/matrixome/bm/home.html), isoforms of laminin expressed in mouse fetal neural retinal tissue were examined. As a result, it was revealed that as isoforms of laminin, laminin α is 1, 4, and 5, laminin β is 1 and 2, and laminin γ is 1 (Figure 26).

From this result, the possibility was suggested that 511, 521, 411, 421, 111, and 121 (particularly, 511, 521, 411) of laminin in terms of the combination of laminin α, laminin β, and laminin γ are useful as an extracellular matrix suitable for neural retinal tissue.

Specifically, the possibility was suggested that Laminin511, Laminin521, Laminin411, fibronectin, collagen IV, and the like are useful as an extracellular matrix for reforming a sheet-shaped retina sheet by adhesion culture.

### <Example 8 Seeding onto Transwell; extracellular matrix study>

In the studies of Examples 1 to 6, an approach of reforming aggregates containing retinal tissue having a layer structure by suspension culture from a single cell suspension of retinal cells was studied. In Example 7, a candidate of an extracellular matrix for use in adhesion culture was studied. These were combined to study a method for reforming (preparing into a sheet again) a sheet-shaped retinal tissue by adhesion culture from a single cell suspension of retinal cells.

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of Day 16 from initiation of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting.

As an extracellular matrix promoting preparation into a sheet again, Matrigel (Corning, Inc.) and Laminin511-E8 (trade name: iMatrix511, manufactured by Nippi, Inc.) were used. The single cells of the dispersed retinal cells were suspended in 300 µL of a serum-free medium containing 10 µM Y-27632 (FUJIFILM Wako Pure Chemical Corp.), 300 nM SAG (Enzo Life Sciences, Inc.) and 3 µM CHIR99021 (FUJIFILM Wako Pure Chemical Corp.) and seeded so as to become 0.5 to 4 × 10⁵ cells per well of 24-well Transwell (Corning, Inc.) under three conditions given below. 3 days or later from seeding, the medium was replaced with a serum-free medium (gfCDM), which did not contain Y-27632, SAG or CHIR99021, once every 3 to 4 days.
Condition 1: Seeded into 24-well Transwell precoated with Matrigel (Matrigel (Pre coat))
Condition 2: Seeded into 24-well Transwell precoated with Laminin511-E8 (iMatrix511 (Pre coat))
Condition 3: Seeding of cells to non-precoated 24-well Transwell using a culture solution supplemented with Laminin511-E8 (Mix method).

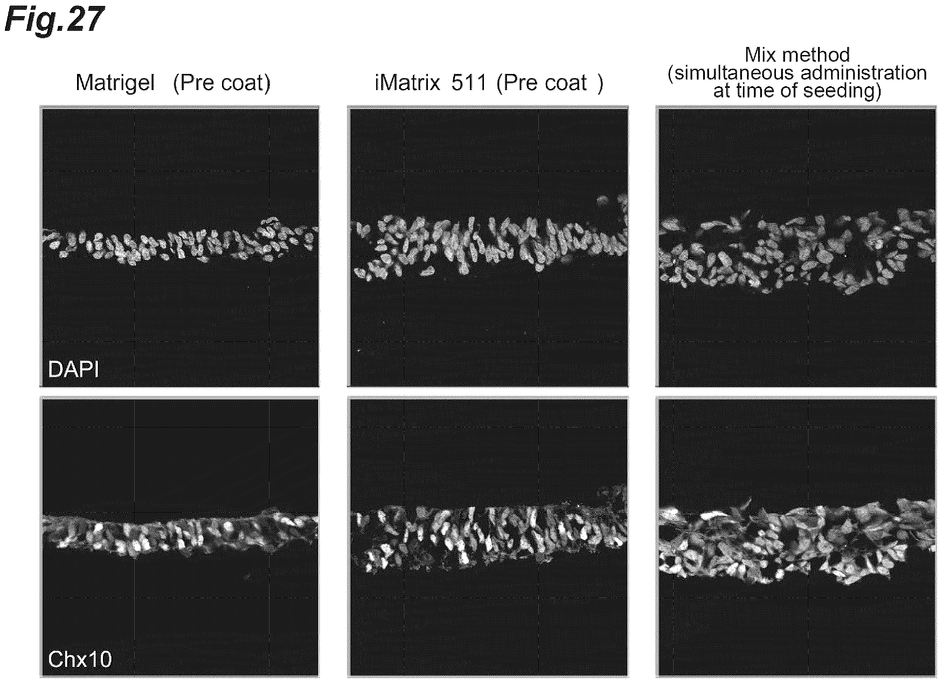

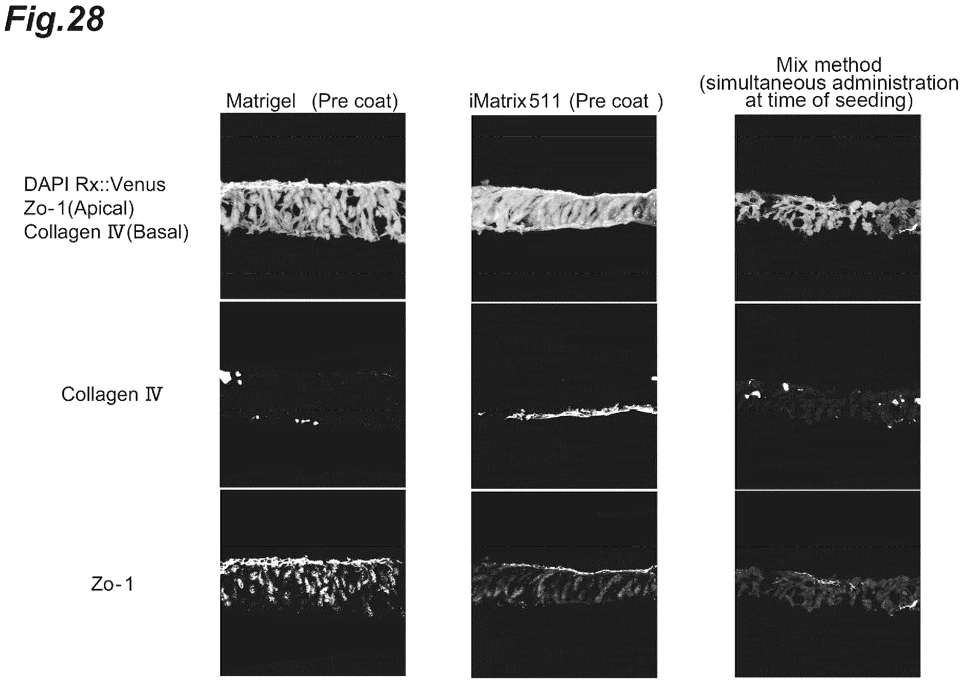

Culture Day 14 or later for aggregate reforming, sheet-shaped cell aggregates (cell sheets) were formed under all the conditions. The sheet-shaped cell aggregates were fixed in 4% PFA, and frozen sections were prepared. These frozen sections were subjected to immunostaining using DAPI and anti-Chx10 antibody (Exalpha Biologicals Inc.), anti-Zo-1 antibody (trade name: Anti Zo-1 Antibody, Invitrogen Corp.), and anti-collagen IV antibody (Abeam plc). These immunostained sections were observed under a confocal laser scanning fluorescence microscope. As a result, it was confirmed that Chx10-positive neural retinal progenitor cells were present in all of Matrigel (Pre coat), iMatrix511 (Pre coat), and Mix method (Figure 27). Under the conditions of Matrigel (Pre coat) and iMatrix511 (Pre coat), Zo-1, a marker for the apical surface (tight junction of epithelial tissue is formed on the apical surface), was confirmed on the upper side (the side in no contact with wells) of the sheet-shaped cell aggregates, and collagen IV, a marker for the basal surface, was confirmed on the lower side (the side in contact with wells) of the sheet-shaped cell aggregates (Figure 28). It was confirmed that sheets of Rx:: Venus-positive retinal progenitor cells having apical and basal polarities were reformed. On the other hand, it was confirmed that a polarity was not formed in Mix method of simultaneously administering an extracellular matrix.

From these results, it was found that a sheet-shaped retinal tissue having an apical-basal polarity can be prepared again (prepared into a sheet again) by coating retinal cells (NR) dispersed into single cells with Laminin511-E8 or Matrigel as a scaffold beforehand.

### <Example 9 Seeding onto Transwell and preparation into sheet again; confirmation of effect of CHIR99021>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of Days 15 to 30 from initiation of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting. After dispersion, the cells were suspended in 300 µL of a serum-free medium containing (1) 10 µM Y-27632 (FUJIFILM Wako Pure Chemical Corp.), (2) 10 µM Y-27632 (FUJIFILM Wako Pure Chemical Corp.) and 300 nM SAG (Enzo Life Sciences, Inc.), (3) 10 µM Y-27632 (FUJIFILM Wako Pure Chemical Corp.) and 5 µM CHIR99021 (FUJIFILM Wako Pure Chemical Corp.), or (4) 10 µM Y-27632 (FUJIFILM Wako Pure Chemical Corp.), 300 nM SAG (Enzo Life Sciences, Inc.) and 3 µM CHIR99021 (FUJIFILM Wako Pure Chemical Corp.) so as to become 8 × 10⁵ cells per well of 12-well Transwell coated with Laminin511-E8, and adhesion-cultured at 37°C and 5%CO₂. 3 days or later from seeding, the medium was replaced with a serum-free medium (NucTO), which did not contain Y-27632, SAG or CHIR99021, once every 3 to 4 days.

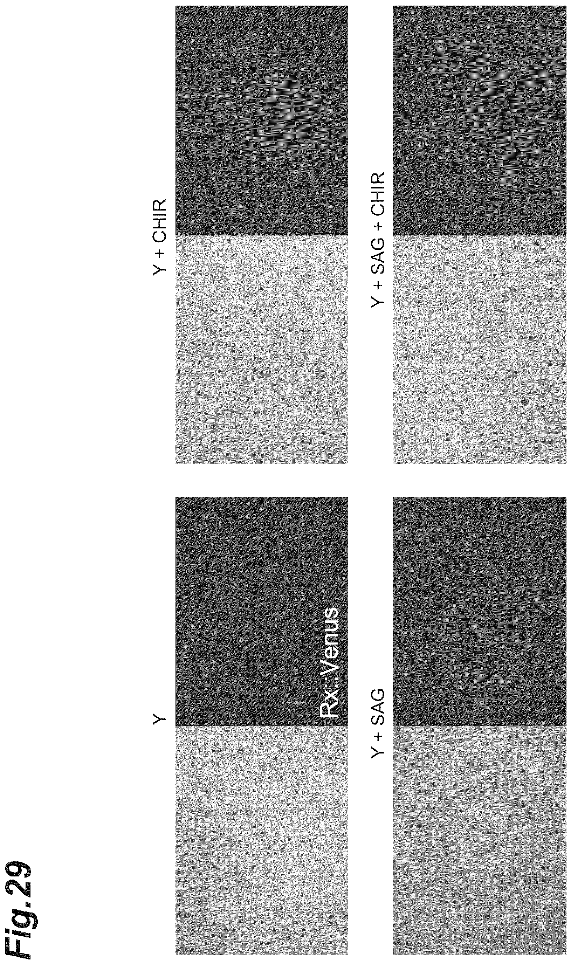

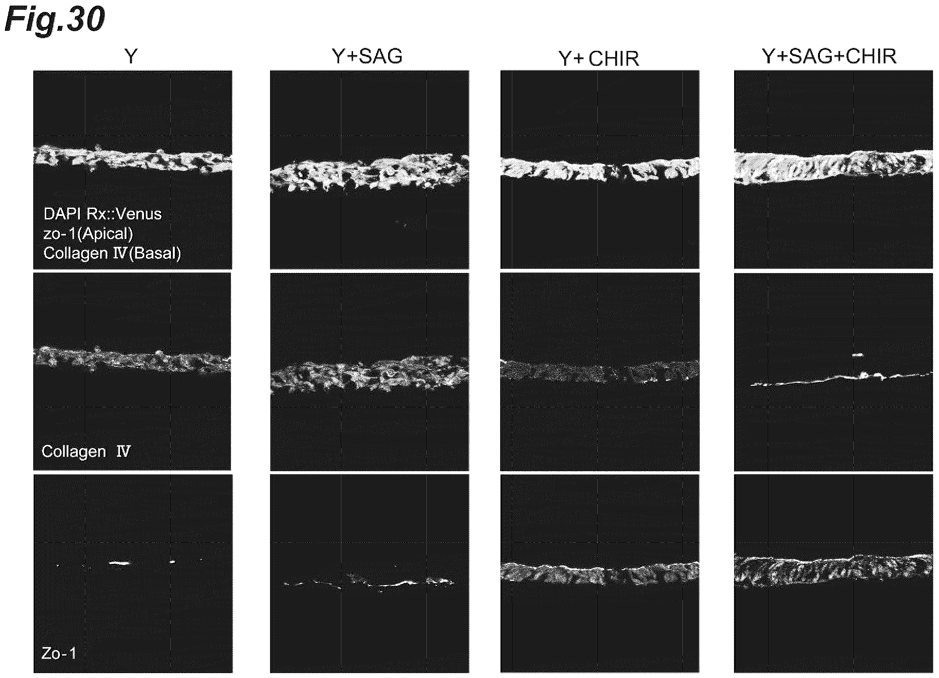

When cell sheets obtained on Culture Day 14 for aggregate reforming were observed, it was confirmed that the cells were engrafted under all the conditions (Figure 29). Accordingly, these cell sheets were fixed in 4% PFA, and frozen sections were prepared. These frozen sections were subjected to immunostaining using DAPI and anti-Zo-1 antibody (trade name: Anti Zo-1 Antibody, manufactured by Invitrogen Corp.) and anti-collagen IV antibody (Abeam plc). These immunostained sections were observed under a confocal laser scanning fluorescence microscope. As a result, it was confirmed that in the case of adding CHIR99021, sheets of Rx:: Venus-positive retinal progenitor cells having apical and basal polarities were formed again. On the other hand, it was confirmed that in the case of adding no CHIR99021, an apical surface was not constituted and a polarity was not formed (Figure 30).

From these results, it was found that a retina sheet having an apical-basal polarity can be prepared again by adding CHIR99021 to retinal cells (NR) dispersed into single cells.

### <Example 10 Seeding onto Transwell and preparation into sheet again; Study on concentration and addition period of CHIR99021>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of 33 days (dd33) after the start of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting. After dispersion, the cells were seeded so as to become 2 to 4 × 10⁵ cells per well of 24-well Transwell coated with Laminin511-E8, and 300 µL of a serum-free medium containing 1 µM, 3 µM or 9 µM CHIR99021 (FUJIFILM Wako Pure Chemical Corp.) in addition to 10 µM Y-27632 (FUJIFILM Wako Pure Chemical Corp.) and 300 nM SAG (Enzo Life Sciences, Inc.) was added, followed by adhesion culture at 37°C and 5% CO₂ for 3 days (Days 0 to 3) or 6 days (Days 0 to 6) or 9 days (Days 0 to 9). 3 days or later from seeding, the medium was replaced once every 3 to 4 days.

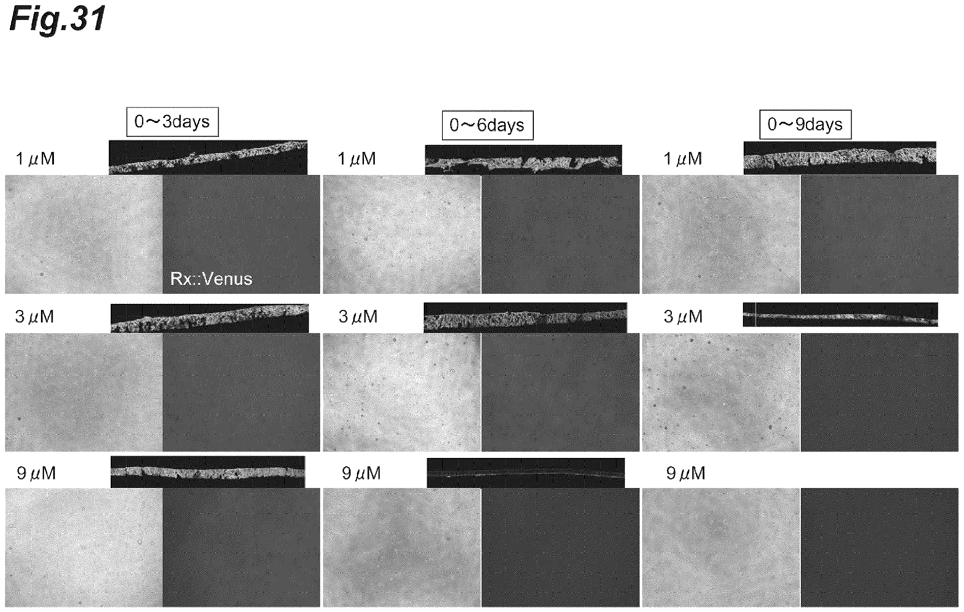

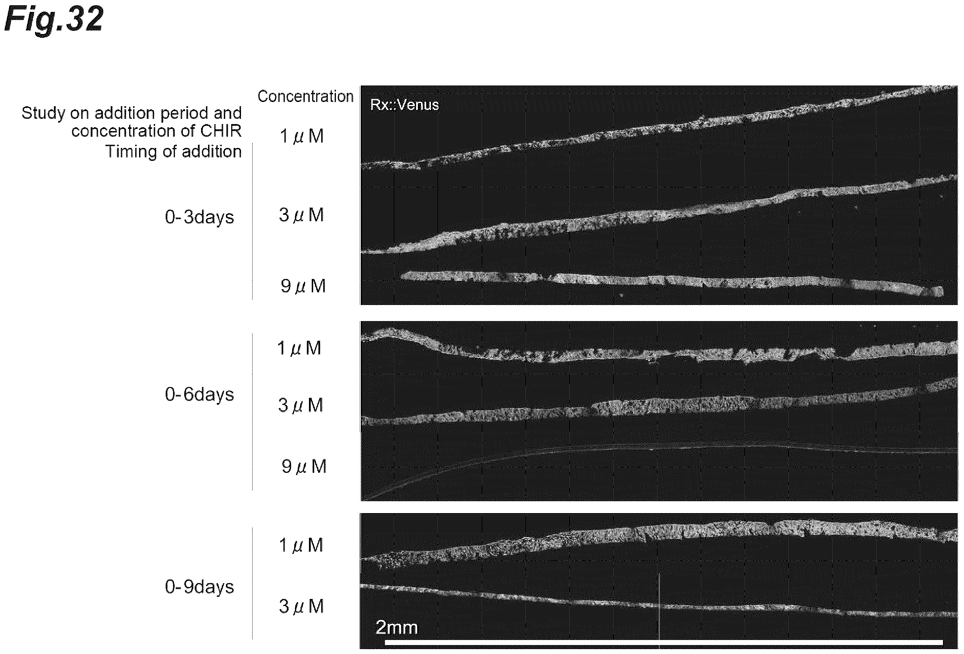

When cell sheets obtained after culture for aggregate reforming for 14 days (Day 14, dd47) were observed, engraftment was observed except for the group supplemented with 9 µM CHIR99021 for 6 days or 9 days (Figure 31). Accordingly, these cell sheets were fixed in 4% PFA, and frozen sections were prepared. These frozen sections were subjected to immunostaining using DAPI. It was confirmed that thick sheets of Rx:: Venus-positive retinal progenitor cells were reformed by adding 1 µM to 9 µM CHIR99021 for 3 days to 9 days (Figure 32).

From these results, it was found that a retina sheet can be prepared again by adding CHIR99021 at at least 1 to 9 µM for 3 to 9 days to retinal cells (NR) dispersed into single cells.

### <Example 11 Study on scaffold at time of seeding onto Transwell>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of 24 days after the start of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting. After dispersion, the cells were seeded so as to become 2.0 × 10⁵ cells per well of 24-well Transwell (1) without coating (Non Coat) or coated with (2) 2 µL of iMatrix511 (trade name, manufactured by Nippi, Inc.), (3) 2 µL of iMatrix411 (trade name, manufactured by Nippi, Inc.), (4) 2 µL of iMatrix211 (trade name, manufactured by Nippi, Inc.), (5) 2 µL Vitronectin (VTN-N) Recombinant Human Protein (Thermo Fisher Scientific Inc.), (6) 2 µL of Cell Start (Thermo Fisher Scientific Inc.), (7) 5 µL of Laminin111, 5 µL of Laminin121, 5 µL of Laminin211, 5 µL of Laminin221, 5 µL of Laminin411, 5 µL of Laminin421, 5 µL of Laminin511, and 5 µL of Laminin521 (BioLamina AB), (8) 5 µL of Laminin332, or (9) 2 µL of Matrigel (Corning, Inc.), and 200 µL of a serum-free medium containing 3 µM CHIR99021 (FUJIFILM Wako Pure Chemical Corp.) and 100 µg/mL FGF8 (FUJIFILM Wako Pure Chemical Corp.) in addition to 10 µM Y-27632 (FUJIFILM Wako Pure Chemical Corp.) and 300 nM SAG (Enzo Life Sciences, Inc.) was added, followed by adhesion culture at 37°C and 5% CO₂ for 3 days. 3 days or later from seeding, the medium was replaced once every 3 to 4 days.

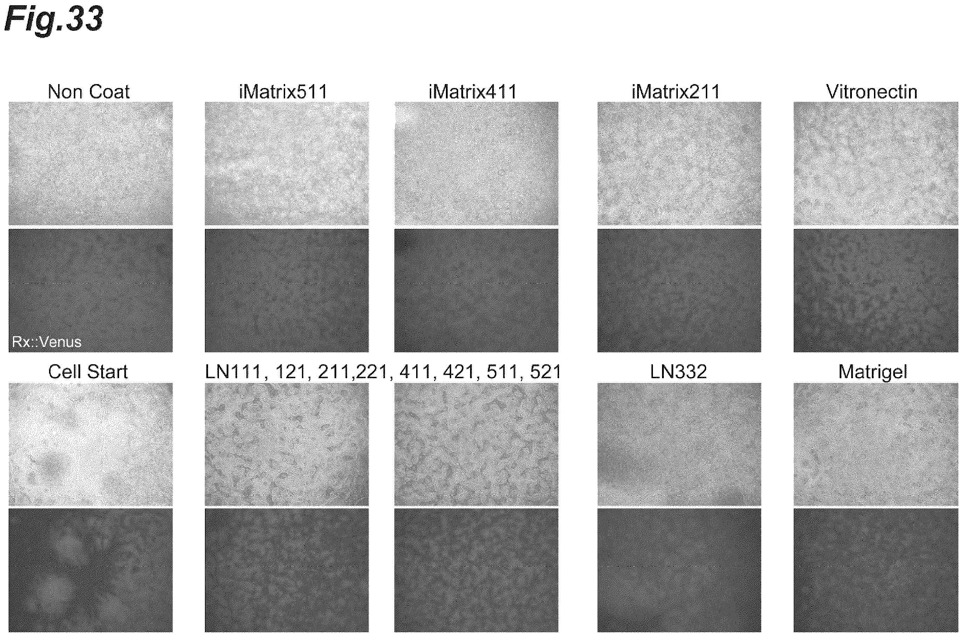

When cell sheets obtained 22 days after seeding were observed, favorable neural retina sheets were obtained in the scaffolds other than Cell Start (Figure 33).

From these results, it was found that in aggregates (NR) dispersed into single cells, a retina sheet can be prepared again using not only iMatrix511 or Matrigel but various scaffold proteins.

### <Example 12 Seeding onto Transwell, preparation into sheet again, and confirmation of retinal differentiation>

Human ES cells (KhES-1 strain (Non Patent Literature 3)) genetically engineered so as to have Crx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of Day 15 from initiation of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting. After dispersion, the cells were suspended in 200 µL of a serum-free medium containing 10 µM Y-27632, 300 nM SAG and 3 µM CHIR99021 so as to become 2.0 × 10⁵ cells per well of 24-well Transwell coated with Laminin511-E8, and cultured at 37°C and 5% CO₂ for 3 days. 3 days or later from seeding, the medium was replaced with a serum-free medium (NucTO), which did not contain Y-27632, SAG or CHIR99021, once every 3 to 4 days.

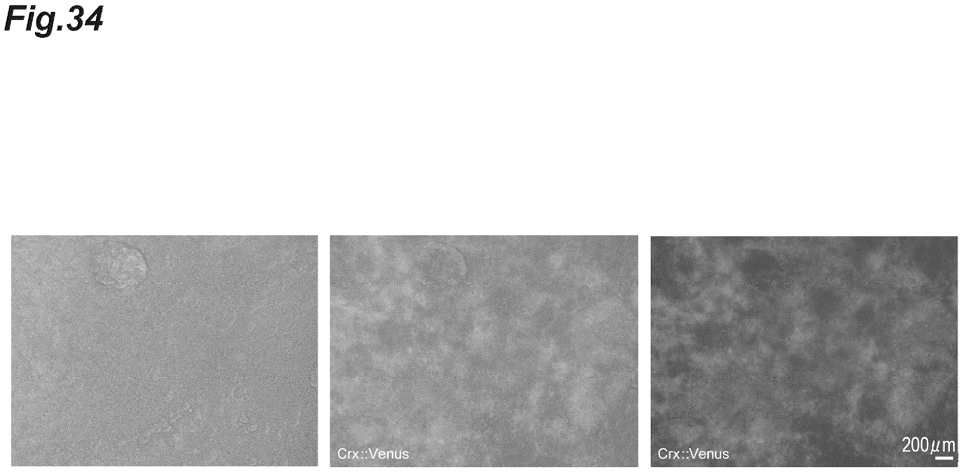

When cell sheets obtained 44 days after seeding (Day 44, dd59) were observed, Crx:: Venus-positive photoreceptor cells were observed to be differentiated (Figure 34).

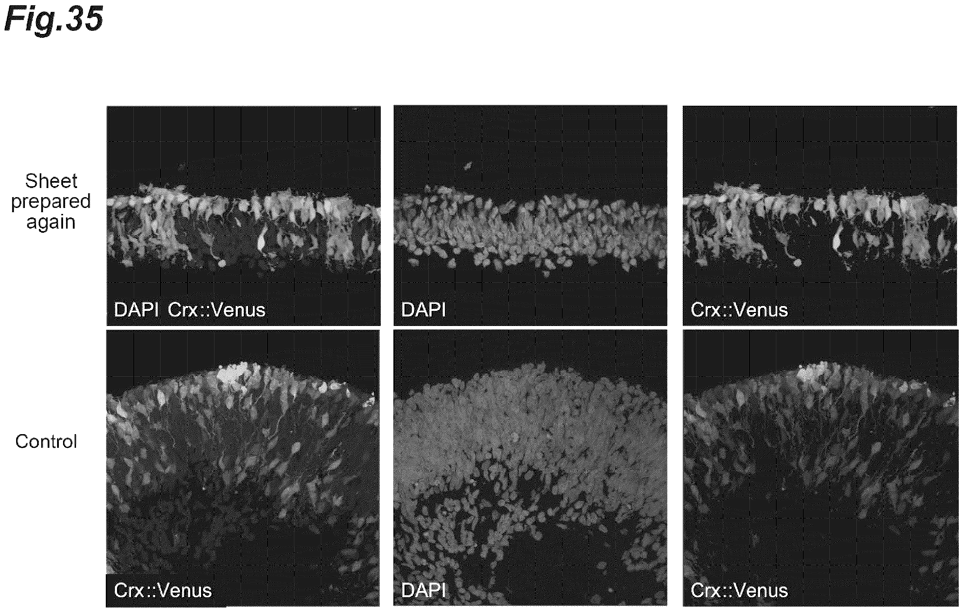

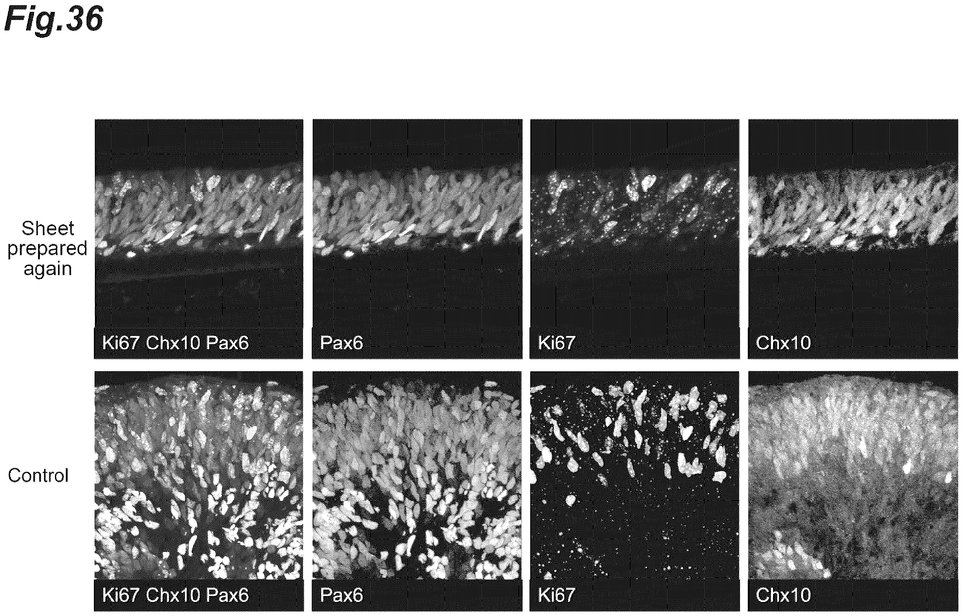

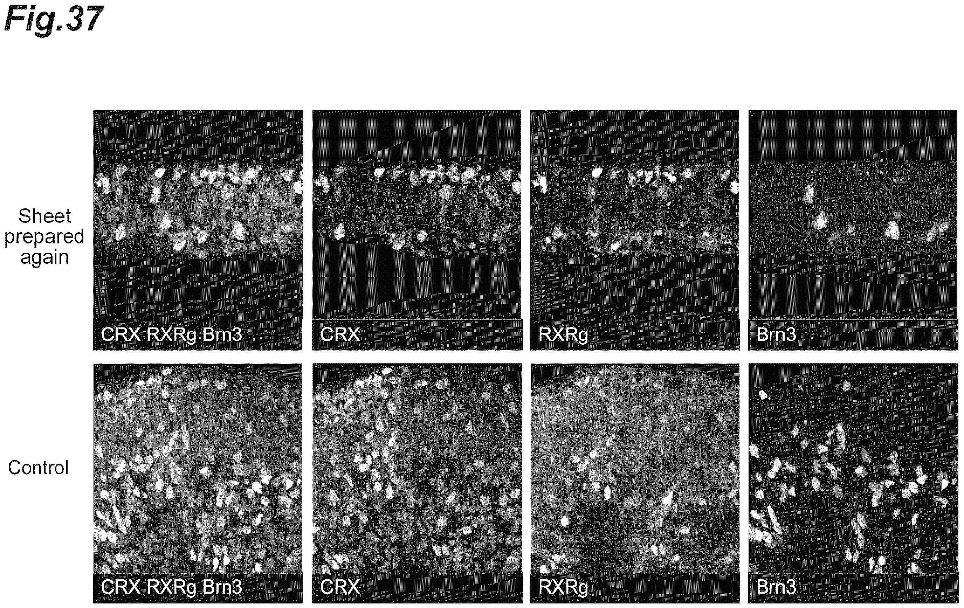

The aggregates of Day 29 from initiation of the suspension culture were used and similarly prepared into a sheet again. Cell sheets of Culture Day 28 for reorganization (Day 67) were fixed in 4% PFA, and frozen sections were prepared. These frozen sections were subjected to immunostaining using DAPI and anti-Ki67 antibody (R&D Systems, Inc.), anti-Chx10 antibody (trade name: Anti CHX10 Antibody, Exalpha Biologicals Inc.), anti-Pax6 antibody (BD Pharmingen), anti-Brn3 antibody (Santa Cruz Biotechnology), anti-RxR-γ (RxRg) antibody (Spring Bioscience Corp.), and anti-Crx antibody (Abnova Corp.). As a control, a 3D retina (cell aggregates) of dd70 that was not planarized (prepared into a sheet again) was also subjected to staining (Figures 35 to 37).

The cell sheets of Day 57 were fixed in 4% PFA and then subjected to whole immunostaining without preparing sections. Immunostaining was performed using DAPI and anti-Chx10 antibody (trade name: Anti CHX10 Antibody, Exalpha Biologicals Inc.), anti-Sox2 antibody (BD Pharmingen), anti-Ki67 antibody (Becton, Dickinson and Company), anti-Pax6 antibody (BioLegend, Inc.), anti-Brn3 antibody (Santa Cruz Biotechnology), anti-TUJ1 antibody (Merck Millipore), anti-Islet-1 antibody (R&D Systems, Inc.), anti-Crx antibody (Abnova Corp.), anti-recoverin antibody (Proteintech Group, Inc.), anti-Zo-1 antibody (Invitrogen Corp.), and anti-collagen IV antibody (Abeam plc).

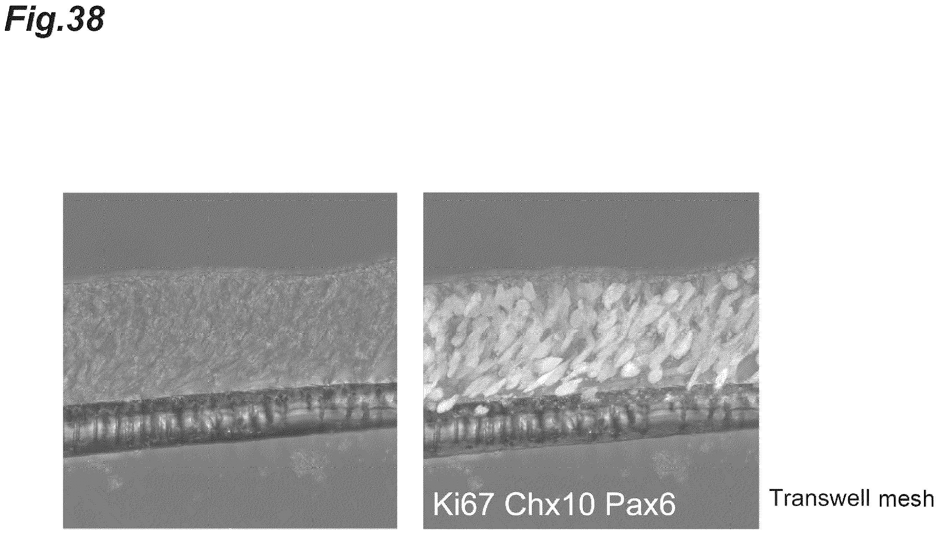

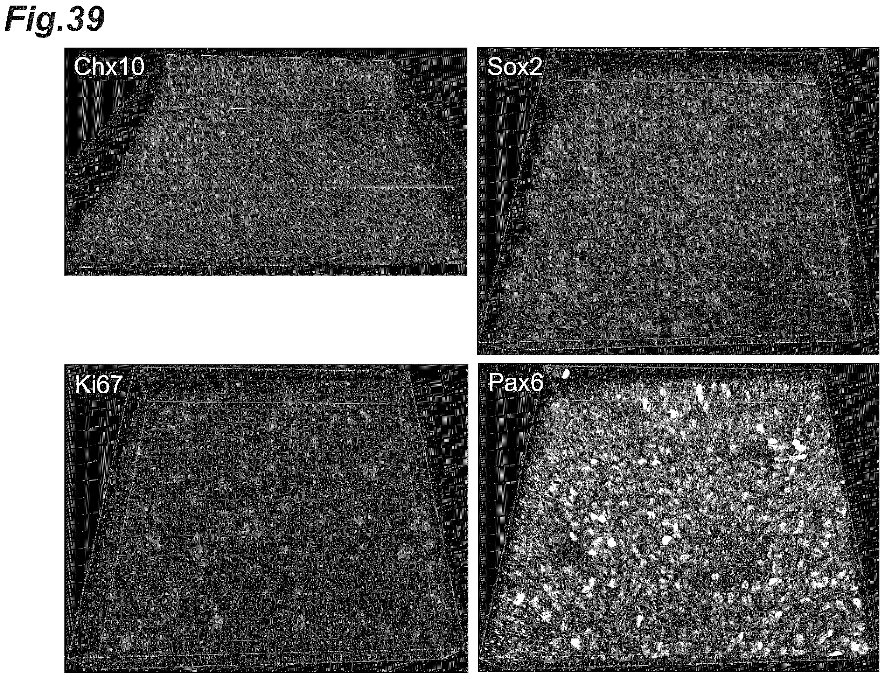

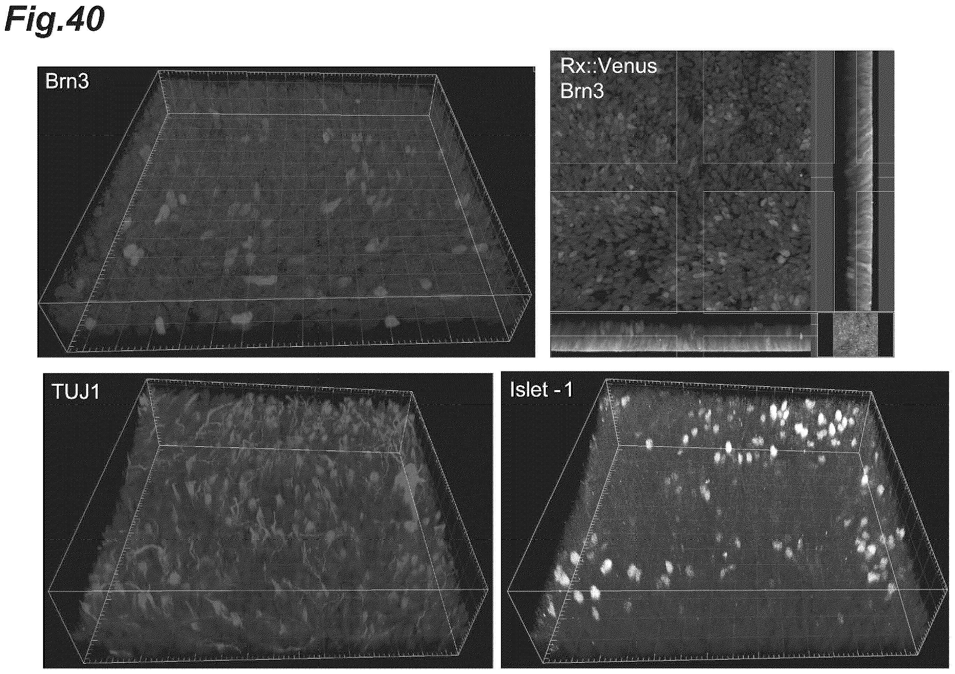

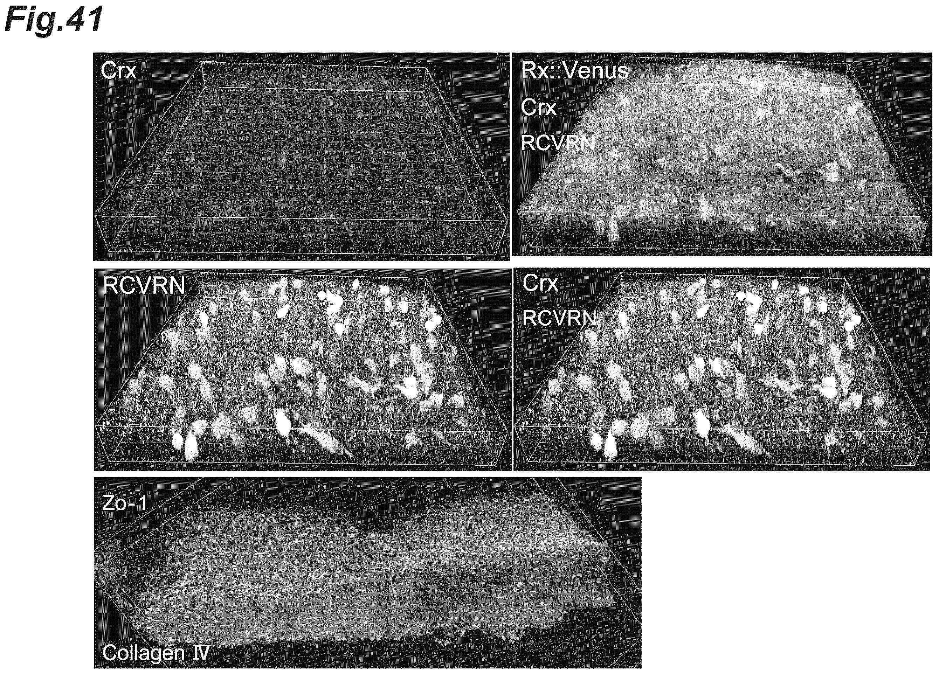

As a result, it was confirmed that Chx10-positive, Pax6-positive and Ki67-positive retinal progenitor cells, Bm3-positive retinal ganglion cells, RxRg-positive, Crx:: Venus-positive and Crx-positive cone cell progenitor cells were differentiated in no way inferior to the control 3D retina. When a positional relationship with Transwell was observed through transmitted light, it was confirmed that retina sheets were formed on Transwell and strongly Pax6-positive and Chx10-negative retinal ganglion cells localized on the basal side were localized on the Transwell side (Figure 38). When the whole was observed under a confocal microscope without preparing sections, various retina cells having apical and basal polarities were differentiated according to the polarities (Figures 39, 40, and 41).

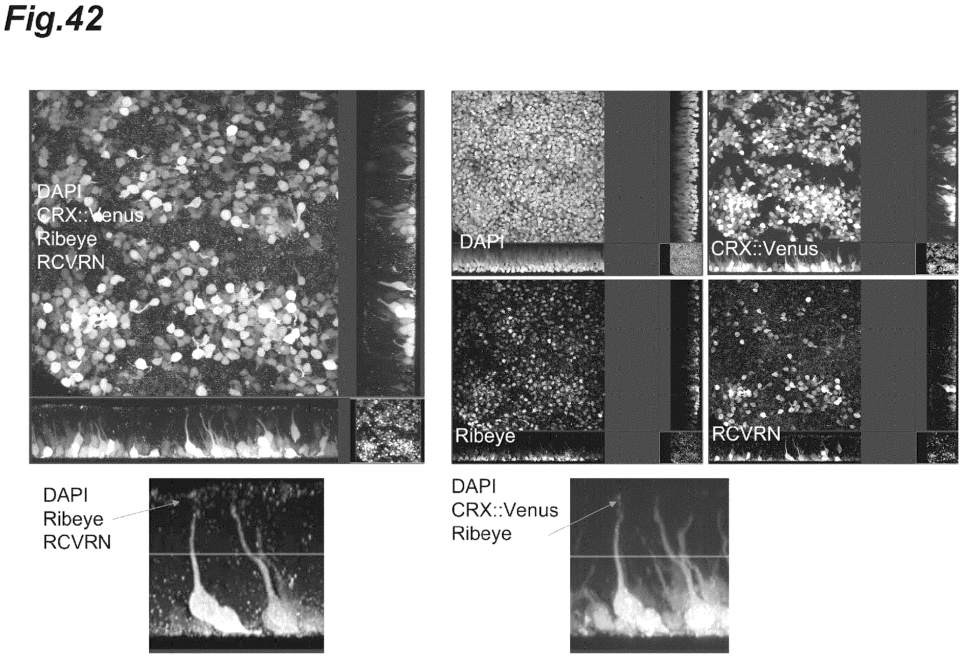

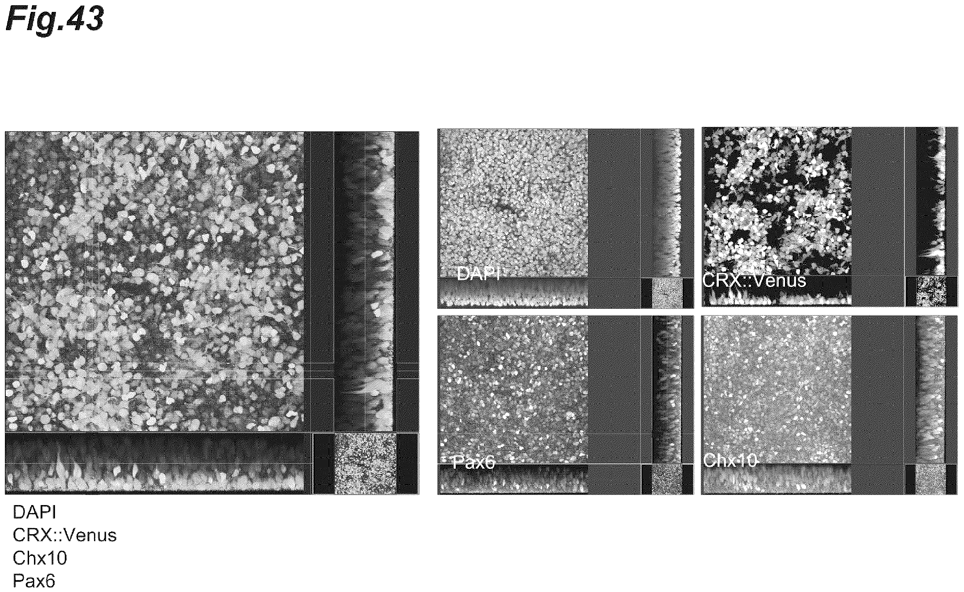

Culture was further performed for a long period, and cell sheets of dd112 were subjected in the state of sheets to immunostaining without preparing sections. When staining was performed using anti-Ribeye antibody (CtBP2, Becton, Dickinson and Company), anti-recoverin antibody (Proteintech Group, Inc.), anti-Pax6 antibody (BioLegend, Inc.) and anti-Chx10 antibody (trade name: Anti CHX10 Antibody, Santa Cruz Biotechnology), the expression of Crx:: Venus and recoverin in the same cells was observed and Ribeye was confirmed to be expressed (Figure 42). Accordingly, it was confirmed that photoreceptor cells differentiated from a retina prepared into a sheet again are matured to the extent that synaptic protein is expressed. It was also confirmed that sheets are formed in a state in which Crx:: Venus was localized on the apical side and Chx10 and Pax6 was localized in the inside (Figure 43).

From these results, the differentiation of retina cells was confirmed in the retina sheets reorganized on Transwell, and a polarity was observed to be maintained.

### <Example 13 Maintenance culture of retinal cell in Rx::Venus strain>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of Day 31 from initiation of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting. After dispersion, the cells were cultured at 37°C and 5% CO₂ for 3 days so as to become 2.0 × 10⁵ cells per well of 12-well Transwell coated with 10 µL of Laminin511-E8, by adding (1) a control, (2) 20 ng/mL FGF2, (3) 20 ng/mL EGF, (4) 100 nM SAG, (5) 1 unit of LIF, (6) 10 ng/mL IGF-1, (7) 100 ng/mL PDGF-AA, (8) 100 ng/mL PDGF-AB, (9) 10 µg/mL GDNF, (10) 20 µg/mL BDNF, (11) 2 µM Pyrintegrin, or (12) 1 µM BMP4 to a culture medium in which N2 was added to 500 µL of serum-free DMEM/F12 containing 10 µM Y-27632, 300 nM SAG and 3 µM CHIR99021. 3 days or later from seeding, the medium was replaced with a serum-free medium (DMEM/F12 supplemented with N2), which did not contain Y-27632, SAG or CHIR99021, once every 3 to 4 days.

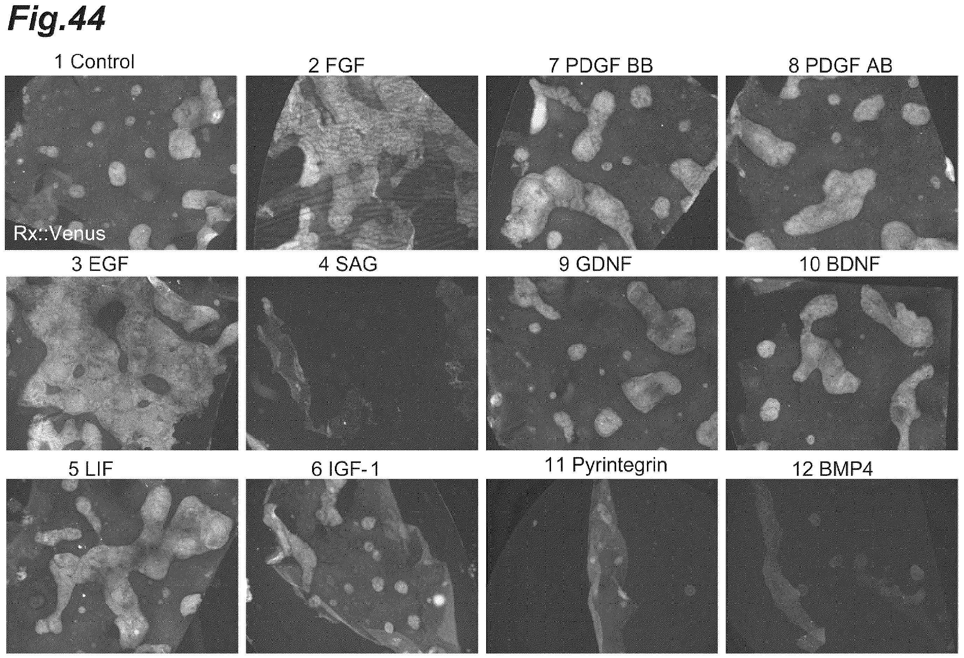

After 34 days, cell sheets were fixed in 4% PFA, and the nuclei were stained using DAPI. Observation was made focusing on the areas of Rx:: Venus-positive cells, and the state of maintenance of retinal cells was studied. When observation was made under a fluorescence microscope (BZ-X810 manufactured by Keyence Corp.), it was confirmed that Rx:: Venus-positive cells were widely maintained by continuously adding 20 ng/mL EGF and FGF2 (Figure 44).

Accordingly, it was found that the addition of EGF or FGF in the reorganization of a retina is effective for the maintenance of Rx:: Venus-positive cells.

### <Example 14 Maintenance culture of retinal progenitor cell at time of planarization culture>

Human ES cells (KhES-1 strain (Non Patent Literature 3)) genetically engineered so as to have Crx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates (NR) of Day 27 from initiation of the suspension culture prepared in Example 1 were washed with PBS, and a neuronal cell dispersion solution (manufactured by FUJIFILM Wako Pure Chemical Corp.) was added. After incubation at 37°C, they were dispersed into single cells by pipetting. After dispersion, the cells were suspended in 500 µL of the following culture medium containing 10 µM Y-27632, 300 nM SAG and 3 µM CHIR99021 and seeded so as to become 8.0 × 10⁵ cells/well of 12-well Transwell coated with 10 µL of Laminin511-E8. In order to study a culture medium that maintained retinal progenitor cells, culture was performed for 1 month by adding 20 ng/mL FGF2 and 20 ng/mL EGF to a culture medium of (1) NeuroCult NS-A (STEMCELL Technologies, Inc.), (2) STEMdiff Neural Progneitor (STEMCELL Technologies, Inc.), (3) StemPro NSC SFM (Thermo Fisher Scientific Inc.), or (4) RHB-A (Takara Bio Inc.). As a control, NucTO medium was established. 3 days or later from seeding, the medium was replaced with a culture medium, which did not contain Y-27632, SAG or CHIR99021, once every 3 to 4 days.

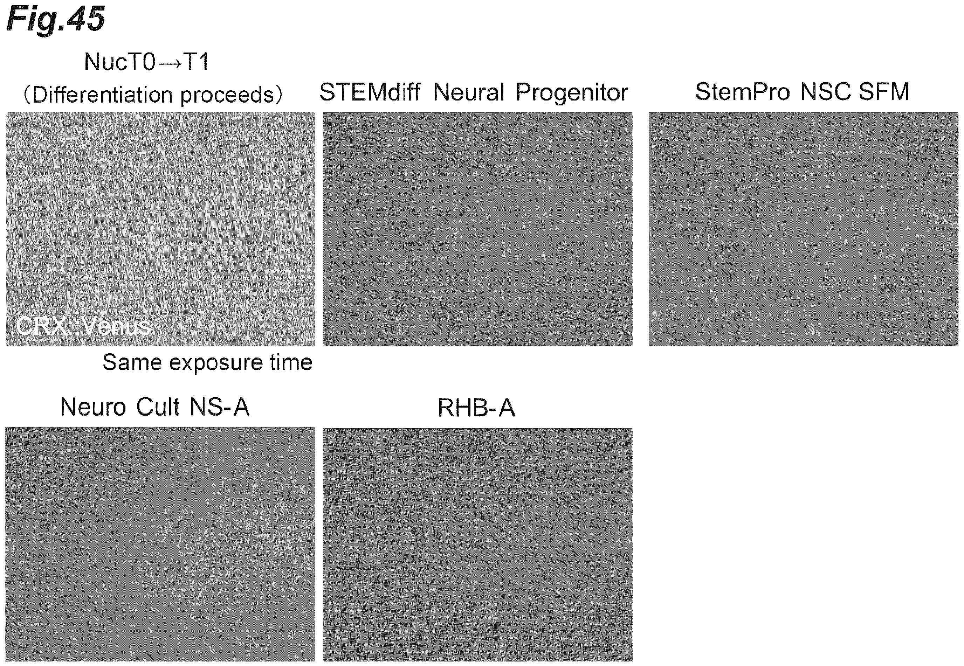

After culture for aggregate reforming for 16 days, when conditions under which differentiation did not occur (conditions where Crx:: Venus did not emit light when observed under a fluorescence microscope) were examined with the photoreceptor progenitor cell marker Crx:: Venus as an index, the NeuroCult NS-A and RHB-A media were found to have an effect of delaying differentiation to some extent (Figure 45).

Accordingly, it was found that the addition of NeuroCult NS-A and RHB-A media and EGF or FGF in preparation into a sheet again has an effect of maintaining retinal progenitor cells and delaying differentiation.

### <Example 15 Culture on collagen gel>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

Collagen gel was prepared using beMatrix low-endotoxin collagen solution (Nitta Gelatin Inc.). As specific operation, collagen AT, 5 × DME, and a buffer solution for reconstitution were mixed at a ratio of 7:2:1, and a mesh of Transwell was coated and incubated at 37°C for 30 minutes in a 5% CO₂ incubator. After incubation, a culture medium was added to the inside and the outside of the insert.

The aggregates (NR) of Day 26 from initiation of the suspension culture prepared in Example 1 were washed with PBS, and a neuronal cell dispersion solution (manufactured by FUJIFILM Wako Pure Chemical Corp.) was added. After incubation at 37°C, they were dispersed into single cells by pipetting. After dispersion, the cells were seeded at 8.0 × 10⁵ cells/well onto the collagen gel of 12-well Transwell. For the first 3 days, culture was performed in the presence of 10 µM Y-27632, 300 nM SAG and 3 µM CHIR99021. 3 days or later from seeding, the medium was replaced with a serum-free medium (NucTO medium), which did not contain Y-27632, SAG or CHIR99021, once every 3 to 4 days.

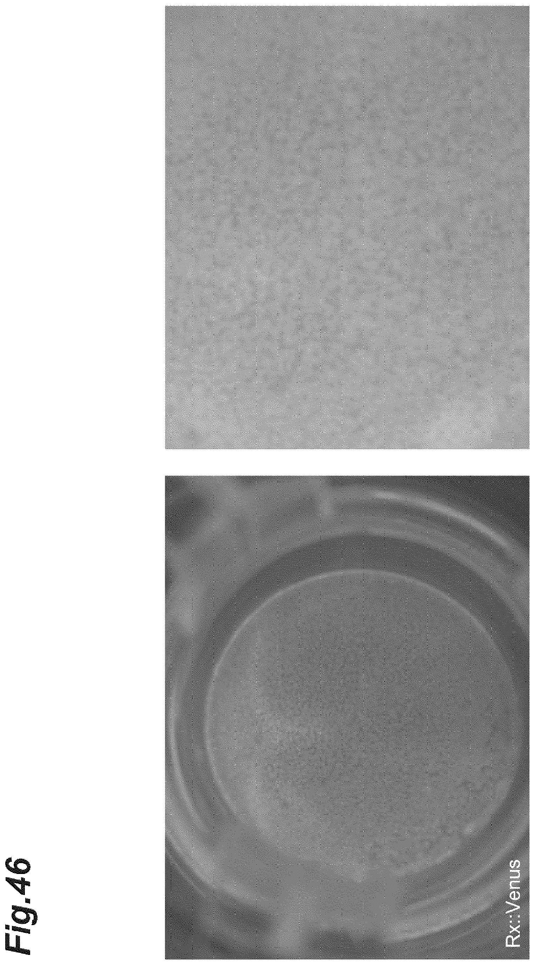

After culture for aggregate reforming for 33 days, when the degree of preparation into a sheet again was observed under a stereo microscope with Rx:: Venus as an index, it was found that Rx:: Venus-positive cells were present at the front on the collagen gel of Transwell (Figure 46).

Accordingly, it was found that preparation into a sheet again is also achieved on collagen gel by adding Y-27632 and SAG and CHIR99021.

### <Example 16 Transplantation study>

Human ES cells (KhES-1 strain (Non Patent Literature 3)) genetically engineered so as to have Crx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates (NR) of Day 26 were washed with PBS on the collagen gel prepared as in Example 15, and a neuronal cell dispersion solution (manufactured by FUJIFILM Wako Pure Chemical Corp.) was added. After incubation at 37°C, they were dispersed into single cells by pipetting. After dispersion, the cells were seeded at 8.0 × 10⁵ cells/well onto the collagen gel of 12-well Transwell. For the first 3 days, culture was performed in the presence of 10 µM Y-27632, 300 nM SAG and 3 µM CHIR99021. 3 days or later from seeding, the medium was replaced with a serum-free medium (NucTO medium), which did not contain Y-27632, SAG or CHIR99021, once every 3 to 4 days.

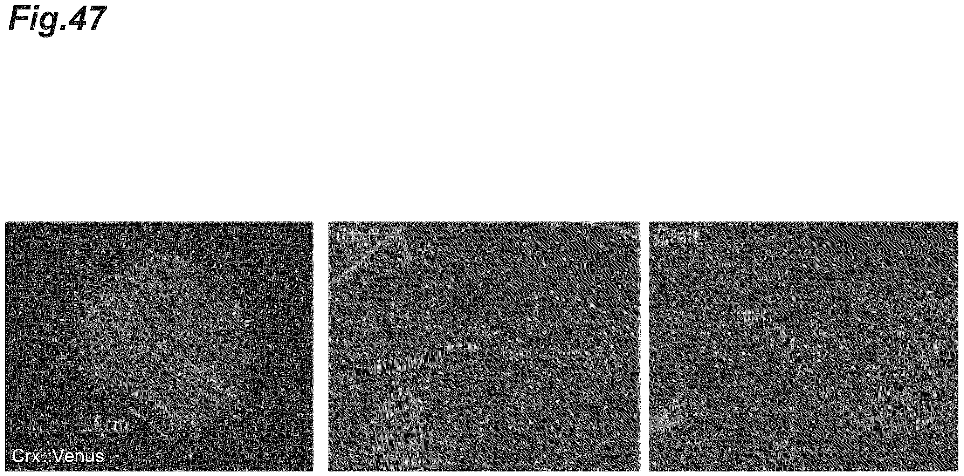

Retina cell sheets of Culture Day 37 and Day 44 for aggregate reforming and Differentiation Day 63 and Day 70 were treated with collagenase (F. Hoffmann-La Roche, Ltd.) at 37°C for 30 minutes and detached. Strips of the detached retina sheets were dissected using tweezers and scissors to prepare grafts for transplantations having a length on the order of 1.8 cm (Figure 47).

The prepared grafts were subretinally transplanted to immunodeficient retina-deficient rats (SD Foxn) using a glass Pasteur pipette.

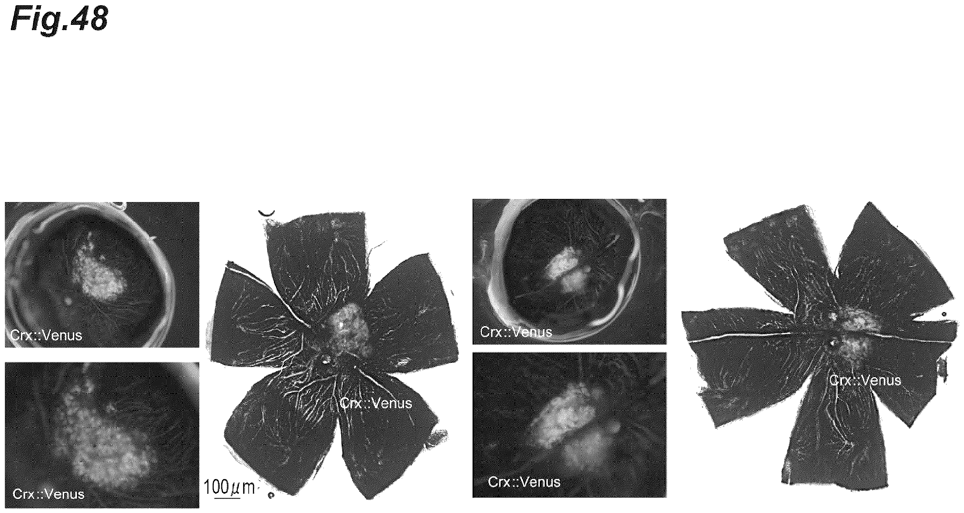

The eyes of the rats 1 year after transplantation were isolated and fixed in 4% PFA. When observation was made under a fluorescence stereo microscope and a fluorescence microscope (Keyence BZ-X810), it was confirmed that Crx:: Venus-positive grafts were engrafted (Figure 48).

Accordingly, it was found that a retina sheet prepared again can be engrafted after transplantation.

### <Example 17 Sorting study>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

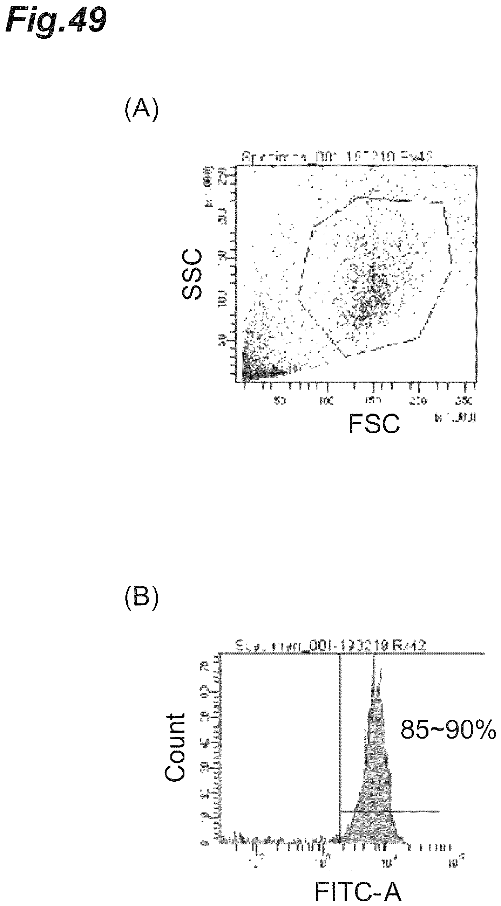

The aggregates of Days 14 to 25 from initiation of the suspension culture prepared in Example 1 were washed with PBS, and a neuronal cell dispersion solution (manufactured by FUJIFILM Wako Pure Chemical Corp.) was added. After incubation at 37°C, they were dispersed into single cells by pipetting, and a target cell population was gated with FSC and SSC using Cell Sorter ARIAII (Becton, Dickinson and Company). Then, Rx::Venus-positive fractions were separated (Figure 49).

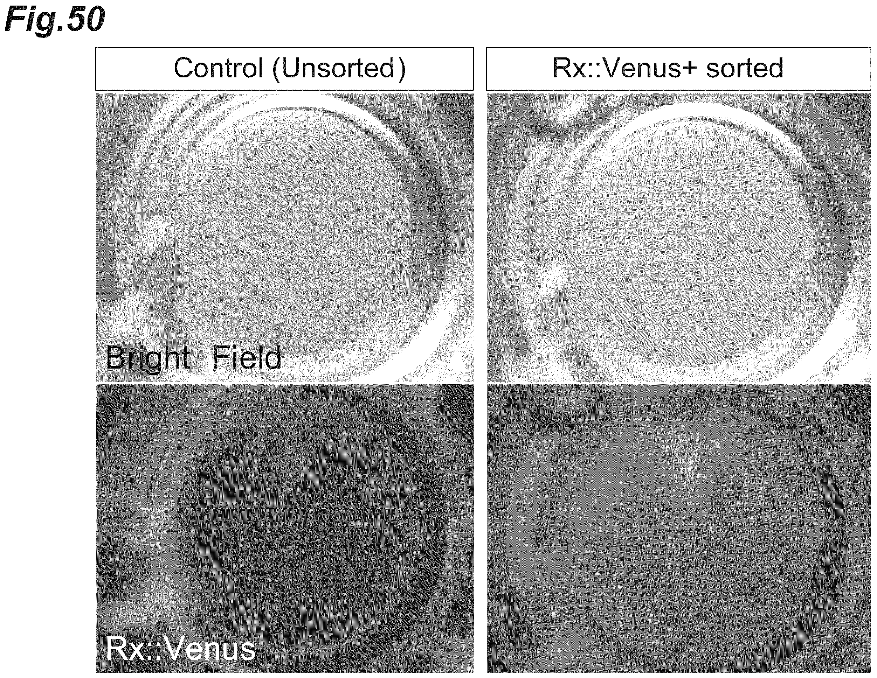

The separated cells were seeded at 1.6 to 8.0 × 10⁵ cells per well onto 12-well Transwell (Corning, Inc.) coated with Laminin511-E8. As a control, unsorted sheets were also prepared. For the first 3 days, culture was performed in the presence of 10 µM Y-27632, 300 nM SAG and 3 µM CHIR99021. 3 days or later from seeding, the medium was replaced with a serum-free medium (NucTO medium), which did not contain Y-27632, SAG or CHIR99021, once every 3 to 4 days. Day 26 after seeding, observation was made under a fluorescence stereo microscope (Figure 50).

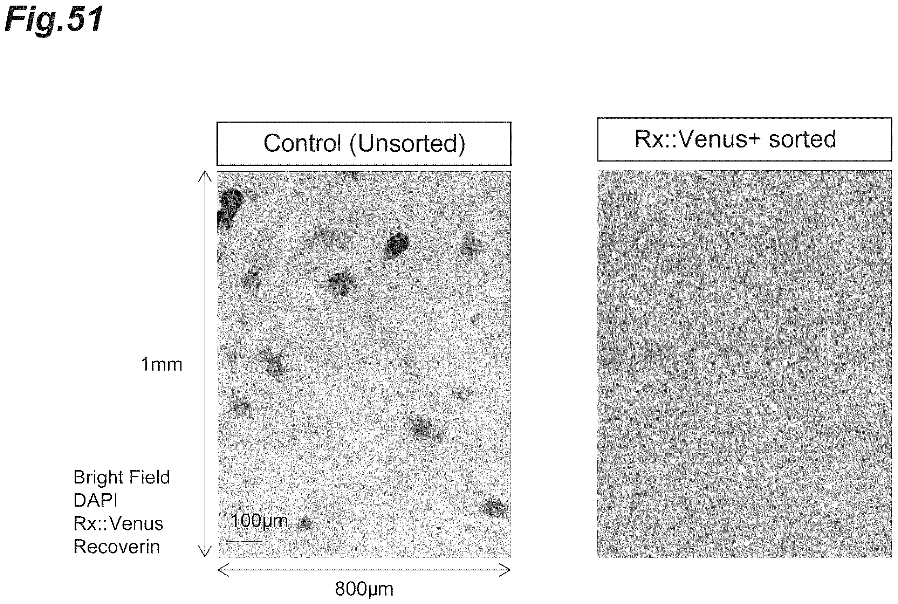

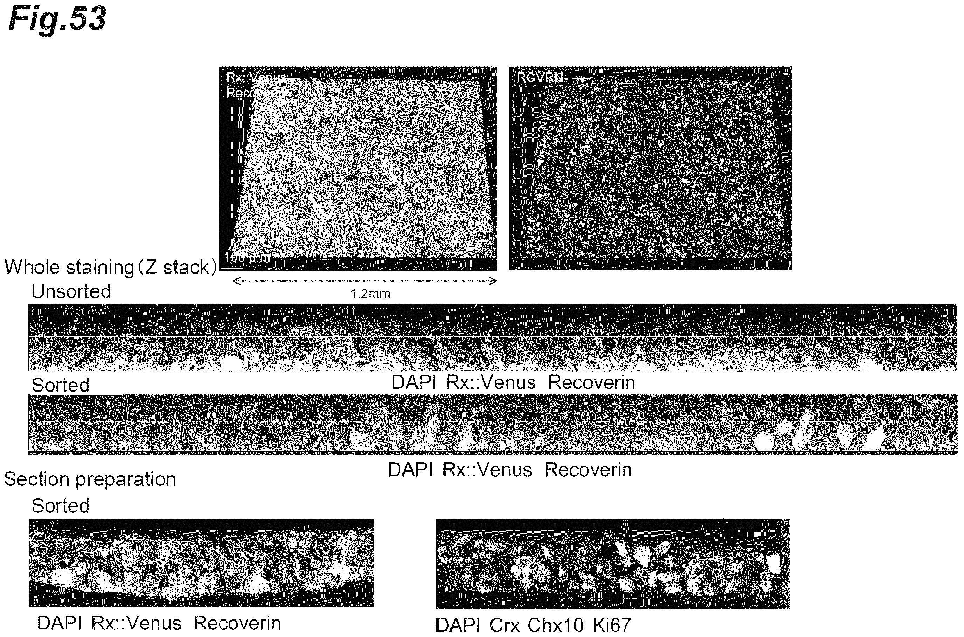

Cell sheets purified with Rx:: Venus by sorting on Differentiation Day 82 were fixed in 4% PFA and then subjected in the state of sheets to immunostaining without preparing sections. Immunostaining was performed using DAPI and anti-recoverin antibody (Proteintech Group, Inc.). As a control, the unsorted retina sheets were also subjected to staining (Figures 51, 52, and 53). Then, frozen sections were prepared and subjected to immunostaining using DAPI and anti-Ki67 antibody (Becton, Dickinson and Company), anti-Chx10 antibody (trade name: Anti CHX10 Antibody, Santa Cruz Biotechnology), and anti-CRX antibody (Takara Bio Inc.) (Figure 53).

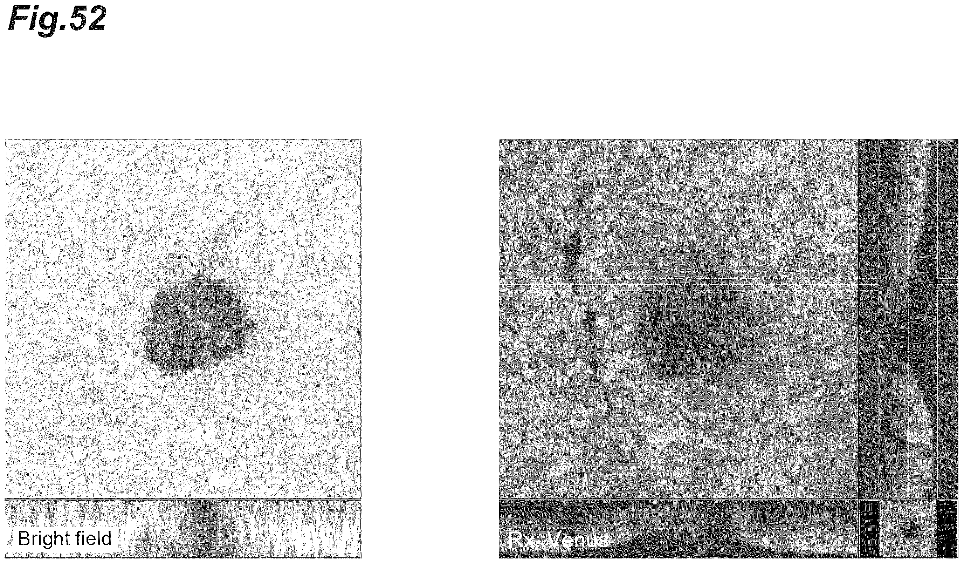

As a result, when the Rx:: Venus-positive fractions were seeded without sorting, a mass of RPE cells was partially observed. It is naturally possible to remove RPE cells because they can be visually confirmed. On the other hand, in the case of sorting the Rx:: Venus-positive fractions, Rx:: Venus-positive retina sheets were observed to be formed. It was further confirmed that a majority of RPE cells were able to be removed (Figures 49 and 50). However, even if sorting was performed, contamination with RPE cells occurred in some times. It is uncertain whether this was because RPE cells failed to be completely removed by sorting or were differentiated after sorting. However, it was able to be confirmed that the contaminating RPE cells did not intermingle with neural retinal tissue (Figure 52). It is possible to remove RPE cells because they can be visually confirmed.

It was confirmed that recoverin-positive photoreceptor cells were differentiated in no way inferior to the unsorted retina sheets serving as a control, in the sorted retina sheets (Figures 51 and 53). From Z Stack analysis and immunohistological analysis on the sections, it was confirmed that the layer structure of retinal tissue was reformed, and the presence of Chx10-positive and Ki67-positive retinal progenitor cells and Crx-positive photoreceptor progenitor cells was confirmed (Figure 53).

Accordingly, a majority of RPE cells can be removed by gating a target cell population with FSC and SSC and then performing sorting with Rx:: Venus as an index, and retina cells were observed to be differentiated without problems when prepared into a sheet again.

### <Example 18 Search of factor that maintains property of retina at time of preparation into sheet again>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates (NR) of Day 25 from initiation of the suspension culture prepared in Example 1 were washed with PBS, and a neuronal cell dispersion solution (manufactured by FUJIFILM Wako Pure Chemical Corp.) was added. After incubation at 37°C, they were dispersed into single cells by pipetting, and sorting was carried out with Rx:: Venus as an index using Cell sorter ARIAII (Becton, Dickinson and Company). The cells after sorting were seeded at 5 × 10⁴ cells per well to a 96-well glass-bottom plate (Greiner Bio-One International GmbH) coated with Laminin511-E8 using Easy iMatrix, by adding Y-27632, CHIR99021, and SAG to 100 µL of NucTO. Simultaneously with seeding, the proteins shown in Table 1 or the low-molecular compounds shown in Table 2 were added.

**[Table 1]**

| | 1 | 2 | 3 | 4 | 5 | 6 | 7 | 8 | 9 | 10 | 11 | 12 |
|---|---|---|---|---|---|---|---|---|---|---|---|---|
| A | PBS/0.1% BSA | | | Endorepellin (ng/ml) | | | IGF-1 (ng/ml) | | | SCF (ng/ml) | | |
| | | | | 100 | 50 | 25 | 40 | 20 | 10 | 100 | 50 | 25 |
| B | Activin A (ng/ml) | | | FGF2 (ng/ml) | | | LIF (Unit) | | | TGF-b1 (ng/ml) | | |
| | 100 | 50 | 25 | 40 | 20 | 10 | x500 | x1000 | x2000 | 100 | 50 | 25 |
| C | BDNF (ng/ml) | | | FGF4 (ng/ml) | | | NGF (ng/ml) | | | TGF-b2 (ng/ml) | | |
| | 100 | 50 | 25 | 100 | 50 | 25 | 40 | 20 | 10 | 100 | 50 | 25 |
| D | BMP2 (ng/ml) | | | FGF8 (ng/ml) | | | PDGF-AB (ng/ml) | | | Thbs1 (ng/ml) | | |
| | 100 | 50 | 25 | 100 | 50 | 25 | 100 | 50 | 25 | 100 | 50 | 25 |
| E | BMP4 (ng/ml) | | | GDF7 (ng/ml) | | | PDGF-BB (ng/ml) | | | Thbs 2 (ng/ml) | | |
| | 100 | 50 | 25 | 100 | 50 | 25 | 100 | 50 | 25 | 100 | 50 | 25 |
| F | BMP7 (ng/ml) | | | GDF11 (ng/ml) | | | PEDF (ng/ml) | | | SAG (ng/ml) | | |
| | 100 | 50 | 25 | 100 | 50 | 25 | 100 | 50 | 25 | 100 | 50 | 25 |
| G | CXCL12 (ng/ml) | | | GDF15 (ng/ml) | | | PODN (ng/ml) | | | VEGF (ng/ml) | | |
| | 100 | 50 | 25 | 100 | 50 | 25 | 100 | 50 | 25 | 100 | 50 | 25 |
| H | EGF (ng/ml) | | | GDNF (ng/ml) | | | R-Spondin (ng/ml) | | | Wnt2b (ng/ml) | | |
| | 100 | 50 | 25 | 40 | 20 | 10 | 100 | 50 | 25 | 100 | 50 | 25 |

**[Table 2]**

| | 1 | 2 | 3 | 4 | 5 | 6 | 7 | 8 | 9 | 10 | 11 | 12 |
|---|---|---|---|---|---|---|---|---|---|---|---|---|
| A | DMSO | | | SIP (µM) | | | XMUMP 1 (XMU) (µM) | | | Ascorbic acid (Asco) (µM) | | |
| | | | | 20 | 10 | 5 | 10 | 5 | 2.5 | 400 | 200 | 100 |
| B | Y-27632 (Y) (µM) | | | FTY 720 (FTY) (µM) | | | Adenosine (Ade) (µM) | | | Go 6983 (Go) (µM) | | |
| | 40 | 20 | 10 | 20 | 10 | 5 | 200 | 100 | 50 | 4 | 2 | 1 |
| C | SB-431542 (SB) (µM) | | | VPC-23019 (VPC) (µM) | | | ATP (µM) | | | PMA (µM) | | |
| | 20 | 10 | 5 | 20 | 10 | 5 | 200 | 100 | 50 | 0.5 | 0.25 | 0.125 |
| D | LDN-193189 (LDN) (µM) | | | AMD-3100 (AMD) (µM) | | | ZM-241385 (ZM) (µM) | | | PD-0325901 (PD) (µM) | | |
| | 2 | 1 | 0.5 | 10 | 5 | 2.5 | 10 | 5 | 2.5 | 2 | 1 | 0.5 |
| E | Cyclopamine KAAD (Cyc) (nM) | | | LPA (µM) | | | CP-724714 (CP) (µM) | | | TSA (µM) | | |
| | 40 | 20 | 10 | 20 | 10 | 5 | 5 | 2.5 | 1.25 | 0.2 | 0.1 | 0.05 |
| F | GI-254023X (GI) (µM) | | | DHA (µM) | | | Marimastat (Mari) (µM) | | | OAC2 (µM) | | |
| | 2 | 1 | .5 | 20 | 10 | 5 | 5 | 2.5 | 1.25 | 20 | 10 | 5 |
| G | IWR1 endo (IWR) (µM) | | | SF-1670 (SF) (µM) | | | AGN-193109 (AGN) (µM) | | | Chetomin (CTM) (nM) | | |
| | 6 | 3 | 1.5 | 20 | 10 | 5 | 0.2 | 0.1 | 0.05 | 80 | 40 | 20 |
| H | Pyriintegrin (Pyr) (µM) | | | Rapamycin (Rapa) (µM) | | | Retinoic acid (RA) (µM) | | | ML-792 (ML) (µM) | | |
| | 4 | 2 | 1 | 1 | 0.5 | 10.25 | 1 | 0.5 | 10.25 | 5 | 2.5 | 1.25 |

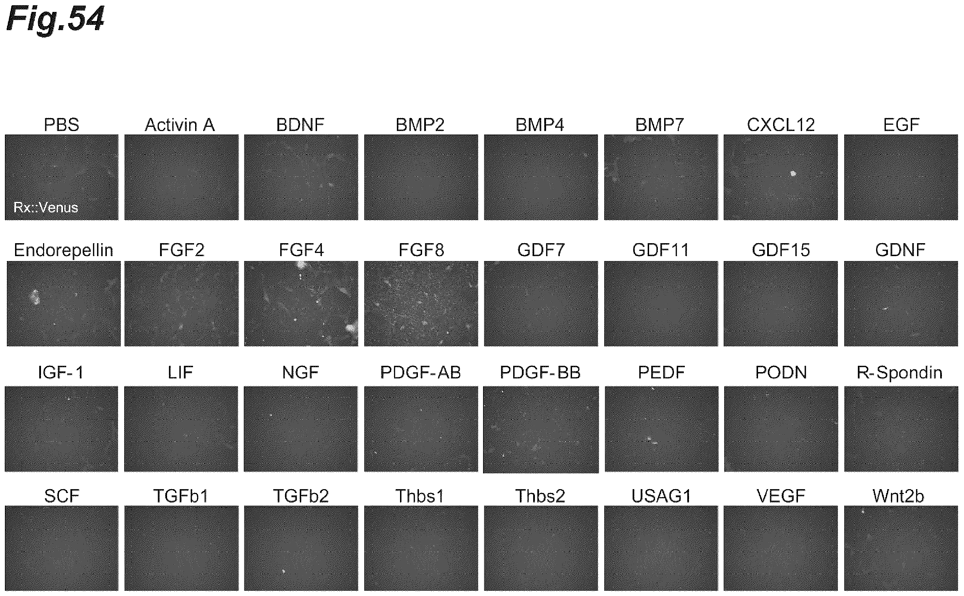

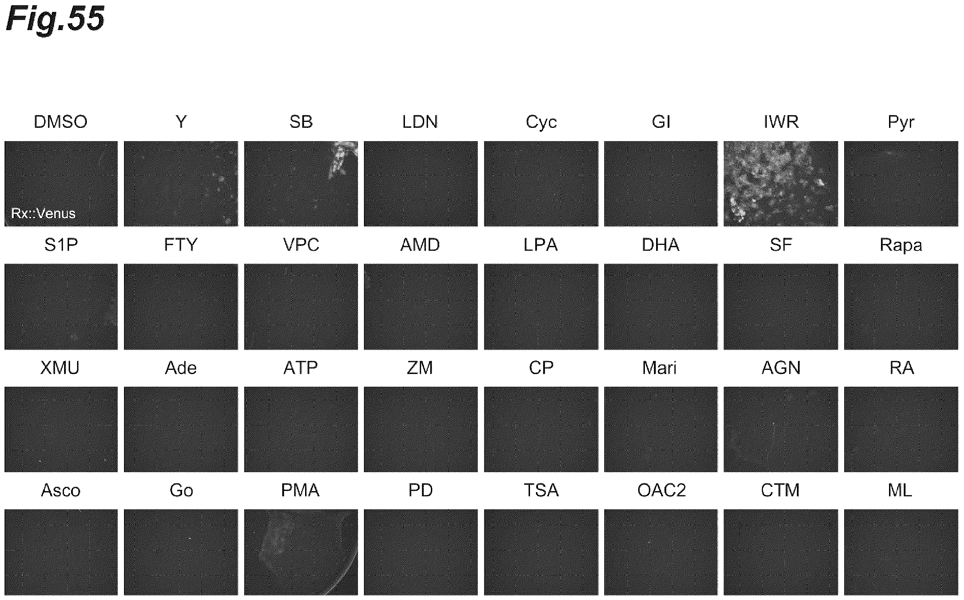

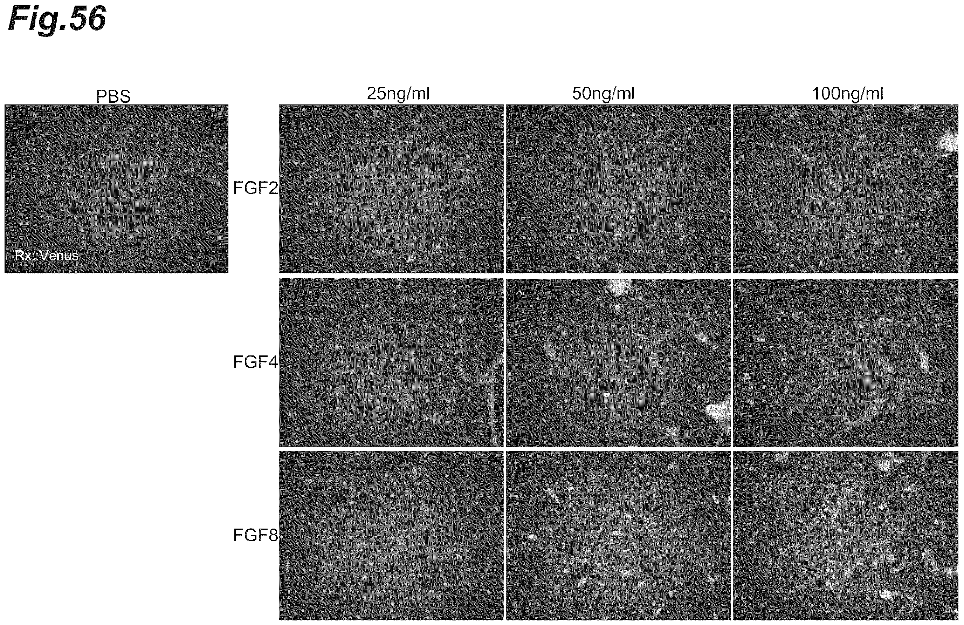

3 days after seeding, the medium was similarly replaced so as to maintain the concentrations of the low-molecular compounds or the proteins described in Tables 1 and 2. Then, on Day 7, medium replacement was carried out with NucTO medium, which did not contain the low-molecular compounds or the proteins described in Tables 1 and 2. 10 days after seeding, fixation was performed in 4% PFA, and the evaluation of the cells after sorting was conducted with Rx:: Venus as an index under a fluorescence microscope (BZ-X810 manufactured by Keyence Corp.). As a result, the sheets supplemented with FGF2, FGF4, or FGF8 among the proteins were observed to have high expression intensity of Rx:: Venus as compared with PBS serving as a control (Figure 54). The sheets supplemented with IWR1 endo among the low-molecular compounds were observed to have high expression intensity of Rx:: Venus (Figure 55). Further, concentration dependency was confirmed, and the highest expression intensity of Rx:: Venus was observed when 100 µg/mL FGF8 was added (Figure 56).

Accordingly, it was found that a Rx:: Venus-positive cell population can be maintained by activating FGF signals immediately after cell seeding.

### <Example 19 Aggregate reforming by addition of FGF8, and confirmation of retinal differentiation>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates (NR) of Day 24 from initiation of the suspension culture prepared in Example 1 were washed with PBS, and a neuronal cell dispersion solution (manufactured by FUJIFILM Wako Pure Chemical Corp.) was added. After incubation at 37°C, they were dispersed into single cells by pipetting, and sorting was carried out with Rx:: Venus as an index using Cell sorter ARIAII (Becton, Dickinson and Company). The cells after sorting were seeded at 2.0 to 5.0 × 10⁴ cells per well to a low adhesive V plate. As controls, a group that was not supplemented with FGF8 (FGF8-) and a group that was not sorted (unsorted) were also established.

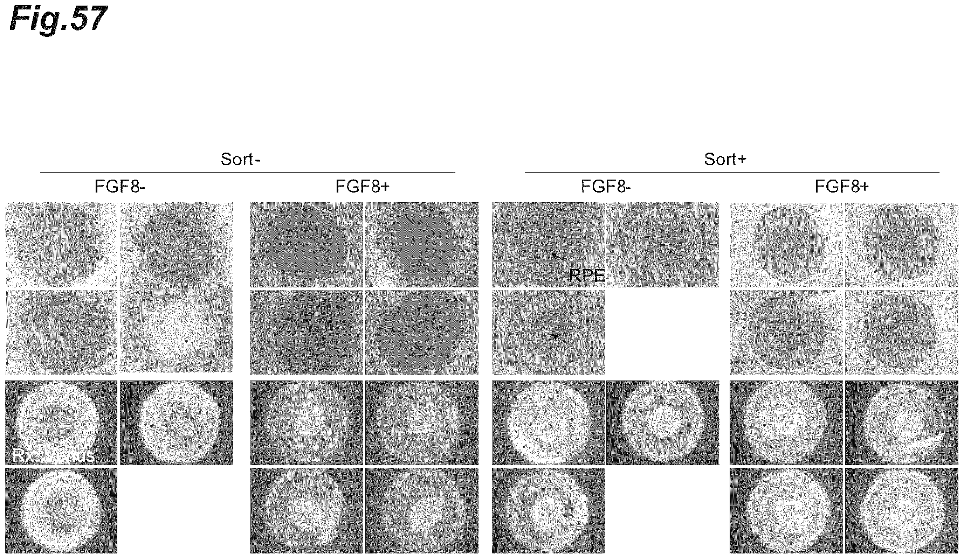

Medium replacement was carried out once every 3 to 4 days. FGF8 was also added at the time of medium replacement. Day 10 after reseeding, when observation was made under a microscope, Rx:: Venus-negative cell masses or RPE cells were observed in the unsorted FGF8-group (Figure 57). On the other hand, these cell masses were observed to decrease in the group supplemented with FGF8 (FGF8+). RPE cells were partially observed in the sorted FGF8- group. The RPE cells were removed by sorting, suggesting that a partial population of Rx:: Venus-positive cells differentiated into RPE cells. On the other hand, in the sorted FGF8+ group, Rx:: Venus-positive aggregates were formed, and RPE cells or Rx:: Venus-negative masses were not observed.

From these results, it was found that the differentiation of RPE cells or Rx:: Venus-negative cells can be reduced by adding FGF8.

Further, the aggregates of Culture Day 54 for aggregate reforming (Day 30 after reseeding) were washed with PBS and dispersed into single cells using a neuronal cell dispersion solution (manufactured by FUJIFILM Wako Pure Chemical Corp.). Then, formaldehyde fixation was performed using Fixation buffer (Becton, Dickinson and Company). Thereafter, after washing with Perm/Wash buffer (Becton, Dickinson and Company), staining was performed with anti-CHX10-Alexa Fluor 647 conjugate antibody (Santa Cruz Biotechnology), anti-Sox2-BV421 antibody (BioLegend, Inc.), anti-Ki67-Alexa Fluor 647 conjugate antibody (Becton, Dickinson and Company), and anti-CRX-Alexa Fluor 647 conjugate antibody (Santa Cruz Biotechnology). After washing of the antibodies with Perm/Wash buffer (Becton, Dickinson and Company), measurement was performed using FACSCantoII (BD Biosciences), and analysis was conducted using FlowJo. As a control, neural retinal tissue having the same number of differentiation days that was not reorganized was also established.

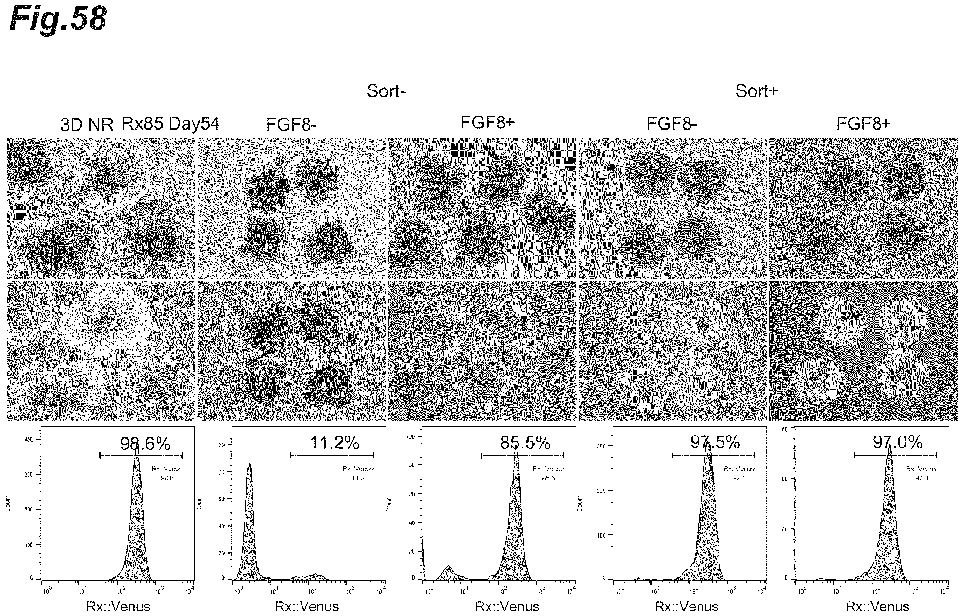

As a result, in the unsorted group that was not supplemented with FGF8, many Rx:: Venus-negative cell populations were observed, and a Rx:: Venus positive rate tended to be low. On the other hand, in the unsorted group supplemented with FGF8, it was shown that the Rx:: Venus positive rate tended to be high (Figure 58).

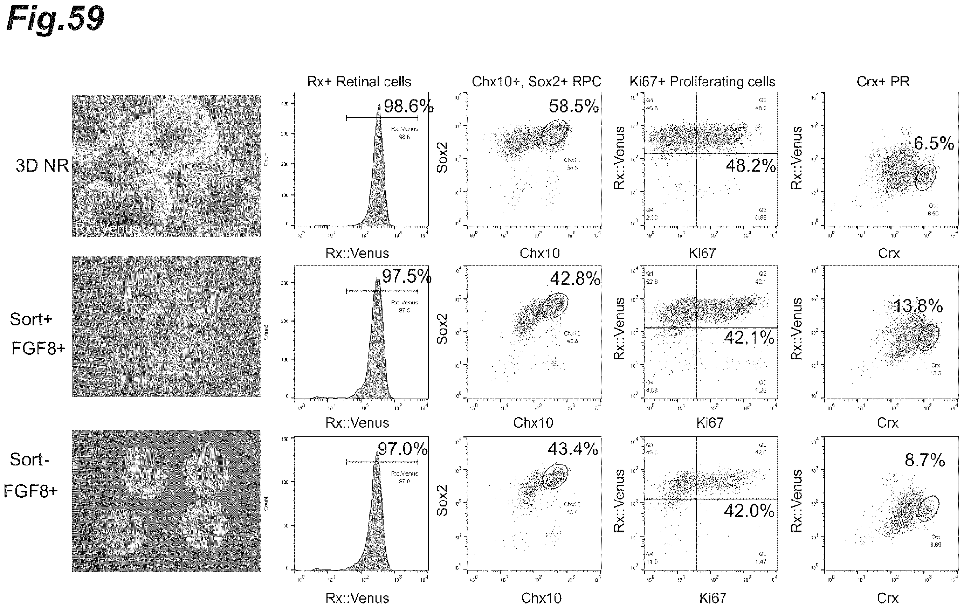

When sorting was performed, the Rx:: Venus positive rate also tended to be high in the group that was not supplemented with FGF8 (Figure 58). On the other hand, in the sorted group supplemented with FGF8, the percentage of Chx10-positive and Sox2-positive neural retinal progenitor cells was high, and the percentage of Crx-positive photoreceptor progenitor cells tended to be low (Figure 59).

From these results, it was found that the differentiation of RPE or Rx:: Venus-negative cells can be reduced and homogeneous and favorable aggregates can be obtained, by not only performing sorting but adding FGF8.

### <Example 20 Confirmation of effect of FGF8 at time of preparation into sheet again>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates (NR) of Day 24 from initiation of the suspension culture prepared in Example 1 were washed with PBS, and a neuronal cell dispersion solution (manufactured by FUJIFILM Wako Pure Chemical Corp.) was added. After incubation at 37°C, they were dispersed into single cells by pipetting, and sorting was carried out with Rx:: Venus as an index using Cell sorter ARIAII (Becton, Dickinson and Company). The cells after sorting were suspended and seeded at 2.0 × 10⁵ cells per well of 24-well Transwell (Corning, Inc.) using NucTO medium containing Y-27632, CHIR99021 and SAG, FGF8. As a control, a group that was not supplemented with FGF8 (FGF8-) was also established.

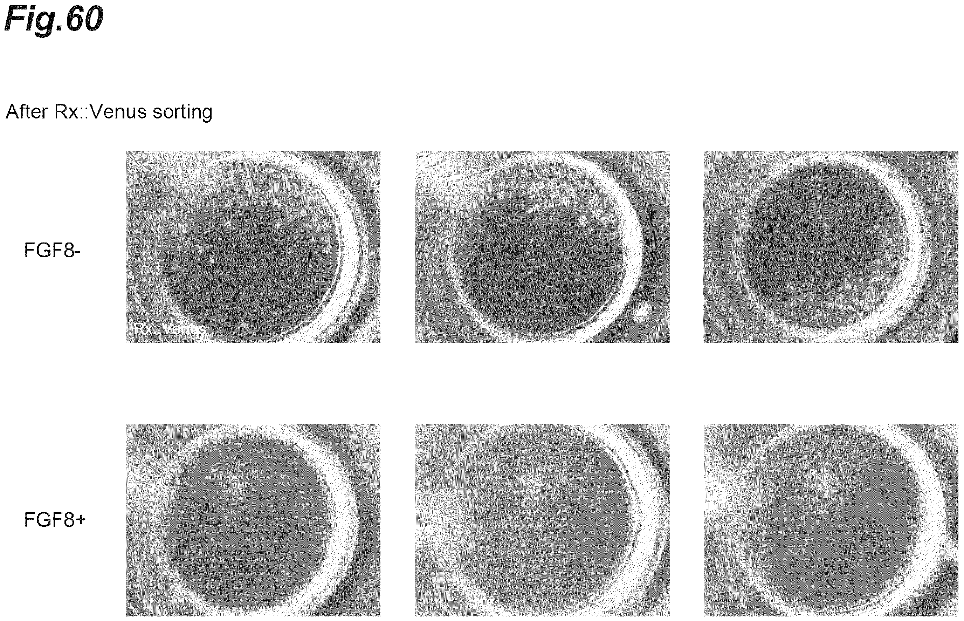

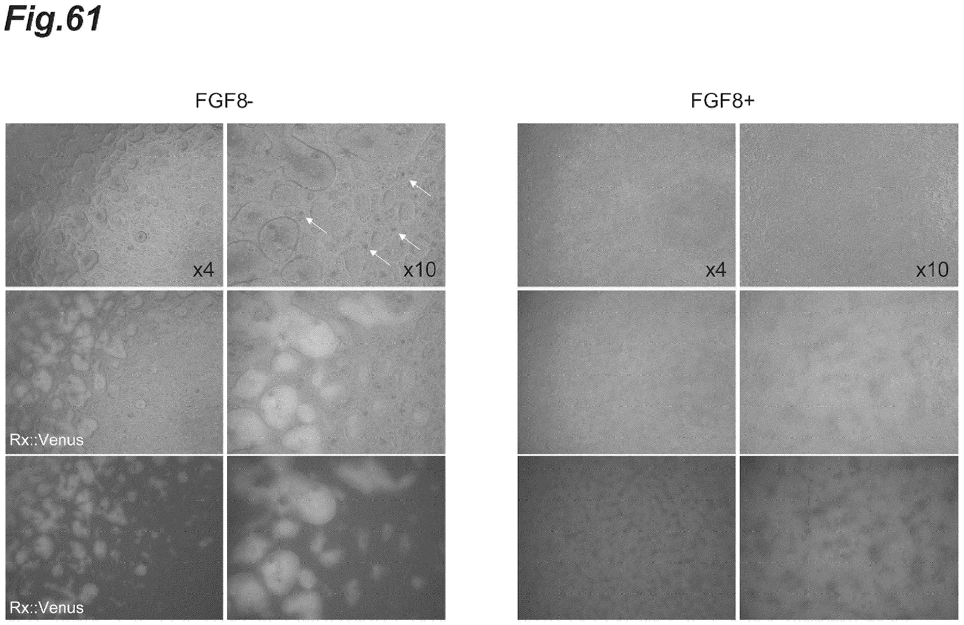

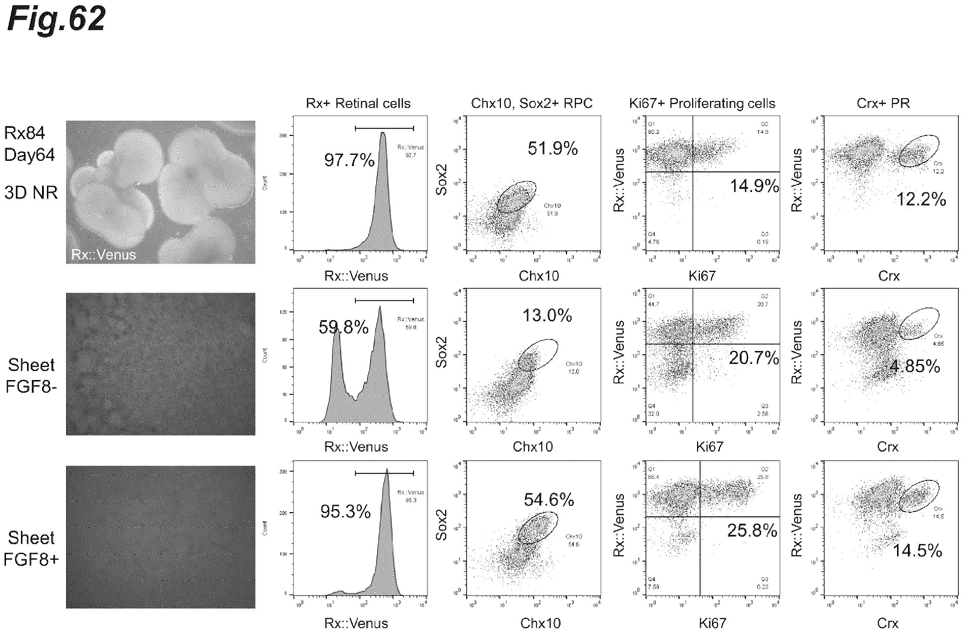

Medium replacement was carried out once every 3 to 4 days. FGF8 was also added at the time of medium replacement. Sheets after a lapse of differentiation for 63 days and 40 days after reseeding were observed under a fluorescence stereo microscope (Figures 60 and 61). For FACS analysis, the aggregates of Differentiation Day 64 and Day 41 after reseeding were washed with PBS and dispersed into single cells using a neuronal cell dispersion solution (manufactured by FUJIFILM Wako Pure Chemical Corp.). Then, formaldehyde fixation was performed using Fixation buffer (Becton, Dickinson and Company). Thereafter, after fixation using Fixation buffer (Becton, Dickinson and Company) and washing with Perm/Wash buffer (Becton, Dickinson and Company), staining was performed with anti-CHX10-Alexa Fluor 647 conjugate antibody (Santa Cruz Biotechnology), anti-Sox2-BV421 conjugate antibody (BioLegend, Inc.), anti-Ki67 antibody (Becton, Dickinson and Company), and anti-CRX-Alexa Fluor 647 conjugate antibody (Santa Cruz Biotechnology). After washing of the antibodies with Perm/Wash buffer (Becton, Dickinson and Company), measurement was performed using FACSCantoII (BD Biosciences), and analysis was conducted using FlowJo. As a control, neural retinal tissue having the same number of differentiation days that was not reorganized was also established (Figure 62).

As a result, in the sheets that were sorted and were then not supplemented with FGF8, many Rx::Venus-negative cell populations were observed, and Rx:: Venus-positive cells tended to be biased toward neighboring parts of the wells and present on a mass. On the other hand, in the sheets that were sorted and then supplemented with FGF8, Rx:: Venus-positive cells were uniformly present in the whole wells, and favorable sheets were observed to be prepared (Figures 60 and 61). Black RPE (Figure 61, arrow) was observed in the sheets that were not supplemented with FGF8, and on the other hand, was hardly observed in the sheets supplemented with FGF8.

In FACS analysis, it was found that a Rx::Venus positive rate was 60% and thus decreased in the sheets that were not supplemented with FGF8, whereas the positive rate was 95% or more in the group supplemented with FGF8 and was thus able to be maintained in no way inferior to neural retinal tissue (Figure 62). The percentage of Chx10-positive and Sox2-positive neural retinal progenitor cells or Crx-positive photoreceptor progenitor cells was also equivalent as compared with neural retinal tissue. The percentage of Rx:: Venus-positive and Ki67-positive proliferative retinal cells tended to be large as compared with neural retinal tissue (Figure 62).

From these results, it was found that the differentiation of RPE or Rx:: Venus-negative cells can be reduced and homogeneous and favorable sheets can be obtained by adding FGF8 after sorting when prepared into a sheet again.

### <Example 21 Confirmation of time-dependent change after seeding onto Transwell and preparation into sheet again>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates of Day 27 from initiation of the suspension culture prepared as in Example 1 were washed with PBS, and a neuronal cell dispersion solution (manufactured by FUJIFILM Wako Pure Chemical Corp.) was added. After incubation at 37°C, they were dispersed into single cells by pipetting. After dispersion, the cells were suspended in 200 µL of a serum-free medium containing 10 µM Y-27632, 300 nM SAG, 3 µM CHIR99021 and 100 ng/mL FGF8 so as to become 2.0 × 10⁵ cells per well of 24-well Transwell coated with Laminin511-E8, and cultured at 37°C and 5% CO₂ for 3 days. 3 days or later from seeding, the medium was replaced with a serum-free medium (NucTO), which did not contain Y-27632, SAG, CHIR99021, or FGF8, once every 3 to 4 days.

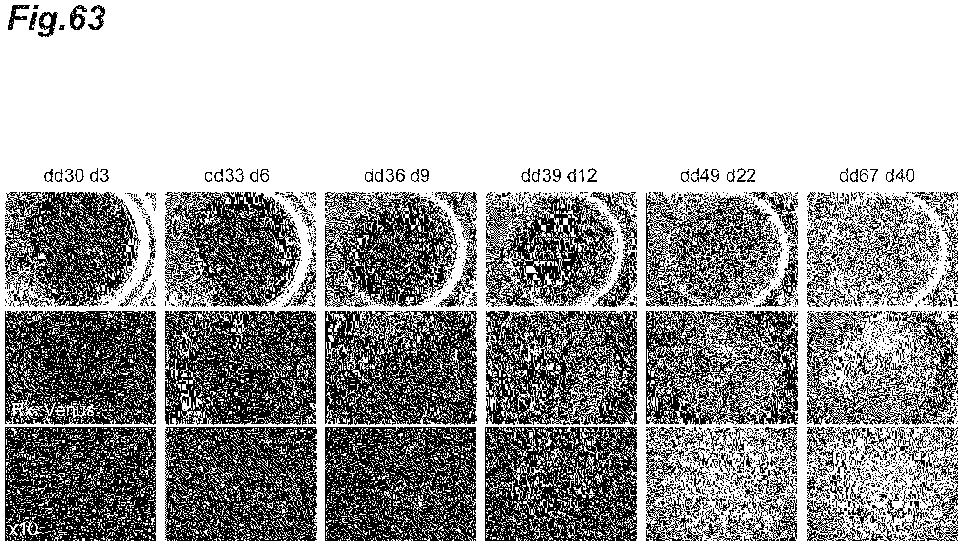

Cell sheets obtained after culture for aggregate reforming for 3 days, 6 days, 9 days, 12 days, 22 days, and 40 days (dd30, dd33, dd36, dd39, dd49, dd67) were fixed in 4% PFA. Then, when preparation into a sheet was observed with Rx:: Venus as an index under a fluorescence stereo microscope and a fluorescence microscope, Rx:: Venus-positive retina cells proliferated and sheets having a thickness were observed to be formed (Figure 63). Thick cell sheets whose whole surface was covered with Rx:: Venus-positive cells were confirmed, particularly, after 22 days to 40 days.

From these results, reorganized retina sheets for use in transplantation, assay, or the like were observed to be obtained after 22 days to 40 days.

### <Example 22 RPC surface antigen screening - comparison of hES cell with NR ->

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

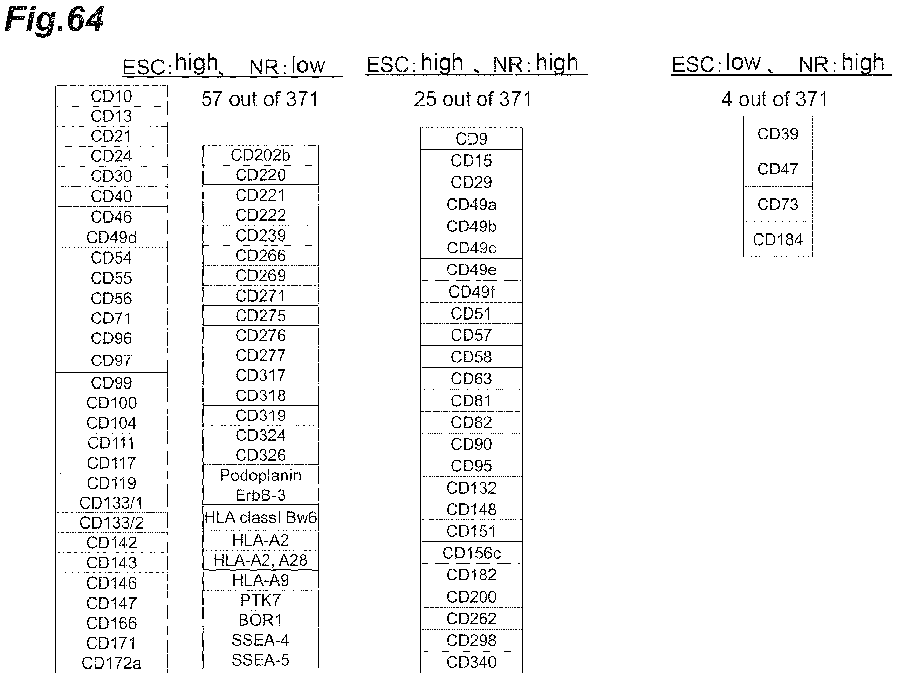

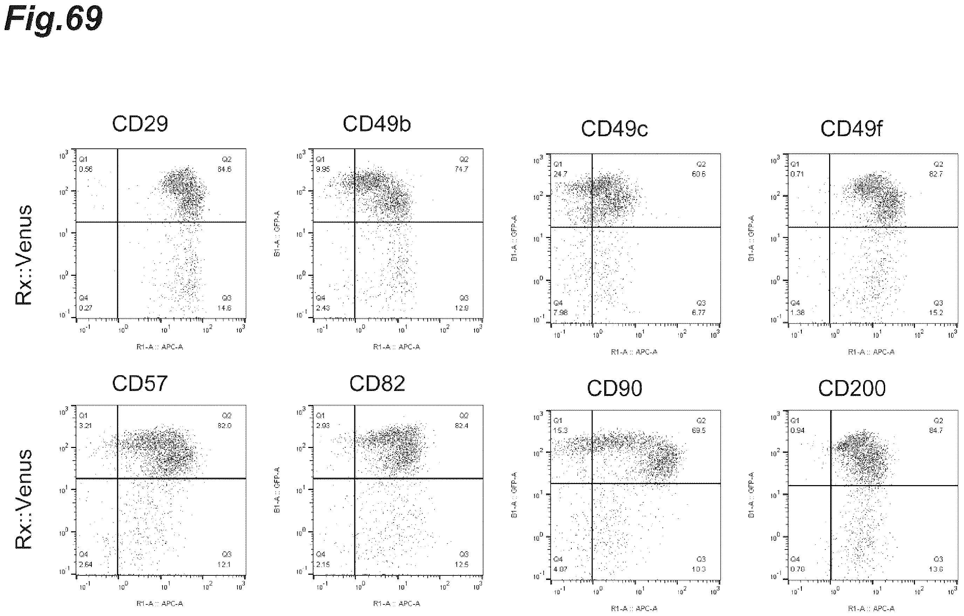

Suspension culture for differentiation was started as described in Example 1 using the cells. 300 nM SAG was added at the start of differentiation. The aggregates (NR) of Days 19 and 26 from initiation of the suspension culture were washed with PBS, and a neuronal cell dispersion solution (manufactured by FUJIFILM Wako Pure Chemical Corp.) was added. After incubation at 37°C, they were dispersed into single cells by pipetting, and surface antigen screening was carried out using surface antigen screening kit MACS(R) Marker Screen, human (Miltenyi Biotec) (Alexa 647 fluorescent dye conjugate antibody group). As a control, human ES cells (hESC) were stained. The number of markers used in screening was 371 in total (Figures 64 to 69).

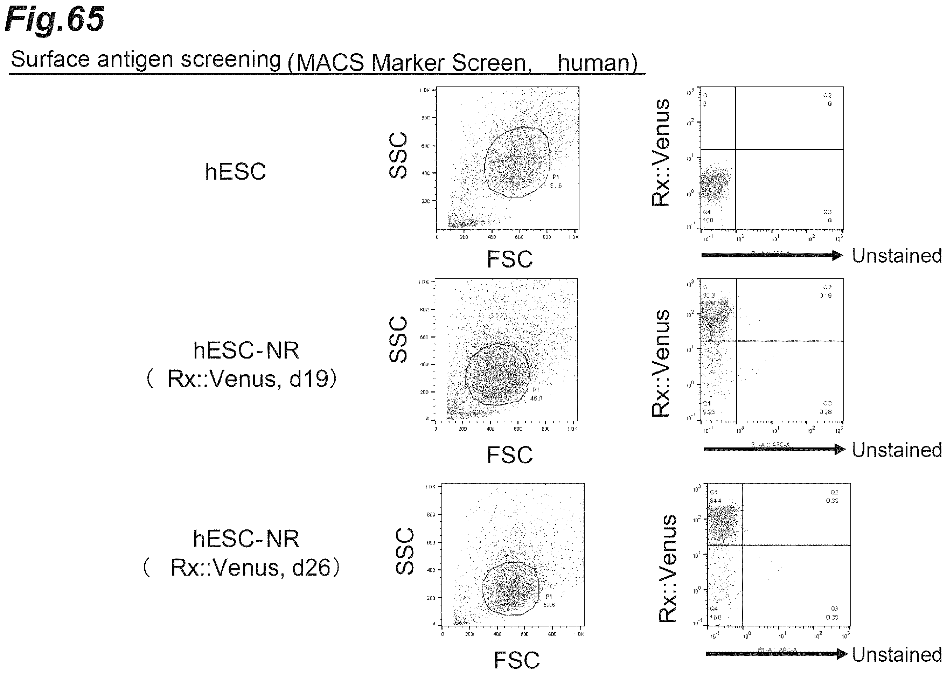

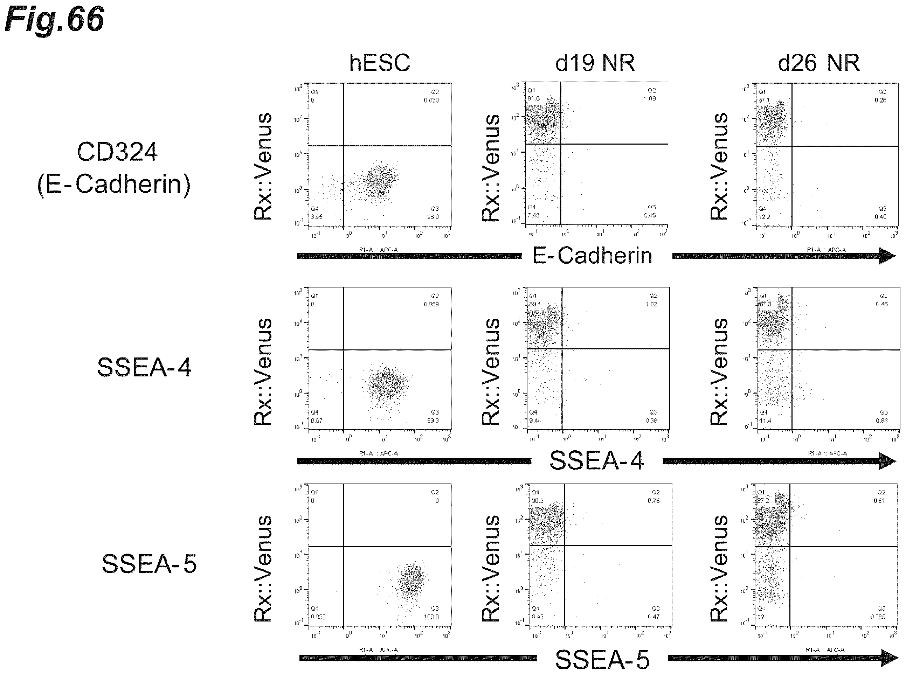

In FACS analysis, measurement was performed using MACS Quant10 (Miltenyi Biotec), and analysis was conducted using FlowJo. Analysis was conducted by performing gating with the populations indicated by FSC and SSC shown in Figure 65. First, it was confirmed that staining and analysis were accurately performed using E-cadherin, SSEA-4, and SSEA-5, which were expressed in hESC and whose expression disappeared by differentiation (Figure 66). Subsequent analysis results are summarized in Figure 64. "hESC: high" means that in human ES cells, an expression level is high or expression is present, "hESC: low" means that in human ES cells, an expression level is low or expression is absent, "NR: high" means that in neural retina cells, an expression level is high, and "NR: low" means that in neural retina cells, an expression level is low or expression is absent.

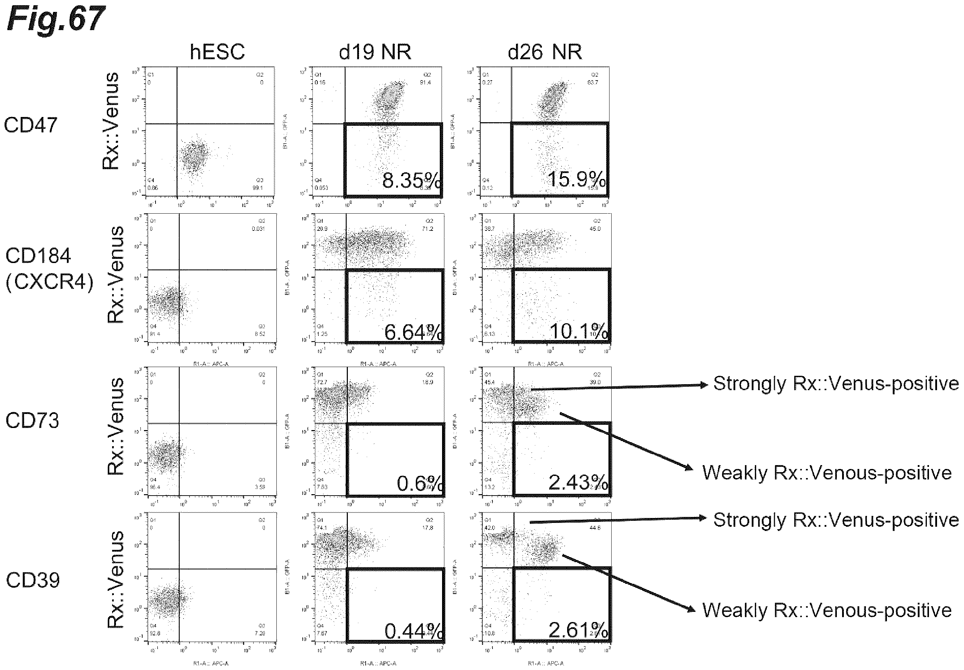

When a surface antigen was searched for which was not expressed (or was expressed) in hESC and was expressed (or was not expressed) by a Rx:: Venus-positive population on Day 19 or Day 26 from initiation of the suspension culture, the possibility was suggested that CD39, CD73, CXCR4, and further, CD29, CD49b, CD49c, CD49f, CD57, CD82, CD90, and CD200 were markers capable of distinguishing Rx:: Venus-positive cells from other cells (Rx:: Venus-negative cells, hESC) (Figure 67).

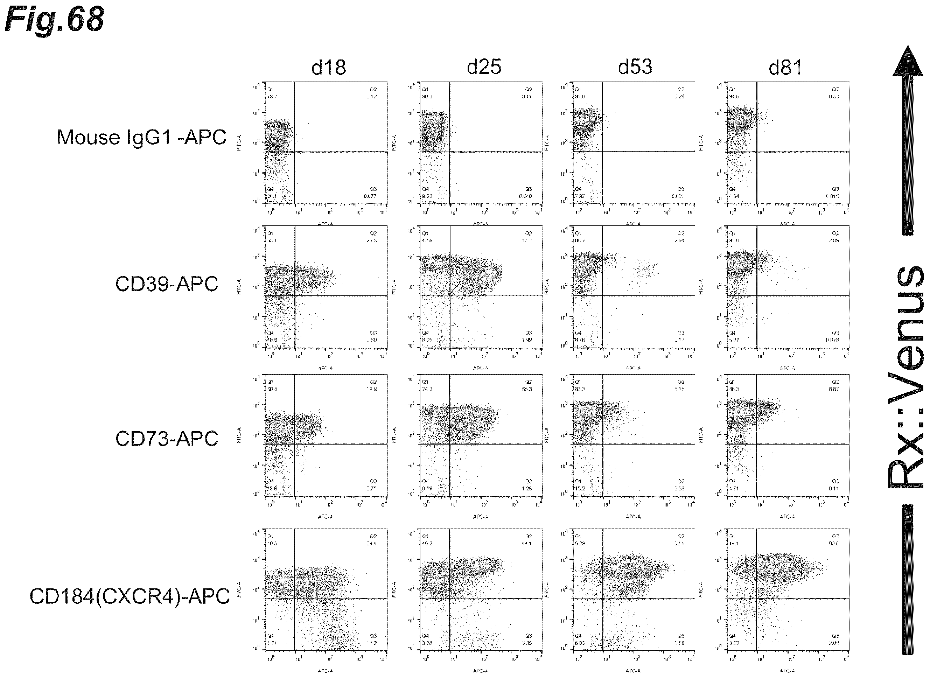

The expression of CD39, CD73, and CD184 (CXCR4) on dd18, dd25, dd53, and dd81 (in Figure 68, d18, d25, d53, d81) from initiation of the suspension culture was analyzed by FACS using Mouse IgG1-APC-conjugate antibody (Miltenyi Biotec), anti-CD39-APC-conjugate antibody (Miltenyi Biotec), anti-CD73-APC-conjugate antibody (Miltenyi Biotec), and anti-CD184(CXCR4)-APC-conjugate antibody (Miltenyi Biotec) (Figure 68).

As a result, it was found that the percentage of cells in which CD39 and CD73 were expressed was highest on dd25 and as for CD184, the percentage of cells with low expression was high on dd18 and dd25.

Accordingly, it was found that in the case of using surface antigens CD39, CD73, and CD184 for purifying neural retinal progenitor cells, Day 18 or later and around Day 25 from initiation of suspension culture are preferable (Figure 68).

### <Example 23 Expression analysis of CD39, CD73, and CXCR4>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

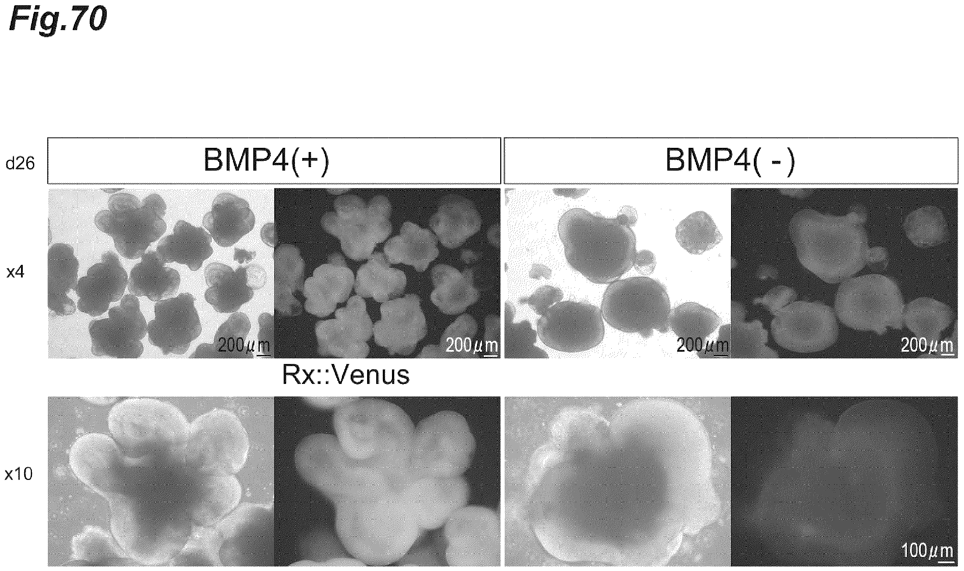

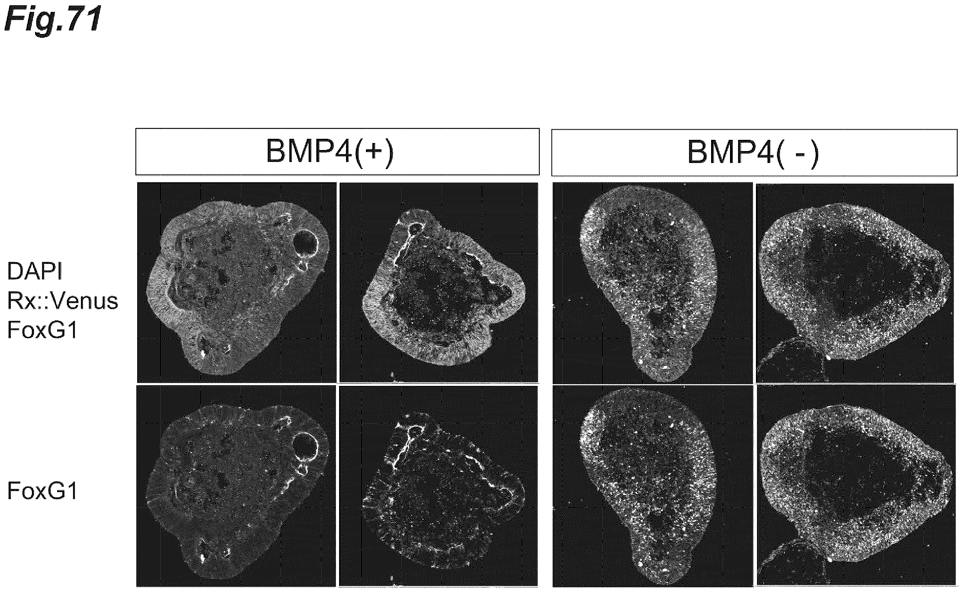

300 nM SAG was added at the start of suspension culture (at the start of differentiation), and brain organoid was prepared on Day 3 from initiation of the suspension culture without adding BMP4. As a control, a group supplemented with BMP4 was established. Fluorescence microscope observation was performed as to the brain organoid of Day 26 from initiation of the suspension culture prepared in this way (Figure 70). As a result, in the group supplemented with BMP4, Rx:: Venus-positive retinal tissue was observed to be differentiated. On the other hand, in the group that was not supplemented with BMP4, Rx:: Venusnegative neuroepithelial cells were observed to be differentiated. Further, these aggregates were fixed in 4% PFA. Sections were prepared and stained with DAPI and anti-FoxG1 antibody (Takara Bio Inc.), and observation was performed under a fluorescence microscope (BZ-X810 manufactured by Keyence Corp.). As a result, in the group supplemented with BMP4, FoxG1 was negative. On the other hand, in the group that was not supplemented with BMP4, FoxG1 positive was confirmed, and it was confirmed that brain organoid was differentiated (Figure 71).

The brain organoid of Day 25 from initiation of the suspension culture prepared in this way was washed with PBS, and a neuronal cell dispersion solution (manufactured by FUJIFILM Wako Pure Chemical Corp.) was added. After incubation at 37°C, it was dispersed into single cells by pipetting and stained with anti-CD39-APC conjugate antibody (Miltenyi Biotec), anti-CD73-APC conjugate antibody (Miltenyi Biotec), and anti-CXCR4-APC conjugate antibody (Miltenyi Biotec). In FACS measurement, the percentage of an expressing cell population was measured using FACSCantoII (BD Biosciences), and analysis was conducted using FlowJo.

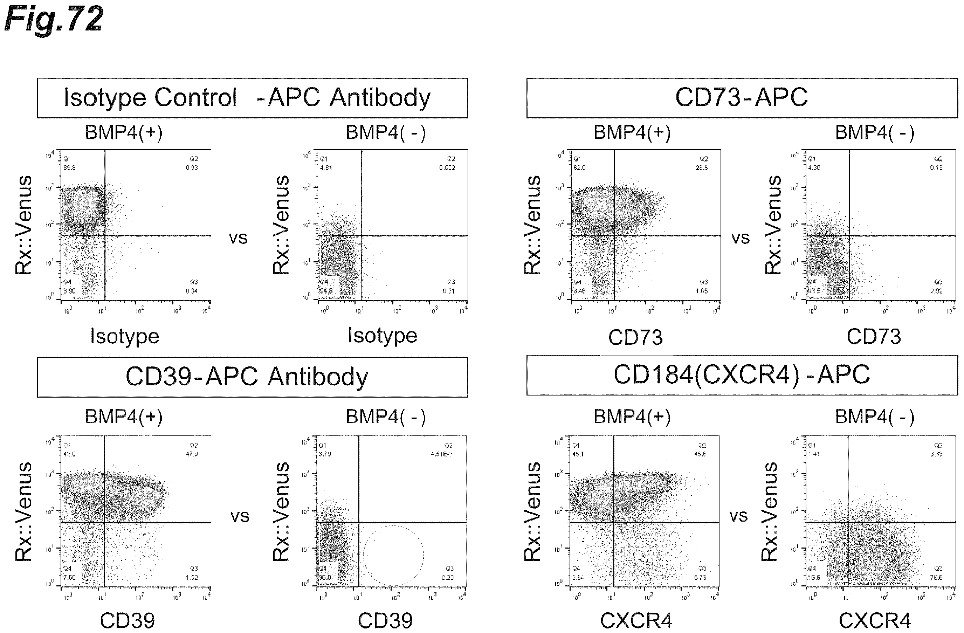

As a result, CD39 or CD73 was not expressed in the brain organoid, whereas CXCR4 was observed to be expressed in a majority of cells in the brain organoid (Figure 72).

Accordingly, it was confirmed that a population of Rx:: Venus-positive cells can be distinguished and separated from brain organoid by using a CD39-positive, CD73-positive, and CXCR4-negative cell population as an index.

### <Example 24 CD39 expression analysis>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

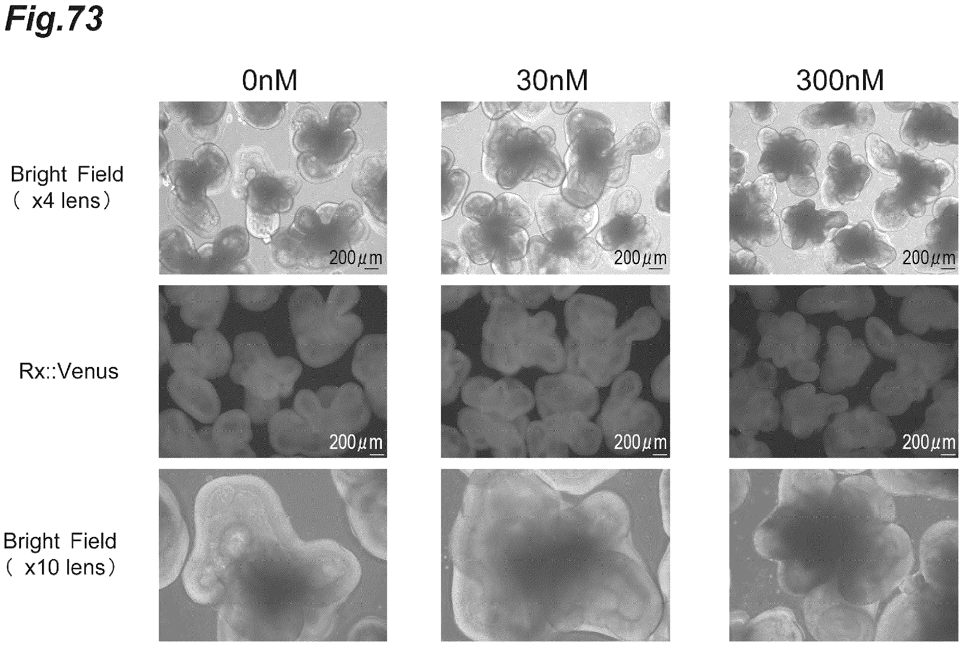

The morphology of aggregates (NR) of Day 26 from initiation of the suspension culture prepared by adding (1) 0 nM, (2) 30 nM, or (3) 300 nM SAG at the start of differentiation was observed under a microscope. As a result, it was confirmed that in the case of adding 0 nM SAG and 30 nM SAG as compared with the case of adding 300 nM SAG at the start of suspension culture (dd0), aggregates were larger and a layer structure was clearer (Figure 73).

The aggregates were dispersed into single cells using a neuronal cell dispersion solution, and FACS analysis was conducted as to the expression of CD39 and CXCR4 using anti-CD39-APC conjugate antibody (Miltenyi Biotec) and anti-CXCR4-APC conjugate antibody (Miltenyi Biotec). The percentage of an expressing cell population was measured using FACSCantoII (BD Biosciences), and analysis was conducted using FlowJo. These aggregates were fixed in 4% PFA, and sections were prepared and then subjected to staining using anti-ALDH1A1 antibody (R&D Systems, Inc.), anti-CoupTF1 antibody (Perseus Proteomics Inc. (PPMX)), anti-LHX2 antibody (manufactured by Merck Millipore), anti-Chx10 antibody (manufactured by Exalpha Biologicals Inc.), anti-Pax2 antibody (manufactured by Covance), and anti-NKX2.1 antibody (manufactured by Leica).

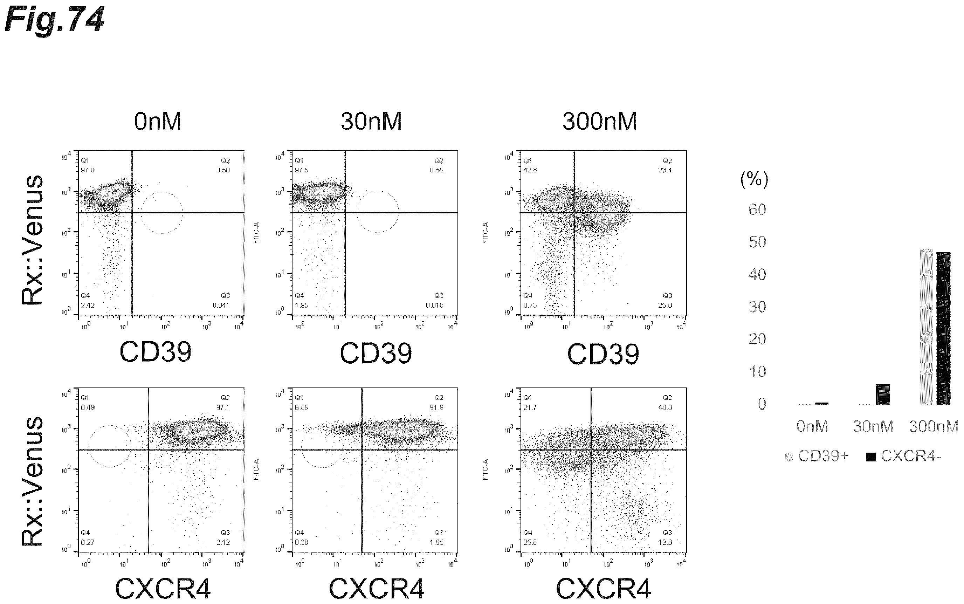

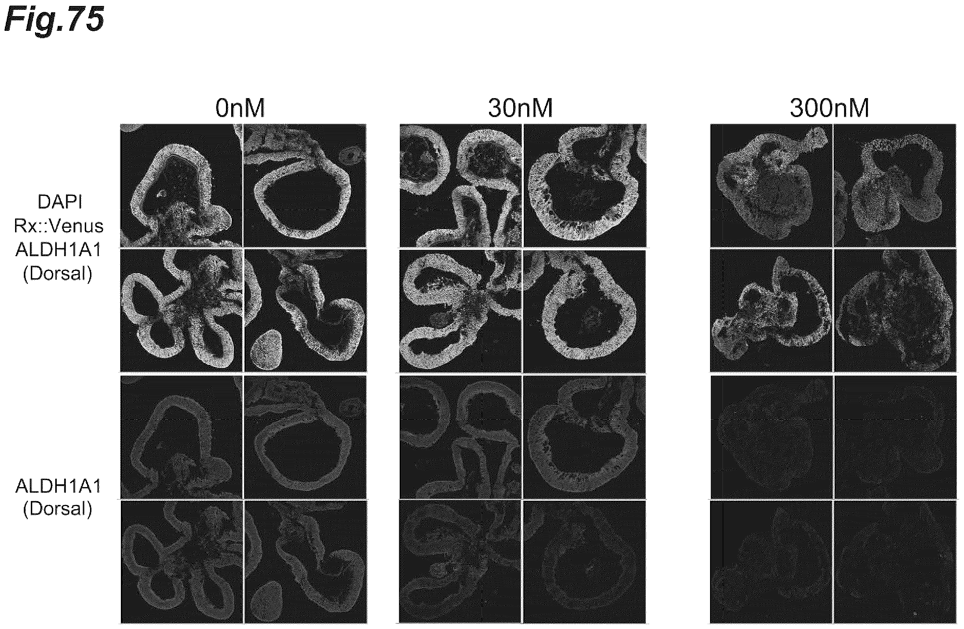

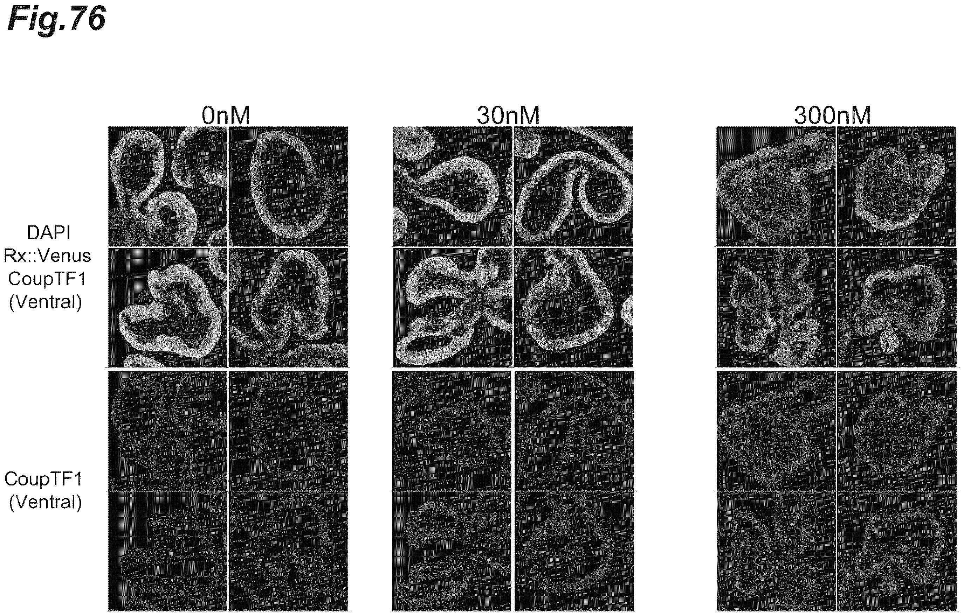

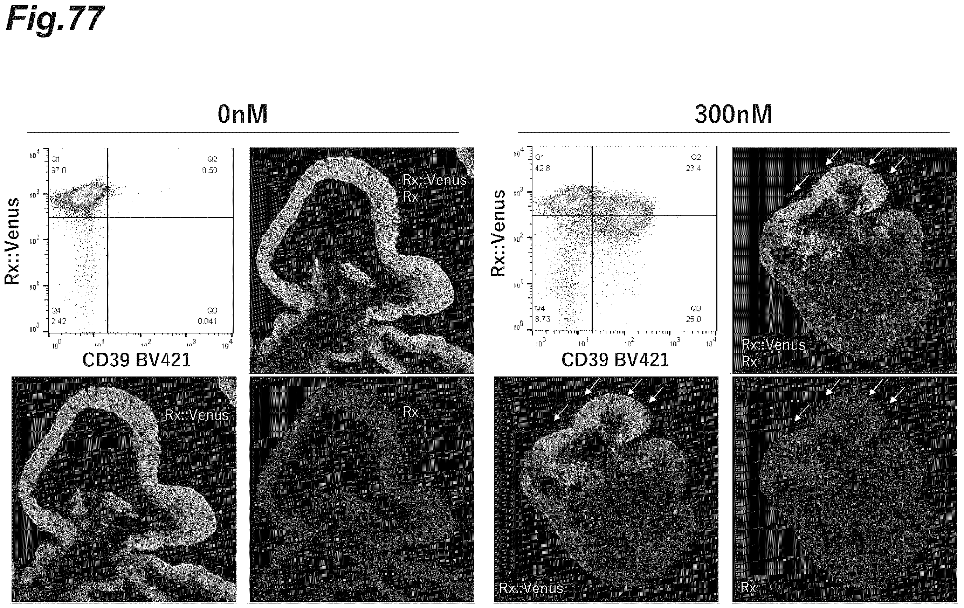

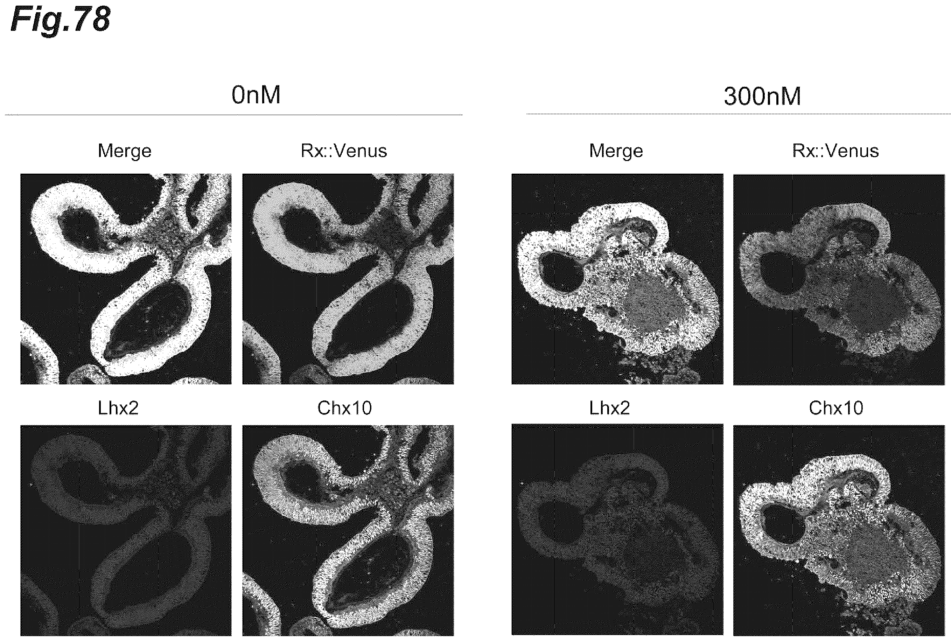

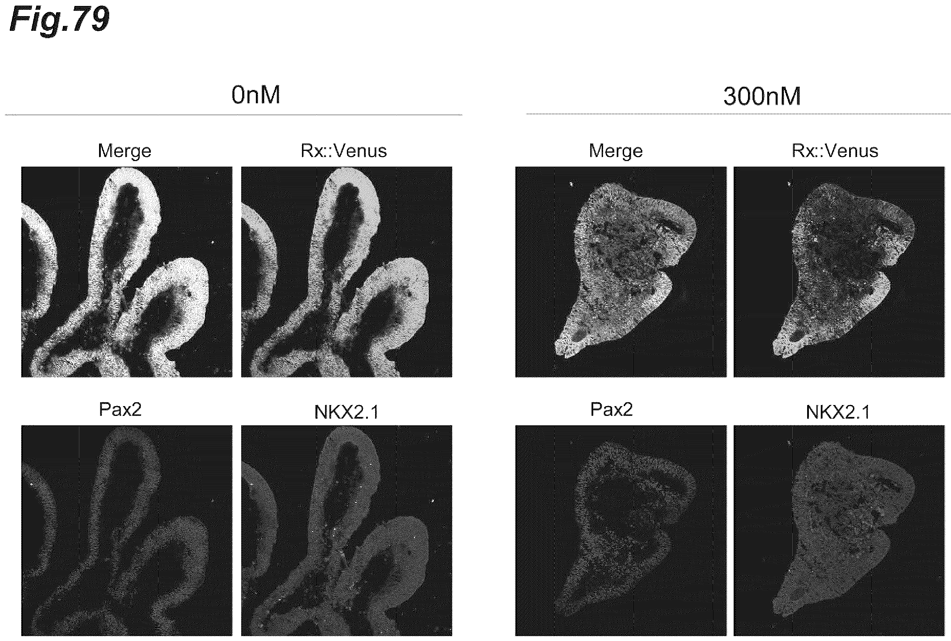

As a result, in the aggregates to which 300 nM SAG was added at the start of suspension culture (dd0), CD39 was expressed, and low expression of CXCR4 was observed (Figure 74). Decrease in expression of a dorsal marker ALDH1A1 and increase in expression of a ventral marker CoupTF1 were found by the addition of SAG on dd0 (Figures 75 and 76). The degree of expression of Rx in the cell aggregates was found uniform when stimulation with SAG was not applied (Figure 77). However, the expression of LHX2, Chx10, and Pax2 was also confirmed in a region with low expression of Rx in the aggregates supplemented with 300 nM, and NKX2.1 was negative. Therefore, neural retinal tissue was confirmed (Figures 78 and 79).

Accordingly, it was found that a population of CD39-positive and CXCR4-negative neural retinal progenitor cells can be obtained by adding 300 nM SAG at the start of differentiation.

### <Example 25 Study on enhancement in CD39 expression>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

For aggregates (NR) prepared by adding 300 nM SAG at the start of differentiation (dd0), 10 neural retinal tissues were transferred to low adhesive 60-mm Petri dish for suspension culture (manufactured by Sumitomo Bakelite Co., Ltd.) on Day 17 from initiation of the suspension culture. 1 mM or 0.2 mM adenosine (A2A receptor agonist, manufactured by Sigma-Aldrich Co. LLC), 1 mM, 0.2 mM, or 0.04 mM AMP-PNP (manufactured by Tocris Bioscience), 5 mM, 1 mM, 0.2 mM, or 0.04 mM ATP disodium salt (manufactured by Tocris Bioscience), and 1 µM, 0.2 µM, or 0.04 µM CGS 21680 (Adenosine A2A Receptor Agonist, manufactured by Tocris Bioscience) were added into 4 mL of NucTO medium. Culture was performed up to Day 25 from initiation of the suspension culture. Change in expression of CD39 and CXCR4 was stained using Mouse IgG1-APC conjugate antibody (Miltenyi Biotec), anti-CD39-BV421 conjugate antibody (BD Biosciences) and anti-CXCR4-APC conjugate antibody (Miltenyi Biotec) and measured using FACSCantoII (BD Biosciences), and analysis was conducted using FlowJo. As a control, a DMSO addition group was established.

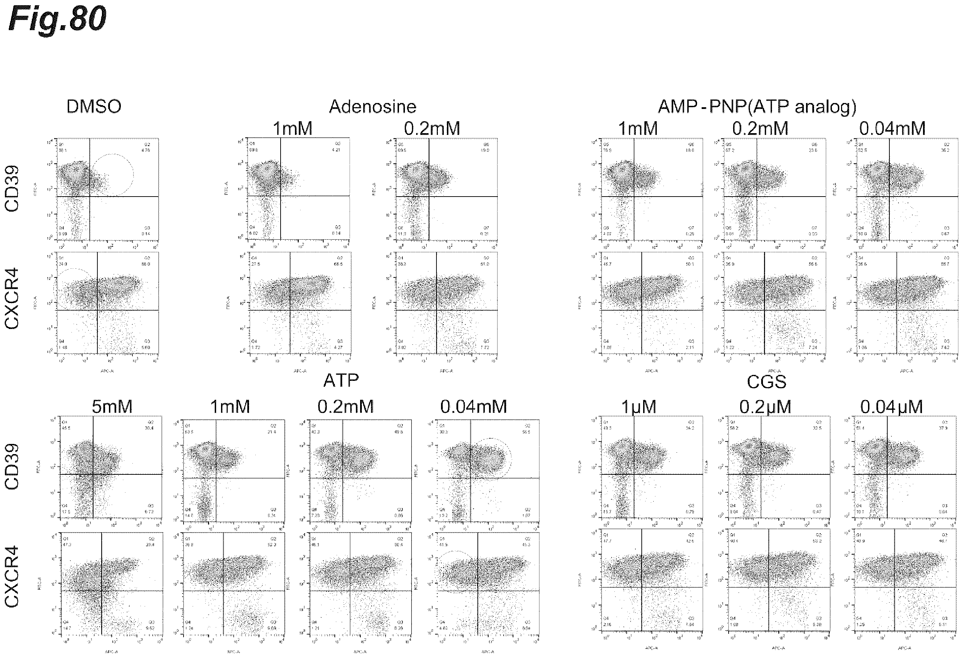

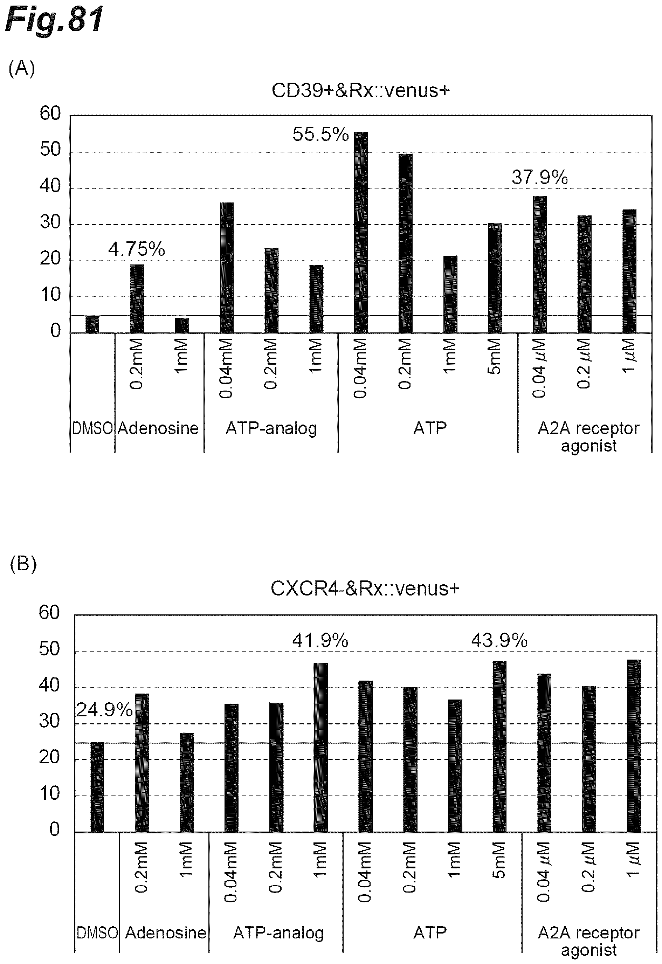

As a result, it was found that ATP and an A2A receptor agonist have an effect of enhancing the expression of CD39 and decreasing the expression of CXCR4 (Figures 80 and 81).

Accordingly, it was found that the expression of CD39 can be enhanced by adding ATP and an A2A receptor agonist.

### <Example 26 Transplantation of Islet-1 KO hESC-retina sheet prepared by sorting with CD39 and addition of FGF8>

Human ES cells (KhES-1 strain (Non Patent Literature 3)) genetically engineered so as to have Rx:: Venus reporter gene, in which Islet-1 gene was knocked out, were cultured under feeder-free conditions in accordance with the methods described in "Scientific Reports 4, 3594 (2014)" and WO2018/097253. As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

For aggregates differentiated with 300 nM SAG at the start of suspension culture (at the start of differentiation, dd0), the aggregates (NR) of Day 25 from initiation of the suspension culture were dispersed into single cells and stained at 37°C for 15 minutes using anti-CD39-APC conjugate antibody (Miltenyi Biotec). Then, sorting was carried out with CD39-APC as an index using Cell sorter ARIAII (Becton, Dickinson and Company). The cells after sorting were seeded at 8.0 × 10⁵ cells per well into 12-well Transwell coated with collagen gel. Y-27632 (FUJIFILM Wako Pure Chemical Corp.), SAG (Enzo Life Sciences, Inc.), CHIR99021 (FUJIFILM Wako Pure Chemical Corp.), and 50 ng/mL FGF8 (FUJIFILM Wako Pure Chemical Corp.) were added at the time of seeding. As a control, a group that was not supplemented with FGF8 (FGF8-) was also established.

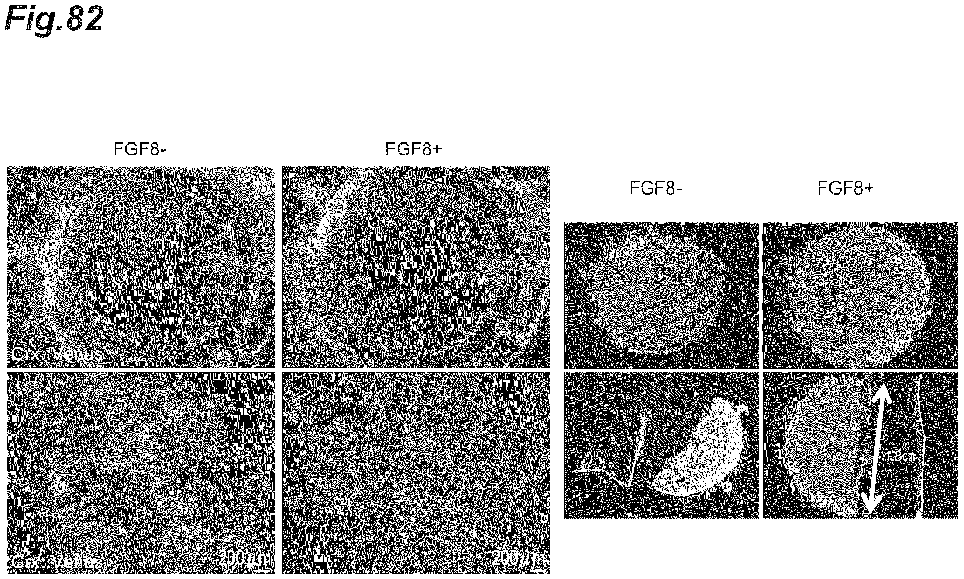

Medium replacement was carried out once every 3 to 4 days. FGF8 was also added at the time of medium replacement. When observation was made under a microscope on Day 73 from initiation of the suspension culture, Crx: : Venus cell populations were sparsely observed in the group that was not supplemented with FGF8 and by contrast, were observed to be uniformly present in the group supplemented with FGF8 (Figure 82).

The sheets prepared in this way were recovered using collagenase on Day 88 from initiation of the suspension culture. Then, long grafts for transplantations were dissected using microscissors (Figure 82), and subretinal transplantation to immunodeficient retina-deficient rats (SD Foxn) was carried out.

Accordingly, it was confirmed that retina sheets manufactured from cells sorted with CD39 were transplantable.

### <Example 27 RPC surface antigen screening - Comparison of brain organoid with NR ->

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

Aggregates (NR) of Days 24 to 26 from initiation of the suspension culture prepared by adding 30 nM SAG at the start of the suspension culture (at the start of differentiation, dd0) were dispersed into single cells using a neuronal cell dispersion solution (FUJIFILM Wako Pure Chemical Corp.) and stained using surface antigen screening kit MACS(R) Marker Screen, human (Miltenyi Biotec), and surface antigen screening was carried out (Alexa 647 fluorescent dye conjugate antibody group). As a control, brain organoid prepared without adding BMP4 on Day 3 from initiation of the differentiation was stained.

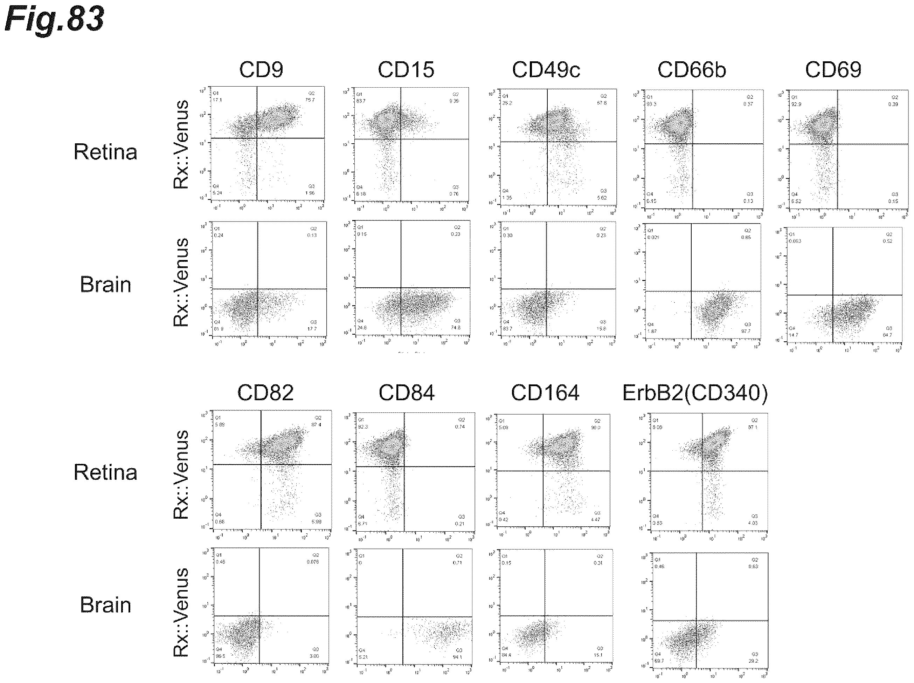

In FACS analysis, measurement was performed using MACS Quant10 (Miltenyi Biotec), and analysis was conducted using FlowJo. When a surface antigen was searched for which was not expressed (or was expressed) in the brain organoid and was expressed (or was not expressed) by a Rx:: Venus-positive population on Days 24 to 26 from initiation of the suspension culture, the possibility was suggested that CD9, CD15, CD49c, CD66b, CD69, CD82, CD164, EpCAM, and ErbB2 (CD340) were capable of distinguishing it from the brain organoid (Figure 83).

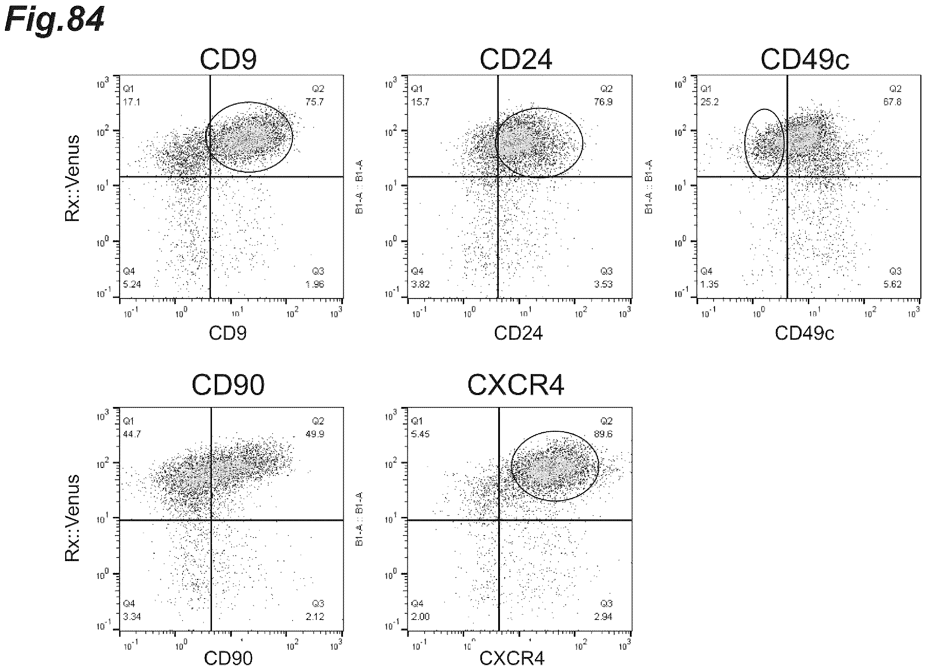

The possibility was indicated that CD9, CD24, CD49c, CD90, CXCR4, and EpCAM are useful as markers that distinguish Rx:: Venus-positive cells from negative cells (Figure 84).

### <Example 28 Confirmation of time-dependent change in expression of CD9>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

hESC and aggregates (NR) of dd4, dd11, dd18, dd25, dd32, and dd46 prepared by adding 30 nM SAG at the start of suspension culture (at the start of differentiation, dd0) were dispersed into single cells using a neuronal cell dispersion solution (FUJIFILM Wako Pure Chemical Corp.) and stained with APC-conjugated anti-CD9 antibody (BioLegend, Inc.). After the antibody was washed, FACS analysis was conducted. In FACS analysis, measurement was performed using MACS Quant10 (Miltenyi Biotec), and analysis was conducted using FlowJo.

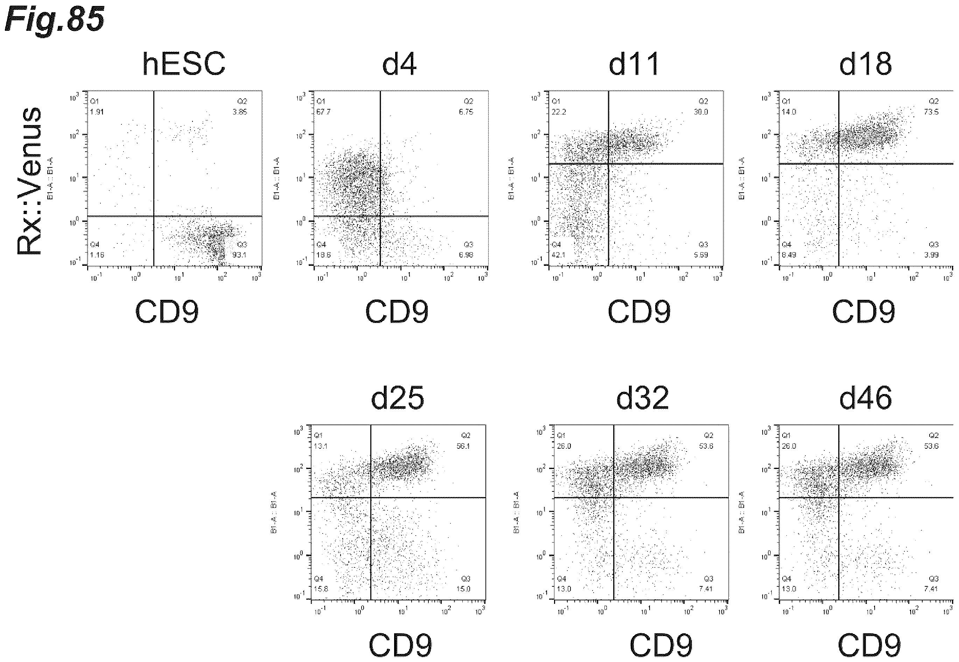

As a result, it was found that although CD9 was expressed in hESC, the expression was once decreased on Day 4 from initiation of the differentiation and started from around Day 11 (d11 in Figure 85) and the percentage of positive cells increased from dd18 (d18 in Figure 85) to dd25 (d25 in Figure 85) and around dd32 (d32 in Figure 85). It was confirmed that dd11 or later is preferable as the timing of purification and more preferably purification is performed on dd18 to dd32 or later (Figure 85).

### <Example 29 Study on removal of non-target cell with SSEA1 as index>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

Aggregates (NR) of Day 25 prepared by adding 30 nM SAG at the start of suspension culture (at the start of differentiation, dd0) were dispersed into single cells using a neuronal cell dispersion solution (FUJIFILM Wako Pure Chemical Corp.) and stained with BV421-conjugated anti-SSEA1 antibody (BioLegend, Inc.) in addition to APC-conjugated anti-CD9 antibody (BioLegend, Inc.). Measurement was performed using FACSCantoII (BD Biosciences), and analysis was conducted using FlowJo.

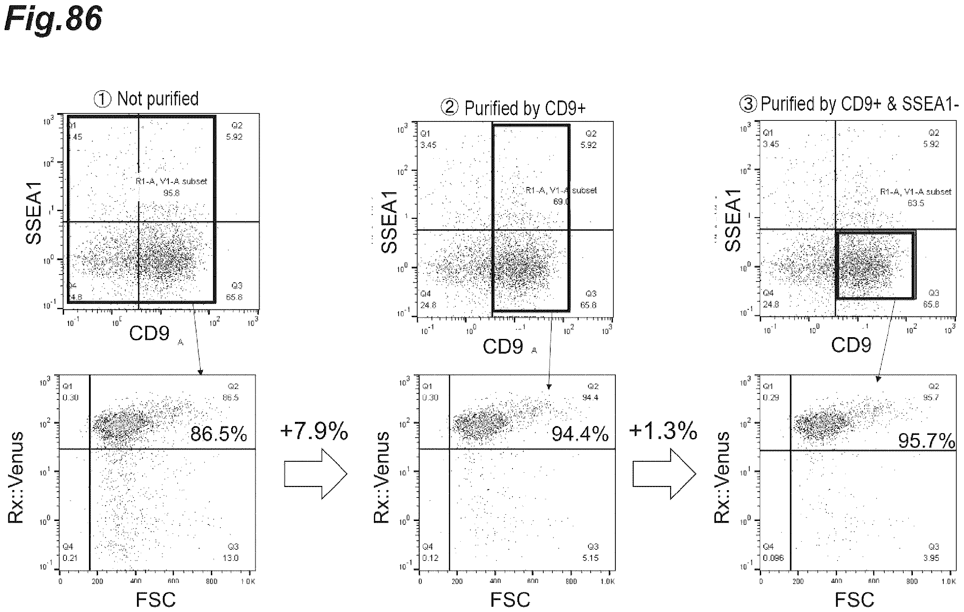

As a result, Rx-positive cells were about 87% in the whole aggregates. When CD9-positive cells were gated, the Rx-positive cells were found to be increased to 94%. When CD9-positive and SSEA1-negative cells were further gated, the Rx-positive cells were found to be increased to about 96% (Figure 86).

Accordingly, it was found that retinal cells can be more purified by combining SSEA1 in addition to CD9.

### <Example 30 Study on combination of CD9, CD90, CXCR4, and SSEA1>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

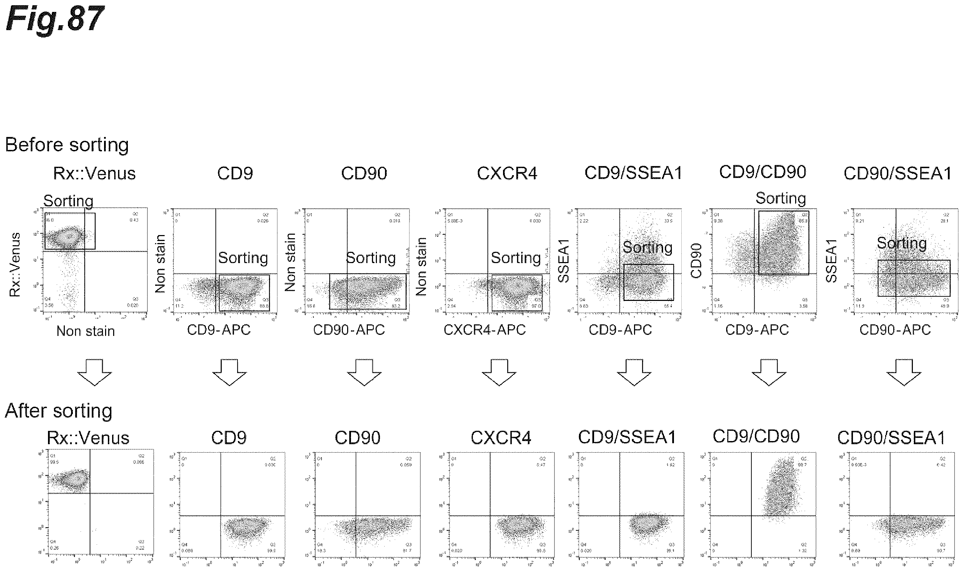

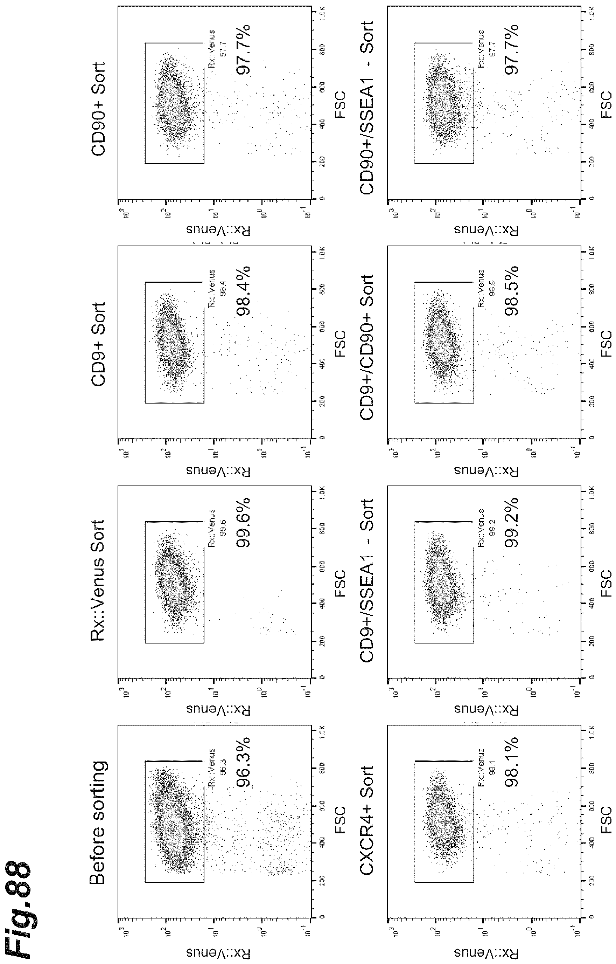

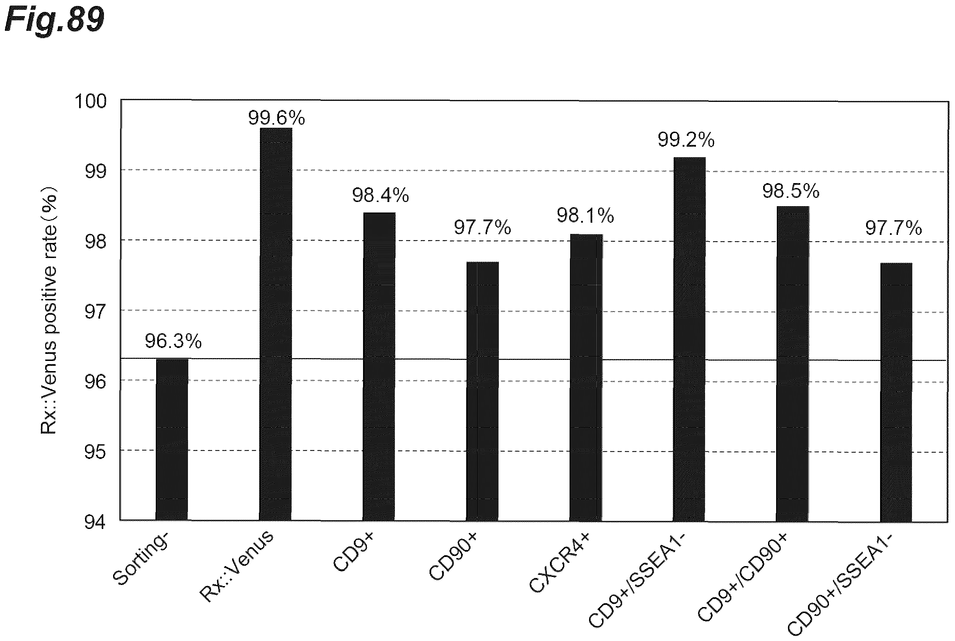

200 aggregates (NR) of Day 25 prepared by adding 30 nM SAG at the start of suspension culture (at the start of differentiation, dd0) were dispersed into single cells using a neuronal cell dispersion solution (FUJIFILM Wako Pure Chemical Corp.), and the cells were equally divided into 7. Surface antigens were stained at 4°C for 1 hour under the following 7 conditions: staining was performed using (1) non-staining (purification with Rx:: Venus as an index), (2) anti-CD9-APC-conjugate antibody (BioLegend, Inc.), (3) anti-CD90-APC-conjugate antibody (Becton, Dickinson and Company), (4) anti-CD184 (CXCR4)-APC-conjugate antibody (Miltenyi Biotec), (5) anti-CD9-APC-conjugate antibody (BioLegend, Inc.) and anti-SSEA1-BV421-conjugate antibody (BioLegend, Inc.), (6) anti-CD9-APC-conjugate antibody (BioLegend, Inc.) and anti-CD90-BV421-conjugate antibody (BioLegend, Inc.), and (7) anti-CD90-APC-conjugate antibody (Becton, Dickinson and Company) and anti-SSEA1-BV421-conjugate antibody (BioLegend, Inc.). After staining, sorting was carried out with the boxes shown in Figure 87 as indexes using ARIA II (Becton, Dickinson and Company). Cells recovered by sorting were subjected to FACS analysis using MACS Quant10 (Miltenyi Biotec) to confirm that target cell fractions were able to be purified (Figure 87). When the percentage of Rx:: Venus-positive cells in the populations of cells after purification was measured, the purity was highest in the (1) Rx::Venus-positive fraction among the sorted samples as compared with before sorting. However, the Rx:: Venus positive rate was high in no way inferior to the sample purified with Rx::Venus, in the samples purified with CD9-positive and SSEA-1-negative fractions (Figures 88 and 89).

These cells after sorting were seeded at 2.0 × 10⁵ cells per well into 24-well Transwell coated with Laminin511-E8. 10 µM Y-27632 (FUJIFILM Wako Pure Chemical Corp.), 300 nM SAG (Enzo Life Sciences, Inc.), 3 µM CHIR99021 (FUJIFILM Wako Pure Chemical Corp.), and 100 ng/mL FGF8 (FUJIFILM Wako Pure Chemical Corp.) were added at the time of seeding. After seeding, medium replacement was carried out once every 3 to 4 days.

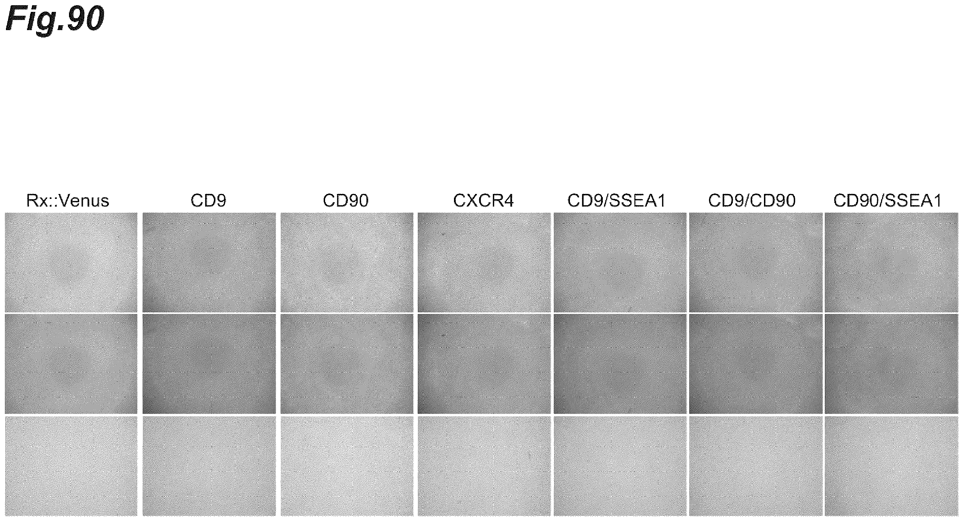

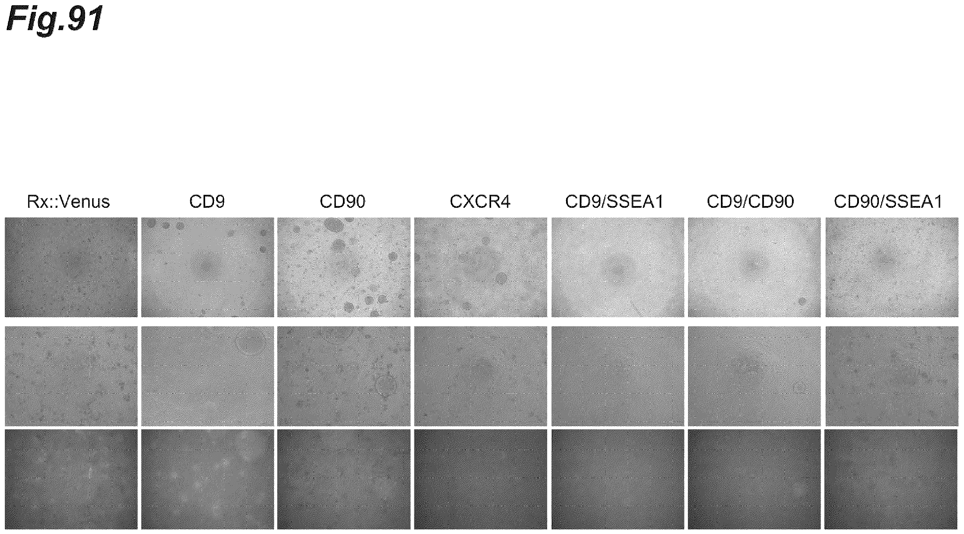

When bright field and fluorescent observation was made under a fluorescence microscope on Day 1 and Day 12 from initiation of the culture for preparation into a sheet again, cells were observed to be engrafted on the mesh of Transwell as to all the cells after purification on Day 1 after seeding (Figure 90). In observation further 12 days later, some black RPEs were observed in the Rx:: Venus-positive samples purified, whereas black RPE was not observed in the sheets purified with CD9 in the CD9-positive and CD9-positive/SSEA1-negative (CD9/SSEA1) populations and the CD9-positive/CD90-positive (CD9/CD90) populations (Figure 91).

Accordingly, it was confirmed that retina sheets sorted with CD9 and with CD9 and SSEA1-negative or CD90-positive fractions can remove RPE fractions more than Rx:: Venus-positive sorting and are better.

### <Example 31 Confirmation of time-dependent change in expression of CD9 and SSEA1>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

hESC and aggregates (NR) of dd4, dd11, dd18, dd25, dd32, and dd46 prepared by adding 30 nM SAG at the start of suspension culture (at the start of differentiation, dd0) were dispersed into single cells using neuronal cell dispersion solution (FUJIFILM Wako Pure Chemical Corp.), stained with anti-CD9-APC-conjugate antibody (BioLegend, Inc.) and anti-SSEA1-BV421-conjugate antibody (BioLegend, Inc.), and subjected to FACS analysis. Measurement was performed using FACSCantoII (BD Biosciences), and analysis was conducted using FlowJo.

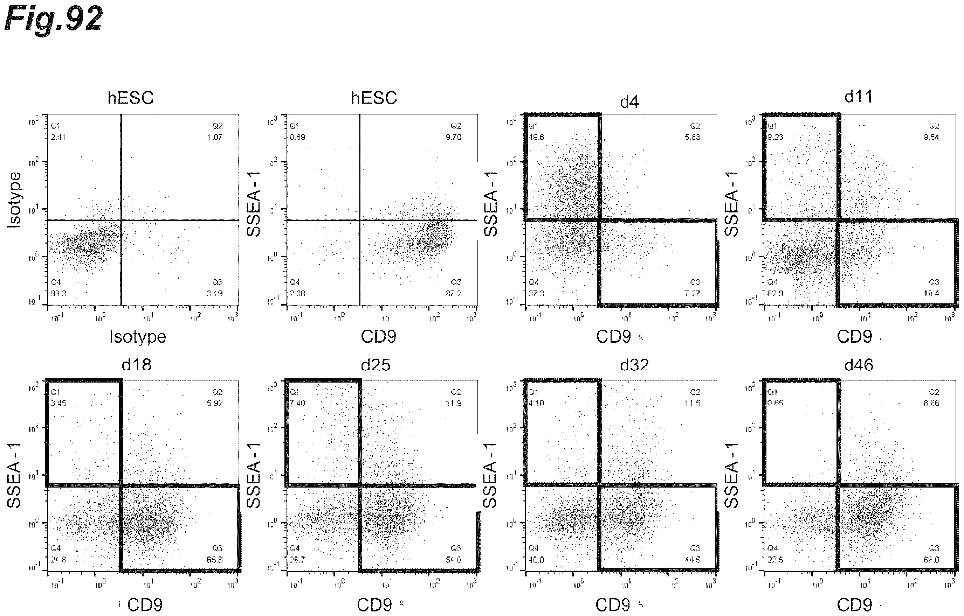

As a result, in hESC, CD9 was positive, and SSEA1 was negative. However, on Day 4 from initiation of the differentiation, CD9 became negative, and SSEA1 became positive (Figure 92). From Differentiation Day 11, a CD9-positive and SSEA1-negative population started to appear, and it was found that the percentage of the target population increased on Day 18 and Day 25. Continuous expression was also measured on Day 32 and Day 46.

Accordingly, it was confirmed that dd11 (d11 in Figure 92) is preferable as the timing of purification and it is preferable to perform purification on dd18 (d18 in Figure 92) to dd32 (d32 in Figure 92) or later (Figure 92).

The retina sheets on 12-well Transwell prepared in this way by performing sorting with CD9 and SSEA1 as indexes and adding 10 µM Y-27632, 3 µM CHIR99021, 300 nM SAG and 100 ng/mL FGF8 were recovered using a scalpel or scissors on Days 48 and 62 from initiation of the suspension culture. Then, long grafts for transplantations were dissected using microscissors, and subretinal transplantation to immunodeficient retina-deficient rats (SD Foxn) was carried out (not shown).

Accordingly, it was confirmed that retina sheets manufactured using cells sorted with CD9 and SSEA1 are transplantable.

### <Example 32 RPE-retinal tissue complex formation study using gelatin>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

The aggregates (NR) of Days 18 to 30 from initiation of the suspension culture prepared in Example 1 were washed with PBS, and a neuronal cell dispersion solution (manufactured by FUJIFILM Wako Pure Chemical Corp.) was added. After incubation at 37°C, they were dispersed into single cells by pipetting and seeded at 4.0 to 8.0 × 10⁵ cells per well into 12-well Transwell. Y-27632, SAG, and CHIR99021 were added to NucTO medium at the time of seeding. Then, medium replacement was carried out with NucTO medium, which did not contain Y-27632, SAG, or CHIR99021, once every 3 to 4 days, and culture was performed for 1 month or more.

The aggregates of 80 days or later from initiation of the suspension culture in which RPE was also simultaneously differentiated, prepared in Example 1 were seeded into a culture medium in which B27 (Gibco), L glutamine, 10 ng/mL FGF2, and SB431542 were added to DMEM (Sigma-Aldrich Co. LLC) and F12 Ham (Sigma-Aldrich Co. LLC) media in a dish coated with Laminin511-E8, and cultured. After RPE adhered, then proliferated and spread, only RPE was scraped off using a 1-mL tip, seeded into another dish coated with Laminin511-E8, and subjected to purification. Then, after expansion culture, 1.0 × 10⁶ cells of RPE were seeded into 12-well Transwell coated with Laminin511-E8. Medium replacement was carried out once every 3 to 4 days, and culture was performed for 1 month or more.

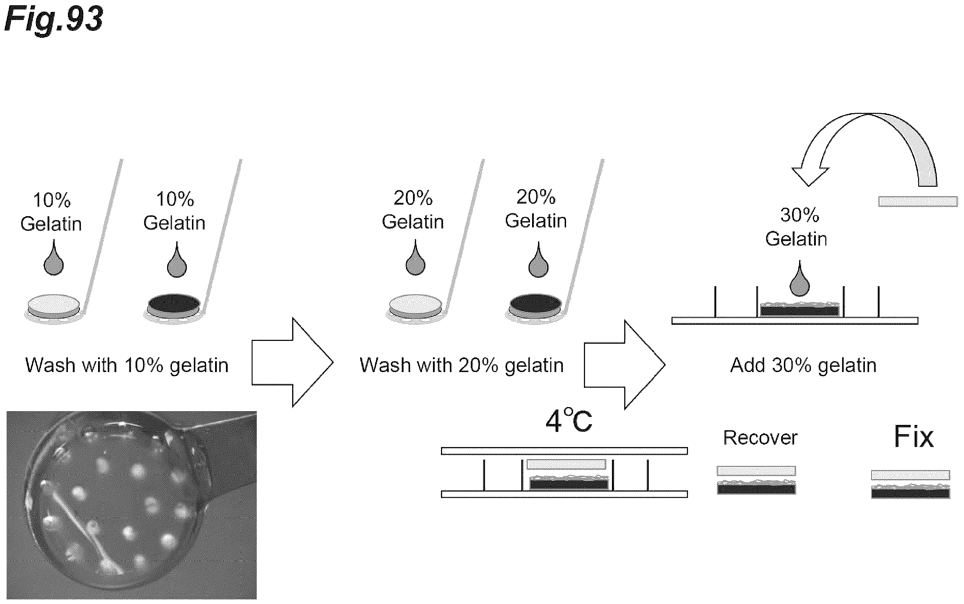

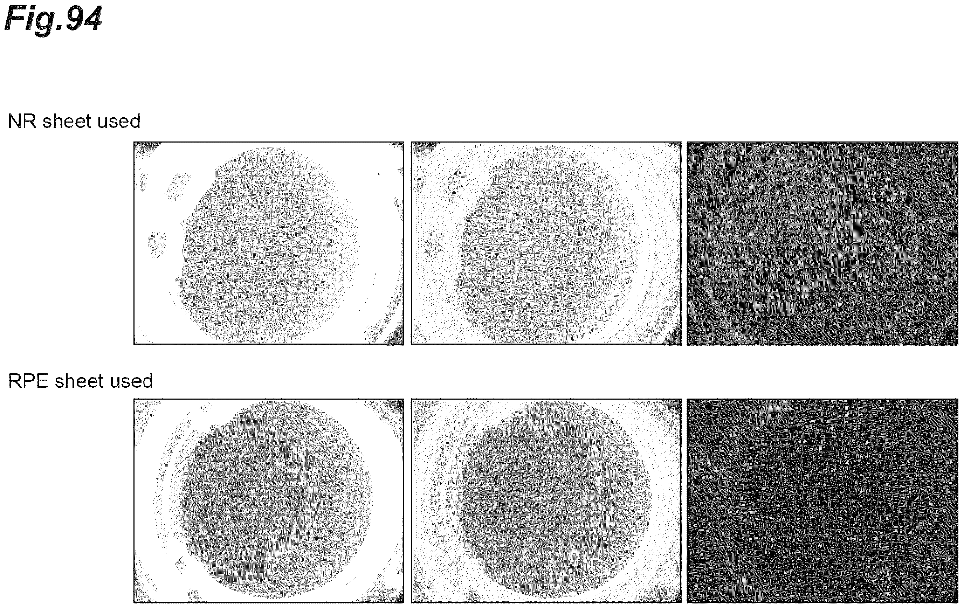

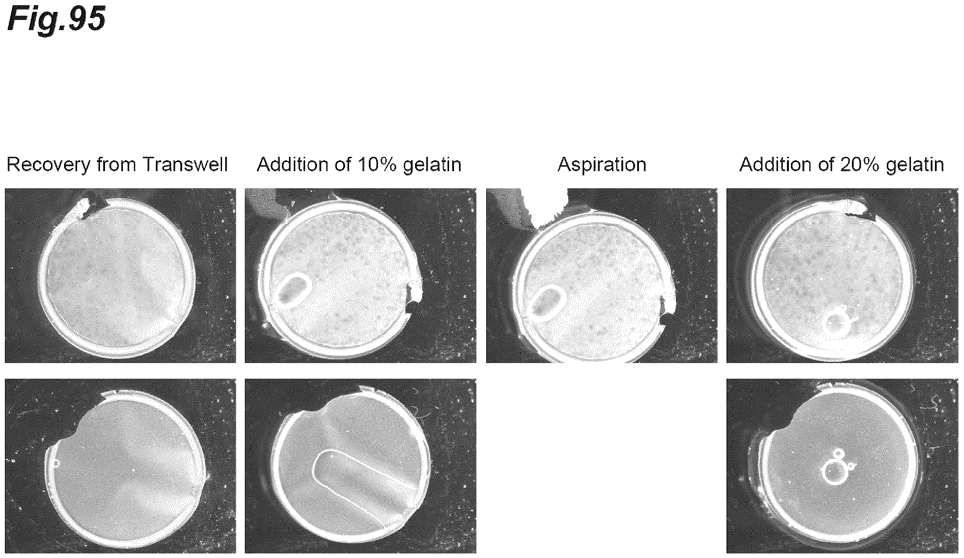

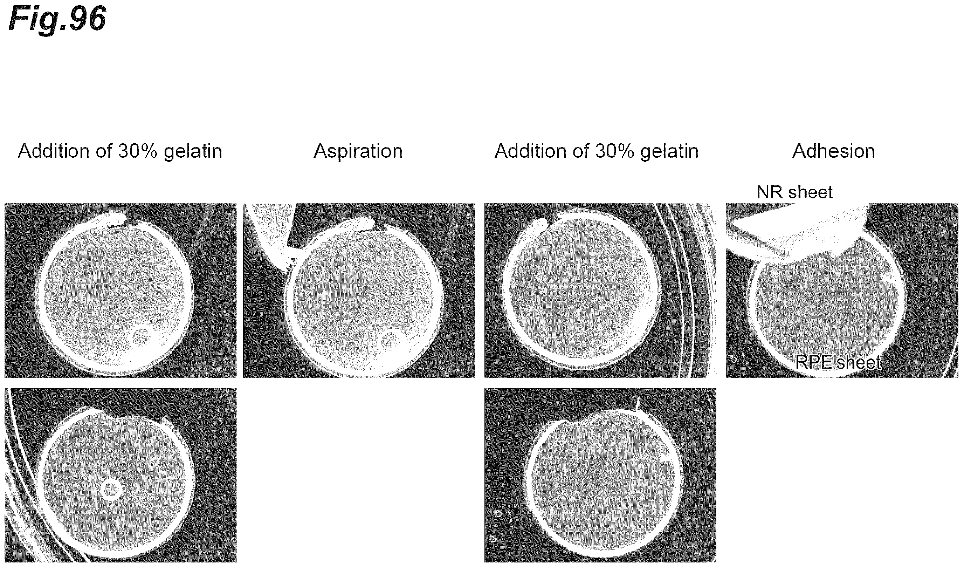

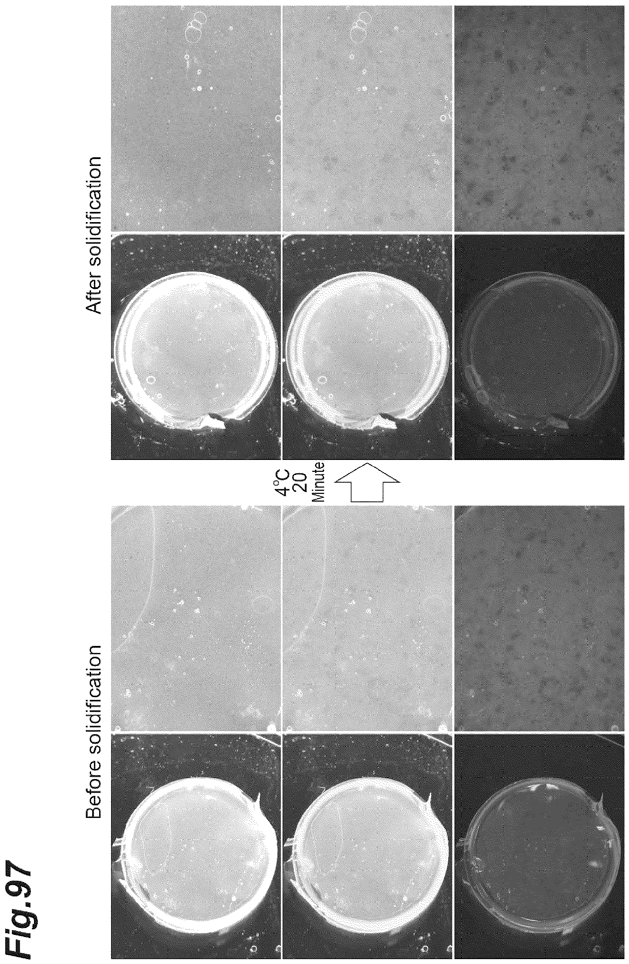

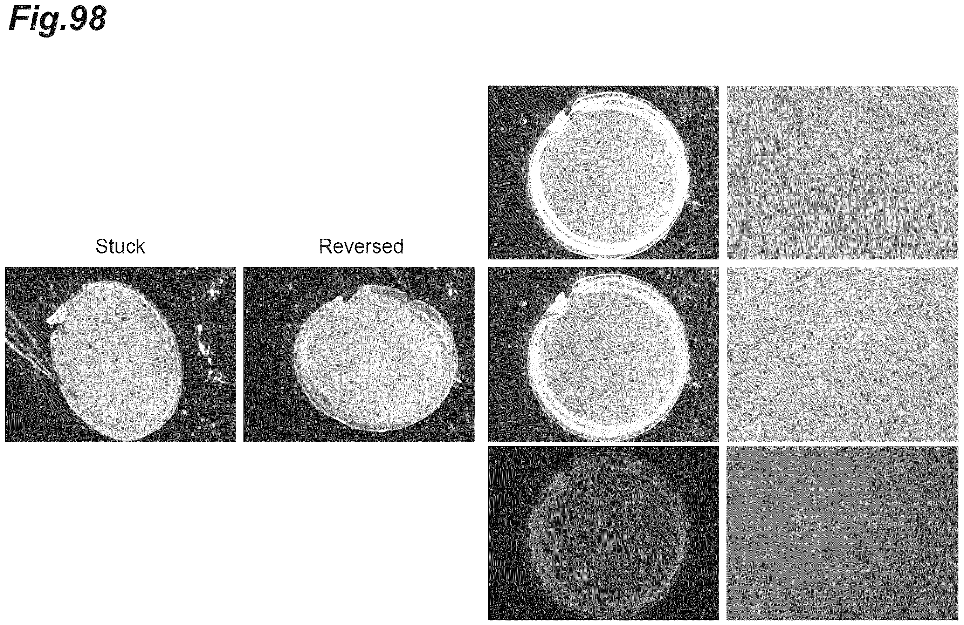

The adhesion procedures of the RPE sheet and the sheet-shaped retinal tissue cultured as described above were performed using BeMatrix LS-W gelatin (Nitta Gelatin Inc.). As specific operation, each sheet was recovered from Transwell using tweezers or scissors. To the sheet-shaped retinal tissue and the RPE sheet, 10% (w/v) gelatin was applied, then 20% gelatin was applied, and finally 30% gelatin was added so that the sheet-shaped retinal tissue and the RPE sheet were allowed to adhere. After adhesion, solidification was performed by rapid cooling to 4°C and incubation for 20 minutes, and a complex sheet in which the sheet-shaped retinal tissue and the RPE sheet were allowed to adhere was prepared by recovery (Figures 93 to 98).

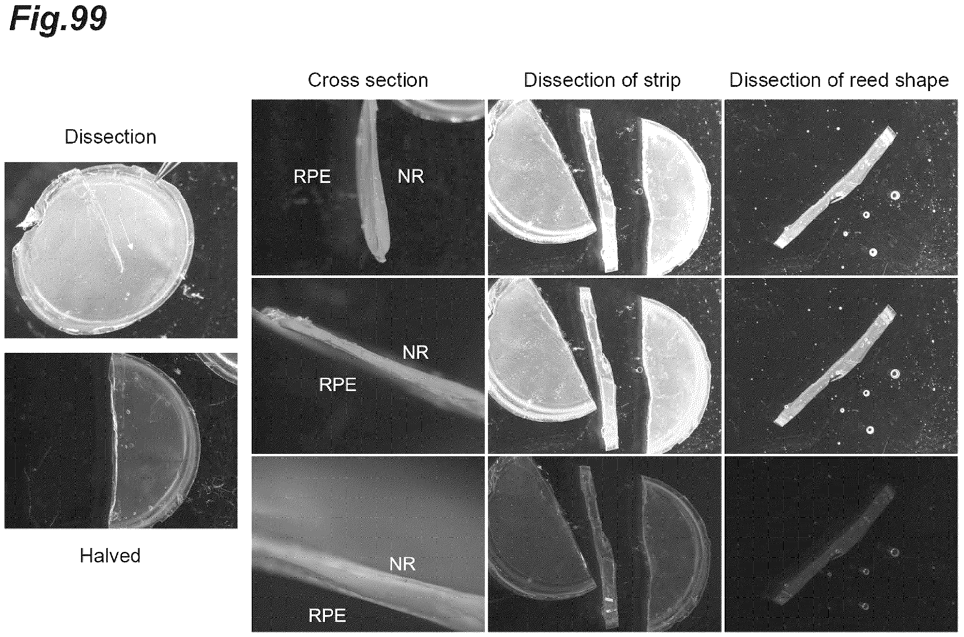

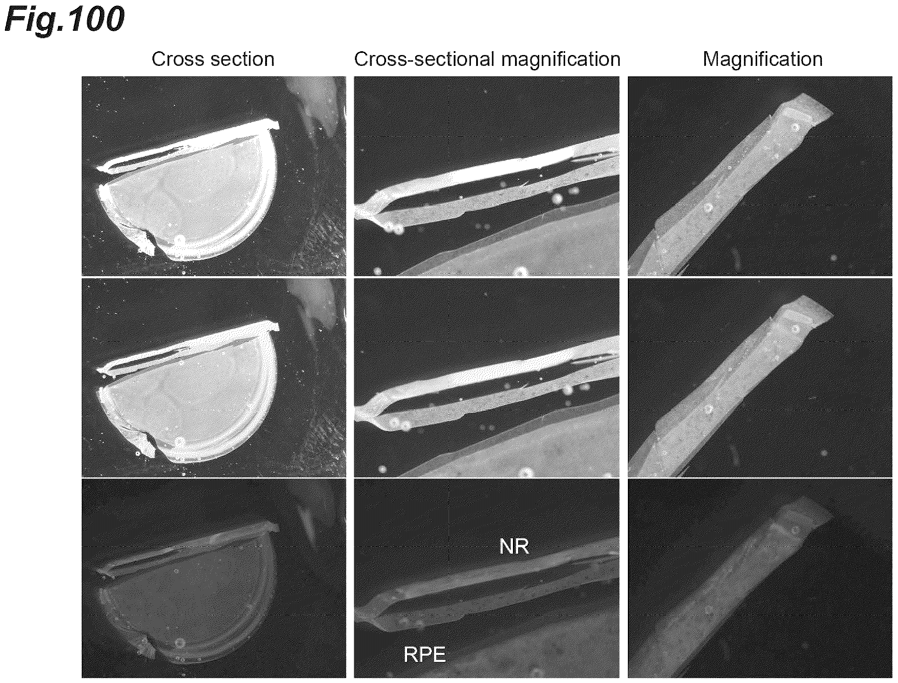

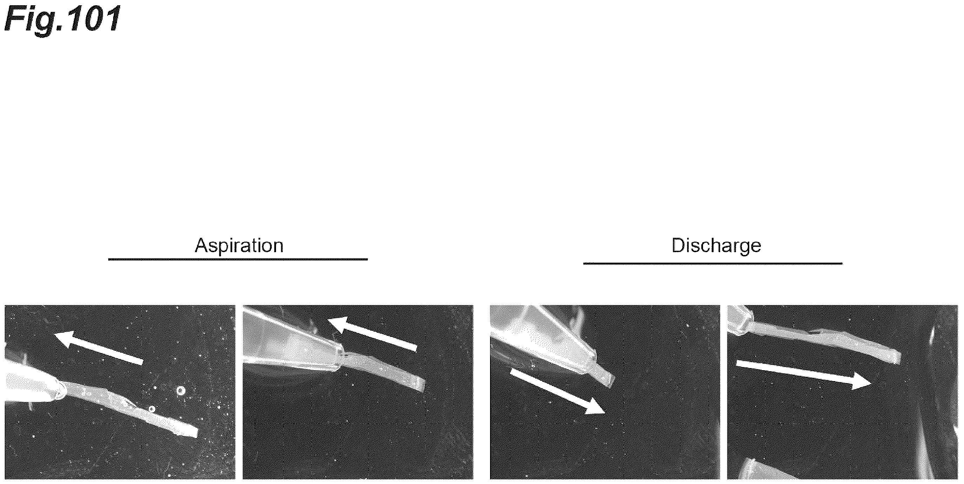

Dissection was performed in the retinal tissue-RPE complex sheet prepared in this way using tweezers and scissors. First, the complex sheet was halved, and strips were then dissected along the dissected surface (Figure 99). As a result, dissection was achieved in a state in which both the sheets adhered via gelatin. When the cross section of this dissected complex sheet was observed, RPE having a black dye was confirmed on one side and a Rx:: Venus-positive sheet-shaped retinal tissue was confirmed on the opposite side, between which gelatin was observed to be packed (Figure 100). In order to examine whether this dissected complex sheet would come off by the action of aspiration and discharge, which are procedures of transplantation, the operation of aspiration and discharge was performed using a 1-mL tip whose top was chopped off. As a result, the complex sheet was able to be aspirated and discharged many time without separating (Figure 101).

Accordingly, it was found that a transplantable retinal tissue-RPE complex sheet can be prepared by using a sheet-shaped retinal tissue, a RPE sheet, and gelatin.

### <Example 33 RPE-retinal tissue complex formation study using fibrin>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

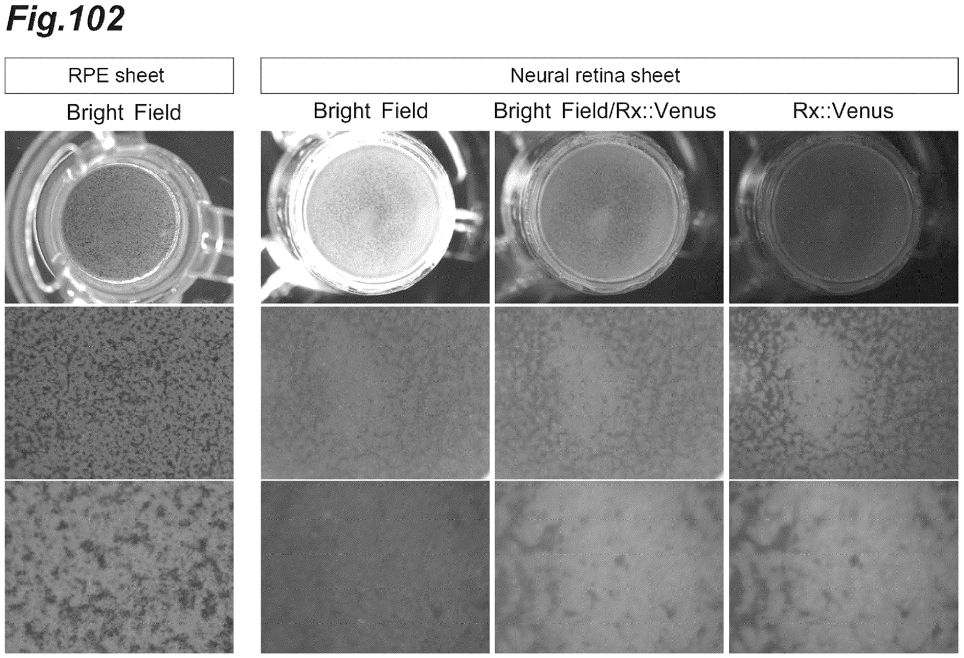

The aggregates (NR) of Days 18 to 30 from initiation of the suspension culture prepared in Example 1 were washed with PBS, and a neuronal cell dispersion solution (manufactured by FUJIFILM Wako Pure Chemical Corp.) was added. After incubation at 37°C, they were dispersed into single cells by pipetting and seeded at 4.0 to 8.0 × 10⁵ cells per well into 12-well Transwell. Y-27632, SAG, and CHIR99021 were added to NucTO medium at the time of seeding. Then, medium replacement was carried out with NucTO medium, which did not contain Y-27632, SAG, or CHIR99021, once every 3 to 4 days, and culture was performed for 1 month or more (Figure 102).

The aggregates of 80 days or later from initiation of the suspension culture in which RPE was also simultaneously differentiated, prepared in Example 1 were seeded into a culture medium in which B27 (Gibco), L glutamine, 10 ng/mL FGF2, and SB431542 were added to DMEM (Sigma-Aldrich Co. LLC) and F12 Ham (Sigma-Aldrich Co. LLC) media in a dish coated with iMatrix511, and cultured. After RPE adhered, then proliferated and spread, only RPE was scraped off using a tip, seeded into another dish coated with iMatrix511, and subjected to purification. Then, after expansion culture, 1.0 × 10⁶ cells of RPE were seeded into 12-well Transwell coated with iMatrix511. Medium replacement was carried out once every 3 to 4 days, and culture was performed for 1 month or more (Figure 102).

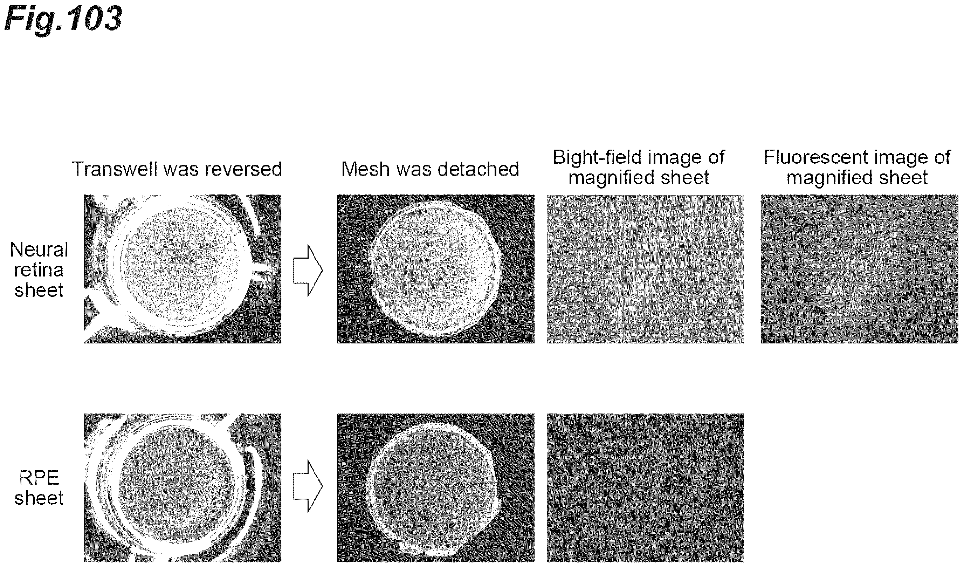

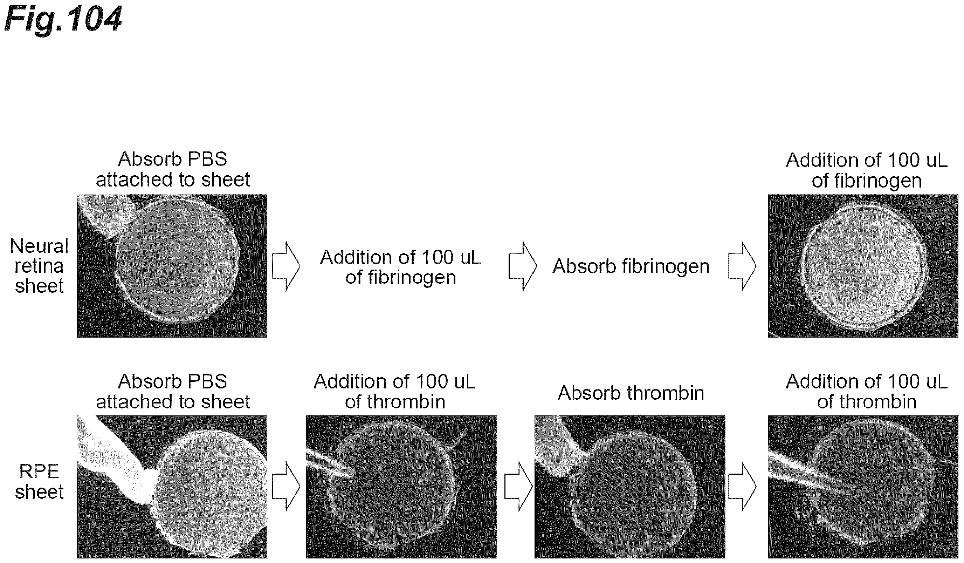

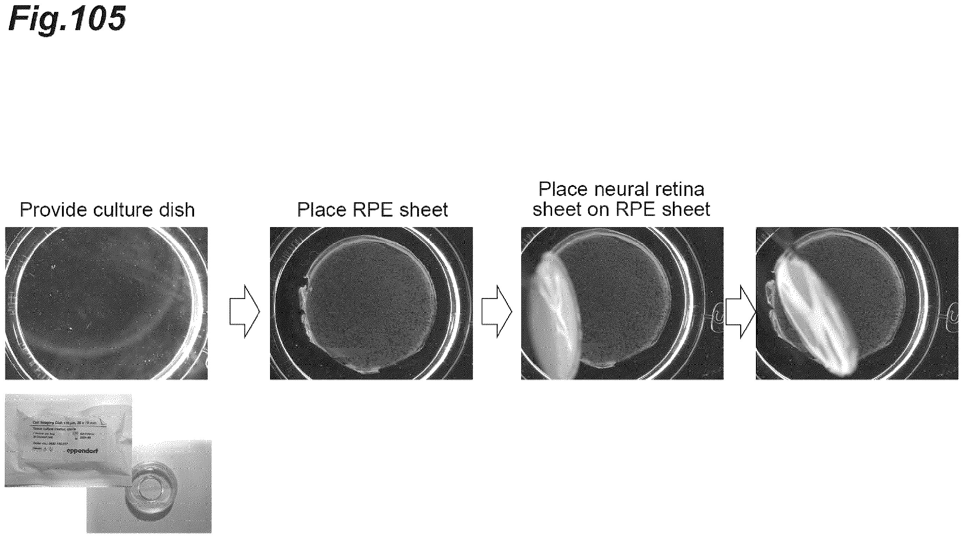

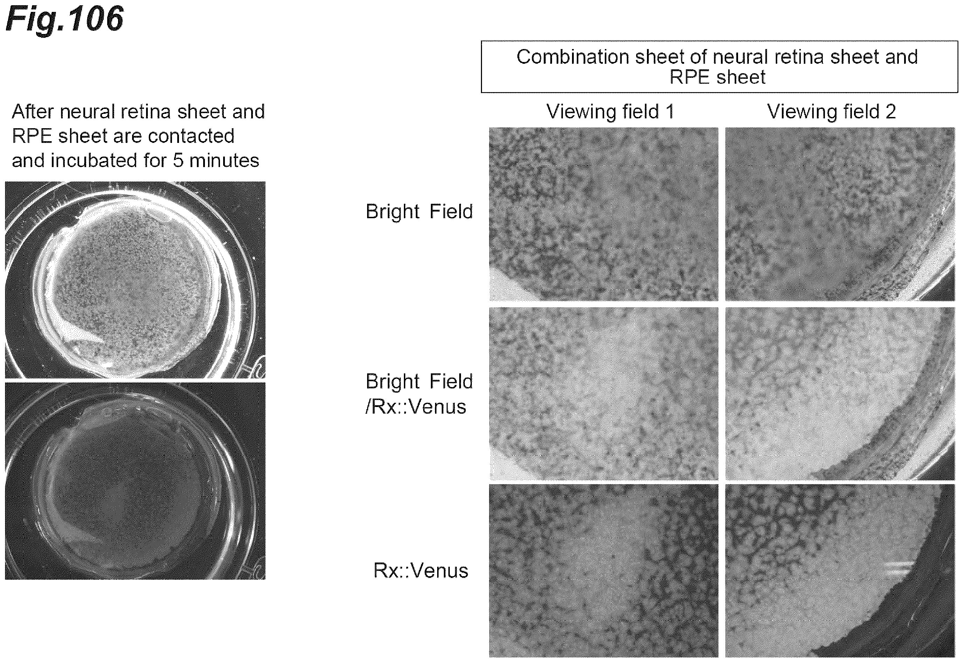

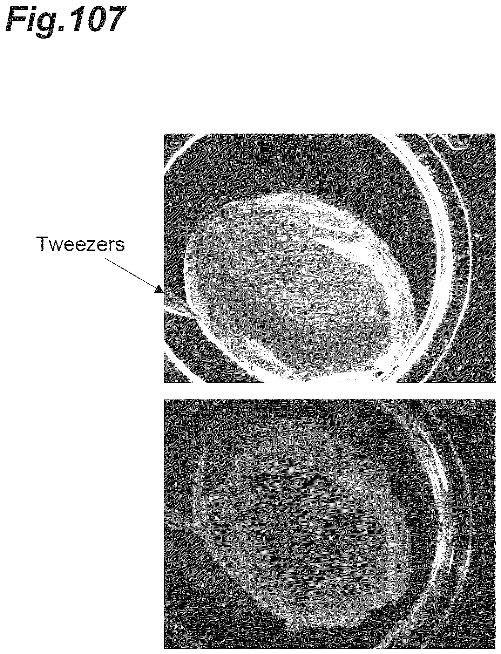

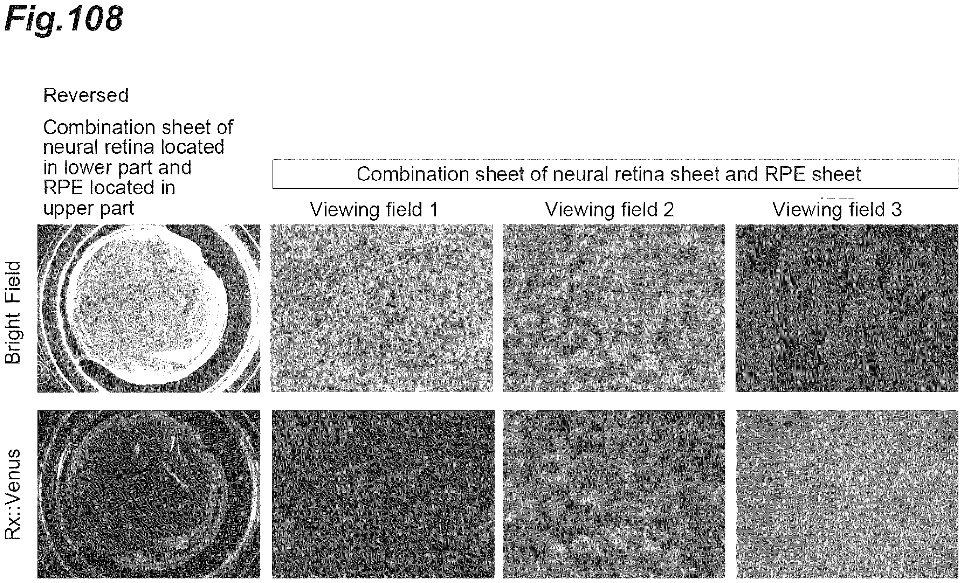

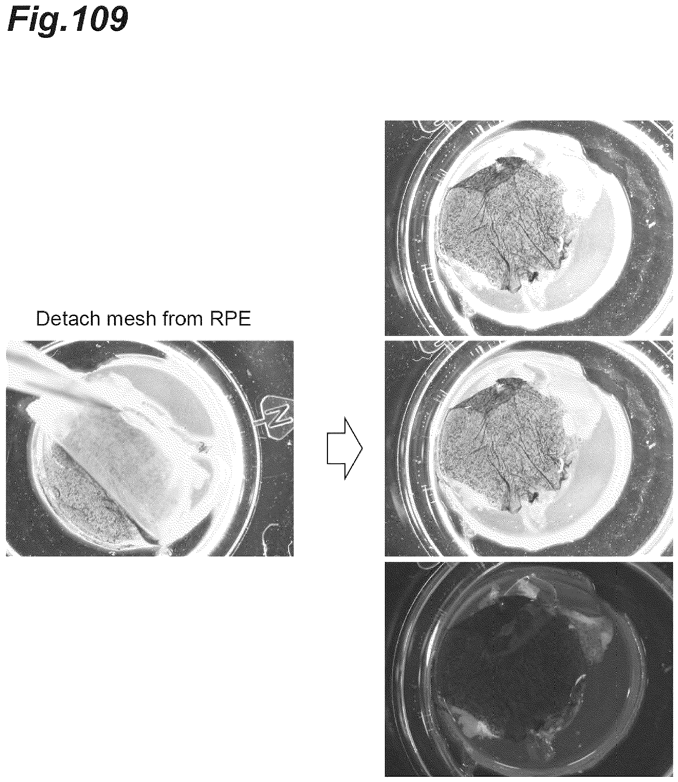

The adhesion procedures of the sheet-shaped retinal tissue and the RPE sheet cultured as described above were performed using Bolheal for tissue adhesion (Teijin Pharma Ltd.). As specific operation, each sheet was recovered from Transwell using tweezers or scissors. 100 µL of fibrinogen was added to the sheet-shaped retinal tissue, and 100 µL of thrombin was added to the RPE sheet. They were applied and then removed (Figures 103 and 104). Again, 100 µL of fibrinogen was added to the sheet-shaped retinal tissue, and 100 µL of thrombin was added to the RPE sheet (Figure 104). The RPE sheet was placed on a culture dish such that the apical side was located on the upper side and the mesh side was located on the culture dish side. The sheet-shaped retinal tissue was placed from above such that the apical surface faced the RPE side (Figure 105). After adhesion, solidification was performed by incubation at room temperature for 5 minutes, and a sheet that adhered was prepared by recovery (Figures 106 and 107). The retinal tissue-RPE complex sheet prepared in this way was reversed using tweezers, placed such that the sheet-shaped retinal tissue faced the lower side and the retinal pigment epithelial sheet faced the upper side, and observed (Figure 108). The mesh of Transwell that adhered was detached (Figure 109).

Accordingly, it was found that a retinal tissue-RPE complex sheet can be prepared by using a sheet-shaped retinal tissue, a RPE sheet, and fibrin.

### <Example 34 Study on removal of unnecessary gelatin between two sheets using CellShifter>

In the formed complex sheet studied in Example 32, it was found that a large amount of gelatin was contained between two sheets and caused thickening. Accordingly, study was made on a complex formation device in which a passage through which gelatin would escape was formed such that unnecessary gelatin between two sheets was able to be removed by pushing at the time of the complex formation step.

Two, four, or five levee-shaped passages were prepared on a glass slide using a silicon sheet, and two pieces of CellShifter (CellSeed Inc.) between which gelatin was added were used and pushed from above to observe whether gelatin ran out thereof.

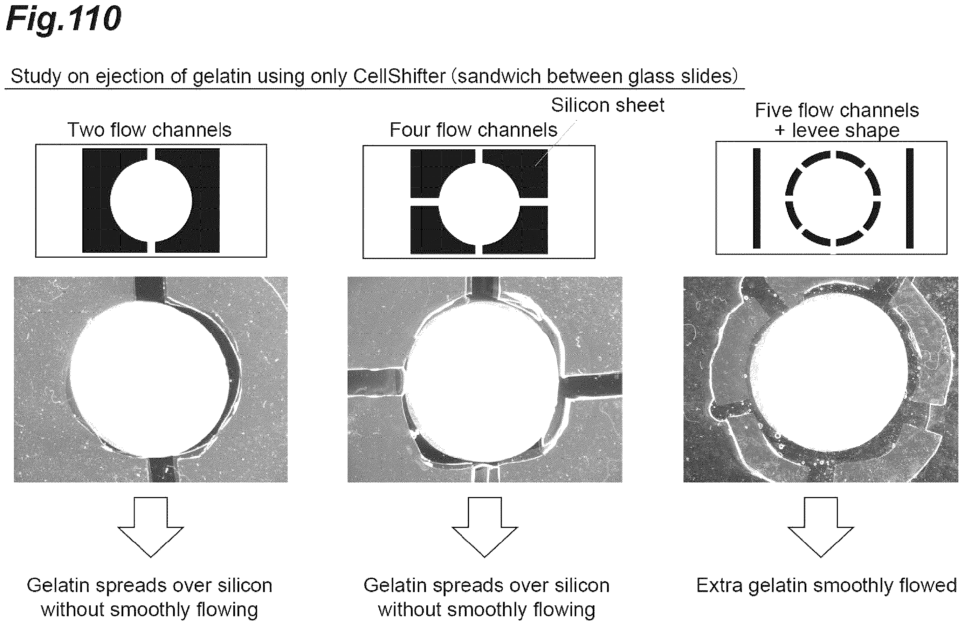

As a result, it was found that extra gelatin ran out most in the device having five flow channels in which a silicon sheet was placed in a levee shape (Figure 110).

### <Example 35 Preparation of planarized sheet on temperature-responsive culture dish, and detachment and transplantation study>

Human ES cells (KhES-1 strain (Non Patent Literature 3)) genetically engineered so as to have Rx:: Venus reporter gene were cultured under feeder-free conditions in accordance with the method described in "Scientific Reports 4, 3594 (2014)". As a feeder-free medium, StemFit medium (trade name: AK03N, manufactured by Ajinomoto Co., Inc.) was used, and as a scaffold in place of feeder cells, Laminin511-E8 (trade name, manufactured by Nippi, Inc.) was used.

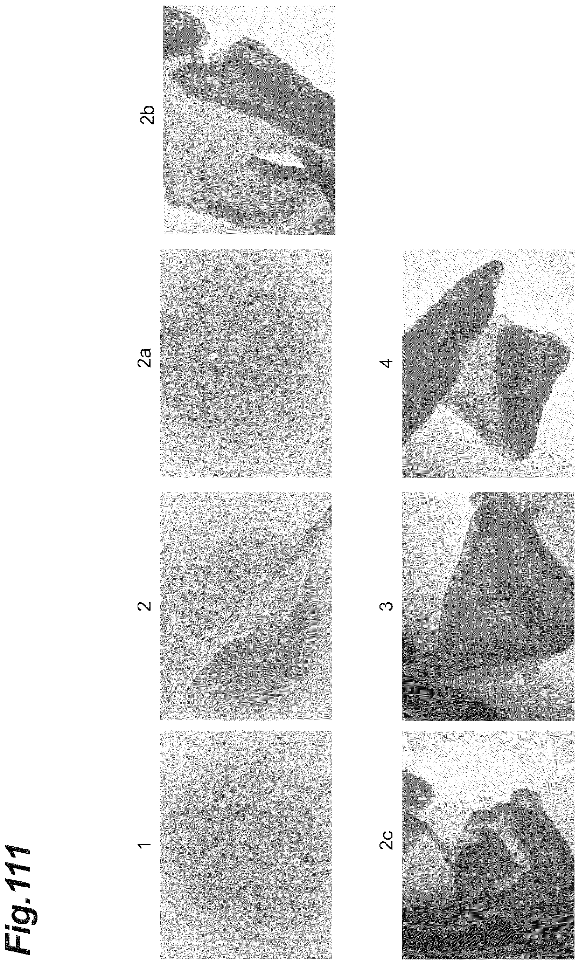

The aggregates (NR) of Days 18 to 30 prepared in Example 1 were washed with PBS, and a neuronal cell dispersion solution (manufactured by FUJIFILM Wako Pure Chemical Corp.) was added. After incubation at 37°C, they were dispersed into single cells by pipetting, and the cells were seeded at 8.0 × 10⁵ cells/well into a 24-well temperature-responsive culture dish (CellSeed Inc.) with 7 different degrees of detachment coated with Laminin511-E8. Medium replacement was carried out once every 3 to 4 days. 30 days or later after seeding, a sheet was formed with a thickness, then incubated with the temperature lowered to room temperature for 2 hours, and observed. As a result, in a plurality of wells of 2b, 2c, 3, and 4 in the temperature-responsive culture dish with 7 degrees of detachment, the retina sheet was observed to be detachable by creating room temperature (Figure 111).

Accordingly, it was found that a retina sheet can be recovered by performing culture in a temperature-responsive culture dish.

The retina sheet on the temperature-responsive culture dish prepared in this way by performing sorting with Rx:: Venus as an index and adding 10 µM Y-27632, 3 µM CHIR99021, 300 nM SAG and 100 ng/mL FGF8 was incubated at 4°C for 1 hour on Day 62 from initiation of the suspension culture, and recovered. Then, long grafts for transplantations were dissected using microscissors, and subretinal transplantation to immunodeficient retina-deficient rats (SD Foxn) was carried out (not shown).

Accordingly, it was confirmed that a retina sheet cultured on a temperature-responsive culture dish is transplantable.

## Claims

1. A method for man
ufacturing retinal tissue having an epithelial structure, comprising
suspension-culturing or adhesion-culturing a dispersed retinal cell population in a culture medium comprising a Wnt signaling pathway agonist, wherein
the retinal cell population comprises one or more cells selected from the group consisting of a retinal progenitor cell and a photoreceptor progenitor cell.

2. The manufacturing method according to claim 1, wherein the Wnt signaling pathway agonist is one or more substances selected from the group consisting of CHIR99021, BIO, Wnt2b and Wnt3a.

3. The manufacturing method according to claim 1 or 2, wherein the culture medium further comprises one or more substances selected from the group consisting of a ROCK inhibitor, a SHH signaling pathway agonist and an FGF signaling pathway agonist.

4. The manufacturing method according to claim 3, wherein the ROCK inhibitor is one or more substances selected from the group consisting ofY-27632, fasudil (HA1077) and H-1152.

5. The manufacturing method according to claim 3 or 4, wherein the SHH signaling pathway agonist is one or more substances selected from the group consisting of SAG, PMA and SHH.

6. The manufacturing method according to any one of claims 3 to 5, wherein the FGF signaling pathway agonist is one or more fibroblast growth factors selected from the group consisting of FGF2, FGF4 and FGF8.

7. The manufacturing method according to any one of claims 1 to 6, comprising performing the adhesion culture in the culture medium comprising the Wnt signaling pathway agonist, wherein the retinal tissue having an epithelial structure is a sheet-shaped retinal tissue.

8. The manufacturing method according to claim 7, wherein the adhesion culture is performed using a culture container coated with an extracellular matrix and/or a temperature-responsive polymer.

9. The manufacturing method according to claim 8, wherein a culture surface of the culture container is coated with the temperature-responsive polymer, and an upper surface of the temperature-responsive polymer is coated with the extracellular matrix.

10. The manufacturing method according to claim 8 or 9, wherein the extracellular matrix is one or more substances selected from the group consisting of collagen, laminin, fibronectin, Matrigel and vitronectin.

11. The manufacturing method according to any one of claims 8 to 10, further comprising the step of exposing the culture container coated with the temperature-responsive polymer to a temperature that changes the properties of the temperature-responsive polymer to thereby detach the sheet-shaped retinal tissue from the culture container.

12. The manufacturing method according to any one of claims 1 to 11, comprising the step of dispersing a cell aggregate comprising one or more cells selected from the group consisting of a retinal progenitor cell and a photoreceptor progenitor cell to obtain the dispersed retinal cell population.

13. The manufacturing method according to claim 12, comprising the step of differentiating pluripotent stem cells to obtain the cell aggregate comprising one or more cells selected from the group consisting of the retinal progenitor cell and the photoreceptor progenitor cell.

14. The manufacturing method according to any one of claims 1 to 13, further comprising the step of increasing the percentage of a retinal progenitor cell comprised in the dispersed retinal cell population before suspension-culturing or adhesion-culturing the dispersed retinal cell population.

15. The method according to claim 14, wherein contamination with or differentiation into retinal pigment epithelial cells is suppressed.

16. The manufacturing method according to claim 14 or 15, wherein the step of increasing the percentage of a retinal progenitor cell comprises the step of contacting the dispersed retinal cell population with a substance that binds to one or more antigens selected from the group consisting of CD9, CD39, CD90 and CXCR4 to obtain a cell population expressing the antigens.

17. The manufacturing method according to claim 16, wherein the step of increasing the percentage of a retinal progenitor cell comprises the step of further contacting the dispersed retinal cell population with a substance that binds to one or more antigens selected from the group consisting of SSEA1, CD66b, CD69 and CD84 to obtain a cell population having expression levels of the antigens that are equal to or less than a reference.

18. The manufacturing method according to any one of claims 14 to 17, wherein the step of increasing the percentage of a retinal progenitor cell comprises the following steps:
(1) culturing pluripotent stem cells in the presence of one or more selected from the group consisting of a Shh signaling pathway agonist, ATP and an A2A receptor agonist to manufacture a cell aggregate;
(2) differentiating the cell aggregate into a retinal progenitor cell; and
(3) dispersing the cell aggregate and contacting it with a substance that binds to CD39.

19. The manufacturing method according to any one of claims 1 to 18, wherein the retinal progenitor cell and/or the photoreceptor progenitor cell occupies 50% or more of the total number of cells comprised in the retinal cell population.

20. The manufacturing method according to any one of claims 1 to 18, wherein the retinal progenitor cell and/or the photoreceptor progenitor cell occupies 80% or more of the total number of cells comprised in the retinal cell population.

21. The manufacturing method according to any one of claims 1 to 20, wherein from the start of the suspension culture or the adhesion culture, the dispersed retinal cell population is cultured in the culture medium comprising the Wnt signaling pathway agonist.

22. The manufacturing method according to any one of claims 1 to 21, wherein in the epithelial structure, the orientation of cells is a direction roughly perpendicular to the layer direction.

23. The manufacturing method according to any one of claims 1 to 22, further comprising the step of dissecting a size necessary for transplantation from the retinal tissue having an epithelial structure obtained by the suspension culture or the adhesion culture.

24. The manufacturing method according to any one of claims 1 to 23, wherein the epithelial structure is a multilayered structure.

25. A sheet-shaped retinal tissue consisting of a retinal cell layer having a multilayered structure, wherein
(1) the retinal cell layer having a multilayered structure has polarities of a basal surface and an apical surface,
(2) the retinal cell layer having a multilayered structure comprises one or more cells selected from the group consisting of a retinal progenitor cell, a photoreceptor progenitor cell and a photoreceptor cell,
(3) in each layer of the retinal cell layer, the orientation of cells is a direction roughly perpendicular to the layer direction, and
(4) a diameter is 8 mm or more.

26. The sheet-shaped retinal tissue according to claim 25, wherein the retinal cell layer having a multilayered structure further comprises a sheet-shaped retinal pigment epithelial cell joined to the retinal cell layer, wherein the tangent directions of the respective surfaces of the retinal cell layer and the sheet-shaped retinal pigment epithelial cell are roughly in parallel, the apical surface of the retinal cell layer and the apical surface of the sheet-shaped retinal pigment epithelial cell face each other, and the retinal cell layer and the sheet-shaped retinal pigment epithelial cell are joined through an adhesion factor present therebetween.

27. The sheet-shaped retinal tissue according to claim 26, wherein the adhesion factor is an extracellular matrix or a hydrogel.

28. The sheet-shaped retinal tissue according to claim 27, wherein the adhesion factor is one or more substances selected from gelatin, fibrin, fibronectin, hyaluronic acid, laminin, IV-type collagen, heparan sulfate proteoglycan and entactin.

29. The sheet-shaped retinal tissue according to claim 27, wherein the adhesion factor is gelatin or fibrin.

30. A pharmaceutical composition comprising the sheet-shaped retinal tissue according to any one of claims 25 to 29.

31. A method for treating a disease caused by the damage of a retinal cell or retinal tissue or the injury of retinal tissue, comprising transplanting the sheet-shaped retinal tissue according to any one of claims 25 to 29 to a subject in need of transplantation.

32. A method for increasing the percentage of a retinal progenitor cell in a cell population, comprising the step of contacting the cell population comprising the retinal progenitor cell with a substance that binds to one or more antigens selected from the group consisting of CD9, CD24, CD29, CD39, CD47, CD49b, CD49c, CD49f, CD57, CD73, CD82, CD90, CD164, CD200, CD340 and CXCR4.

33. The method according to claim 32, comprising the step of contacting the cell population comprising the retinal progenitor cell with the substance that binds to one or more antigens selected from the group consisting of CD9, CD39, CD90 and CXCR4.

34. The method according to claim 32 or 33, comprising the step of contacting the cell population comprising the retinal progenitor cell with a substance that binds to one or more antigens selected from the group consisting of SSEA1, CD66b, CD69 and CD84.

35. A cell population comprising 90% or more of a retinal progenitor cell that is positive to at least one factor selected from the group consisting of CD9, CD39, CD90 and CXCR4 and is Rx-positive with respect to the total number of cells in the cell population.

36. The cell population according to claim 35, wherein the retinal progenitor cell is negative to one or more antigens selected from the group consisting of SSEA1, CD66b, CD69 and CD84.
